# EUROPEAN PATENT APPLICATION

(11) **EP 2 433 635 A1**
(43) Date of publication of application: **28.03.2012**
(21) Application number: 11185523.5
(22) Date of filing: 09.11.2007
(51) Int. Cl.: A61K 31/454, A61K 31/395, A61P 25/00

(54) **PAK Modulators**

(30) Priority: 10.11.2006 US 858108 P
(62) Divisional of application: 07871432.6
(71) Applicant: Massachusetts Institute Of Technology, Cambridge, MA 02139-4307 (US)
(72) Inventor: Tonegawa, Susumu, Chestnut Hill, MA 02467 (US); Hayashi, Mansuo, Winchester, MA 01890 (US); Dolan, Bridget, Boston, MA 02114 (US)
(74) Representative: Hollywood, Jane Constance

(57) **Abstract**

The present invention provides methods for treating fragile X syndrome and/or other neurodevelopmental disorders by administering p21-activated kinase (PAK) modulators to a patient suffering from, susceptible to, and/or exhibiting one or more symptoms of FXS and/or other neurodevelopmental disorders. The present invention provides PAK modulators and pharmaceutical compositions comprising PAK modulators. The present invention further provides methods for identifying and/or characterizing PAK modulators.

## Description

### Related Applications

This application claims priority under 35 U.S.C. § 119(e) to U.S. provisional patent application, U.S.S.N. 60/858,108, filed November 10, 2006 ("the 108 application"). The entire contents of the `108 application are incorporated herein by reference.

### Government Support

The United States Government has provided grant support utilized in the development of the present invention. In particular, the National Institutes of Health (contract number MH78821) and the National Institute of Mental Health Center (contract number MH58880) have supported development of this invention. The United States Government has certain rights in the invention.

### Background of the Invention

Fragile X syndrome (FXS) is the most commonly inherited form of mental retardation, with symptoms of hyperactivity, stereotypy, anxiety, seizure, impaired social behavior, and/or cognitive delay. FXS results from the loss of expression of the *fragile X mental retardation 1 (FMR1)* gene, which encodes the fragile X mental retardation protein (FMRP). *FMR1* knockout (*FMR1* KO) mice and FXS patients show similar behavioral phenotypes, as well as similar abnormalities in synaptic morphology in the brain (O'Donnell et al., 2002, Annu. Rev. Neurosci., 25:315; incorporated herein by reference). Their brains have more dendritic spines and/or a higher proportion of longer and/or thinner spines compared to normal individuals (Hinton et al., 1991, Am. J. Med. Genet., 41:289; Comery et al., 1997, Proc. Natl. Acad. Sci., USA, 94:5401; and Irwin et al., 2001, Am. J. Med. Genet., 98:161; all of which are incorporated herein by reference). Dendritic spines are the protrusions from dendritic shafts that serve as postsynaptic sites of the majority of excitatory synapses in mammals. Correlated with altered spine morphology, *FMR1* KO mice display abnormal synaptic function, including enhanced long-term depression (LTD) mediated by metabotropic glutamate receptor in the hippocampus and/or impaired long-term potentiation (LTP) in the cortex, compared to wild type mice (Huber et al., 2002, Neuropharmacology, 37:571; and Li et al., 2002, Mol. Cell Neurosci., 19:138; both of which are incorporated herein by reference). Thus, these findings demonstrate that FMRP functions in regulating spine morphology, synaptic function, and/or animal behavior.

FMRP is a selective RNA-binding protein that associates with polyribosomes (Corbin et al., 1997, Hum. Mol. Genet., 6:1465; and Stefani et al., 2004, J. Neurosci., 24:7272; both of which are incorporated herein by reference) and with Argonaute2 (AG02) and Dicer, two members of RISC, a complex that is required for RNAi-mediated gene silencing (Ishizuka et al., 2002, Genes Dev., 16:2497; incorporated herein by reference). FMRP is thought to regulate synaptic morphology and/or function because of its ability to repress translation of its RNA binding partners (Laggerbauer et al., 2001, Hum. Mol. Genet., 10:329; Li et al., 2001, Nucleic Acids Res., 29:2276; and Mazroui et al., 2002, Hum. Mol. Genet., 11:3007; all of which are incorporated herein by reference), perhaps using an RNAi-mediated mechanism. Some of these RNAs encode proteins that are involved in synaptic morphology and/or function, such as Rac1, microtubule-associated protein 1B, activity-regulated cytoskeleton-associated protein, and alpha-calcium/calmodulin-dependent protein kinase II (Zhang et al., 2001, Cell, 107:591; Lee et al., 2003, Development, 130:5543; and Zalfa et al., 2003, Cell, 112:317; all of which are incorporated herein by reference).

### Summary of the Invention

The present invention encompasses the recognition that signaling pathways mediated by p21-activated kinases (PAK) and FMRP may antagonize each other to regulate synaptic morphology and/or function. The present invention encompasses the recognition that abnormalities in cortical spine morphology of FXS patients and *FMR1* knockout mice are opposite to those we found in transgenic mice in which PAK activity is inhibited by its dominant negative form (dnPAK). The present invention encompasses the recognition that FMRP binds to PAK.

The present invention provides systems, including methods, reagents, and/or compositions, for treating fragile X syndrome (FXS) and/or other neurodevelopmental disorders. For example, according to the present invention, administration of PAK modulators may be used to treat patients suffering from, susceptible to, and/or exhibiting symptoms of FXS and/or other neurodevelopmental disorder.

In some embodiments, the present invention provides modulators of PAK activity and/or levels. In some embodiments, a modulator of PAK stimulates PAK activity and/or increases its levels. In some embodiments, a modulator of PAK inhibits PAK activity and/or decreases its levels. In accordance with some embodiments, compositions are provided comprising at least one PAK modulator and a pharmaceutically acceptable excipient. In certain embodiments, PAK modulators function by modulating the interaction between PAK and the fragile X mental retardation protein (FMRP).

The present invention provides systems, including methods, reagents and/or compositions, for identifying PAK modulators. In some embodiments, such methods include high throughput screening methods. In some embodiments, the methods include *in vitro, in cyto,* and/or *in vivo* assays.

The present invention provides specific PAK modulators. In some embodiments, specific PAK modulators include small molecule therapeutics. In some embodiments, small molecule modulators of PAK include Emodin, OSU-03012, combinations thereof, and/or derivatives thereof. The present invention provides pharmaceutical compositions comprising Emodin, OSU-03012, combinations thereof, and/or derivatives thereof. The present invention provides methods of treating FXS and/or other neurodevelopmental disorders comprising administering Emodin, OSU-03012, combinations thereof, and/or derivatives thereof to a patient susceptible to, suffering from, and/or exhibiting symptoms of FXS and/or other neurodevelopmental disorder.

This application refers to various patent publications and non-patent publications, the contents of all of which are incorporated herein by reference.

### Brief Description of the Drawing

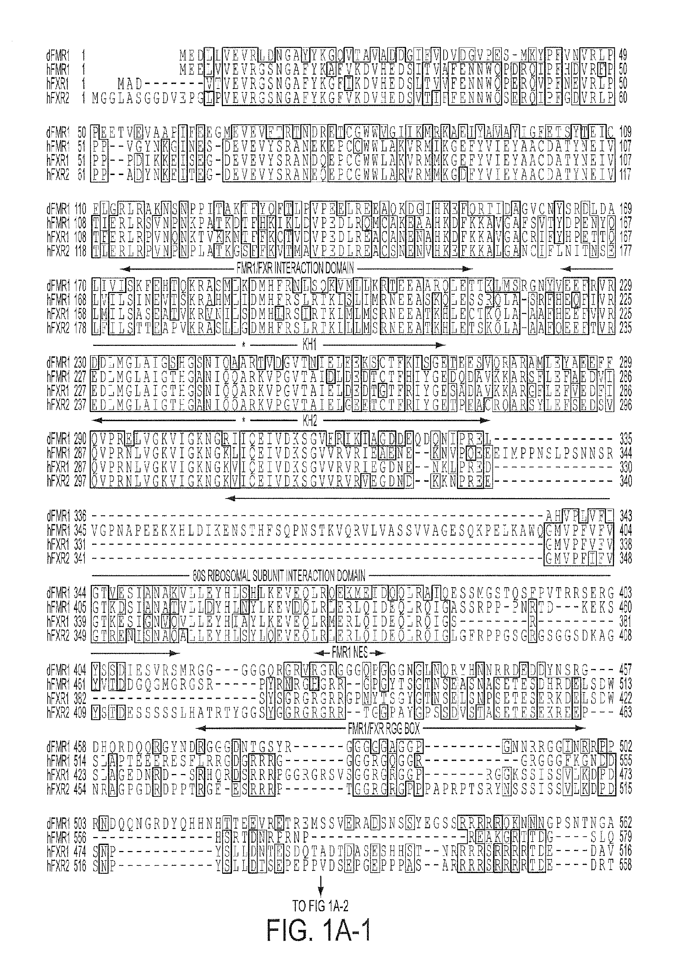

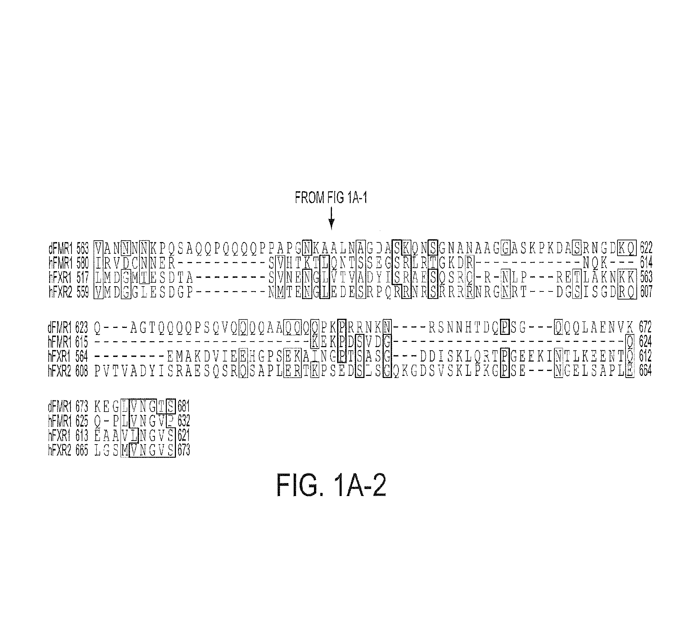

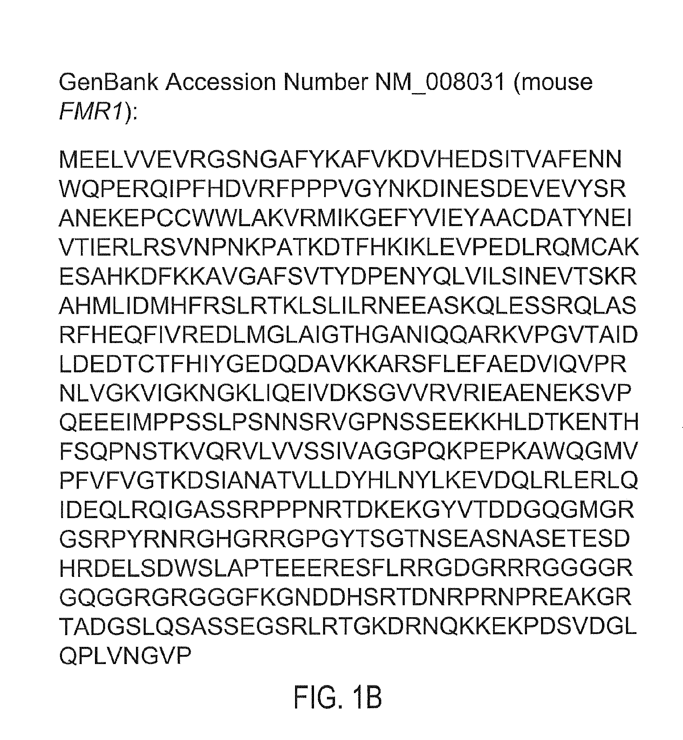

*Figure 1**. Representative FMR and FXR amino acid sequences.* Figure 1A shows an alignment of representative examples of *Drosophila melanogaster* FMR1 (dFMR1; GenBank AAG22045), human FMR1 (hFMR1; GenBank AAB 18829), human FXR1 (hFXR1; GenBankAAC50155), and human FXR2 (hFXR2; GenBank AAC50292) amino acid sequences. Dark gray boxes mark identical, amino acids, and light gray boxes denote conservative amino acid substitutions. Insertions are denoted by a dash. The FMR1/FXR interaction domain is depicted. "KH1" and "KH2": KH domains; "NES": nuclear export signal; "RGG": domain that mediates protein-protein interactions, RNA-binding, and/or may be methylated. Figure modified from Wan et al., 2000, Mol. Cell. Biol., 20:8536 (incorporated herein by reference). Figure 1B shows an amino acid sequence for mouse *FMR1* (GenBank NM_008031).

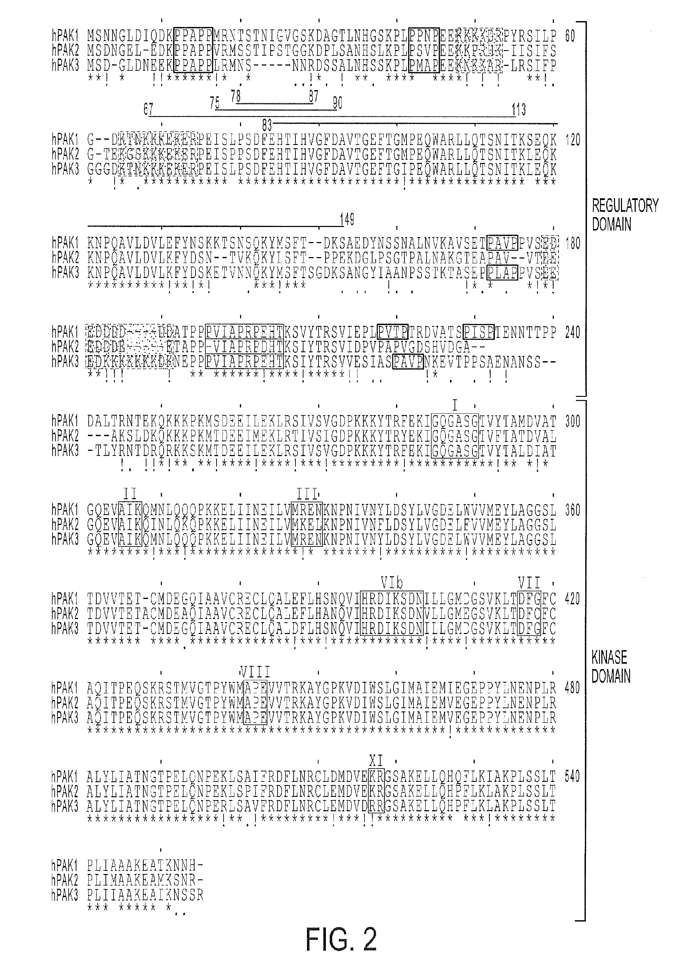

*Figure 2**. Representative human PAK1, PAK2, and PAK3 amino acid sequences.* Shown is an alignment of representative examples of human PAK1 (GenBank AAA65441), human PAK2 (GenBank AAA65442), and human PAK3 (GenBank AAC36097) amino acid sequences. Asterisks below the sequences mark identical amino acids, and dots below the sequences denote conservative amino acid substitutions. Insertions are denoted by a dash. Black boxes mark proline-rich regions containing putative SH3-binding PXXP motifs. The open box marks the noncanonical PIX binding site. Gray boxes indicate highly charged basic or acidic tracts. The dark overhead lines indicate the homodimerization domain (amino acids 78 - 87), CRIB motif (amino acids 75 - 90), p21-binding domain (PBD; amino acids 67 - 113), and autoinhibitory switch domain (amino acids 83 - 139) for PAK1. Diagnostic kinase motifs in the catalytic domain are boxed and numbered per convention. Figure modified from (Bokoch et al., 2003, Annu. Rev. Biochem., 72:743 (incorporated herein by reference).

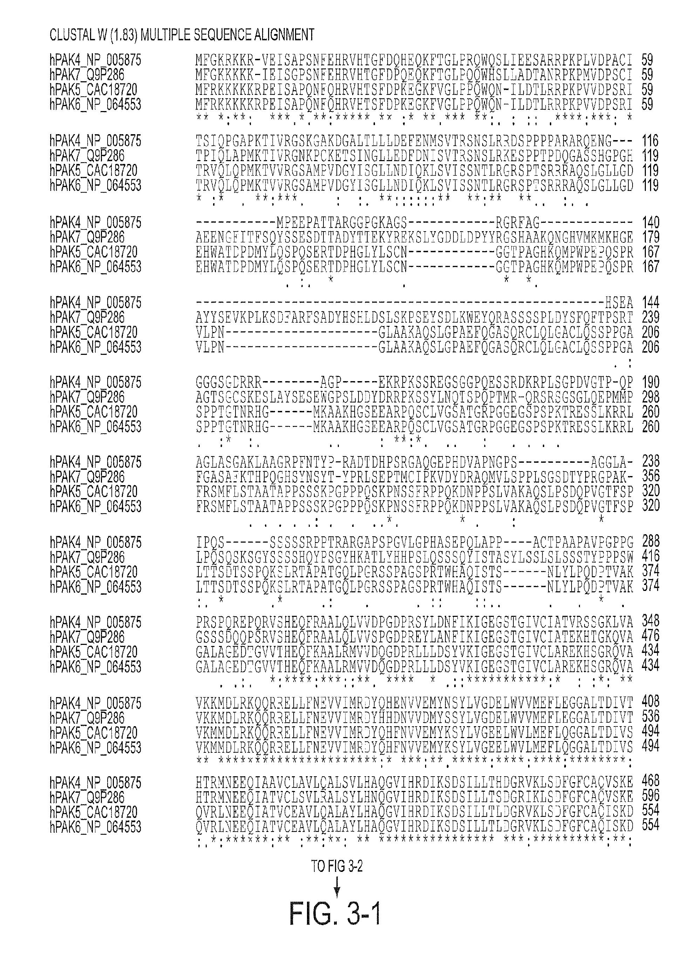

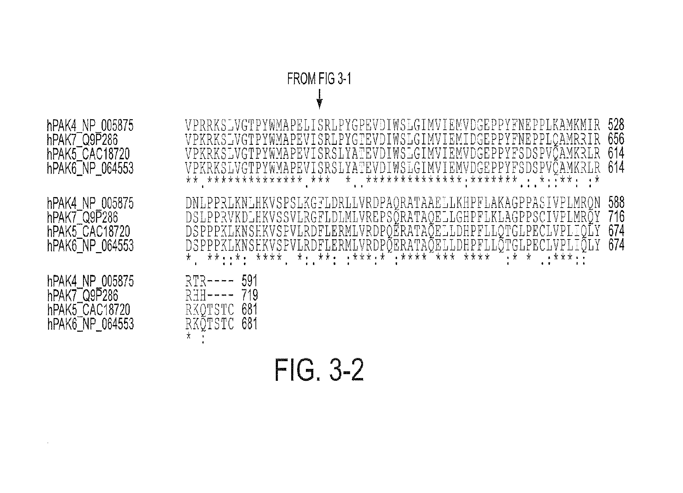

*Figure 3**. Representative human PAK4, PAK5, PAK6, and PAK7 amino acid sequences.* Shown is an alignment of representative examples of human PAK4 (GenBank NP_005875), human PAK5 (GenBank CAC18720), human PAK6 (GenBank NP_064553), and human PAK7 (GenBank Q9P286) amino acid sequences. Asterisks below the sequences mark identical amino acids, and dots below the sequences denote conservative amino acid substitutions. Insertions are denoted by a dash.

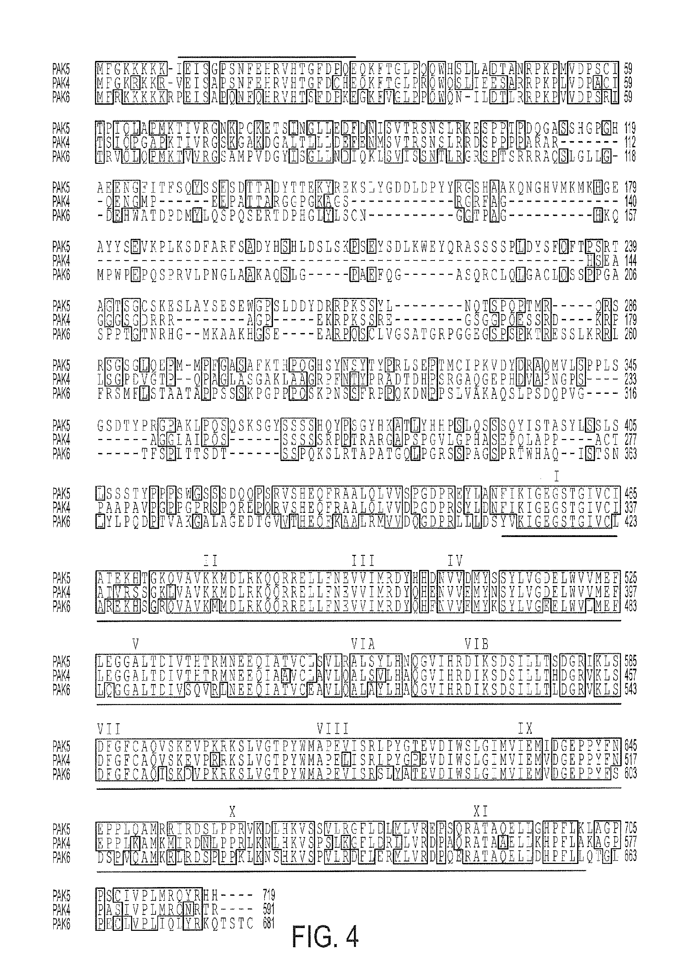

*Figure 4**. Representative human PAK4, PAK5, and PAK6 amino acid sequences.* Shown is an alignment of representative examples of human PAK4 (GenBank CAA09820), human PAK5 (GenBank BAA94194), and human PAK6 (GenBank AAF82800) amino acid sequences. Black boxes mark identical amino acids. Insertions are denoted by a dash. Diagnostic kinase motifs in the catalytic domain are boxed and numbered per convention. Figure modified from Dan et al., 2002, Mol. Cell. Biol., 22:567 (incorporated herein by reference).

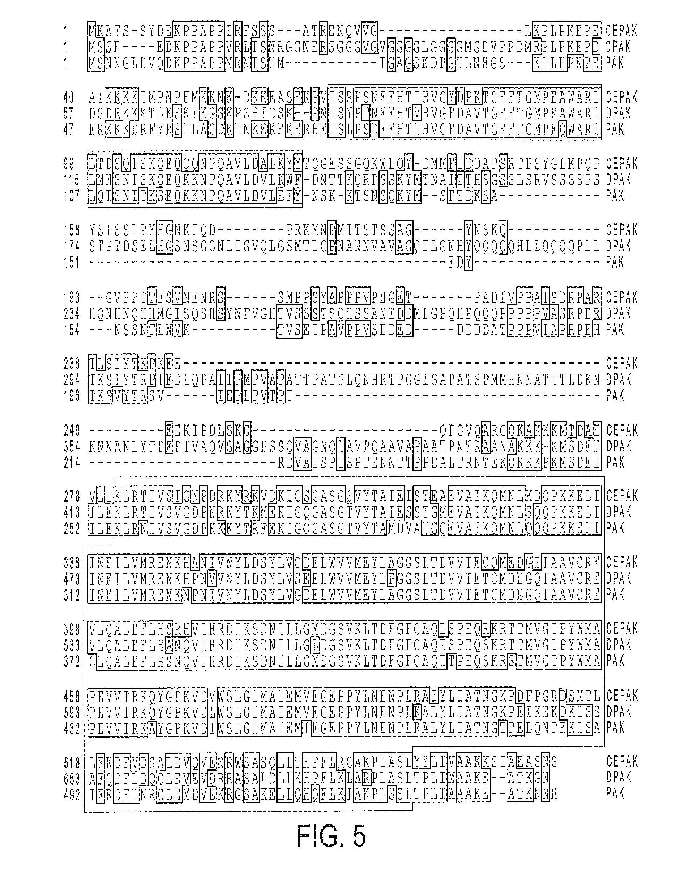

*Figure 5**. Representative* Caenorhabditis elegans, Drosophila melanogaster, *and rat PAK1 amino acid sequences.* Shown is an alignment of representative examples of PAK1 amino acid sequences from *C. elegans* (CEPAK; GenBank BAA11844), *D. melanogaster,* (DPAK; GenBank AAC47094), and rat (PAK; GenBank AAB95646). Black boxes mark identical amino acids. Insertions are denoted by a dash. The N-terminal box marks the p21-binding domain, and the C-terminal box denotes the C-terminal kinase domain. Figure modified from Chen et al., 1996, J. Biol. Chem., 271:26362 (incorporated herein by reference).

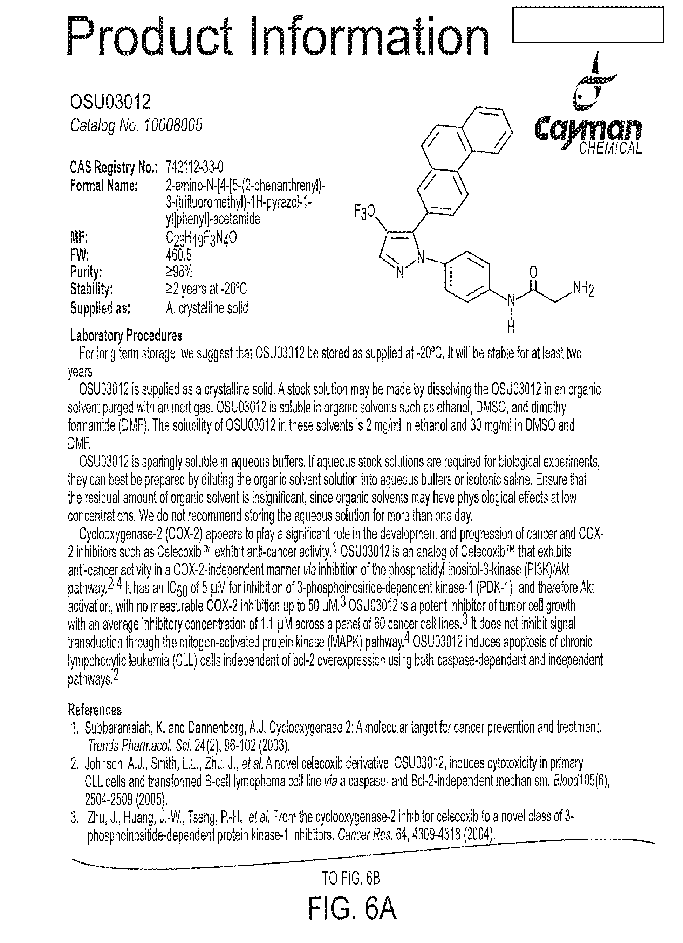

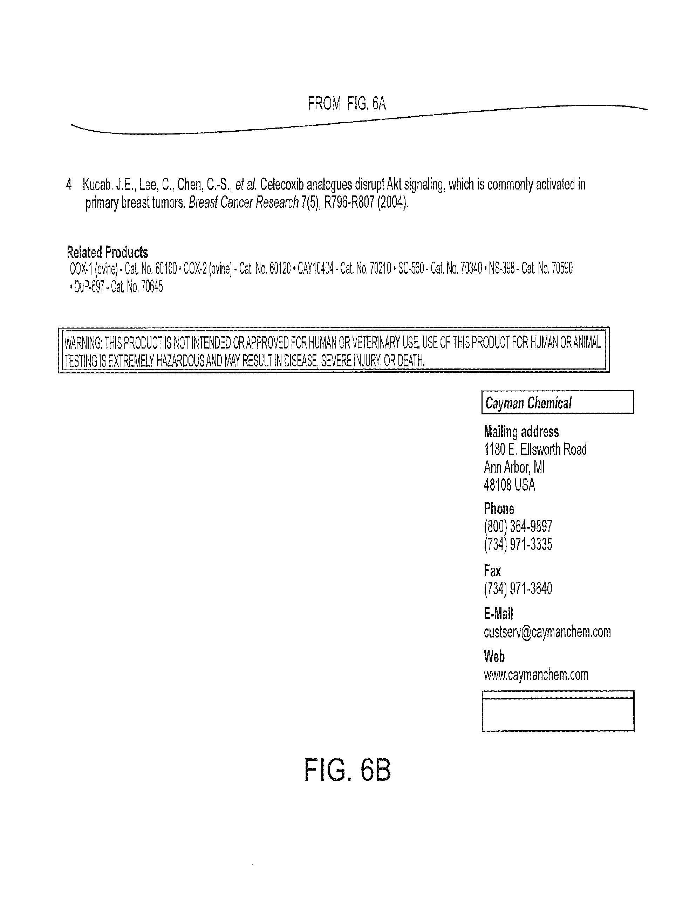

*Figure 6**.* OSU-03012 product information from Cayman Chemical catalog (May 2006).

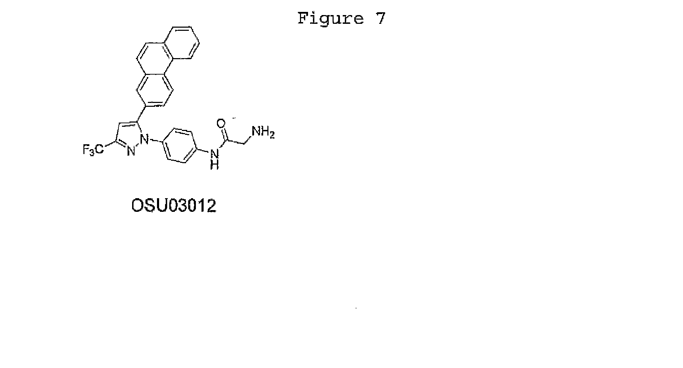

*Figure 7**.* Chemical structure of OSU-03012.

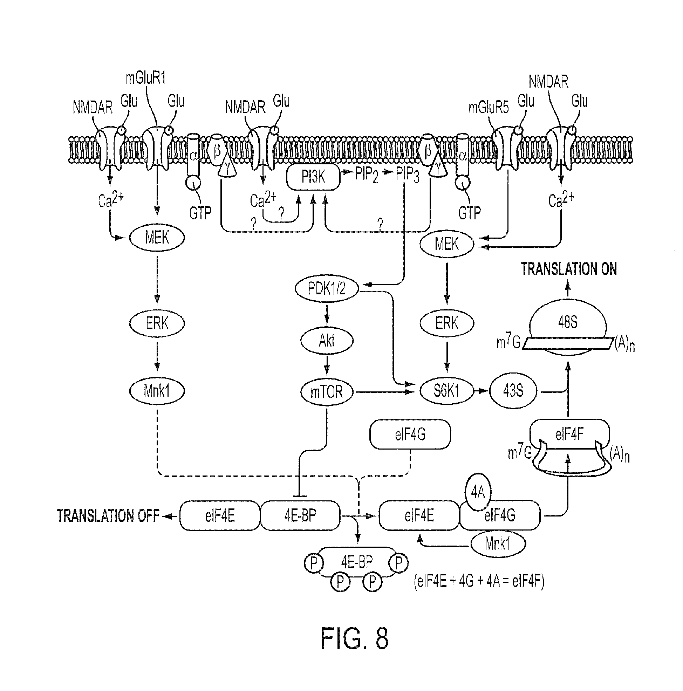

*Figure 8**. PAK is involved in multiple signaling pathways.* PAK participates upstream of Raf, mitogen-activated protein kinase kinase (MEK), extracellular signal-regulated kinase (ERK), and MAPK-interacting serine/threonine kinase 1 (Mnk1) in the ERK signaling pathway. PAK participates downstream of phosphoinositide-dependent kinase 1 (PDK1), PDK2, and phosphatidylinositol 3-kinase (PI3K) in the PI3K signaling pathway. Figure modified from Klann and Dever (2004, Nat. Rev. Neurosci., 5:931*).*

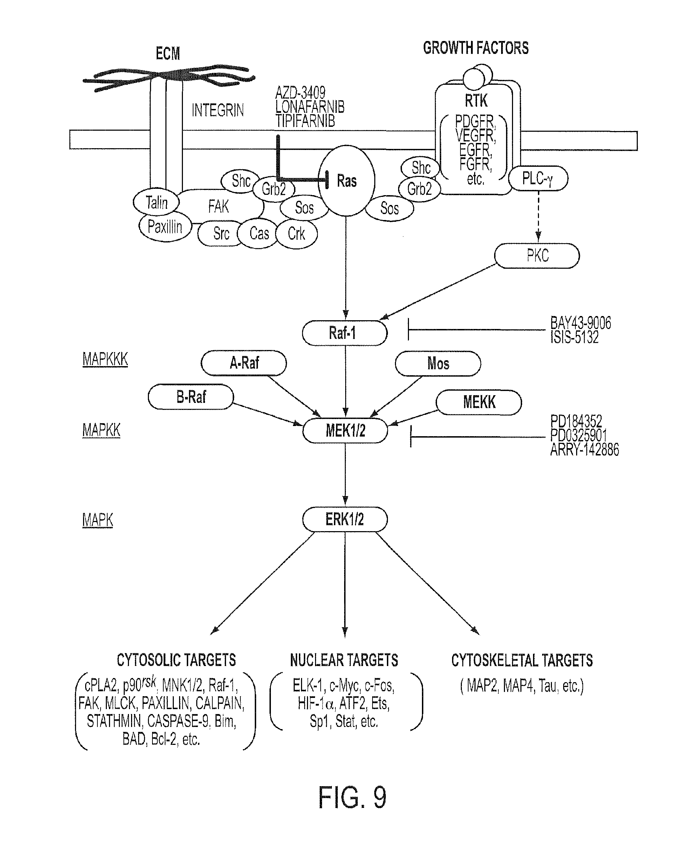

*Figure 9**. Schematic representation of the extracellular signal-regulated kinase (ERK) pathway.* The mitogen-activated protein (MAP) kinase cascade comprises three sequential kinases: MAP kinase kinase kinase (MAPKKK), MAP kinase kinase (MAPKK), and MAP kinase (MAPK). ERK1/2 phosphorylated a variety of nuclear, cytosolic and cytoskeletal targets. Integrin-focal adhesion kinase (FAK) pathway, which is activated by adhesion of integrins to specific extracellular matrix (ECM) molecules, is involved in activating the ERK pathway. Activation of the ERK pathway is often associated with cell proliferation, cell survival and cell migration. Exemplary inhibitors of the ERK pathway are shown. Several negative regulators of the ERK pathway exist, such as MAP kinase phosphatases (MKPs/DUSPs) and Sprouty proteins. Expression of MKPs and Sprouty proteins is induced in an ERK-dependent manner, and thus these proteins participate in the negative feedback regulatory loop of the ERK pathway.

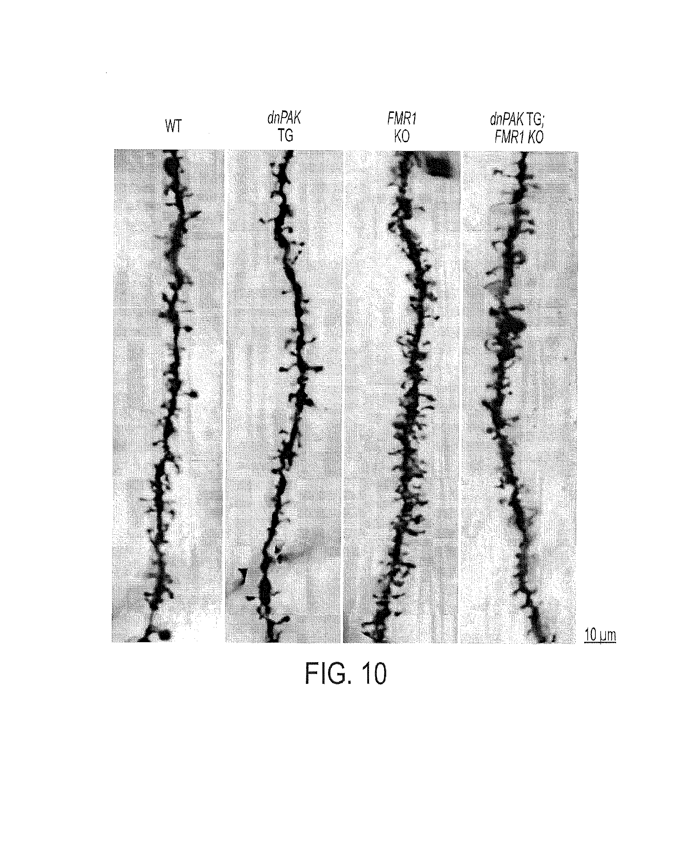

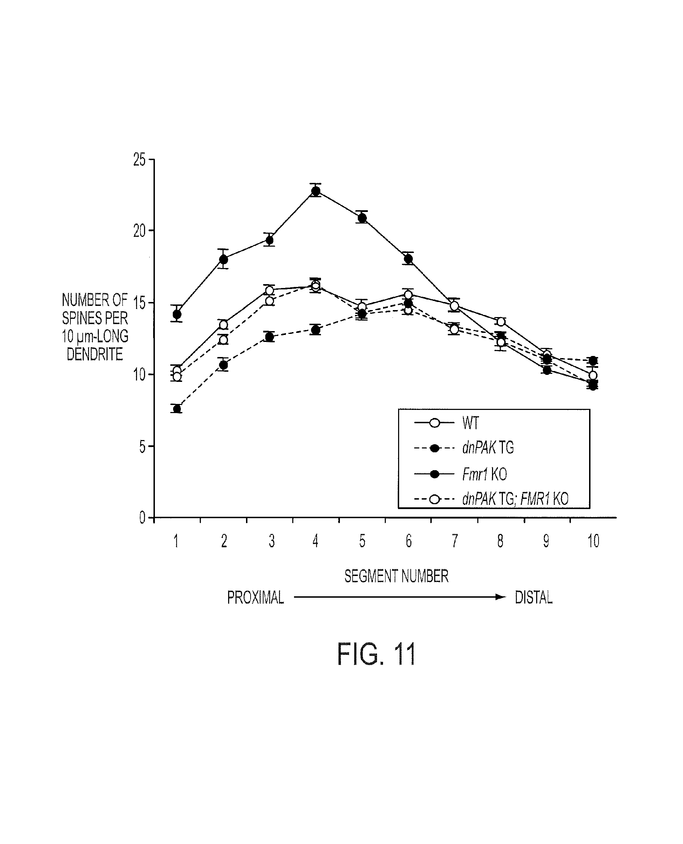

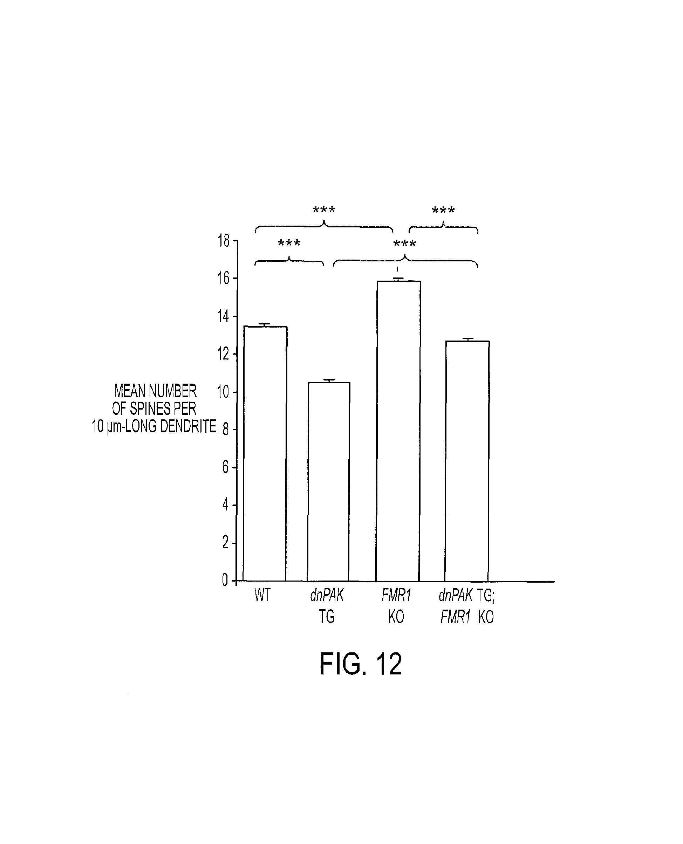

*Figure 10**. Golgi analysis.* Representative dendritic segments of layer II/III pyramidal neurons from wild-type (WT; n = 20 neurons, 2 mice), *dnPAK* TG mice (n = 30 neurons, 3 mice), *FMR1* KO mice (n = 20 neurons, 2 mice), and double mutant *dnPAK* TG; *FMR1* KO mice (dMT; n = 40 neurons, 4 mice). On each primary apical dendritic branch, ten consecutive 10 µm-long dendritic segments were analyzed to quantify spine density per 10 µm-long dendritic segment (Figure 11) and mean spine density (Figure 12).

*Figure 11**. Quantification of spine density.* On each primary apical dendritic branch, ten consecutive 10 µm-long dendritic segments were analyzed to quantify spine density per 10 µm-long dendritic segment. Spine density in dMTs was comparable to wild-type controls in all dendritic segments except segment 7 and 8 (*p* > 0.05 in segments 1 - 6, 9, and 10; *p* < 0.01 in segments 7 and 8).

*Figure 12**. Quantification of mean spine density.* (A) On each primary apical dendritic branch, ten consecutive 10 µm-long dendritic segments were analyzed to quantity mean spine density per 10 µm-long dendritic segment. Mean spine density in dMTs (1.28 ± 0.02) was significantly lower than that in *FMR1* KO mice (1.60 ± 0.02; *p* < 0.001) and significantly higher than that in *dnPAK TG* mice (1.06 ± 0.01; *p* < 0.001). ANOVA, *p* < 0.0001. ***: *p* < 0.001.

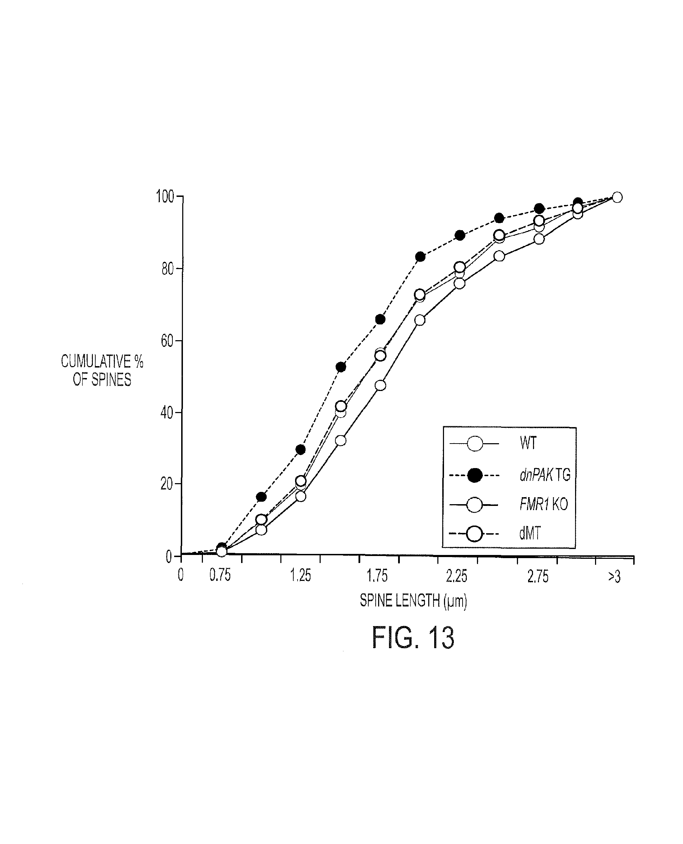

*Figure 13**. Quantification of spine length. FMR1* KO neurons (444 spines) exhibited a significant shift in the overall spine distribution towards spines of longer length compared to wild-type neurons (406 spines; Kolmogorov-Smirnov test: *p* < 0.05), while *dnPAK* TG neurons (630 spines) exhibited the opposite shift to shorter spines (*p* < 0.01). In contrast, spine length distribution in dMT neurons (785 spines) overlapped well with wild-type neurons and is significantly different from *FMR1* KO neurons (*p* < 0.01).

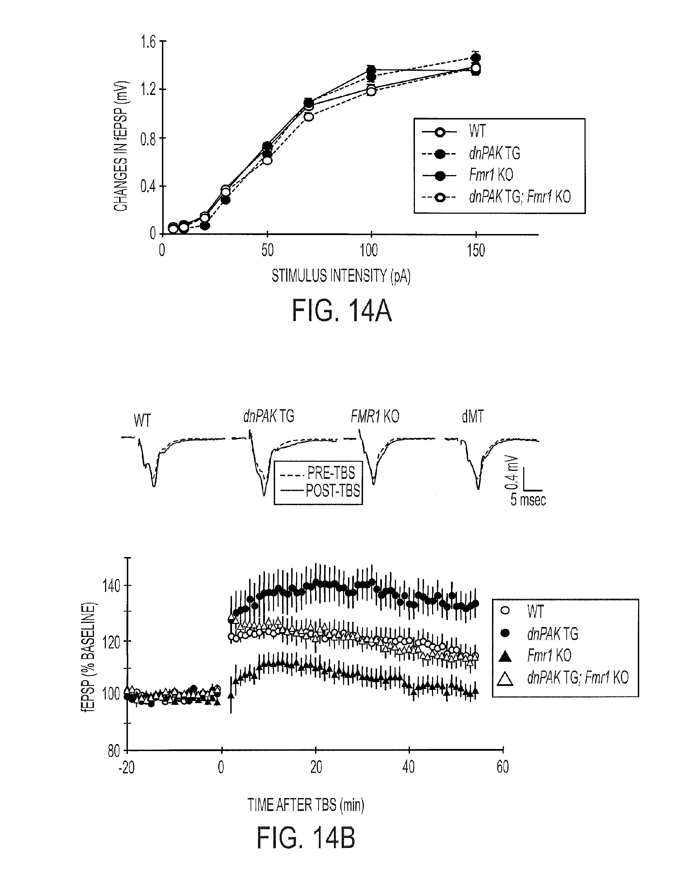

*Figure 14**. PAK inhibition rescues reduced cortical LTP in FMR1 KO mice.* (A) Input-output curves plotting the changes in field excitatory post-synaptic potential (fEPSP) amplitude and their corresponding presynaptic stimulus intensity in wild-type (n = 45 slices, 16 mice), *dnPAK* TG (n = 30 slices, 10 mice), *FIMR1* KO (n = 57 slices, 19 mice) and dMT mice (n = 24 slices, 8 mice). (B) Cortical LTP induced by TBS was enhanced in *dnPAK*TG (n = 13 slices, 11 mice), but reduced in *FMR1* KO (n = 17 slices, 11 mice), relative to wild-type controls (n = 17 slices, 11 mice; for responses at 55 minutes post stimulation, ANOVA, *p < 0.05;* for both *dnPAK* TG versus WT and *FMR1* KO versus WT, *p* < 0.04). By contrast, the magnitude of LTP was indistinguishable between dMT slices (n = 13 slices, 9 mice) and wild-type controls (*p* > 0.05 for responses at 55 minutes post stimulation). An overlay of representative field potential traces taken during baseline of recording and at 55 minutes post stimulation is shown for each genotype.

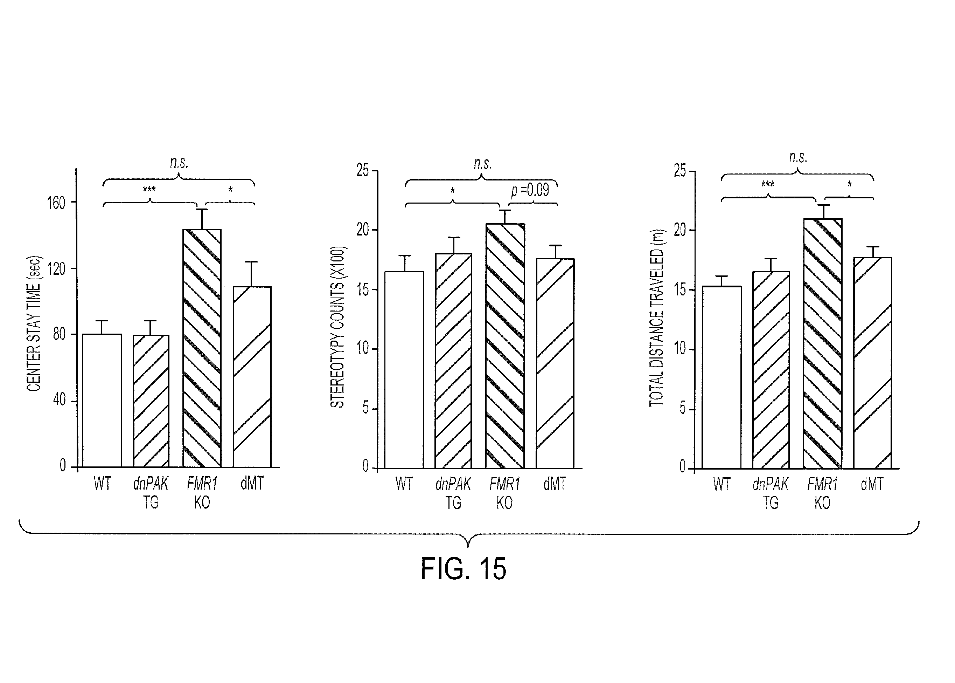

*Figure 15**. Open field test.* Mice of different genotypes were subjected to the open field test according to standard procedures. Each mouse ran for 10 minutes in an activity monitor chamber. Open field activity was detected by photobeam breaks and analyzed by VersaMax software. Activities measured were the amount of time the mouse spent in the center of the field, the number of times the mouse exhibited repetitive behaviors ("stereotypy"), and the total distance traveled by the mouse. Genotypes are as follows: (1) wild-type (n = 10 mice); (2) *dnPAK* TG (n = 10 mice); (3) *FMR1* KO (n = 11 mice); and (4) *dnPAK* TG; *FMR1* KO *("dMT* mice" n = 11 mice). *"n.s."*: not statistically different. *: *p* < 0.05; *****: *p* < 0.001. *(A) FMR1* KO traveled a longer distance compared to wild-type mice (ANOVA, *p* < 0.01. WT: 15.29 ± 0.92 m; *FMR1* KO: 20.99 ± 1.10 m, *p* < 0.001). *(B) FMR1* KO exhibited a higher number of repetitive behaviors than wild-type mice *(stereotypy counts:* ANOVA, *p* < 0.05. WT: 1636 ± 119; *FMR1* KO: 2049 ± 125, *p* < 0.05). (C) *FMR1* KO stayed a longer period of time in the center of the open field than wild-type mice (ANOVA, *p* < 0.001. WT: 79.8 ± 8.5 sec; *FMR1* KO: 143.1 ± 12.0 sec, *p* < 0.001). In all of these three behaviors, dMT mice exhibited comparable performance to wild-type controls (*p* > 0.05 for all of the following parameters: *distance traveled:* 17.76 ± 0.91 m; *stereotypy counts:* 1756 ± 102; *center time:* 108.8 ± 14.6 sec).

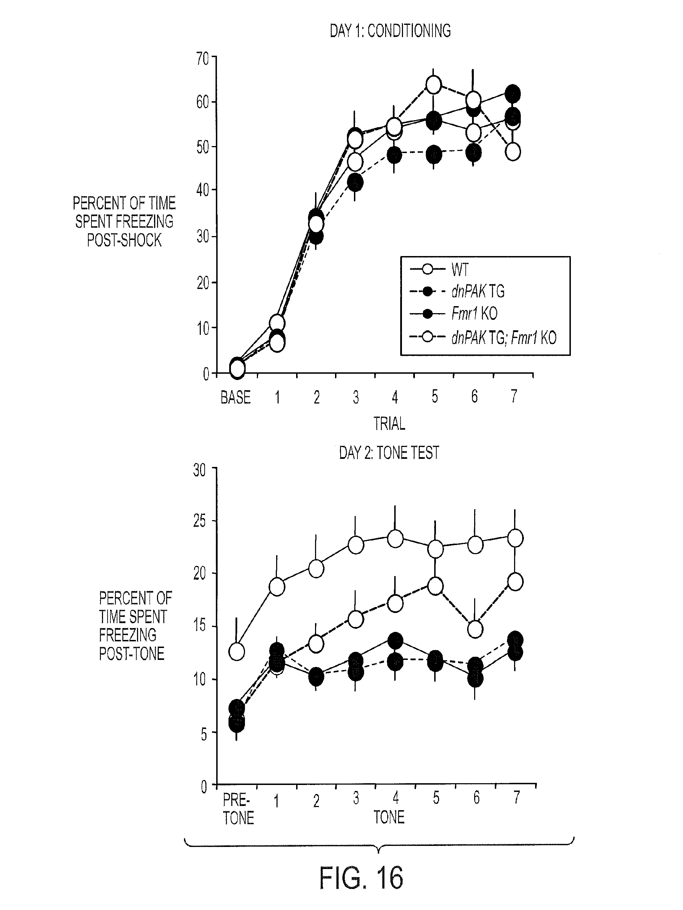

*Figure 16**. Trace fear conditioning task.* Mice of different genotypes (WT, n = 15 mice; *dnPAK TG,* n = 12 mice; *FMR1* KO, n = 15 mice; dMT, n = 9 mice) were subjected to the trace fear conditioning task according to standard procedures. On day 1 ("conditioning"), mice were placed into a training chamber for 60 seconds before the onset of a 15-second white noise tone. Another 30 seconds later, mice received a 1-second shock (0.7 mA intensity). Thus, one trial is composed of tone, 30 seconds blank time (also called "trace"), and then shock. Seven trials with an intertrial interval (ITI) of 210 seconds were performed. To examine whether mice remember this association, on day 2 ("tone test"), mice were placed into a new chamber with a different shape and smell from the first chamber. After 60 seconds, a 15-second tone was repeated for seven times with an ITI of 210 seconds. Video images were digitized and the percentage of freezing time during each ITI was analyzed by Image FZ program. Freezing was defined as the absence of all but respiratory movement for a 1-second period. On day 1 ("Conditioning"), the four genotypes of mice exhibited comparable amounts of freezing pre-conditioning ("Baseline") and post-conditioning in all trials. At the 24-hour tone test, the four genotypes exhibit comparable amounts ofpre-tone freezing (ANOVA *p* > 0.05). However, for tone-dependent freezing, *FMR1* KO mice and *dnPAK* TG mice exhibited a significant reduction compared to wild-type controls (ANOVA for each tone session, *p* < 0.05; for *FMR1* KO versus WT, *p* < 0.05 for session 1 and *p* < 0.01 for sessions 2 to 7; for *dnPAK* TG versus WT, *p* > 0.05 for session 1 and *p* < 0.01 for sessions 2 to 7). dMT mice also showed freezing deficits during the first several tone sessions (sessions 1 to 4) compared to wild-type controls (*p* < 0.05). However, with additional tone sessions (sessions 5 to 7), freezing by dMT mice caught up to that of wild-type controls (*p* > 0.05). *"n.s."*: not statistically different. ***: *p < 0.05*; ****: *p <* 0.01; ***: *p* < 0.001.

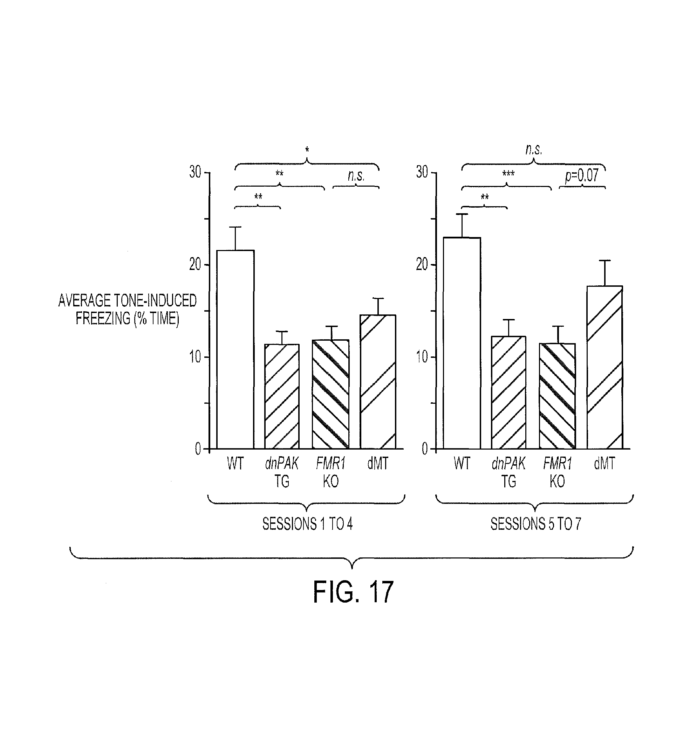

*Figure 17**. Quantification of mean tone-induced freezing.* Average freezing for tone sessions 1 to 4: ANOVA *p* < 0.05. dMT mice showed freezing deficits compared to wild-type controls (*p* < 0.05), but the deficits in dMT mice were less pronounced compared to *dnPAK* TG (*p* < 0.01) or *FMR1* KO mice (*p* < 0.01). Average freezing for tone sessions 5 to 7: ANOVA *p* < 0.05. Freezing level in dMT mice was not significantly different from wild-type controls (p > 0.05) and there were trends in its difference from *dnPAK* TG (*p* = 0.12) or *FMR1* KO mice (*p* = 0.07).

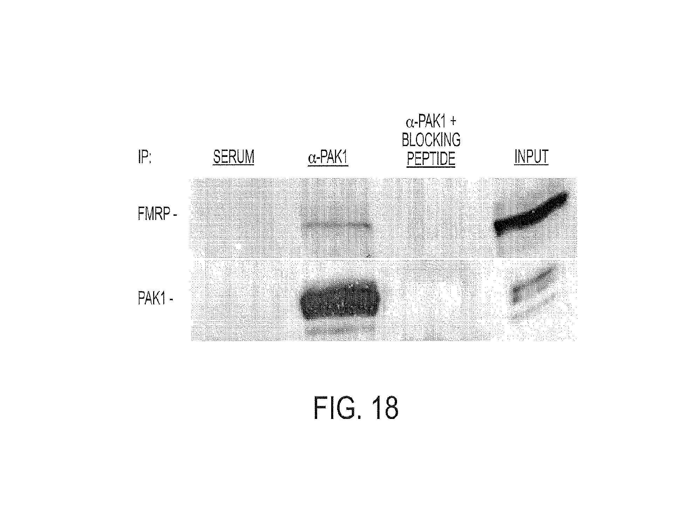

*Figure 18**. Immunoprecipitation.* Immunoprecipitations were performed using either rabbit serum (negative control), α-PAK1, or α-PAK1 plus a blocking peptide. Western blots were probed for either PAK1 or FMRP. α-PAK1, but not rabbit serum, immunoprecipitates FMRP in this assay. The specificity of the interaction was tested by including a blocking peptide specific for α-PAK1 in the immunoprecipitation reaction. Input: 2% of the extract used for a single immunoprecipitation was loaded on the gel.

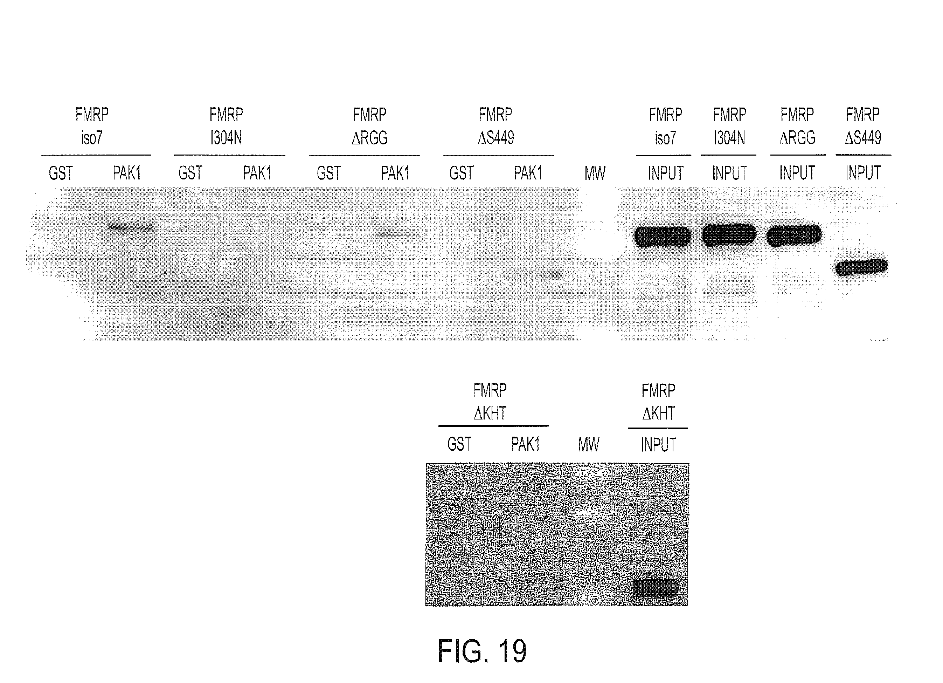

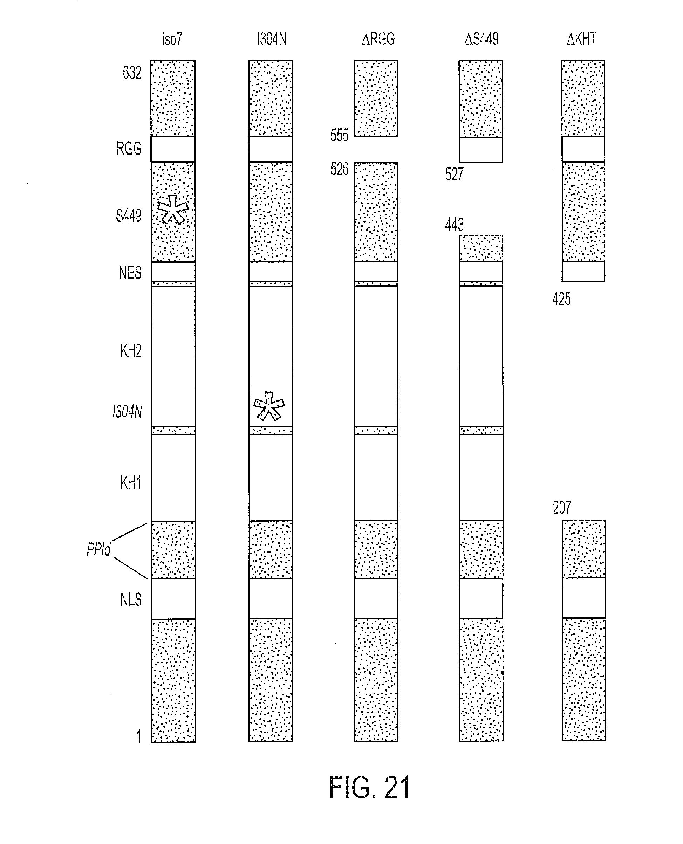

*Figure 19**. GST pull down.* FMRP was produced by *in vitro*-translation. GST and GST-tagged PAK1 were purified from a bacterial expression system. Wild type FMRP and the FMRP mutants depicted in Figure 21 were used in the GST-pull down assay. "Input": *in vitro* translated FMRP sample before the reaction was carried out; "MW": molecular weight standard.

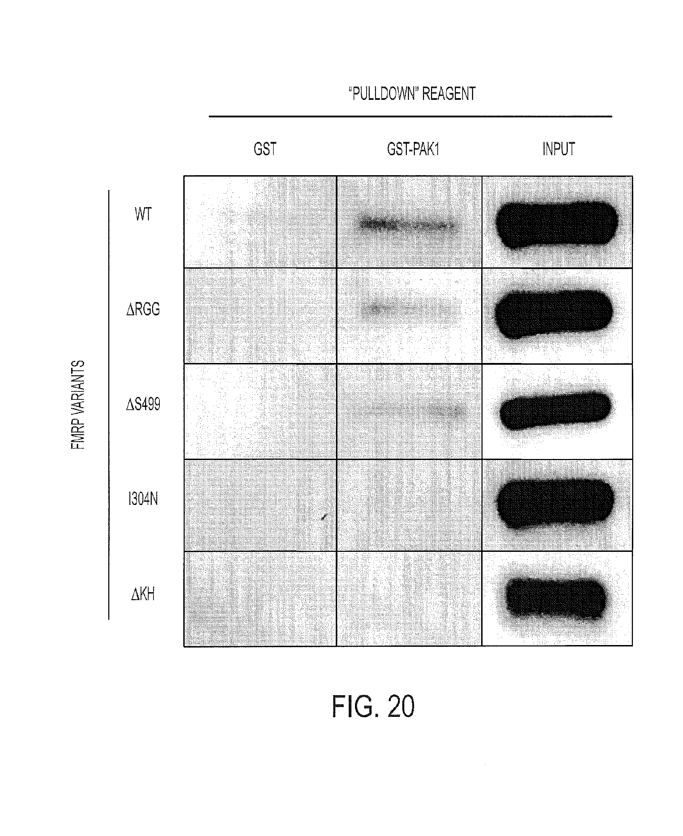

*Figure 20**. Characterization of the interaction between PAK1 and various FMRP variants* in vitro. *In vitro*-translated FMRP variants were incubated with GST or GST-PAK1 and glutathione sepharose beads. The complexes isolated by this method were subjected to SDS-PAGE and Western blotted for FMRP. "Input": 10% of *in vitro*-translated FMRP sample before the binding reaction was carried out was loaded on the gel.

*Figure 21**. Wild type and mutant* FMRP *domain structure.* Figure 21 (adapted from Mazroui *et al.,* 2003, *Hum. Mol. Genet.* 12:3087; incorporated herein by reference) shows a schematic structure of FMRP, highlighting various functional domains including three RNA-binding motifs (RGG, KH1, and KH2) and the phosphorylation site (S499, represented by a white asterisk). The constructs used for *in vitro* binding included full length (WT), truncated (ΔRGG, ΔS499 and ΔKH), or mutated (I304N) FMRP. ΔRGG refers to the FMRP variant with a deletion of the RGG box at amino acids 526 - 555. The deleted area in ΔS499 spans amino acids 443 - 527 and includes the phosphorylation site, S499, as well as putative phosphorylation sites. The isoleucine to asparagine missense mutation in the KH2 domain mimics that previously reported in a human FXS patient (I304N, represented by a black asterisk). The ΔKH deletion mutant lacks both KH domains in tandem corresponding to amino acids 207 - 425. Adapted from Mazroui et al., 2003, Hum. Mol. Genet., 12:3087; incorporated herein by reference; numbering refers to the amino acid positions designated by the SwissProt Q06787 entry.

### Definitions

*Amino acid:* As used herein, term "amino acid," in its broadest sense, refers to any compound and/or substance that can be incorporated into a polypeptide chain. In some embodiments, an amino acid has the general structure H₂N-C(H)(R)-COOH. In some embodiments, an amino acid is a naturally-occurring amino acid. In some embodiments, an amino acid is a synthetic amino acid; in some embodiments, an amino acid is a D-amino acid; in some embodiments, an amino acid is an L-amino acid. "Standard amino acid" refers to any of the twenty standard L-amino acids commonly found in naturally occurring peptides. "Nonstandard amino acid" refers to any amino acid, other than the standard amino acids, regardless of whether it is prepared synthetically or obtained from a natural source. As used herein, "synthetic amino acid" encompasses chemically modified amino acids, including but not limited to salts, amino acid derivatives (such as amides), and/or substitutions. Amino acids, including carboxy- and/or amino-terminal amino acids in peptides, can be modified by methylation, amidation, acetylation, and/or substitution with other chemical groups that can change the peptide's circulating half-life without adversely affecting their activity. Amino acids may participate in a disulfide bond. The term "amino acid" is used interchangeably with "amino acid residue," and may refer to a free amino acid and/or to an amino acid residue of a peptide. It will be apparent from the context in which the term is used whether it refers to a free amino acid or a residue of a peptide.

*Animal:* As used herein, the term "animal" refers to any member of the animal kingdom. In some embodiments, "animal" refers to humans, at any stage of development. In some embodiments, "animal" refers to non-human animals, at any stage of development. In certain embodiments, the non-human animal is a mammal (*e.g.*, a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, insects, and/or worms. In some embodiments, an animal may be a transgenic animal, genetically-engineered animal, and/or a clone.

*Antibody*: As used herein, the term "antibody" refers to any immunoglobulin, whether natural or wholly or partially synthetically produced. All derivatives thereof which maintain specific binding ability are also included in the term. The term also covers any protein having a binding domain which is homologous or largely homologous to an immunoglobulin binding domain. Such proteins may be derived from natural sources, or partly or wholly synthetically produced. An antibody may be monoclonal or polyclonal. An antibody may be a member of any immunoglobulin class, including any of the human classes: IgG, IgM, IgA, IgD, and IgE. As used herein, the terms "antibody fragment" or "characteristic portion of an antibody" are used interchangeably and refer to any derivative of an antibody which is less than full-length. In general, an antibody fragment retains at least a significant portion of the full-length antibody's specific binding ability. Examples of antibody fragments include, but are not limited to, Fab, Fab', F(ab')2, scFv, Fv, dsFv diabody, and Fd fragments. An antibody fragment may be produced by any means. For example, an antibody fragment may be enzymatically or chemically produced by fragmentation of an intact antibody and/or it may be recombinantly produced from a gene encoding the partial antibody sequence. Alternatively or additionally, an antibody fragment may be wholly or partially synthetically produced. An antibody fragment may optionally comprise a single chain antibody fragment. Alternatively or additionally, an antibody fragment may comprise multiple chains which are linked together, for example, by disulfide linkages. An antibody fragment may optionally comprise a multimolecular complex. A functional antibody fragment typically comprises at least about 50 amino acids and more typically comprises at least about 200 amino acids.

*Approximately:* As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Biologically active:* As used herein, the phrase "biologically active" refers to a characteristic of any substance that has activity in a biological system and/or organism. For instance, a substance that, when administered to an organism, has a biological effect on that organism, is considered to be biologically active. In particular embodiments, where a protein or polypeptide is biologically active, a portion of that protein or polypeptide that shares at least one biological activity of the protein or polypeptide is typically referred to as a "biologically active" portion.

*Candidate substance:* As used herein, the term "candidate substance" refers to any substance that may potentially inhibit FXS and/or modulate PAK activity. A candidate substance may be a protein, a nucleic acid, a small molecule, a lipid, a carbohydrate, a glycoprotein, a proteoglycan, combinations thereof, and/or characteristic portions thereof. It may prove to be the case that the most useful candidate substances will be substances that are structurally related to FMRP, PAK, their binding partners, their upstream effectors, and/or their downstream effectors (*e.g.*, mimics). However, it may be the case that the most useful candidate substances will have little or no structural relationship to FMRP, PAK, their binding partners, their upstream effectors, and/or their downstream effectors. In some embodiments, candidate substances are provided as individual substances. In some embodiments, candidate substances are provided in the form of a library, collection, and/or set of candidate substances. In some embodiments, candidate substances can be screened for their ability to modulate PAK.

*Characteristic portion:* As used herein, the term a "characteristic portion" of a substance, in the broadest sense, is one that shares some degree of sequence and/or structural identity and/or at least one functional characteristic with the relevant intact substance. For example, a "characteristic portion" of a protein or polypeptide is one that contains a continuous stretch of amino acids, or a collection of continuous stretches of amino acids, that together are characteristic of a protein or polypeptide. In some embodiments, each such continuous stretch generally will contain at least 2, 5, 10, 15, 20, 50, or more amino acids. A "characteristic portion" of a nucleic acid is one that contains a continuous stretch of nucleotides, or a collection of continuous stretches of nucleotides, that together are characteristic of a nucleic acid. In some embodiments, each such continuous stretch generally will contain at least 2, 5, 10, 15, 20, 50, or more nucleotides. In general, a characteristic portion of a substance (*e.g.* of a protein, nucleic acid, small molecule, *etc.*) is one that, in addition to the sequence and/or structural identity specified above, shares at least one functional characteristic with the relevant intact substance. In some embodiments, a characteristic portion may be biologically active.

*Expression:* As used herein, "expression" of a nucleic acid sequence refers to one or more of the following events: (1) production of an RNA template from a DNA sequence (*e.g.*, by transcription); (2) processing of an RNA transcript (*e.g.*, by splicing, editing, 5' cap formation, and/or 3' end formation); (3) translation of an RNA into a polypeptide or protein; (4) post-translational modification of a polypeptide or protein.

*FMR1:* As used herein, the *"FMR1"* gene refers to any nucleotide sequence that encodes the fragile X mental retardation protein (FMRP) and/or a characteristic portion thereof. Representative examples of *FMR1* nucleotide sequences depicted in Figure 1 include GenBank Accession Number AF305881 and GenBank Accession Number L29074. The *"FXR"* gene is homologous to *FMR1* and may compensate for loss of *FMR1* function. Representative examples of *FXR* nucleotide sequences include, but are not limited to, *FXR1* (GenBank Accession Number U25165) and *FXR2* (GenBank Accession Number U31501). *FXR1 and FXR2* genes depicted in Figure 1 are homologous to *FMR1* and may compensate for loss of *FMR1* function. In some embodiments, an *FMR1* gene comprises any nucleotide sequence that shares about 70%, about 80%, about 90%, about 95%, or greater than 95% sequence identity with the sequences of GenBank Accession Numbers AF305881, L29074, U25165, and/or U31501.

*Fragile X mental retardation proteins (FMRP):* As used herein, "fragile X mental retardation protein," or "FMRP," refers to any protein product of the *fragile X mental retardation (FMR1)* nucleotide sequence, and/or a characteristic portion thereof. Representative examples of FMRP amino acid sequences depicted in Figure 1 include, but are not limited to, GenBank Accession Number AAG22045 and GenBank Accession Number AAB 18829. The "FXR" protein is homologous to FMRP and may compensate for loss of FMRP function. Representative examples of FXR amino acid sequences include, but are not limited to, FXR1 (GenBank Accession Number AAC50155) and FXR2 (GenBank Accession Number AAC50292). In some embodiments, FMRP comprises any amino acid sequence that shares about 60%, about 70%, about 80%, about 90%, about 95%, or greater than 95% sequence identity with the sequences of GenBank Accession Numbers AAG22045, AAB18829, AAC50155, and/or AAC50292.

*Fragile X Syndrome (FXS):* As used herein, in some embodiments "fragile X syndrome," or "FXS," refers to a disease, disorder, and/or condition characterized by one or more of the following symptoms: (1) behavioral symptoms, including but not limited to hyperactivity, stereotypy, anxiety, seizure, impaired social behavior, and/or cognitive delay; (2) defective synaptic morphology, such as an abnormal number, length, and/or width of dendritic spines; and/or (3) defective synaptic function, such as enhanced long-term depression (LTD) and/or reduced long-term potentiation (LTP). In some embodiments, FXS refers to a disease, disorder, and/or condition caused by and/or associated with one or more of the following: (1) a mutation in *FMR1* (the nucleotide sequence encoding FMRP); (2) defective *FMR1* expression; (3) increased and/or decreased expression of FMRP; (4) defective FMRP function; (5) increased and/or decreased expression of FMRP's natural binding partners; (6) an increased and/or decreased ability of FMRP to bind to its natural binding partners; (7) decreased or absent arginine methylation of FMRP; (8) the increased methylation of *FMR1* CpG repeats in the 5' UTR of exon 1; (9) the mislocalization or misexpression of FMRP within the cell or within the organism; (10) the modulation of function of *FMR1* transcription factors Sp1 and/or NRF1; (11) an increased and/or decreased ability of FMRP to associate with polysomes; (12) the loss of function of *FXR1* and/or *FXR2,* which are homologous to *FMR1* and may compensate for loss of *FMR1* function; (13) the decreased ability of FMRP to recognize RNA secondary structures that FMRP normally recognizes (such as intramolecular G quartet and/or FMRP "kissing complex"); (14) an increased and/or decreased ability of FMRP to interact with miRNAs and/or members of the miRNA pathway; (15) an increased and/or decreased ability of FMRP to interact with its known target RNAs, such as RNAs encoding Rac1, microtubule-associated protein 1B, activity-regulated cytoskeleton-associated protein, and/or alpha-calcium/calmodulin-dependent protein kinase II; (16) an increased and/or decreased ability of phosphatase PP2A to act on FMRP (PP2A is thought to bring about translational repression activity of FMRP); (17) the increased and/or decreased activity of mGluR5, which is known to decrease activity of phosphatase PP2A (18) exaggerated signaling in mGluR pathways (Bear et al., 2004, Trends Neurosci., 27:370; incorporated herein by reference); (19) disruption of a KH domain of FMRP, which decreases the ability of FMRP to interact with PAK; and/or (20) an I304N mutation in FMRP, which decreases the ability of FMRP to interact with PAK. Those of ordinary skill in the art will appreciate that the teachings of the present invention described herein with respect to FXS are in fact applicable to other neurodevelopmental disorders including, for example, premature ovarian failure (POF), fragile X-associated tremor ataxia (FXTAS), and/or other neurodevelopmental disorders, including, but not limited to, various forms of mental retardation and/or autism spectrum disorders (ASD). Furthermore, those of ordinary skill in the art will appreciate that the teachings of the present invention described herein with respect to FXS are applicable to any disease, disorder, and/or condition caused by and/or associated with one or more of the following: (1) a mutation in *FMR1* (the nucleotide sequence encoding FMRP); (2) defective *FMR1* expression; (3) increased and/or decreased expression of FMRP; (4) defective FMRP function; (5) increased and/or decreased expression of FMRP's natural binding partners; (6) an increased and/or decreased ability of FMRP to bind to its natural binding partners; (7) decreased or absent arginine methylation of FMRP; (8) the increased methylation of *FMR1* CpG repeats in the 5' UTR of exon 1; (9) the mislocalization or misexpression of FMRP within the cell or within the organism; (10) the modulation of function of *FMR1* transcription factors Sp1 and/or NRF1; (11) an increased and/or decreased ability of FMRP to associate with polysomes; (12) the loss of function of *FXR1* and/or *FXR2,* which are homologous to *FMR1* and may compensate for loss of *FMR1* function; (13) the decreased ability of FMRP to recognize RNA secondary structures that FMRP normally recognizes (such as intramolecular G quartet and/or FMRP "kissing complex"); (14) an increased and/or decreased ability of FMRP to interact with miRNAs and/or members of the miRNA pathway; (15) an increased and/or decreased ability of FMRP to interact with its known target RNAs, such as RNAs encoding Rac1, microtubule-associated protein 1B, activity-regulated cytoskeleton-associated protein, and/or alpha-calcium/calmodulin-dependent protein kinase II; (16) an increased and/or decreased ability of phosphatase PP2A to act on FMRP (PP2A is thought to bring about translational repression activity of FMRP); (17) the increased and/or decreased activity of mGluR5, which is known to decrease activity of phosphatase PP2A; (18) exaggerated signaling in mGluR pathways (Bear et al., 2004, Trends Neurosci., 27:370; incorporated herein by reference); (19) disruption of a KH domain of FMRP, which decreases the ability of FMRP to interact with PAK; and/or (20) an I304N mutation in FMRP, which decreases the ability of FMRP to interact with PAK.

*Functional:* As used herein, a "functional" biological molecule is a biological molecule in a form in which it exhibits a property and/or activity by which it is characterized.

*Gene:* As used herein, the term "gene" has its meaning as understood in the art. It will be appreciated by those of ordinary skill in the art that the term "gene" may include gene regulatory sequences (*e.g.*, promoters, enhancers, *etc.*) and/or intron sequences. It will further be appreciated that definitions of gene include references to nucleic acids that do not encode proteins but rather encode functional RNA molecules such as tRNAs, RNAi-inducing agents, *etc.* For the purpose of clarity we note that, as used in the present application, the term "gene" generally refers to a portion of a nucleic acid that encodes a protein; the term may optionally encompass regulatory sequences, as will be clear from context to those of ordinary skill in the art. This definition is not intended to exclude application of the term "gene" to non-protein-coding expression units but rather to clarify that, in most cases, the term as used in this document refers to a protein-coding nucleic acid.

*Gene product* or *expression product:* As used herein, the term "gene product" or "expression product" generally refers to an RNA transcribed from the gene (pre-and/or post-processing) or a polypeptide (pre- and/or post-modification) encoded by an RNA transcribed from the gene.

*Homology:* As used herein, the term "homology" refers to the overall relatedness between polymeric molecules, *e.g.* between nucleic acid molecules (*e.g.* DNA molecules and/or RNA molecules) and/or between polypeptide molecules. In some embodiments, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% identical. In some embodiments, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% similar.

*Identity:* As used herein, the term "identity" refers to the overall relatedness between polymeric molecules, *e.g.* between nucleic acid molecules (*e.g.* DNA molecules and/or RNA molecules) and/or between polypeptide molecules. Calculation of the percent identity of two nucleic acid sequences, for example, can be performed by aligning the two sequences for optimal comparison purposes (*e.g.,* gaps can be introduced in one or both of a first and a second nucleic acid sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or substantially 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using the algorithm of Meyers and Miller (CABIOS, 1989, 4:11-17), which has been incorporated into the ALIGN program (version 2.0) using a PAM120 weight residue table, a gap length penalty of 12 and a gap penalty of 4. The percent identity between two nucleotide sequences can, alternatively, be determined using the GAP program in the GCG software package using an NWSgapdna.CMP matrix.

*Isolated:* As used herein, the term "isolated" refers to a substance and/or entity that has been (1) separated from at least some of the components with which it was associated when initially produced (whether in nature and/or in an experimental setting), and/or (2) produced, prepared, and/or manufactured by the hand of man. Isolated substances and/or entities may be separated from at least about 10%, about 20%, about 30%, about 40%, about 50%, about 60%, about 70%, about 80%, about 90%, or more of the other components with which they were initially associated. In some embodiments, isolated agents are more than about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components.

*Natural binding partner:* As used herein, the term "natural binding partner" refers to any substance that binds to PAK and/or FMRP. In some embodiments, the substance binds directly, and in some embodiments, the substance binds indirectly. A natural binding partner substance may be a protein, nucleic acid, lipid, carbohydrate, glycoprotein, proteoglycan, and/or small molecule that binds to either PAK and/or FMRP. A change in the interaction between PAK and/or FMRP and a natural binding partner may manifest itself as an increased and/or decreased probability that the interaction forms. A change in the interaction between PAK and/or FMRP and a natural binding partner may manifest itself as an increased and/or decreased concentration of PAK and/or FMRP/natural binding partner complex within the cell. This can result in an increased and/or decreased activity of PAK and/or FMRP. The present invention identifies FMRP as a novel natural binding partner of PAK. Other natural binding partners of PAK include PAK substrates and/or other substances that interact with PAK. Examples of natural binding partners of PAK include; but are not limited to; Myosin light chain kinase (MLCK); regulatory Myosin light chain (R-MLC); Myosin I heavy chain; Myosin II heavy chain; Myosin VI; Caldesmon; Desmin; Op18/stathmin; Merlin; Filamin A; LIM kinase (LIMK); Ras; Raf; Mek; p47^{phox}; BAD; caspase 3; estrogen and/or progesterone receptors; RhoGEF; GEF-H1; NET1; Gαz; phosphoglycerate mutase-B; RhoGDI; prolactin; p41^{Arc} ; Aurora-A; Rac/Cdc42; CIB; sphingolipids; G-protein β and/or γ subunits; PIX/COOL; GIT/PKL; Paxillin; Nef; NESH; SH3-containing proteins (*e.g*. Nck and/or Grb2); kinases (*e.g.* Akt, PDK1, PI 3-kinase/p85, Cdk5, Cdc2, Src kinases, Abl, and/or protein kinase A (PKA)); and/or phosphatases (*e.g*. phosphatase PP2A, POPX1, and/or POPX2) (Bokoch et al., 2003, Annu. Rev. Biochem., 72:743; and Hofmann et al., 2004, J. Cell Sci., 117:4343; both of which are incorporated herein by reference). Prominent upstream effectors of PAK include, but are not limited to, PDK1, PDK2, PI3K, and/or NMDARs.

*Nucleic acid:* As used herein, the term "nucleic acid," in its broadest sense, refers to any compound and/or substance that is or can be incorporated into an oligonucleotide chain. In some embodiments, a nucleic acid is a compound and/or substance that is or can be incorporated into an oligonucleotide chain via a phosphodiester linkage. In some embodiments, "nucleic acid" refers to individual nucleic acid residues (*e.g.* nucleotides and/or nucleosides). In some embodiments, "nucleic acid" refers to an oligonucleotide chain comprising individual nucleic acid residues. As used herein, the terms "oligonucleotide" and "polynucleotide" can be used interchangeably. In some embodiments, "nucleic acid" encompasses RNA as well as single and/or double-stranded DNA and/or cDNA. Furthermore, the terms "nucleic acid," "DNA," "RNA," and/or similar terms include nucleic acid analogs, *i.e*. analogs having other than a phosphodiester backbone. For example, the so-called "peptide nucleic acids," which are known in the art and have peptide bonds instead of phosphodiester bonds in the backbone, are considered within the scope of the present invention. The term "nucleotide sequence encoding an amino acid sequence" includes all nucleotide sequences that are degenerate versions of each other and/or encode the same amino acid sequence. Nucleotide sequences that encode proteins and/or RNA may include introns. Nucleic acids can be purified from natural sources, produced using recombinant expression systems and optionally purified, chemically synthesized, *etc.* Where appropriate, *e.g.*, in the case of chemically synthesized molecules, nucleic acids can comprise nucleoside analogs such as analogs having chemically modified bases or sugars, backbone modifications, *etc.* A nucleic acid sequence is presented in the 5' to 3' direction unless otherwise indicated. The term "nucleic acid segment" is used herein to refer to a nucleic acid sequence that is a portion of a longer nucleic acid sequence. In many embodiments, a nucleic acid segment comprises at least 3, 4, 5, 6, 7, 8, 9, 10, or more residues. In some embodiments, a nucleic acid is or comprises natural nucleosides (*e.g.* adenosine, thymidine, guanosine, cytidine, uridine, deoxyadenosine, deoxythymidine, deoxyguanosine, and deoxycytidine); nucleoside analogs (*e.g.*, 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (*e.g*., methylated bases); intercalated bases; modified sugars (*e.g.*, 2'-fluororibose, ribose, 2'-deoxyribose, arabinose, and hexose); and/or modified phosphate groups (*e.g.*, phosphorothioates and 5'-N-phosphoramidite linkages). In some embodiments, the present invention is specifically directed to "unmodified nucleic acids," meaning nucleic acids (*e.g.* polynucleotides and residues, including nucleotides and/or nucleosides) that have not been chemically modified in order to facilitate or achieve delivery.

*PAK activity:* As used herein, the term "PAK activity" refers to any activity and/or function of p21-activated kinase. Examples of "PAK activity" include, but are not limited to, PAK binding to other substances, PAK kinase activity, autophosphorylation, translocation, *etc.* "PAK activity" is used interchangeably with "PAK function."

*PAK Modulator:* As used herein, the term "PAK modulator" refers to any substance that directly and/or indirectly changes, affects, alters, increases, and/or decreases the activity and/or levels of PAK. PAK modulators may modulate the level of PAK mRNA and/or protein; an activity of PAK; the half-life of PAK RNA and/or protein; and/or the interaction between PAK and its natural binding partners (*e.g.*, a substrate for a PAK kinase, a Rac protein, a cdc42 protein, and/or FMRP), as measured using standard methods. "PAK inhibitors" may inhibit, decrease, and/or abolish the level of PAK mRNA and/or protein; an activity of PAK; the half-life of PAK mRNA and/or protein; and/or the interaction between PAK and its natural binding partners (*e.g.*, a substrate for a PAK kinase, a Rac protein, a cdc42 protein, and/or FMRP), as measured using standard methods. "PAK activators" may activate and/or increase the level of PAK mRNA and/or protein; an activity of PAK; the half-life of PAK mRNA and/or protein; and/or the interaction between PAK and its natural binding partners (*e.g.*, a substrate for a PAK kinase, a Rac protein, a cdc42 protein, and/or FMRP), as measured using standard methods. mRNA expression levels may be determined using standard RNase protection assays and/or *in situ* hybridization assays, and/or the level of protein may be determined using standard Western and/or immunohistochemistry analysis. The phosphorylation levels of signal transduction proteins downstream of PAK activity may be measured using standard assays. PAK modulators may modulate binding between PAK and FMRP. "PAK inhibitors" may reduce, abolish, and/or remove the binding between these two entities, and "PAK activators" may increase and/or strengthen the binding between these two entities. Thus, binding between PAK and FMRP is stronger in the absence of the inhibitor than in its presence and is weaker in the absence of the activator than in its presence. Put another way, a PAK inhibitor increases the Kₘ of binding between PAK and FMRP, and a PAK activator decreases the Kₘ of binding between PAK and FMRP. Alternatively or additionally, PAK modulators may inhibit and/or activate the kinase activity of PAK. In particular, PAK inhibitors and/or activators may modulate the ability of PAK to phosphorylate FMRP. PAK modulators may be inorganic and/or organic. PAK modulators may comprise one or more of the following: proteins, peptides, antibodies, nucleic acids, RNAi-inducing entities, antisense oligonucleotides, ribozymes, small molecules, glycoproteins, proteoglycans, viruses, lipids, and/or carbohydrates. PAK modulators may be in the form of monomers, dimers, oligomers, and/or in a complex. In some embodiments, as used herein, a "PAK modulator" is a substance that modulates all isoforms of PAK. In some embodiments, as used herein, a "PAK modulator" is a substance that modulates a single isoform of PAK. In some embodiments, as used herein, a "PAK modulator" is a substance that modulates two or more isoforms of PAK. In some embodiments, as used herein, a "PAK modulator" is a substance that modulates a subset of PAK isoforms. In some embodiments, as used herein, a "PAK modulator" is a substance that modulates any of the isoforms of PAK.

*PAK protein:* As used herein the term "PAK protein" refers to a protein that belongs in the family of PAK serine/threonine protein kinases. These include mammalian isoform identified, *e.g.*, PAK1, PAK2, PAK3, PAK4, PAK5, PAK6, and/or PAK7; and/ or lower eukaryotic isoforms, such as the yeast Ste20 (Leberter *et al.,* 1992, *EMBO J.,* 11:4805; incorporated herein by reference) and/or the Dictyostelium single-headed myosin I heavy chain kinases (Wu *et al.,* 1996, *J. Biol. Chem.,* 271:31787; incorporated herein by reference). Representative examples of PAK amino acid sequences depicted in Figures 2, 3, 4, and 5 include, but are not limited to, human PAK1 (GenBank Accession Number AAA65441), human PAK2 (GenBank Accession Number AAA65442), human PAK3 (GenBank Accession Number AAC36097), human PAK 4 (GenBank Accession Numbers NP_005875 and CAA09820), human PAK5 (GenBank Accession Numbers CAC18720 and BAA94194), human PAK6 (GenBank Accession Numbers NP_064553 and AAF82800), human PAK7 (GenBank Accession Number Q9P286), *C. elegans* PAK (GenBank Accession Number BAA11844), *D. melanogaster* PAK (GenBank Accession Number AAC47094), and rat PAK1 (GenBank Accession Number AAB95646). Representative examples of *PAK* genes encoding PAK proteins include, but are not limited to, human *PAK1* (GenBank Accession Number U24152), human *PAK2* (GenBank Accession Number U24153), human *PAK3* (GenBank Accession Number AF068864), human *PAK4* (GenBank Accession Number AJ011855), human *PAK5* (GenBank Accession Number AB040812), and human *PAK6* (GenBank Accession Number AF276893). In some embodiments, a PAK protein comprises any amino acid sequence that shares about 60%, about 70%, about 80%, about 90%, about 95%, or greater than 95% sequence identity with the sequences of GenBank Accession Numbers AAA65441, AAA65442, AAC36097, NP_005875, CAA09820, CAC18720, BAA94194, NP_064553, AAF82800, Q9P286, BAA11844, AAC47094, and/or AAB95646. In some embodiments, a *PAK* gene comprises any nucleotide sequence that shares about 60%, about 70%, about 80%, about 90%, about 95%, or greater than 95% sequence identity with the sequences of GenBank Accession Numbers U24152, U24153, AF068864, AJ011855, AB040812, and/or AF276893. In some embodiments, as used herein, "PAK" refers to all isoforms of PAK. In some embodiments, as used herein, "PAK" refers to a single isoform of PAK. In some embodiments, as used herein, "PAK" refers to two or more isoforms of PAK. In some embodiments, as used herein, "PAK" refers to a subset of PAK isoforms. In some embodiments, as used herein, "PAK" refers to any of the isoforms of PAK.

*Protein:* As used herein, the term "protein" refers to a polypeptide (*i.e*., a string of at least two amino acids linked to one another by peptide bonds). Proteins may include moieties other than amino acids (*e.g.*, may be glycoproteins, proteoglycans, *etc*.) and/or may be otherwise processed or modified. Those of ordinary skill in the art will appreciate that a "protein" can be a complete polypeptide chain as produced by a cell (with or without a signal sequence), or can be a characteristic portion thereof. Those of ordinary skill will appreciate that a protein can sometimes include more than one polypeptide chain, for example linked by one or more disulfide bonds or associated by other means. Polypeptides may contain L-amino acids, D-amino acids, or both and may contain any of a variety of amino acid modifications or analogs known in the art. Useful modifications include, *e.g*., terminal acetylation, amidation, *etc.* In some embodiments, proteins may comprise natural amino acids, non-natural amino acids, synthetic amino acids, and combinations thereof. The term "peptide" is generally used to refer to a polypeptide having a length of less than about 100 amino acids.

*Small Molecule:* In general, a "small molecule" is understood in the art to be an organic molecule that is less than about 5 kilodaltons (Kd) in size. In some embodiments, the small molecule is less than about 4 Kd, 3 Kd, about 2 Kd, or about 1 Kd. In some embodiments, the small molecule is less than about 800 daltons (D), about 600 D, about 500 D, about 400 D, about 300 D, about 200 D, or about 100 D. In some embodiments, a small molecule is less than about 2000 g/mol, less than about 1500 g/mol, less than about 1000 g/mol, less than about 800 g/mol, or less than about 500 g/mol. In some embodiments, small molecules are non-polymeric. In some embodiments, small molecules are not proteins, peptides, or amino acids. In some embodiments, small molecules are not nucleic acids or nucleotides. In some embodiments, small molecules are not saccharides or polysaccharides.

*Subject:* As used herein, the term "subject" or "patient" refers to any organism to which a composition of this invention may be administered, *e.g.*, for experimental, diagnostic, prophylactic, and/or therapeutic purposes. Typical subjects include animals (*e.g.*, mammals such as mice, rats, rabbits, non-human primates, and humans; insects; worms; *etc.*).

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Suffering from:* An individual who is "suffering from" FXS and/or other neurodevelopmental disorders has been diagnosed with or displays one or more symptoms of FXS and/or other neurodevelopmental disorders.

*Susceptible to:* An individual who is "susceptible to" FXS has not been diagnosed with FXS and/or may not exhibit symptoms of FXS but may be characterized by one or more of the following: (1) a mutation in *FMR1* (the nucleotide sequence encoding FMRP); (2) defective *FMR1* expression; (3) increased and/or decreased expression of FMRP; (4) defective FMRP function; (5) increased and/or decreased expression of FMRP's natural binding partners; (6) an increased and/or decreased ability of FMRP to bind to its natural binding partners; (7) decreased or absent arginine methylation of FMRP; (8) the increased methylation of *FMR1* CpG repeats in the 5' UTR of exon 1; (9) the mislocalization or misexpression of FMRP within the cell or within the organism; (10) the modulation of function of *FMR1* transcription factors Sp1 and/or NRF1; (11) an increased and/or decreased ability of FMRP to associate with polysomes; (12) the loss of function of *FXR1* and/or *FXR2*, which are homologous to *FMR1* and may compensate for loss of *FMR1* function; (13) the decreased ability of FMRP to recognize RNA secondary structures that FMRP normally recognizes (such as intramolecular G quartet and/or FMRP "kissing complex"); (14) an increased and/or decreased ability of FMRP to interact with miRNAs and/or members of the miRNA pathway; (15) an increased and/or decreased ability of FMRP to interact with its known target RNAs, such as RNAs encoding RacI, microtubule-associated protein 1B, activity-regulated cytoskeleton-associated protein, and/or alpha-calcium/calmodulin-dependent protein kinase, II; (16) an increased and/or decreased ability of phosphatase PP2A to act on FMRP (PP2A is thought to bring about translational repression activity of FMRP); (17) the increased and/or decreased activity of mGluR5, which is known to decrease activity of phosphatase PP2A; (18) exaggerated signaling in mGluR pathways (Bear *et al.,* 2004, *Trends Neurosci.,* 27:370; incorporated herein by reference); (19) disruption of a KH domain of FMRP, which decreases the ability of FMRP to interact with PAK; and/or (20) an I304N mutation in FMRP, which decreases the ability of FMRP to interact with PAK. In some embodiments, an individual who is susceptible to FXS will develop FXS and/or other neurodevelopmental disorder. In some embodiments, an individual who is susceptible to FXS will not develop FXS and/or other neurodevelopmental disorder.

*Test substance:* As used herein, the phrase "test substance" refers to any substance that may be utilized in the systems, methods, assays, and/or compositions described herein. A "test substance" may refer to one or more of the following: (I) a PAK protein, a nucleic acid encoding PAK, and/or homolog, portion, variant, mutant, and/or derivative thereof; (2) a natural binding partner of PAK, a nucleic acid encoding a natural binding partner of PAK, and/or a homolog, portion, variant, mutant, and/or derivative thereof; and/or (3) an FMRP protein, a nucleic acid encoding FMRP, and/or a homolog, portion, variant, mutant, and/or derivative thereof; (4) a substrate of PAK kinase, a nucleic acid encoding a substrate of PAK, and/or a homolog, portion, variant, mutant, and/or derivative thereof; and/or (5) a substance related to PAK signal transduction, and/or a homolog, portion, variant, mutant, and/or derivative thereof. In some embodiments, a test substance is a protein; nucleic acid; small molecule; carbohydrate; lipid; library, collection, and/or set of any of these; and/or combination of any of these.

*Therapeutically effective amount:* As used herein, the term "therapeutically effective amount" means an amount of inventive PAK modulator that is sufficient, when administered to a subject suffering from or susceptible to FXS and/or other neurodevelopmental disorders, to treat, diagnose, prevent, and/or delay the onset of the FXS and/or other neurodevelopmental disorders symptom(s) and/or condition(s).

*Therapeutic agent:* As used herein, the phrase "therapeutic agent" refers to any agent that, when administered to a subject, has a therapeutic effect and/or elicits a desired biological and/or pharmacological effect.

*Treating:* As used herein, the term "treat," "treatment," or "treating" refers to any method used to partially or completely alleviate, ameliorate, relieve, inhibit, prevent, delay onset of, reduce severity of and/or reduce incidence of one or more symptoms or features of a particular disease, disorder, and/or condition (*e.g.*, FXS and/or other neurodevelopmental disorders). Treatment may be administered to a subject who does not exhibit signs of a disease and/or exhibits only early signs of the disease for the purpose of decreasing the risk of developing pathology associated with the disease.

*Vector:* As used herein, "vector" refers to a nucleic acid molecule capable of transporting another nucleic acid to which it has been linked. In some embodiment, vectors are capable of extra-chromosomal replication and/or expression of nucleic acids to which they are linked in a host cell such as a eukaryotic and/or prokaryotic cell. Vectors capable of directing the expression of operatively linked genes are referred to herein as "expression vectors."

### Detailed Description of Certain Preferred Embodiments of the Invention

In some embodiments, the present invention provides methods for treating fragile X syndrome (FXS) and/or other neurodevelopmental disorders. In certain embodiments, treatment of patients with one or more PAK modulators may be used to treat FXS.

In some embodiments, the present invention provides modulators of PAK activity and/or levels. In some embodiments, a modulator of PAK increases PAK activity and/or levels. In some embodiments, a modulator of PAK inhibits PAK activity and/or levels. In accordance with some embodiments, compositions are provided comprising at least one PAK modulator and a pharmaceutically acceptable excipient. In certain embodiments, PAK modulators function by modulating the interaction between PAK and the fragile X mental retardation protein (FMRP).

In some embodiments, the present invention provides methods for identifying PAK modulators. In some embodiments, such methods include high throughput screening methods. In some embodiments, the methods include *in vitro, in cyto,* and/or *in vivo* assays.

### p21-activated kinase (PAK)

PAK, a family of serine-threonine kinases that is composed of at least three members, PAK1, PAK2 and/or PAK3, functions downstream of the small GTPases Rac and/or Cdc42 to regulate multiple cellular functions, including motility, morphogenesis, angiogenesis, and/or apoptosis (Bokoch et al., 2003, Annu. Rev. Biochem., 72:743; and Hofmann et al., 2004, J. Cell Sci., 117:4343; both of which are incorporated herein by reference). GTP-bound Rac and/or Cdc42 bind to inactive PAK, releasing steric constraints imposed by a PAK autoinhibitory domain and/or permitting PAK auto-phosphorylation and/or activation. Numerous autophosphorylation sites have been identified that serve as markers for activated PAK.

In some embodiments, as used herein, "PAK" refers to all isoforms of PAK (e.g. PAK1, PAK2, PAK3, PAK4, PAK5, PAK6, PAK7, etc.). In some embodiments, as used herein, "PAK" refers to a single isoform of PAK. In some embodiments, as used herein, "PAK" refers to two or more isoforms of PAK. In some embodiments, as used herein, "PAK" refers to a subset of PAK isoforms. In some embodiments, as used herein, "PAK" refers to any of the isoforms of PAK. In some embodiments, as used herein, "PAK" is a substance that is at least 60%, at least 70%, at least 80%, at least 90%, at least 95%, or at least 98% identical to any and/or all of PAK1, PAK2, PAK3, PAK4, PAK5, PAK6, and/or PAK7.

Prominent downstream targets of mammalian PAK include, but are not limited to, substrates of PAK kinase, such as Myosin light chain kinase (MLCK), regulatory Myosin light chain (R-MLC), Myosins I heavy chain, myosin II heavy chain, Myosin VI, Caldesmon, Desmin, Op18/stathmin, Merlin, Filamin A, LIM kinase (LIMK), Ras, Raf, Mek, p47^{Phox}, BAD, caspase 3, estrogen and/or progesterone receptors, RhoGEF, GEF-H1, NET1, Gαz, phosphoglycerate mutase-B, RhoGDI, prolactin, p41^{Arc}, and/or Aurora-A (Bokoch et al., 2003, Annu. Rev. Biochem., 72:743; and Hofmann et al., 2004, J. Cell Sci., 117:4343; both of which are incorporated herein by reference). Other substances known to bind to PAK in cells include CIB; sphingolipids; G-protein β and/or γ subunits; PIX/COOL; GIT/PKL; Nef; Paxillin; NESH; SH3-containing proteins (*e.g.* Nck and/or Grb2); kinases (*e.g.* Akt, PDK1, PI 3-kinase/p85, Cdk5, Cdc2, Src kinases, Abl, and/or protein kinase A (PKA)); and/or phosphatases (*e.g.* phosphatase PP2A, POPX1, and/or POPX2)

Prominent upstream effectors of PAK include, but are not limited to, PDK1, PDK2, PI3K, and/or NMDARs.

The present invention encompasses the recognition that the abnormalities in cortical spine morphology of FXS patients and/or *FMR1* KO mice are substantially opposite of those found in transgenic mice in which activity of p21-activated kinase (PAK) is inhibited by its dominant negative form (*dnPAK* TG mice), for example, in the postnatal forebrain.

### PAK modulators

PAK inhibitors have been described in the art as possible substances for use in the treatment of cancer, endometriosis, urogenital disorders, macropinocytosis, viral infection, vascular permeability, joint disease, lymphocyte activation, muscle contraction, and/or diabetes (see, for example, U.S. Patents 5,863,532, 6,191,169, and 6,248,549; U.S. Patent Applications 2002/0045564, 2002/086390, 2002/106690, 2002/142325, 2003/124107, 2003/166623, 2004/091992, 2004/102623, 2004/208880, 2005/0203114, 2005/037965, 2005/080002, and 2005/233965; EP Patent Publication 1492871; Kumar et al., 2006, Nat. Rev. Cancer, 6:459; Eswaren et al., 2007, Structure, 15:201; all of which are incorporated herein by reference).

PAK inhibitors have also been described in the art as possible substances for use in the treatment of neurological disorders characterized by nerve damage and/or neurodegeneration. Such disorders include Parkinson's disease, Alzheimer's disease, prion-related diseases, neurofibromatosis, stroke-induced nerve damage, and/or diseases associated with nerve injury due to ischaemia and/or anoxia (see, for example, U.S. Patents 5,952,217 and 6,046,224; U.S. Patent Application 2006/088897; and PCT applications WO 99/02701 and WO 04/07507; all of which are incorporated herein by reference).

However, PAK modulators have not previously been described in the art as possible substances for use in the treatment of neurodevelopmental disorders, including but not limited to FXS, premature ovarian formation (POF), fragile X-associated tremor ataxia (FXTAS), various forms of mental retardation, and/or autism spectrum disorders (ASD). The present invention encompasses the use of PAK modulators in the treatment of FXS, POF, FXTAS, and/or other neurodevelopmental disorders.

In some embodiments, a PAK modulator refers to a substance, wherein the substance may be inorganic and/or organic; a biological effector and/or a nucleic acid encoding an agent such as a biological effector; a protein, polypeptide, or peptide including, but not limited to, a structural protein, an enzyme, a cytokine (such as an interferon and/or an interleukin), an antibiotic, a polyclonal or monoclonal antibody and/or an effective part thereof, such as an Fv fragment, which antibody or part thereof may be natural, synthetic, or humanized, a peptide hormone, a receptor, a signaling molecule and/or other protein; a nucleic acid, as defined below, including, but not limited to, an oligonucleotide and/or modified oligonucleotide, an RNAi-inducing entity, an antisense oligonucleotide and/or modified antisense oligonucleotide, cDNA, genomic DNA, an artificial and/or natural chromosome (*e.g.* a yeast artificial chromosome, bacterial artificial chromosome, *etc.*) and/or portion thereof, RNA, including mRNA, tRNA, rRNA and/or a ribozyme, and/or a peptide nucleic acid (PNA); a virus or virus-like particle; a nucleotide, ribonucleotide, and/or synthetic analogue thereof, which may be modified or unmodified; an amino acid and/or analogue thereof, which may be modified or unmodified; a non-peptide (*e.g*., steroid) hormone; a glycoprotein; a proteoglycan; a lipid; and/or a carbohydrate. Small molecules, including inorganic and/or organic chemicals, which bind to and/or occupy the active site of the polypeptide thereby making the catalytic site inaccessible to substrate such that normal biological activity is prevented, are included. A PAK modulator may be in solution and/or in suspension (*e.g.*, in crystalline, colloidal, or other particulate form). A PAK modulator may be in the form of a monomer, dimer, oligomer, *etc.*, and/or in a complex.

In some embodiments, as used herein, a "PAK modulator" is a substance that modulates all isoforms of PAK (e.g. PAK1, PAK2, PAK3, PAK4, PAK5, PAK6, PAK7, etc.). In some embodiments, as used herein, a "PAK modulator" is a substance that modulates a single isoform of PAK. In some embodiments, as used herein, a "PAK modulator" is a substance that modulates two or more isoforms of PAK. In some embodiments, as used herein, a "PAK modulator" is a substance that modulates a subset of PAK isoforms. In some embodiments, as used herein, a "PAK modulator," is a substance that modulates any of the isoforms of PAK. In some embodiments, as used herein, a "PAK modulator" is a substance that modulates any of PAK1, PAK2, PAK3, PAK4, PAK5, PAK6, and/or PAK7. In some embodiments, as used herein, a "PAK modulator" is a substance that modulates any subset of PAK1, PAK2, PAK3, PAK4, PAK5, PAK6, and/or PAK7. In some embodiments, as used herein, a "PAK modulator" is a substance that modulates all of PAK1, PAK2, PAK3, PAK4, PAK5, PAK6, PAK7. In some embodiments, as used herein, a "PAK modulator" is a substance that modulates any entity that is at least 60%, at least 70%, at least 80%, at least 90%, at least 95%, or at least 98% identical to any and/or all of PAK1, PAK2, PAK3, PAK4, PAK5, PAK6, and/or PAK7.

Candidate PAK modulators may be isolated from natural sources, such as animals, bacteria, fungi, plants, and/or marine samples. It will be understood that candidate PAK modulators can be derived and/or synthesized from chemical compositions and/or man-made substances.

### Rational Drug Design

As used herein the term "candidate substance" refers to any substance that may potentially inhibit FXS and/or modulate PAK activity. A candidate substance may be a protein, a nucleic acid, a small molecule, a lipid, a carbohydrate, a glycoprotein, a proteoglycan, combinations thereof, and/or characteristic portions thereof. It may prove to be the case that the most useful candidate substances will be substances that are structurally related to FMRP, PAK, their binding partners, their upstream effectors, and/or their downstream effectors (*e.g.*, mimics). Using lead compounds to help develop improved compounds is known as "rational drug design" and includes not only comparisons with known inhibitors and/or activators, but predictions relating to the structure of target substances.

In some embodiments, rational drug design may be used to predict and/or produce structural analogs of known biologically-active PAK modulators (*i.e*. "PAK modulator starting materials"). By creating such analogs, it is possible to fashion therapeutic agents which may be more active and/or stable than the PAK modulator starting material, may have different susceptibility to alteration, and/or may affect the function of various the PAK modulator starting material. In some embodiments, a three-dimensional structure for a known candidate substance and/or characteristic portion thereof could be generated. In some embodiments, this is accomplished by x-ray crystallography, computer modeling, and/or by a combination of these approaches.

In some embodiments, antibodies are used to ascertain the structure of a PAK modulator starting material. In principle, this approach yields a pharmacore upon which subsequent drug design can be based. It is possible to bypass protein crystallography altogether by generating anti-idiotypic antibodies to a functional, pharmacologically active antibody. As a mirror image of mirror image, the binding site of anti-idiotype would be expected to be an analog of the original antigen. The anti-idiotype could then be used to identify and/or isolate peptides from banks of chemically- and/or biologically-produced peptides. Selected peptides would then serve as pharmacores. Anti-idiotypes may be generated using the methods described herein for producing antibodies, using an antibody as the antigen.

Alternatively or additionally, the present invention encompasses the recognition that other sterically similar substances may be formulated to mimic the key portions of the structure of rationally-designed PAK modulators. Such substances may be used in the same manner as or in a different manner from the PAK modulator starting material.

### Libraries

In some embodiments, libraries of candidate substances may be employed in the methods and/or compositions described herein. The phrase "library of candidate substances," as used herein, refers to a collection and/or set of multiple species of substances that consist of randomly- and/or systematically-selected subunits and/or members. Screening libraries of candidate substances is a rapid and/or efficient way to screen large number of related and/or unrelated substances for activity. Combinatorial approaches lend themselves to rapid evolution of potential drugs by the creation of second, third, fourth, *etc*. generation substances modeled of active, but otherwise undesirable substances.

In certain embodiments, combinatorial libraries (also known as "combinatorial chemical libraries"), small molecule libraries, peptides and/or peptide mimetics, defined chemical entities, oligonucleotides, and/or natural product libraries are screened for activity. In some embodiments, a library of candidate substances may comprise a synthetic combinatorial library (*e.g.*, a combinatorial chemical library), a cellular extract, a bodily fluid (*e.g.*, urine, blood, tears, sweat, and/or saliva), or other mixture of synthetic and/or natural substances (*e.g.*, a library of small molecules and/or a fermentation mixture).

In some embodiments, libraries of candidate substances may include, for example, proteins (*e.g.* peptides, polypeptides, antibodies, amino acids, *etc.*), nucleic acids (*e.g.*, DNA, RNA, oligonucleotides, RNAi-inducing entities, antisense nucleic acids, ribozymes, peptide nucleic acids, aptamers, *etc.*), carbohydrates (*e.g.* monosaccharides, disaccharides, polysacharides, *etc*.), small molecules (*e.g.* organic small molecules, inorganic small molecules, *etc*.), combinations thereof, and/or characteristic portions thereof. Each member of a library may be singular and/or may be a part of a mixture (*e.g.*, a "compressed library"). A library may comprise substantially purified substances and/or may be "dirty" (*i.e.*, containing a significant quantity of impurities).

In some embodiments, candidate substances may be used in an initial screen in batches (*e.g.* batches of types of substances). The substances of those batches which show enhancement and/or reduction of the activity being assayed may subsequently be tested individually.

In some embodiments, libraries are acquired from various commercial sources in an effort to "brute force" the identification of useful substances. In some embodiments, commercially available libraries are obtained from Affymetrix, ArQule, Neose Technologies, Sarco, Ciddco, Oxford Asymmetry, Maybridge, Aldrich, Panlabs, Pharmacopoeia, Sigma, and/or Tripose. A comprehensive review of combinatorial libraries, in particular their construction and/or uses is provided in Dolle et al. (1999, J. of Comb. Chem., 1:235; incorporated herein by reference). Reference is made to combinatorial peptide library protocols (Cabilly, ed., Methods in Molecular-Biology, Humana Press, Totowa, NJ, 1998; incorporated herein by reference).

Further references describing combinatorial libraries, their production and/or use include those available from the URL http://www.netsci.org/Science/Combichem/, including The Chemical Generation of Molecular Diversity. Michael R. Pavia, Sphinx Pharmaceuticals, A Division of Eli Lilly (Published July, 1995); Combinatorial Chemistry: A Strategy for the Future-MDL Information Systems discusses the role its Project Library plays in managing diversity libraries (Published July, 1995); Solid Support Combinatorial Chemistry in Lead Discovery and SAR Optimization, Adnan M. M. Mjalli and Barry E. Toyonaga, Ontogen Corporation (Published July, 1995); Non-Peptidic Bradykinin Receptor Antagonists From a Structurally Directed Non-Peptide Library. Sarvajit Chakravarty, Babu J. Mavunkel, Robin Andy, Donald J. Kyle*, Scios Nova Inc. (Published July, 1995); Combinatorial Chemistry Library Design using Pharmacophore Diversity Keith Davies and Clive Briant, Chemical Design Ltd. (Published July, 1995); A Database System for Combinatorial Synthesis Experiments-Craig James and David Weininger, Daylight Chemical Information Systems, Inc. (Published July, 1995); An Information Management Architecture for Combinatorial Chemistry, Keith Davies and Catherine White, Chemical Design Ltd. (Published July, 1995); Novel Software Tools for Addressing Chemical Diversity, R. S. Pearlman, Laboratory for Molecular Graphics and Theoretical Modeling, College of Pharmacy, University of Texas (Published June/July, 1996); Opportunities for Computational Chemists Afforded by the New Strategies in Drug Discovery: An Opinion, Yvonne Connolly Martin, Computer Assisted Molecular Design Project, Abbott Laboratories (Published June/July, 1996); Combinatorial Chemistry and Molecular Diversity Course at the University of Louisville: A Description, Arno F. Spatola, Department of Chemistry, University of Louisville (Published June/July, 1996); Chemically Generated Screening Libraries: Present and Future. Michael R. Pavia, Sphinx Pharmaceuticals, A Division of Eli Lilly (Published June/July, 1996); Chemical Strategies For Introducing Carbohydrate Molecular Diversity Into The Drug Discovery Process. Michael J. Sofia, Transcell Technologies Inc. (Published June/July, 1996); Data Management for Combinatorial Chemistry. Maryjo Zaborowski, Chiron Corporation and Sheila H. DeWitt, Parke-Davis Pharmaceutical Research, Division of Warner-Lambert Company (Published November, 1995); and/or The Impact of High Throughput Organic Synthesis on R&D in Bio-Based Industries, John P. Devlin (Published March, 1996); all of which are incorporated herein by reference.

Selection protocols for isolating desired members of large libraries are known in the art. In some embodiments, selection protocols involve phage display techniques. Such systems, in which diverse peptide sequences are displayed on the surface of filamentous bacteriophage have proven useful for creating libraries of antibody fragments (and the nucleotide sequences that encoding them) for *in vitro* selection and/or amplification of specific antibody fragments that bind a target antigen. Nucleotide sequences encoding the V_{H} and/or V_{L} regions are linked to gene fragments which encode leader signals that direct them to the periplasmic space of *E*. *coli* and, as a result, the resultant antibody fragments are displayed on the surface of the bacteriophage, typically as fusions to bacteriophage coat proteins (*e.g.*, pIII and/or pVIII). Alternatively or additionally, antibody fragments are displayed externally on lambda phage capsids (phagebodies). An advantage of phage-based display systems is that, because they are biological systems, selected library members may be amplified simply by growing the phage containing the selected library member in bacterial cells. Furthermore, since the nucleotide sequence that encodes the polypeptide library member is contained on a phage and/or phagemid vector, sequencing, expression, and/or subsequent genetic manipulation is relatively straightforward.

Methods for the construction of bacteriophage antibody display libraries and/or lambda phage expression libraries are well known in the art (Kang et al., 1991. Proc. Natl. Acad. Sci. USA., 88: 4363; Clackson et al., 1991, Nature, 352: 624; Lowman et al., 1991, Biochemistry, 30: 10832; Burton et al., 1991, Proc. Natl. Acid. Sci., USA, 88: 10134; Hoogenboom et al., 1991, Nucleic Acids Res, 19: 4133; Chang et al., 1991, J. Immunol., 147: 3610; Breitling et al., 1991, Gene, 104: 147; Hawkins et al., 1992, J. Immunol., 22: 867; Marks et al., 1992, J. Biol. Chem., 267: 16007; and Lerner et al., 1992, Science, 258: 1313; all of which are incorporated herein by reference). Such techniques may be modified if necessary for the expression generally of polypeptide libraries.

One approach has been the use of scFv phage-libraries (Bird et al., 1988, Science, 242: 423; Huston et al., 1988, Proc. Natl. Acad. Sci., USA, 85:5879; Chaudhary et al., 1990, Proc. Natl. Acad. Sci, USA, 87:1066; and Chiswell et al., 1992, Trends Biotech., 10:80; all of which are incorporated herein by reference). Various embodiments of scFv libraries displayed on bacteriophage coat proteins have been described. Refinements of phage display approaches are known, for example as described in PCT Publications WO 92/01047, WO 96/06213, and WO 97/08320 (all of which are incorporated herein by reference).

In certain embodiments, alternative library selection technologies include bacteriophage lambda expression systems, which may be screened directly as bacteriophage plaques and/or as colonies of lysogens, as previously described (Huse et al., 1989, Science, 246:1275; Caton et al., 1990, Proc. Natl. Acad. Sci., USA, 87; Mullinax et al., 1990, Proc. Natl. Acad. Sci., USA, 87:8095; and Persson et al., 1991, Proc. Natl. Acad. Sci., USA, 88: 2432; all of which are incorporated herein by reference) and are of use. These expression systems may be used to screen a large number of different members of a library, in the order of about 10⁶ or more.

In some embodiments, screening systems rely, for example, on direct chemical synthesis of library members. One method involves the synthesis of peptides on a set of pins and/or rods, such as described in PCT Publication WO 84/03564 (incorporated herein by reference). A similar method involving peptide synthesis on beads, which forms a peptide library in which each bead is an individual library member, is described in U.S. Patent 4,631,211 (incorporated herein by reference) and a related method is described in PCT Publication WO 92/00091 (incorporated herein by reference). A significant improvement of the bead-based methods involves tagging each bead with a unique identifier tag, such as an oligonucleotide, so as to facilitate identification of the amino acid sequence of each library member. These improved bead-based methods are described in PCT Publication WO 93/06121 (incorporated herein by reference).

Another chemical synthesis method involves the synthesis of arrays of peptides (or peptidomimetics) on a surface in a manner that places each distinct library member (*e.g.*, unique peptide sequence) at a discrete, predefined location in the array. The identity of each library member is determined by its spatial location in the array. The locations in the array where binding interactions between a predetermined molecule (*e.g.*, a receptor) and reactive library members occur is determined, thereby identifying the sequences of the reactive library members on the basis of spatial location. These methods are described in U.S. Patent 5,143,854; PCT Publications WO 90/15070 and WO 92/10092; Fodor et al., 1991, Science, 251: 767; and Dower et al., 1991, Ann. Rep. Med. Chem., 26: 271 (all of which are incorporated herein by reference).

Other systems for generating libraries of polypeptides or nucleotides involve the use of cell-free enzymatic machinery for the *in vitro* synthesis of the library members. In one method, RNA molecules are selected by alternate rounds of selection against a target ligand and PCR amplification (Tuerk et al., 1990, Science, 249: 505; and Ellington et al., 1990, Nature, 346: 818; both of which are incorporated herein by reference). A similar technique may be used to identify DNA sequences which bind a predetermined human transcription factor (Thiesen et al., 1990, Nucleic Acids Res., 18: 3203; Beaudry et al., 1992 Science, 257:635; and PCT Publications WO 92/05258 and WO 92/14843; all of which are incorporated herein by reference). In a similar way, *in vitro* translation may be used to synthesize polypeptides as a method for generating large libraries. These methods which generally comprise stabilized polysome complexes, are described further in PCT Publications WO 88/08453, WO 90/05785, WO 90/07003, WO 91/02076, WO 91/05058, and WO 92/02536 (all of which are incorporated herein by reference). Alternative display systems which are not phage-based, such as those disclosed in PCT Publications WO 95/22625 and WO 95/11922 (both of which are incorporated herein by reference), use the polysomes to display polypeptides for selection.

One combinatorial approach in use is based on a strategy involving the synthesis of libraries containing a different structure on each particle of the solid phase support, interaction of the library with a soluble test substance, identification of the "bead" which interacts with the test substance, and determination of the structure carried by the identified "bead" (Lam et al., 1991, Nature, 354:82; incorporated herein by reference). An alternative to this approach is the sequential release of defined aliquots of the structures from the solid support, with subsequent determination of activity in solution, identification of the particle from which the active structure was released, and elucidation of its structure by direct sequencing (Salmon et al., 1993, Proc. Natl. Acad. Sci., USA, 90:11708; incorporated herein by reference) and/or by reading its code (Kerr et al., 1993, J. Am. Chem. Soc., 115:2529; Nikolaiev et al., 1993, Pept. Res., 6:161; and Ohlmeyer et al., 1993, Proc. Natl. Acad. Sci., USA, 90:10922; all of which are incorporated herein by reference).

In some embodiments, soluble random combinatorial libraries may be synthesized using a simple principle for the generation of equimolar mixtures of peptides which was first described by Furka et al. (1988, Xth International Symposium on Medicinal Chemistry, Budapest 1988; Furka et al., 1988, 14th International Congress of Biochemistry, Prague 1988; and Furka et al., 1991, Int. J. Peptide Protein Res., 37:487; all of which are incorporated herein by reference). The construction of soluble libraries for iterative screening has been described (Houghten et al., 1991, Nature, 354:84; incorporated herein by reference). *Lam et al.* disclosed the novel and unexpectedly powerful technique of using insoluble random combinatorial libraries. Lam synthesized random combinatorial libraries on solid phase supports, so that each support had a test compound of uniform molecular structure, and screened the libraries without prior removal of the test compounds from the support by solid phase binding protocols (Lam et al., 1991, Nature, 354:82; incorporated herein by reference).

In some embodiments, special libraries called "diversity files" may be used to assess the specificity, reliability, and/or reproducibility of the new methods. Diversity files contain a large number of compounds (*e.g*., 1000 or more small molecules) representative of many classes of compounds that could potentially result in nonspecific detection in an assay. Diversity files are commercially available and/or can be assembled from individual substances that are commercially available.

The present invention provides for screening of libraries of candidate substances. Such libraries may be exposed to a test substance (*e.g*. PAK protein, PAK gene, and/or characteristic portion thereof) with or without FMRP, and the relevant assay(s), described in detail below, carried out. Such libraries may be used in the *in vitro, in cyto,* and/or *in vivo* assay(s) described in detail below.

### Protein and Peptide PAK Modulators

In some embodiments, PAK modulators may comprise proteins and/or peptides. In certain embodiments, peptides are proteins having fewer than about 100 amino acids. In some embodiments, peptides range from about 5 to about 100, from about 5 to about 50, from about 5 to about 40, from about 5 to about 35, from about 5 to about 30, from about 5 to about 25, or from about 20 to about 25 amino acids in length. In some embodiments, a peptide sequence can be based on the sequence of a protein. In some embodiments, a peptide sequence can be a random arrangement of amino acids. Peptides from panels of peptides comprising random sequences and/or sequences which have been varied consistently to provide a maximally diverse panel of peptides may be used.

Peptide inhibitors of protein kinases, such as myosin light chain kinase (MLCK) and/or CAM kinase, are well known in the art (Kemp et al., 1991, Methods in Enzymology, 201:287; Saitoh et al., 1987, J. Biol. Chem., 262:7796; Nakanishi et al., 1992, J. Biol. Chem., 267:2157; and Strauss et al., 1992, Am. J. Physiol., 262:C1437; all of which are incorporated herein by reference). Many protein kinases regulate themselves through intermolecular auto inhibition (*e.g*., Johnson et al., 1996, Cell, 85:149; and Kemp et al., 1994, Trends in Biochem. Sci., 19:440; both of which are incorporated herein by reference), in which inhibition is achieved via the interaction of amino acid sequences within the kinase which act as pseudosubstrates which block the catalytic domain of the kinase. The kinase remains inhibited until a specific Ser, Thr, and/or Tyr residue(s) located in the pseudosubstrate is phosphorylated (autophosphorylation), and/or until interaction with an effector eliminates binding of the pseudosubstrate. In the case of PAK, autophosphorylation occurs following binding of Cdc42/Rac1 and the subsequent conformation changes due to this interaction. Regions around these phosphorylation sites are regulatory domains that bind at the substrate binding site, and autophosphorylation fully opens the catalytic site. Thus, peptide modulators of PAK may comprise peptides having at least one amino acid sequence corresponding to at least one of these regulatory domains, but with the phosphorylatable residue replaced by another amino acid that is not phosphorylatable, such that the peptide acts as a "dead-end" pseudosubstrate and modulates PAK activity. Amino acids used to replace the phosphorylatable residues may be Asp, Glu, and/or Ala. Since PAK cannot phosphorylate such peptides, they act as competitive inhibitors.

In some embodiments, candidate substances may comprise FMRP proteins and/or characteristic portions thereof. Accordingly, when applied to cells at high concentrations, *i.e*., in excess of the amount of endogenous FMRP, the FMRP peptides may act as competitors and bind to endogenous PAK, thereby inhibiting binding, and/or phosphorylation of, endogenous FMRP. The skilled artisan would know how much of a competitive peptide inhibitor to add such that the peptide is added to cells and/or cell lysates "in excess."

In some embodiments, peptide modulators of PAK are synthesized based on PAK substrates, either exogenous (*e.g*. Myosin light chain kinase (MLCK), regulatory Myosin light chain (R-MLC), Myosins I heavy chain, Myosin II heavy chain, Myosin VI, Caldesmon, Desmin, Op18/stathmin, Merlin, Filamin A, LIM kinase (LIMK), Ras, Raf, Mek, p47^{phox}, BAD, caspase 3, estrogen and/or progesterone receptors, RhoGEF, GEF-H1, NET1, Gaz, phosphoglycerate mutase-B, RhoGDI, prolactin, p41^{Arc}, and/or Aurora-A), and/or intramolecular (*e.g*., autophosphorylation and/or autoinhibitory sites). In certain embodiments, peptide modulators of PAK comprise peptides that compete with PAK's natural binding partners (e.g., FMRP, Cdc42, and/or Rac1) for binding to PAK protein. In some embodiments, a peptide comprising the sequence Pro-Pro-Val-Ile-Ala-Pro-Arg-Pro-Glu-His-Thr-Lys-Ser-Val-Tyr-Thr-Arg-Ser (*i.e.* the Pro-rich PIX-interacting motif of PAK) disrupts the PIX-PAK interaction and reduces PAK autophosphorylation (Maruta et al., 2002, Methods Mol. Biol., 189:75; incorporated herein by reference). In some embodiments, peptide modulators of PAK may be non-phosphorylatable analogues and/or pseudosubstrates of PAK substrates.

In some embodiments, protein modulators of PAK comprise peptide mimetics. As used herein, the term "peptide mimetics" refers to structures which substitute for peptides in interactions with natural binding partners, receptors, and/or enzymes. The mimetic may possess affinity, efficacy, and/or substrate function. In certain embodiments, a peptide mimetic exhibits function(s) of a particular peptide, without restriction of structure. Peptide mimetics may include amino acid residues and/or other chemical moieties which provide the desired functional characteristics. Peptide mimetics of the present invention include, but are not limited to, analogs of structural motifs such as the PAK autophosphorylation site, the substrate binding site(s) of PAK, the binding site(s) of PAK's natural binding partners, and/or the FMRP binding site of PAK.

In specific embodiments, protein modulators of PAK comprise a FMRP protein, including mutants described herein, a peptide mimetic, a substance that binds to an FMRP protein, and/or characteristic portions thereof. In some embodiments, a PAK modulator comprises a PAK protein, including mutants described herein, a peptide mimetic, and/or an agent that binds to a PAK protein. Such agents can be identified by, for example, direct binding assays, such as two-hybrid assays, GST pulldowns, *etc.* described in detail below.

Inventive protein modulators of PAK (or characteristic portions thereof) may be produced, for example, by utilizing a host cell system engineered to express an inventive *PAK* modulator-encoding nucleic acid.

Any system may be used to produce protein modulators of PAK (and/or characteristic portions thereof), such as eggs, baculovirus, plant cells, fungal cells (*e.g*. yeast cells), insect cells (*e.g.* SFM cells, Sf21 cells, Sf9 cells, Schneider cells, S2 cells, *etc*.), avian cells (*e.g*. chicken embryo fibroblasts, *etc*.), mammalian cells (*e.g.* Chinese hamster ovary (CHO) cells, HeLa cells, Madin-Darby canine kidney (MDCK) cells, baby hamster kidney (BHK cells), NS0 cells, MCF-7 cells, MDA-MB-438 cells, U87 cells, A172 cells, HL60 cells, A549 cells, SP10 cells, DOX cells, DG44 cells, HEK 293 cells, SHSY5Y, Jurkat cells, BCP-1 cells, COS cells, Vero cells (African green monkey kidney), GH3 cells, 9L cells, 3T3 cells, MC3T3 cells, C3H-10T1/2 cells, NIH-3T3 cells, C6/36 cells, *etc.*), and/or hybrid cells. Alternatively or additionally, protein modulators of PAK (and/or characteristic portions thereof) may be expressed in cells using recombinant techniques, such as through the use of an expression vector (Sambrook et al., Molecular Cloning: A Laboratory Manual, 3rd ed., Cold Spring Harbor Laboratory Press, Cold Spring Harbor, N.Y., 2001; incorporated herein by reference).

In some embodiments, protein modulators of PAK (and/or characteristic portions thereof) may be produced in the context of intact virus, whether otherwise wild type, attenuated, killed, *etc.* In some embodiments, protein modulators of PAK (and/or characteristic portions thereof) may be produced in the context of virus like particles. For example, virus may be grown in eggs, such as embryonated hen eggs, in which case the harvested material is typically allantoic fluid. Alternatively or additionally, virus may be derived from any method using tissue culture to grow the virus. Suitable cell substrates for growing virus include, for example, dog kidney cells such as MDCK and/or cells from a clone of MDCK, MDCK-like cells, monkey kidney cells such as AGMK cells including Vero cells, cultured epithelial cells as continuous cell lines, 293T cells, BK-21 cells, CV-1 cells, and/or any other cell type suitable for the production of virus (*e.g*., cells available from ATCC, Rockville, Md.). Suitable cell substrates include human cells, such as MRC-5 cells. Suitable cell substrates are not limited to cell lines; for example primary cells such as chicken embryo fibroblasts are included.

Alternatively or additionally, protein modulators of PAK in accordance with the present invention may be readily prepared by standard, well-established techniques, such as solid-phase peptide synthesis (SPPS) as described by Stewart et al. (Solid Phase Peptide Synthesis, 2nd ed., Pierce Chemical Company, Rockford, IL, 1984); and as described by Bodanszky et al. (The Practice of Peptide Synthesis, Springer-Verlag, New York, NY, 1984).

It will be appreciated, of course, that protein modulators of PAK may incorporate amino acid residues which are modified without affecting activity. For example, the termini may be derivatized to include blocking groups, *i.e*. chemical substituents suitable to protect and/or stabilize the N- and/or C-termini from "undesirable degradation," a term meant to encompass any type of enzymatic, chemical, and/or biochemical breakdown of the protein at its termini which is likely to affect the function of the protein, *i.e*. sequential degradation of the compound at a terminal end thereof.

Blocking groups include protecting groups conventionally used in the art of protein chemistry which will not adversely affect the *in vivo* activities of the protein. For example, suitable N-terminal blocking groups may be introduced by alkylation and/or acylation of the N-terminus. Examples of suitable N-terminal blocking groups include C₁ -C₅ branched and/or unbranched alkyl groups, acyl groups such as formyl and/or acetyl groups, as well as substituted forms thereof, such as the acetamidomethyl (Acm) group. Desamino analogs of amino acids are useful N-terminal blocking groups may be coupled to the N-terminus of the protein and/or used in place of the N-terminal reside. Suitable C-terminal blocking groups, in which the carboxyl group of the C-terminus may or may not be incorporated, include esters, ketones and/or amides. Ester and/or ketone-forming alkyl groups (*e.g*. lower alkyl groups, such as methyl, ethyl and/or propyl) and/or amide-forming amino groups (*e.g*. primary amines (NH₂), and/or mono- and/or di-alkylamino groups (*e.g*. methylamino, ethylamino, dimethylamino, diethylamino, methylethylanino, *etc*.) are examples of C-terminal blocking groups. Descarboxylated amino acid analogues such as agmatine are useful C-terminal blocking groups and may be either coupled to the protein's C-terminal residue and/or used in place of it. Further, it will be appreciated that the free amino and/or carboxyl groups at the termini may be removed altogether from the protein to yield desamino and/or descarboxylated forms thereof without adversely affecting protein activity.

Other modifications may be incorporated without adversely affecting the activity and these include, but are not limited to, substitution of one or more of the amino acids in the natural L-isomeric form with amino acids in the D-isomeric form. Thus, a protein may include one or more D-amino acid resides, and/or may comprise amino acids which are all in the D-form. Retro-inverso forms of proteins in accordance with the present invention are contemplated, for example, inverted proteins in which all amino acids are substituted with D-amino acid forms.

To ensure that a protein obtained from either chemical and/or biological synthetic techniques is the desired protein, analysis of the protein should be conducted. Such amino acid composition analysis may be conducted using high-resolution mass spectrometry to determine the molecular weight of the protein. Alternatively or additionally, the amino acid content of the protein may be confirmed by hydrolyzing the protein in aqueous acid and separating, identifying, and/or quantifying the components of the mixture using HPLC and/or an amino acid analyzer. Protein sequenators, which sequentially degrade the protein and identify the amino acids in order, may be used to determine definitely the sequence of the protein. Prior to its use, the protein is usually purified to remove contaminants. In this regard, it will be appreciated that the protein is often purified so as to meet the standards set out by the appropriate regulatory agencies. Any one of a number of a conventional purification procedures may be used to attain the required level of purity including, for example, reversed-phase high-pressure liquid chromatography (HPLC) using an alkylated silica column such as C₄-, C₈- and/or C₁₈- silica. A gradient mobile phase of increasing organic content is generally used to achieve purification, for example, acetonitrile in an aqueous buffer, usually containing a small amount of trifluoroacetic acid. Ion-exchange chromatography may be used to separate proteins based on charge.

Acid addition salts of peptides are contemplated in accordance with the present invention. Thus, a protein in accordance with the present invention treated with an inorganic acid (*e.g*. hydrochloric, hydrobromic, sulfuric, nitric, phosphoric, *etc.)* and/or an organic acid (*e.g*. acetic, propionic, glycolic, pyruvic, oxalic, malic, malonic, succinic, maleic, fumaric, tataric, citric, benzoic, cinnamic, mandelic, methanesulfonic, ethanesulfonic, p-toluenesulfonic, salicyclic *etc*.) to provide a water-soluble salt of the protein, is suitable for use in the invention.

The present invention provides for sequence variants of protein modulators of PAK. Variants may differ from naturally occurring proteins by conservative amino acid sequence differences and/or by modifications which do not affect sequence. For example, conservative amino acid changes may be made, which although they alter the primary sequence of a protein, do not normally alter its function. In some embodiments, about 1 about 2, about 3, about 4, about 5, about 10, or more than about 10 conservative amino acid changes can be made without any adverse effect on protein function. In some embodiments, variants of protein modulators of PAK are approximately 60% identical, approximately 70% identical, approximately 80% identical, approximately 90% identical, approximately 95% identical, or greater than approximately 95% identical to a given protein modulator of PAK.

Modifications which do not normally alter primary amino acid sequence include *in vivo* and/or *in vitro* chemical derivatization of polypeptides, *e.g*., acetylation, and/or carboxylation. Modifications of glycosylation, *e.g*., those made by modifying the glycosylation patterns of a polypeptide during its synthesis, processing, and/or in further processing steps are contemplated, for example, by exposing the polypeptide to enzymes which affect glycosylation (*e.g*., mammalian glycosylating and/or deglycosylating enzymes). Sequences which have phosphorylated amino acid residues are contemplated (*e.g*., phosphotyrosine, phosphoserine, and/or phosphothreonine). In some embodiments, useful modifications include, *e.g*., terminal acetylation, amidation, lipidation, phosphorylation, glycosylation, acylation, farnesylation, sulfation, *etc.*

Proteins and/or characteristic portions thereof which have been modified using ordinary molecular biological techniques so as to improve their resistance to proteolytic degradation and/or to optimize solubility properties and/or to render them more suitable as a therapeutic agent are contemplated. Homologs of such proteins include those containing residues other than naturally occurring L-amino acids, *e.g*., D-amino acids and/or non-naturally occurring synthetic amino acids. Proteins of the invention are not limited to products of any of the specific exemplary processes listed herein.

Substantially pure protein obtained as described herein may be purified by following known procedures for protein purification, wherein an immunological, enzymatic, and/or other assay is used to monitor purification at each stage in the procedure. Protein purification methods are well known in the art, and are described, for example in Deutscher et al. (ed., Guide to Protein Purification, Harcourt Brace Jovanovich, San Diego, CA, 1990).

In some embodiments, PAK modulators comprise antibodies (for example, monoclonal and/or polyclonal antibodies, single chain antibodies, chimeric antibodies, and/or CDR-grafted antibodies) which bind to PAK (*i.e*., an anti-PAK antibody), a PAK modulator, a natural binding partner of PAK (*e.g*. FMRP), and/or an element upstream and/or downstream of PAK to inhibit and/or activate an interaction between PAK and/or a PAK modulator, natural binding partner, upstream element, and/or downstream element.

Antibodies generated in accordance with the present invention may include, but are not limited to, polyclonal, monoclonal, chimeric (*i.e*. "humanized"), and/or single chain (*e.g*. recombinant) antibodies. Antibodies of the present invention may include antibodies with reduced effector functions and/or bispecific molecules. Antibodies of the present invention may include Fab fragments and/or fragments produced by a Fab expression library. In the production of antibodies, screening for the desired antibody may be accomplished by techniques known in the art, *e.g*., ELISA (enzyme-linked immunosorbent assay).

Antibodies directed against proteins may be generated using methods that are well known in the art. U.S. Patent 5,436,157 (incorporated herein by reference) discloses methods of raising antibodies to peptides. For the production of antibodies, various host animals, including but not limited to rabbits, mice, rats, sheep, goats, chickens, chicken eggs, and/or guinea pigs, can be immunized by injection with a polypeptide or peptide fragment thereof. To increase the immunological response, various adjuvants may be used depending on the host species, including but not limited to oil-emulsion and emulsifier-based adjuvants (*e.g*., complete Freund's Adjuvant, incomplete Freund's adjuvant, MF59 [Novartis], SAF, *etc*.), gel-type adjuvants (*e.g*., aluminum hydroxide, aluminum phosphate, calcium phosphate, *etc*.), microbial adjuvants (*e.g*., immunomodulatory DNA sequences that include CpG motifs; endotoxins such as monophosphoryl lipid A; exotoxins such as cholera toxin, *E. coli* heat labile toxin, and pertussis toxin; muramyl dipeptide, BCG [bacille Calmette-Guerin], corynebacterium, *etc*.), particulate adjuvants (*e.g*., liposomes, biodegradable microspheres, saponins, *etc*.), synthetic adjuvants (*e.g*., nonionic block copolymers, muramyl peptide analogues, polyphosphazene, synthetic polynucleotides, *etc*.), polymers (*e.g*. polyphosphazenes, described in U.S. Patent 5,500,161, incorporated herein by reference; pluronic polyols; polyanions; *etc*.), QS21, squalene, tetrachlorodecaoxide, lysolecithin, peptides, keyhole limpet hemocyanins, dinitrophenol, *etc*., and/or combinations thereof.

The generation of polyclonal antibodies is accomplished by inoculating the desired animal with the antigen and/or isolating antibodies which specifically bind the antigen therefrom.

For the preparation of monoclonal antibodies, any technique which provides for the production of antibody molecules by continuous cell lines in culture may be utilized. For example, the hybridoma technique (Kohler et al., 1975, Nature, 256:495; incorporated herein by reference), the trioma technique, the human B-cell hybridoma technique (Kozbor et al., 1983, Immunology Today, 4:72; incorporated herein by reference), and/or the EBV-hybridoma technique (Cole et al., 1985, in Monoclonal Antibodies and Cancer Therapy, Alan R. Liss, Inc.; incorporated herein by reference) may be employed to produce monoclonal antibodies. In some embodiments, monoclonal antibodies are produced in germ-free animals utilizing the technology described in PCT Publication WO 90/13678 (incorporated herein by reference).

A nucleic acid encoding a monoclonal antibody obtained using the procedures described herein may be cloned and/or sequenced using technology which is available in the art (see, *e.g.*, Wright et al., 1992, Critical Rev. Immunol., 12:125 and references therein; incorporated herein by reference).

The term "antibody" refers to any immunoglobulin, whether natural or wholly or partially synthetically produced and to derivatives thereof and characteristic portions thereof. An antibody may be monoclonal or polyclonal. An antibody may be a member of any immunoglobulin class, including any of the human classes: IgG, IgM, IgA, IgD, and IgE.

As used herein, an antibody fragment (*i.e*. characteristic portion of an antibody) refers to any derivative of an antibody which is less than full-length. In general, an antibody fragment retains at least a significant portion of the full-length antibody's specific binding ability. Examples of antibody fragments include, but are not limited to, Fab, Fab', F(ab')2, scFv, Fv, dsFv diabody, and Fd fragments.

An antibody fragment may be produced by any means. For example, an antibody fragment may be enzymatically or chemically produced by fragmentation of an intact antibody and/or it may be recombinantly produced from a gene encoding the partial antibody sequence. Alternatively or additionally, an antibody fragment may be wholly or partially synthetically produced. An antibody fragment may optionally comprise a single chain antibody fragment. Alternatively or additionally, an antibody fragment may comprise multiple chains which are linked together, for example, by disulfide linkages. An antibody fragment may optionally comprise a multimolecular complex. A functional antibody fragment will typically comprise at least about 50 amino acids and more typically will comprise at least about 200 amino acids.

In some embodiments, antibodies may include chimeric (*e.g*. "humanized") and single chain (recombinant) antibodies. In some embodiments, antibodies may have reduced effector functions and/or bispecific molecules. In some embodiments, antibodies may include fragments produced by a Fab expression library.

Single-chain Fvs (scFvs) are recombinant antibody fragments consisting of only the variable light chain (VL) and variable heavy chain (VH) covalently connected to one another by a polypeptide linker. Either VL or VH may comprise the NH2-terminal domain. The polypeptide linker may be of variable length and composition so long as the two variable domains are bridged without significant steric interference. Typically, linkers primarily comprise stretches of glycine and serine residues with some glutamic acid or lysine residues interspersed for solubility.

Diabodies are dimeric scFvs. Diabodies typically have shorter peptide linkers than most scFvs, and they often show a preference for associating as dimers.

An Fv fragment is an antibody fragment which consists of one VH and one VL domain held together by noncovalent interactions. The term "dsFv" as used herein refers to an Fv with an engineered intermolecular disulfide bond to stabilize the VH-VL pair.

A F(ab')2 fragment is an antibody fragment essentially equivalent to that obtained from immunoglobulins by digestion with an enzyme pepsin at pH 4.0-4.5. The fragment may be recombinantly produced.

A Fab' fragment is an antibody fragment essentially equivalent to that obtained by reduction of the disulfide bridge or bridges joining the two heavy chain pieces in the F(ab')2 fragment. The Fab' fragment may be recombinantly produced.

A Fab fragment is an antibody fragment essentially equivalent to that obtained by digestion of immunoglobulins with an enzyme (*e.g*. papain). The Fab fragment may be recombinantly produced. The heavy chain segment of the Fab fragment is the Fd piece.

An antibody and/or characteristic portion thereof produced in a non-human subject may be recognized to varying degrees as foreign when the antibody is administered to a human subject and an immune response against the antibody may be generated in the subject. One approach for minimizing and/or eliminating this problem is to produce chimeric and/or humanized antibody derivatives, i.e., antibody molecules comprising portions which are derived from non-human antibodies and/or portions which are derived from human antibodies. Chimeric antibody molecules may include, for example, the variable region from an antibody of a mouse, rat, and/or other species, with human constant regions. A variety of approaches for making chimeric antibodies have been described (Morrison et al., 1985, Proc. Natl. Acad. Sci., USA, 81:685 1; Takeda et al., 1985, Nature, 314:452; U. S. Patents 4,816,397 and 4,816,567; European publications EP 0173494 and EP 171496; and United Kingdom patent GB 2177096B; all of which are incorporated herein by reference). In some embodiments, humanized antibodies have only the hypervariable domains of the variable region of non-human origin and have other parts of the variable region of the antibody, especially the conserved framework regions of the antigen-binding domain, of human origin. Such humanized antibodies may be made by any of several techniques known in the art, (*e.g*., Teng et al., 1983, Proc. Natl. Acad. Sci., USA, 80:7308; Kozbor et al., 1983, Immunology Today, 4:727; and Olsson et al., 1982, Meth. Enzymol., 92:3; all of which are incorporated herein by reference), and may be made according to the teachings of PCT Publication WO 92/06193 and/or European Publication EP 0239400 (both of which are incorporated herein by reference). Humanized antibodies may be commercially produced by, for example, Scotgen Limited (Twickenham, Great Britain); Antitope (Cambridge, United Kingdom); Fusion Antibodies (Northern Ireland); and Genentech (South San Francisco, CA).

In some embodiments, techniques described for the production of single-chain (e.g. recombinant) antibodies (U.S. Patent 4,946,778; incorporated herein by reference) are adapted to produce protein-specific single-chain antibodies. In some embodiments, techniques described for the construction of Fab expression libraries (*e.g.* Huse et al., 1989, Science, 246:1275; incorporated herein by reference) are utilized to allow rapid and/or easy identification of monoclonal Fab fragments possessing a desired specificity.

Antibody fragments which contain the idiotype of the antibody molecule can be generated by known techniques. For example, such fragments include but are not limited to a F(ab')₂ fragment which can be produced by pepsin digestion of the antibody molecule; Fab' fragments which can be generated by reducing the disulfide bridges of the F(ab')₂ fragment; Fab fragments which can be generated by treating the antibody molecule with papain and/or a reducing agent; and/or Fv fragments.

To generate a phage antibody library, a cDNA library is first obtained from mRNA which is isolated from cells (*e.g*., hybridomas) which express a desired protein (*e.g*., a desired antibody) to be expressed on the phage surface. cDNA copies of the mRNA are produced using reverse transcriptase. cDNA which specifies immunoglobulin fragments are obtained by PCR and the resulting DNA is cloned into a suitable bacteriophage vector to generate a bacteriophage DNA library comprising DNA specifying immunoglobulin genes. Procedures for making a bacteriophage library comprising heterologous DNA are well known in the art and are described in Sambrook et al. (Molecular Cloning: A Laboratory Manual, 3rd ed., Cold Spring Harbor Laboratory Press, Cold Spring Harbor, N.Y., 2001; incorporated herein by reference).

Bacteriophage encoding a desired antibody may be engineered such that the antibody is displayed on the surface thereof in such a manner that it is available for binding to its corresponding binding protein (*e.g*., the antigen against which the antibody is directed). Thus, when bacteriophage which express a specific antibody are incubated in the presence of a cell which expresses the corresponding antigen, the bacteriophage will bind to the cell. Bacteriophage which do not express the antibody will not bind to the cell. Such panning techniques are well known in the art.

Processes such as those described above, have been developed for the production of human antibodies using M13 bacteriophage display (Burton et al., 1994, Adv. Immunol., 57:191; incorporated herein by reference). Essentially, a cDNA library is generated from mRNA obtained from a population of antibody-producing cells. The mRNA encodes rearranged immunoglobulin genes and thus, the cDNA encodes the same. Amplified cDNA is cloned into M13 expression vectors creating a library of phage which express human Fab fragments on their surface. Phage which display the antibody of interest are selected by antigen binding and are propagated in bacteria to produce soluble human Fab immunoglobulin. Thus, in contrast to conventional monoclonal antibody synthesis, this procedure immortalizes DNA encoding human immunoglobulin rather than cells which express human immunoglobulin.

The procedures just presented describe the generation of phage which encode the Fab portion of an antibody molecule. However, the invention should not be construed to be limited solely to the generation of phage encoding Fab antibodies. Rather, phage which encode single chain antibodies (scFv/phage antibody libraries) are included in the invention. Fab molecules comprise the entire Ig light chain, i.e., they comprise the variable and constant region of the light chain, but include only the variable region and/or first constant region domain of the heavy chain. Single chain antibody molecules comprise a single chain of protein comprising the Ig Fv fragment. An Ig Fv fragment includes only the variable regions of the heavy and light chains of the antibody, having no constant region contained therein. Phage libraries comprising scFv DNA may be generated following the procedures described in Marks et al. (1991, J. Mol. Biol., 222:581; incorporated herein by reference). Panning of phage so generated for the isolation of a desired antibody is conducted in a manner similar to that described for phage libraries comprising Fab DNA.

The invention should be construed to include, for example, synthetic phage display libraries in which the heavy and light chain variable regions may be synthesized such that they include nearly all possible specificities (Barbas, 1995, Nature Med., 1:837; and de Kruifet et al., 1995, J. Mol. Biol., 248:97; both of which are incorporated herein by reference).

For use therapeutically, antibody preparations may be unable to fix complement and/or induce other effector functions. Complement fixation may be prevented by deletion of the Fc portion of the antibody, by using an antibody isotype which is not capable of fixing complement, and/or by using a complement fixing antibody in conjunction with a drug which inhibits complement fixation. Alternatively or additionally, amino acid residues within the Fc region which are involved in activating complement (see *e.g*., Tan et al., 1990, Proc. Natl. Acad. Sci., USA, 87:162; and Duncan et al., 1988, Nature, 332:738; both of which are incorporated herein by reference) may be mutated to reduce and/or eliminate the complement-activating ability of an intact antibody. Likewise, amino acids residues within the Fc region which are involved in binding of the Fc region to Fc receptors (see *e.g*., Canfield et al., 1991, J. Exp. Med., 173:1483; and Lund et al., 1991, J. Immunol., 147:2657; both of which are incorporated herein by reference) may be mutated to reduce and/or eliminate Fc receptor binding if an intact antibody is to be used.

In some embodiments, antibodies may be fragmented using conventional techniques and the fragments screened for utility in the same manner as for whole antibodies. For example, F(ab')₂ fragments can be generated by treating antibody with pepsin. The resulting F(ab')₂ fragment can be treated to reduce disulfide bridges to produce Fab' fragments.

### Nucleic Acid PAK Modulators

Nucleic acid modulators of PAK useful in the present invention include, but are not limited to, RNAi-inducing agents, for example, small interfering RNAs ("siRNAs"), short hairpin RNAs ("sbRNAs"), *etc*.; antisense DNAs and/or RNAs; ribozymes; DNA for gene therapy; viral fragments, including viral DNA and/or RNA; DNA and/or RNA chimeras; mRNA; plasmids; cosmids; genomic DNA; cDNA; gene fragments; various structural forms of DNA including single-stranded DNA, double-stranded DNA, supercoiled DNA and/or triple-helical DNA; Z-DNA; *etc.*

In certain embodiments, the present invention provides nucleic acids which encode PAK modulator proteins and/or characteristic portions thereof. In some embodiments, the invention provides nucleic acids which are complementary to nucleic acids which encode PAK modulator protein and/or characteristic portions thereof.

In some embodiments, PAK modulators comprise nucleic acids, including but not limited to PAK-specific RNAi-inducing agents, antisense nucleic acids, and/or ribozymes.

In some embodiments, the invention provides nucleic acids which hybridize to nucleic acids encoding PAK and/or PAK modulators and/or characteristic portions thereof. Such nucleic acids may be used, for example, as primers and/or as probes. To give but a few examples, such nucleic acids may be used as primers in polymerase chain reaction (PCR), as probes for hybridization (including *in situ* hybridization), and/or as primers for reverse transcription-PCR (RT-PCR).

RNA interference (RNAi) is an evolutionarily conserved process in which presence of an at least partly double-stranded RNA molecule in a eukaryotic cell leads to sequence-specific inhibition of gene expression. RNAi was originally described as a phenomenon in which the introduction of long dsRNA (typically hundreds of nucleotides) into a cell results in degradation of mRNA containing a region complementary to one strand of the dsRNA (U.S. Patent 6,506,559; and Fire et al., 1998, Nature, 391:806; both of which are incorporated herein by reference). Subsequent studies in *D. melanogaster* showed that long dsRNAs are processed by an intracellular RNase III-like enzyme called Dicer into smaller dsRNAs primarily comprised of two approximately 21 nucleotide (nt) strands that form a 19 base pair duplex with 2 nt 3' overhangs at each end and 5'-phosphate and 3'-hydroxyl groups (see, *e.g*., PCT Publication WO 01/75164; U.S. Patent Application Publications 2002/0086356 and 2003/0108923; Zamore et al., 2000, Cell, 101:25; and Elbashir et al., 2001, Genes Dev., 15:188; all of which are incorporated herein by reference).

Short dsRNAs having structures such as this, referred to as siRNAs, silence expression of genes that include a region that is substantially complementary to one of the two strands. This strand is referred to as the "antisense" or "guide" strand, with the other strand often being referred to as the "sense" strand. The siRNA is incorporated into a ribonucleoprotein complex termed the RNA-induced silencing complex (RISC) that contains member(s) of the Argonaute protein family. Following association of the siRNA with RISC, a helicase activity unwinds the duplex, allowing an alternative duplex to form the guide strand and a target mRNA containing a portion substantially complementary to the guide strand. An endonuclease activity associated with the Argonaute protein(s) present in RISC is responsible for "slicing" the target mRNA, which is then further degraded by cellular machinery.

Considerable progress towards the practical application of RNAi was achieved with the discovery that exogenous introduction of siRNAs into mammalian cells can effectively reduce the expression of target genes in a sequence-specific manner via the mechanism described above. A typical siRNA structure includes an approximately 19 nucleotide double-stranded portion, comprising a guide strand and an antisense strand. Each strand has a 2 nt 3' overhang. Typically the guide strand of the siRNA is perfectly complementary to its target gene and mRNA transcript over at least 17 - 19 contiguous nucleotides, and typically the two strands of the siRNA are perfectly complementary to each other over the duplex portion. However, as will be appreciated by one of ordinary skill in the art, perfect complementarity is not required. Instead, one or more mismatches in the duplex formed by the guide strand and the target mRNA is often tolerated, particularly at certain positions, without reducing the silencing activity below useful levels. For example, there may be 1, 2, 3, or even more mismatches between the target mRNA and the guide strand (disregarding the overhangs). Thus, as used herein, two nucleic acid portions such as a guide strand (disregarding overhangs) and a portion of a target mRNA that are "substantially complementary" may be perfectly complementary (*i.e*., they hybridize to one another to form a duplex in which each nucleotide is a member of a complementary base pair) or they may have a lesser degree of complementarity sufficient for hybridization to occur. One of ordinary skill in the art will appreciate that the two strands of the siRNA duplex need not be perfectly complementary. In some embodiments, at least 80%, at least 90%, or more of the nucleotides in the guide strand of an effective siRNA are complementary to the target mRNA over at least about 19 contiguous nucleotides. The effect of mismatches on silencing efficacy and the locations at which mismatches may most readily be tolerated are areas of active study (see, *e.g*., Reynolds et al., 2004, Nat. Biotechnol., 22:326; incorporated herein by reference).

It will be appreciated that molecules having the appropriate structure and degree of complementarity to a target gene will exhibit a range of different silencing efficiencies. A variety of additional design criteria have been developed to assist in the selection of effective siRNA sequences. Numerous software programs that can be used to choose siRNA sequences that are predicted to be particularly effective to silence a target gene of choice are available (see, *e.g*., Yuan et al., 2004, Nucl. Acids. Res., 32:W130; and Santoyo et al., 2005, Bioinformatics, 21:1376; both of which are incorporated herein by reference).

As will be appreciated by one of ordinary skill in the art, RNAi may be effectively mediated by RNA molecules having a variety of structures that differ in one or more respects from that described above. For example, the length of the duplex can be varied (*e.g*., from about 17 - 29 nucleotides); the overhangs need not be present and, if present, their length and the identity of the nucleotides in the overhangs can vary (though most commonly symmetric dTdT overhangs are employed in synthetic siRNAs).

Additional structures, referred to as short hairpin RNAs (shRNAs), are capable of mediating RNA interference. An shRNA is a single RNA strand that contains two complementary regions that hybridize to one another to form a double-stranded "stem," with the two complementary regions being connected by a single-stranded loop. shRNAs are processed intracellularly by Dicer to form an siRNA structure containing a guide strand and an antisense strand. While shRNAs can be delivered exogenously to cells, more typically intracellular synthesis of shRNA is achieved by introducing a plasmid or vector containing a promoter operably linked to a template for transcription of the shRNA into the cell, *e.g*., to create a stable cell line or transgenic organism.

While sequence-specific cleavage of target mRNA is currently the most widely used means of achieving gene silencing by exogenous delivery of RNAi-inducing entities to cells, additional mechanisms of sequence-specific silencing mediated by short RNA entities are known. For example, post-transcriptional gene silencing mediated by RNAi-inducing entities can occur by mechanisms involving translational repression. Certain endogenously expressed RNA molecules form hairpin structures containing an imperfect duplex portion in which the duplex is interrupted by one or more mismatches and/or bulges. These hairpin structures are processed intracellularly to yield single-stranded RNA species referred to as known as microRNAs (miRNAs), which mediate translational repression of a target transcript to which they hybridize with less than perfect complementarity. siRNA-like molecules designed to mimic the structure of miRNA precursors have been shown to result in translational repression of target genes when administered to mammalian cells.

Thus the exact mechanism by which an RNAi-inducing entity inhibits gene expression appears to depend, at least in part, on the structure of the duplex portion of the RNAi-inducing entity and/or the structure of the hybrid formed by one strand of the RNAi-inducing entity and a target transcript, RNAi mechanisms and the structures of various RNA molecules known to mediate RNAi, *e.g*., siRNA, shRNA, miRNA and their precursors, have been extensively reviewed (see, *e.g*., Dykxhhorn et al., 2003, Nat. Rev. Mol. Cell Biol., 4:457; Hannon et al., 2004, Nature, 431:3761; and Meister et al., 2004, Nature, 431:343; all of which are incorporated herein by reference). It is to be expected that future developments will reveal additional mechanisms by which RNAi may be achieved and will reveal additional effective short RNAi-inducing entities. Any currently known or subsequently discovered RNAi-inducing entities are within the scope of the present invention.

An RNAi-inducing entity that is delivered according to the methods of the invention and/or is present in a composition of the invention may be designed to silence any eukaryotic gene. The gene can be a mammalian gene, *e.g*., a human gene. The gene can be a wild type gene, a mutant gene, an allele of a polymorphic gene, *etc.* In certain embodiments, a PAK modulator is an RNAi-inducing entity that targets PAK expression. The following sequences may be used to design RNAi-inducing entities that target PAK expression in accordance with the guidelines described herein: (GenBank Accession Numbers U24152, U24153, AF068864, AJ011855, AB040812, AF276893; and/or nucleic acids encoding the proteins of GenBank Accession Numbers AAA65441, AAA65442, AAC36097, NP_005875, CAA09820, CAC18720, BAA94194, NP_064553, AAF82800, Q9P286, BAA11844, AAC47094, AAB95646. In some embodiments, RNAi-inducing entities can be used to induce isoform-specific suppression of PAK expression. In some embodiments, RNAi-inducing entities can be used to induce suppression of all PAK isoforms.

In specific embodiments, a nucleic acid modulator of PAK comprises an antisense molecule that binds to a translational start site, transcriptional start site, and/or splice junction. Antisense approaches involve the design of oligonucleotides (*e.g*. DNA and/or RNA) that are complementary to PAK mRNA. Antisense oligonucleotides typically bind to PAK mRNA and/or prevent translation. Alternatively or additionally, an antisense oligonucleotide may bind to DNA of a PAK gene, such as, for example, a regulatory element. An antisense oligonucleotide may or may not exhibit absolute complementarity. As used herein, a sequence "complementary" to a portion of a nucleic acid refers to a sequence having sufficient complementarity to be able to hybridize with the RNA and/or form a stable duplex. In the case of double-stranded antisense nucleic acids, a single strand of the duplex DNA may thus be tested and/or triplex formation may be assayed. The ability to hybridize will depend on the degree of complementarity and/or the length of the antisense nucleic acids. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

In some embodiments, oligonucleotides that are complementary to the 5' end of PAK mRNA (*e.g*., the 5' untranslated sequence up to and including the AUG initiation codon) may inhibit translation. In some embodiments, oligonucleotides that are complementary to the 3' untranslated sequences of PAK mRNA may inhibit translation (Wagner, 1994, Nature, 372:333; incorporated herein by reference). Therefore, oligonucleotides complementary to the 5' and/or 3' untranslated, non-coding regions of a PAK gene may be used in an antisense approach to inhibit translation of endogenous PAK mRNA. Oligonucleotides complementary to the 5' untranslated region of the mRNA may include the complement of the AUG start codon. Antisense oligonucleotides complementary to mRNA coding regions are generally less efficient inhibitors of translation but may be used in accordance with the invention. Whether designed to hybridize to any of the afore-mentioned regions of a PAK nucleic acid, an antisense nucleic acid of the present invention comprises at least about 6 nucleotides and may comprise about 6 to about 50 nucleotides. In certain embodiments, an antisense nucleic acid comprises least about 10 nucleotides, at least about 17 nucleotides, at least about 25 nucleotides, or at least about 50 nucleotides.

In some embodiments, ribozyme molecules designed to catalytically cleave PAK mRNA transcripts may be used to prevent translation of PAK mRNA and/or expression of PAK (see, *e.g*., PCT Publication WO 90/11364; and Sarver et al., 1990, Science, 247:1222; both of which are incorporated herein by reference). Ribozymes of the present invention may cleave mRNA at site specific recognition sequences in order to destroy PAK mRNAs. Alternatively, ribozymes of the present invention may comprise hammerhead ribozymes, which cleave mRNAs at locations dictated by flanking regions that form complementary base pairs with the target mRNA. When hammerhead ribozymes are employed, the target mRNA may have the following sequence of two bases: 5'-UG-3'. The construction and/or production of hammerhead ribozymes is well known in the art and is described more fully in Haseloff et al. (1988, Nature, 334:585; incorporated herein by reference). There are numerous potential hammerhead ribozyme cleavage sites within the nucleotide sequence of human PAK cDNA. In some embodiments, a ribozyme is engineered so that the cleavage recognition site is located near the 5' end of the PAK mRNA; *i.e*., to increase efficiency and/or minimize the intracellular accumulation of non-functional mRNA transcripts.

In certain embodiments, ribozymes may comprise RNA endoribonucleases (hereinafter "Cech-typa ribozymes"), such as the one which occurs naturally in *Tetrahymena thermophila* (known as the IVS, and/or L-19 IVS RNA) and which has been extensively described by Thomas Cech and collaborators (Zaug et al., 1984, Science, 224:574; Zaug et al., 1986, Science, 231:470; Zaug et al., 1986,. Nature, 324:429; PCT Publication WO 88/04300; and Been et al., 1986, Cell, 47:207; all of which are incorporated herein by reference). The Cech-type ribozymes have an eight base pair active site which hybridizes to a target RNA sequence whereafter cleavage of the target RNA takes place.

Alternatively or additionally, endogenous PAK gene expression may be reduced by targeting deoxyribonucleotide sequences complementary to the regulatory region of the PAK gene (*i.e*., PAK promoter, enhances, *etc*.) to form triple helical structures that prevent transcription of the PAK gene (see generally, Helene, 1991, Anticancer Drug Des., 6:569; Helene et al., 1992, Ann, N.Y. Acad. Sci., 660:27; and Maher, 1992, Bioassays, 14:807; all of which are incorporated herein by reference).

In certain embodiments, nucleic acid molecules to be used in triple helix formation for the inhibition of transcription may be single-stranded and/or composed of deoxyribonucleotides. The base composition of these oligonucleotides may promote triple helix formation via Hoogsteen base pairing rules, which generally require sizable stretches of purines or pyrimidines to be present on one strand of a duplex. Nucleotide sequences may be pyrimidine-based, which will result in TAT and/or CGC triplets across the three associated strands of the resulting triple helix. Pyrimidine-rich molecules provide base complementarity to a purine-rich region of a single strand of the duplex in a parallel orientation to that strand. In some embodiments, nucleic acid molecules may be chosen that are purine-rich, for example, containing a stretch of G residues. These molecules will typically form a triple helix with a DNA duplex that is rich in GC pairs, in which the majority of the purine residues are located on a single stand of the targeted duplex, resulting in CGC triplets across the three strands in the triplex.

Alternatively or additionally, the potential sequences that can be targeted for triple helix formation may be increased by creating a so called "switchback" nucleic acid molecule. Switchback molecules are synthesized in an alternating 5' - 3', 3' - 5' manner, such that they base pair with first one strand of a duplex and then the other, eliminating the necessity for a sizable stretch of either purines or pyrimidines to be present on one strand of a duplex.

As discussed above, nucleic acid modulators of PAK can target PAK directly. However, it will be understood that nucleic acid modulators of PAK may target PAK directly, but instead, might target downstream effectors of PAK, upstream activators of PAK, and/or natural binding partners of PAK. For example, in certain embodiments, a PAK modulator is an RNAi-inducing entity that targets one or more of the following: Myosin light chain kinase (MLCK), regulatory Myosin light chain (R-MLC), Myosins I heavy chain, myosin II heavy chain, Myosin VI, Caldesmon, Desmin, Op18/stathmin, Merlin, Filamin A, LIM kinase (LIMK), Ras, Raf, Mek, p47^{phox}, BAD, caspase 3, estrogen and/or progesterone receptors, RhoGEF, GEF-H1, NET1, Gαz, phosphoglycerate mutase-B, RhoGDI, prolactin, p41^{Arc}, Aurora-A (Bokoch et al., 2003, Annu. Rev. Biochem., 72:743; and Hofmann et al., 2004, J. Cell Sci., 117:4343; both of which are incorporated herein by reference), CIB; sphingolipids; G-protein β and/or γ subunits; PIX/COOL; GIT/PKL; Nef; Paxillin; NESH; SH3-containing proteins (*e.g.* Nck and/or Grb2); kinases (*e.g*. Akt, PDK1, PI 3-kinase/p85, Cdk5, Cdc2, Src kinases, Abl, and/or protein kinase A (PKA)); phosphatases (*e.g*. phosphatase PP2A, POPX1, and/or POPX2); PDK1, PDK2, PI3K, and/or NMDARs.

In some embodiments, nucleic acid modulators of PAK may target FMRP. In certain embodiments, a PAK modulator is an RNAi-inducing entity that targets *FMR1* expression. The following sequences may be used to design RNAi-inducing entities that target *FMR1* expression in accordance with the guidelines described herein: GenBank Accession Numbers AF305881, L29074, U25165, U31501; and/or nucleic acids encoding the proteins of GenBank Accession Numbers AAG22045, AAB18829, AAC50155, AAC50292.

In certain embodiments, a PAK modulator is an RNAi-inducing entity, antisense oligonucleotide, ribozyme, and/or triple-helix inducing agent that targets one or more genes that have been shown to positively regulate expression of PAK and/or FMRP. For example, in certain embodiments, a PAK modulator is an RNAi-inducing entity that targets transcription factors, translation factors, RNA processing factors, protein stabilizing factors, *etc.* that are involved in positive regulation of PAK and/or FMRP expression.

In some embodiments, a candidate nucleic acid (*e.g*. RNAi-inducing agent, antisense oligonucleotide, ribozyme, and/or triple-helix inducing agent) comprises an α-anomeric oligonucleotide. An α-anomeric oligonucleotide forms specific double-stranded hybrids with complementary RNA in which, contrary to the usual β-units, the strands run parallel to each other (Gautier et al., 1987, Nuc. Acids Res., 15:6625; incorporated herein by reference). The oligonucleotide is a 2'-O-methylribonuelcotide (Inoue et al., 1987, Nuc. Acids Res., 15:6131; incorporated herein by reference) and/or a chimeric RNA-DNA analogue (Inoue et al., 1987, FEBS Lett., 215:327; incorporated herein by reference).

In some embodiments, *in vitro* studies are first performed to quantitate the ability of a candidate nucleic acid (*e.g*. RNAi-inducing agent, antisense oligonucleotide, ribozyme, and/or triple-helix inducing agent) to inhibit PAK gene expression. These studies may utilize controls that distinguish between gene inhibition due to the candidate nucleic acid and nonspecific biological effects of nucleic acids. These studies may compare levels of the target RNA and/or protein with that of an internal control RNA and/or protein. Results obtained using the candidate nucleic acid can be compared with those obtained using a control nucleic acid. In some embodiments, a control nucleic acid is of approximately the same length as the candidate nucleic acid, and/or the nucleotide sequence of the candidate nucleic acid differs from the control nucleic acid no more than is necessary to prevent specific hybridization to the target sequence.

In some embodiments, a candidate nucleic acid (*e.g*. RNAi-inducing agent, antisense oligonucleotide, ribozyme, and/or triple-helix inducing agent) may be delivered to cells which express PAK *in vivo* and/or *in vitro.* A number of methods have been developed for delivering nucleic acids to cells. For example, nucleic acids may be injected directly into the tissue site. Alternatively or additionally, nucleic acids, designed to target desired cells (*e.g*., linked to peptides and/or antibodies that specifically bind receptors and/or antigens expressed on a target cell surface), may be administered systemically.

Since it may be difficult to achieve intracellular concentrations of a candidate nucleic acid (*e.g*. RNAi-inducing agent, antisense oligonucleotide, ribozyme, and/or triple-helix inducing agent) sufficient to inhibit gene expression of PAK, a recombinant DNA construct may be used in which the candidate nucleic acid is placed under the control of a strong pol III and/or pol II promoter. The use of such a construct to transfect, for example, target cells in a patient, may result in the transcription of sufficient amounts of single-stranded RNAs that will form complementary base pairs with endogenous PAK transcripts and thereby prevent translation of PAK mRNA. For example, a vector may be introduced *in vivo* such that it is taken up by a target cell and/or directs the transcription of a candidate nucleic acid. Such a vector may remain episomal and/or become chromosomally integrated, as long as it can be transcribed to produce the desired candidate nucleic acid. Such vectors may be constructed by recombinant DNA technology methods standard in the art. Vectors may be plasmid, viral, and/or others known in the art, used for replication and/or expression in mammalian cells. Expression of the sequence encoding the candidate nucleic acid may be by any promoter known in the art to act in mammalian (*e.g*. human) neuronal cells. Such promoters may be inducible and/or constitutive. Such promoters include, but are not limited to, the SV40 early promoter region (Bernoist et al., 1981, Nature, 290:304; incorporated herein by reference), the promoter contained in the 31 long terminal repeat of Rous sarcoma virus (Yamamoto et al., 1980, Cell, 22:787; incorporated herein by reference), the herpes thymidine kinase promoter (Wagner et al., 1981, Proc. Natl. Acad. Sci., USA, 78:1441; incorporated herein by reference), the promoter and/or regulatory sequences of *FMR1* gene, *etc.* Any type of plasmid, cosmid, YAC, and/or viral vector may be used to prepare the recombinant DNA construct which can be introduced directly into a tissue site.

In some embodiments, a nucleic acid PAK modulator may comprise DNA, RNA, chimeric mixtures, derivatives, characteristic portions, and/or modified versions thereof. Nucleic acids of the present invention may be single-stranded and/or double-stranded. A nucleic acid may be modified at the base moiety, sugar moiety, and/or phosphate backbone, for example, to improve stability of the molecule, hybridization, *etc.* A nucleic acid may include other appended groups such agents facilitating transport across the cell membrane (see, *e.g*., Letsinger et al., 1989, Proc. Natl. Acad. Sci., USA, 86:6553; Lemaitre et al., 1987, Proc. Natl. Acad. Sci., USA, 84:648; and PCT Publication WO 88/09810; all of which are incorporated herein by reference).

Nucleic acid PAK modulators of the present invention (including RNAi-inducing agents, antisense oligonucleotides, ribozymes, triple-helix inducing agents, *etc.)* may be prepared according to any available technique including, but not limited to chemical synthesis, enzymatic synthesis, enzymatic or chemical cleavage of a longer precursor, *etc.* Methods of synthesizing RNAs are known in the art (see, *e.g*., Gait, M.J. (ed.) Oligonucleotide synthesis: a practical approach, Oxford [Oxfordshire], Washington, DC: IRL Press, 1984; and Herdewijn, P. (ed.) Oligonucleotide synthesis: methods and applications, Methods in molecular biology, v. 288 (Clifton, N.J.) Totowa, N.J.: Humana Press, 2005; both of which are incorporated herein by reference).

Nucleic acid modulators of PAK may comprise naturally occurring nucleosides, modified nucleosides, naturally occurring nucleosides with hydrocarbon linkers (*e.g*., an alkylene) or a polyether linker (*e.g*., a PEG linker) inserted between one or more nuclcosides, modified nucleosides with hydrocarbon or PEG linkers inserted between one or more nucleosides, or a combination of thereof. In some embodiments, nucleotides or modified nucleotides of a nucleic acid can be replaced with a hydrocarbon linker or a polyether linker provided that the binding affinity, selectivity, and/or other functional characteristics of the nucleic acid are not substantially reduced by the substitution.

It will be appreciated by those of ordinary skill in the art that nucleic acids in accordance with the present invention may comprise nucleotides entirely of the types found in naturally occurring nucleic acids, or may instead include one or more nucleotide analogs or have a structure that otherwise differs from that of a naturally occurring nucleic acid. U.S. Patents 6,403,779; 6,399,754; 6,225,460; 6,127,533; 6,031,086; 6,005,087; 5,977,089; and references therein disclose a wide variety of specific nucleotide analogs and modifications that may be used. See Crooke, S. (ed.) Antisense Drug Technology: Principles, Strategies, and Applications (1st ed), Marcel Dekker; ISBN: 0824705661; 1st edition (2001) and references therein (incorporated herein by reference). For example, 2'-modifications include halo, alkoxy and allyloxy groups. In some embodiments, the 2'-OH group is replaced by a group selected from H, OR, R, halo, SH, SR₁, NH₂, NH_{R}, NR₂ or CN, wherein R is C₁-C₆ alkyl, alkenyl, or alkynyl, and halo is F, Cl, Br or I. Examples of modified linkages include phosphorothioate and 5'-N-phosphoramidite linkages.

Nucleic acids comprising a variety of different nucleotide analogs, modified backbones, or non-naturally occurring internucleoside linkages can be utilized in accordance with the present invention. Nucleic acids of the present invention may include natural nucleosides (*i.e*., adenosine, thymidine, guanosine, cytidine, uridine, deoxyadenosine, deoxythymidine, deoxyguanosine, and deoxycytidine) or modified nucleosides. Examples of modified nucleotides include base modified nucleoside (*e.g*., aracytidine, inosine, isoguanosine, nebularine, pseudouridine, 2,6-diaminopurine, 2-aminopurine, 2-thiothymidine, 3-deaza-5-azacytidine, 2'-deoxyuridine, 3-nitorpyrrole, 4-methylindole, 4-thiouridine, 4-thiothymidine, 2-aminoadenosine, 2-thiothymidine, 2-thiouridine, 5-bromocytidine, 5-iodouridine, inosine, 6-azauridine, 6-chloropurine, 7-deazaadenosine, 7-deazaguanosine, 8-azaadenosine, 8-azidoadenosine, benzimidazole, M1-methyladenosine, pyrrolo-pyrimidine, 2-amino-6-chloropurine, 3-methyl adenosine, 5-propynylcytidine, 5-propynyluridine, 5-bromouridine, 5-fluorouridine, 5-methylcytidine, 7-deazaadenosine, 7-deazaguanosine, 8-oxoadenosine, 8-oxoguanosine, O(6)-methylguanine, and 2-thiocytidine), chemically or biologically modified bases (*e.g*., methylated bases), modified sugars (*e.g*., 2'-fluororibose, 2'-aminoribose, 2'-azidoribose, 2'-O-methylribose, L-enantiomeric nucleosides arabinose, and hexose), modified phosphate groups (*e.g*., phosphorothioates and 5'-N-phosphoramidite linkages), and combinations thereof. Natural and modified nucleotide monomers for the chemical synthesis of nucleic acids are readily available. In some cases, nucleic acids comprising such modifications display improved properties relative to nucleic acids consisting only of naturally occurring nucleotides. In some embodiments, nucleic acid modifications described herein are utilized to reduce and/or prevent digestion by nucleases (*e.g*. exonucleases, endonucleases, *etc*.). For example, the structure of a nucleic acid may be stabilized by including nucleotide analogs at the 3' end of one or both strands order to reduce digestion.

Modified nucleic acids need not be uniformly modified along the entire length of the molecule. Different nucleotide modifications and/or backbone structures may exist at various positions in the nucleic acid. One of ordinary skill in the art will appreciate that the nucleotide analogs or other modification(s) may be located at any position(s) of a nucleic acid such that the function of the nucleic acid is not substantially affected. The modified region may be at the 5 '-end and/or the 3'-end of one or both strands. For example, modified nucleic acids in which approximately 1 to approximately 5 residues at the 5' and/or 3' end of either of both strands are nucleotide analogs and/or have a backbone modification have been employed. The modification may be a 5' or 3' terminal modification. One or both nucleic acid strands may comprise at least 50% unmodified nucleotide, at least 80% unmodified nucleotides, at least 90% unmodified nucleotides, or 100% unmodified nucleotides.

Nucleic acids in accordance with the present invention may, for example, comprise a modification to a sugar, nucleoside, or internucleoside linkage such as those described in U.S. Patent Publications 2003/0175950, 2004/0192626, 2004/0092470, 2005/0020525, and 2005/0032733 (all of which are incorporated herein by reference). The present invention encompasses the use of any nucleic acid having any one or more of the modification described therein. For example, a number of terminal conjugates, *e.g*., lipids such as cholesterol, lithocholic acid, aluric acid, or long alkyl branched chains have been reported to improve cellular uptake. Analogs and modifications may be tested, *e.g*., using any appropriate assay known in the art. In some embodiments, nucleic acids in accordance with the present invention may comprise one or more non-natural nucleoside linkages. In some embodiments, one or more internal nucleotides at the 3'-end, 5'-end, or both 3'- and 5'-ends of the nucleic acid arc inverted to yield linkages such as a 3' - 3' linkage or a 5' - 5' linkage.

Nucleic acids comprising modified internucleoside linkages may be synthesized using reagents and/or methods that are well known in the art. For example, methods for synthesizing nucleic acids containing phosphonate phosphorothioate, phosphorodithioate, phosphoramidate methoxyethyl phosphoramidate, formacetal, thioformacetal, diisopropylsilyl, acetamidate, carbamate, dimethylene-sulfide (-CH₂-S-CH₂), diinethylene-sulfoxide (-CH₂-SO-CH₂), dimethylene-sulfone (-CH₂-SO₂-CH₂), 2'-O-alkyl, and/or 2'-deoxy 2'-fluoro phosphorothioate internucleoside linkages are well known in the art (Uhlmann et al., 1990, Chem. Rev., 90:543; Schneider et al., 1990, Tetrahedron Lett., 31:335; and references therein; all of which are incorporated herein by reference).

In some embodiments, a nucleic acid, may comprise phosphodiester linkages and/or modified linkages such as phosphotriester, phosphoramidate, siloxane, carbonate, carboxymethylester, acetamidate, carbamate, thioether, bridged phosphoramidate, bridged methylene phosphonate, bridged phosphoramidate, bridged phosphoramidate, bridged methylene phosphonate, phosphorothioate, methylphosphonate, phosphorodithioate, bridged phosphorothioate and/or sulfone linkages, and/or combinations of such linkages.

It will further be understood that, where a heterologous polypeptide is to be expressed in a host cell, it will often be desirable to utilize nucleic acid sequences encoding the polypeptide that have been adjusted to accommodate codon preferences of the host cell and/or to link the encoding sequences with regulatory elements active in the host cell. Nucleic acids of the present invention may be chosen for having codons, which may or may not be preferred for a particular expression system. For example, the nucleic acid may be one in which at least one codon, or in which at least 1%, at least 5%, at least 10%, or at least 20% of the codons have been altered such that the sequence is optimized for expression in the host cell.

Nucleic acid variants may be naturally occurring, such as allelic variants (same locus), homologs (different locus), and/or orthologs (different organism) and/or may be non naturally occurring. Non-naturally occurring variants may be made by mutagenesis techniques, including those applied to polynucleotides, cells, and/or organism. Variants may contain nucleotide substitutions, deletions, inversions and insertions. Variation may occur in the coding and/or non-coding regions. Variations may produce conservative and/or non-conservative amino acid substitutions (as compared in the encoded product).

It is not intended that the present invention be limited by the nature of the nucleic acid employed. In some embodiments, nucleic acids in accordance with the present invention are not synthetic, but are naturally-occurring entities that have been isolated from their natural environments. A nucleic acid may be a naturally-occurring and/or non-naturally-occurring (*e.g.* chemically-synthesized, artificial, man-made, *etc*.) nucleic acid.

In some embodiments, nucleic acids are derived and/or obtained from natural sources (*e.g*. viral, fungal, bacterial, animal and/or plant sources). Alternatively or additionally, nucleic acids of the present invention may be prepared by any conventional means typically used to prepare nucleic acids in large quantity. For example, DNAs and/or RNAs may be chemically synthesized using commercially available reagents and/or synthesizers by methods that are well-known in the art (see, *e.g*., Gait, Oligonucleotide Synthesis: A Practical Approach, IRL Press, Oxford, England, 1985; incorporated herein by reference). RNAs may be produce in high yield via *in vitro* transcription using any of a variety of plasmids known in the art that are suitable for *in vitro* transcription, such as pSP72 (Promega Corporation, Madison, WI), pBluescript (Stratagene, La Jolla, CA), *etc.*

Nucleic acids may be purified by any suitable means, as are well known in the art. For example, the nucleic acids may be purified by reverse phase and/or ion exchange HPLC, size exclusion chromatography, and/or gel electrophoresis. Of course, the skilled artisan will recognize that the method of purification will depend in part on the size of the nucleic acid to be purified.

### Small Molecule PAK Modulators

In some embodiments, PAK modulators may comprise small molecules. For example, U.S. Patent Application 2004/007504 (incorporated herein by reference) describes heterobicyclic pyrazole derivatives that inhibit PAK4 or PAK5 and may be used to treat Alzheimer's disease. In some embodiments, heterobicyclic pyrazole derivatives may be tested for use in treatment of FXS and/or other neurodevelopmental disorders.

In some embodiments, Emodin is a small molecule that can be used in the treatment of FXS and/or other neurodevelopmental disorders (see, *e.g*. Example 12). Emodin (also known as 1,3,8-Tri-hydroxy-6-methyl-anthra-quinone; 6-Methyl-1,3,8-tri-hydroxy-anthra-quinone; Emodol; and Frangula-emodin) is a member of a large family of naturally occurring anthraquinones and an active ingredient in Chinese herbal medicine. Emodin inhibits *HER-2*/*neu* tyrosine kinase activity (Zhang et al., 1999, Clin. Cancer Res., 5:343; incorporated herein by reference) and therefore has anti-cancer effects in *HER-2*/*neu-*overexpressing breast cancer cells (Jayasuriya et al., 1992, J. Nat. Prod., 55:696; incorporate herein by reference). More recently, it has been demonstrated that 40 µM Emodin inhibits human cancer cell migration by interfering with the formation of an active Cdc42/Racl and PAK complex (Huang et al., 2005, Cell. Mol. Life Sci., 62:1167; incorporated herein by reference).

In some embodiments, OSU-03012 (Figures 6 and 7) is a small molecule that can be used in the treatment of FXS and/or other neurodevelopmental disorders (see, *e.g*. Example 12). OSU-03012 (Zhu et al., 2004, Cancer Res., 64:4309; incorporated herein by reference; also known as 2-amino-N-{4-[5-(2-phenanthrenyl)-3-(trifluoromethyl)-1H-pyrazol-1-yl]-phenyl} acetamide) is derived from celecoxib. Celecoxib, sold by Pfizer under the brand name CELEBREX^{®}, is a nonsteroidal anti-inflammatory drug that works through the inhibition cyclooxygenase-2 (COX-2). Celecoxib can be used for the treatment of many conditions including but not limited to osteoarthritis, rheumatoid arthritis, analgesia, familial adenomatous polyposis, and cancer. Unlike celecoxib, its analog OSU-03012 is not a COX-2 inhibitor, but instead inhibits 3-phosphoinositide-dependent protein kinase-1 (PDK-1) and PAK (Porchia et al., 2007, Mol. Pharmacol., 72:1124; incorporated herein by reference). OSU-03012 directly inhibits PAK kinase activity. While not wishing to be bound by any one theory, inhibition probably occurs via competitive inhibition of ATP binding, with IC50 value of about 500 nM - 1 µM (Brader and Eccles, 2004, Tumori., 90:2; incorporated herein by reference). OSU-03013 is another derivative of celecoxib that is structurally similar to OSU-03012. Although OSU-03013 has been shown to inhibit PAK activity, it is toxic *in vivo* and, therefore, not desirable for use as a therapeutic agent (Brader and Eccles, 2004, *Tumori*., 90:2; incorporated herein by reference).

### Activities of PAK Modulators

In some embodiments, PAK modulators target PAK directly. In some embodiments, PAK modulators target PAK indirectly. For example, PAK modulators might target downstream effectors of PAK, upstream effectors of PAK, and/or natural binding partners of PAK.

*PAK Participates in Multiple Signaling Pathways*

PAK is known to participate in a variety of signaling pathways. To give but one example, Figure 8 (Klann and Dever, 2004, *Nat. Rev. Neurosci.,* 5:931) shows how PAK fits within the context of the extracellular signal-regulated kinase (ERK) and phosphoinositide-3 kinase (PI3K) signaling pathways. PAK falls downstream of PDK1, PDK2, and PI3K. PAK falls upstream of Ras, Raf, MEK, ERK, and Mnk.

Although not pictured in Figure 8, PAK falls upstream of Myosin light chain kinase (MLCK), regulatory Myosin light chain (R-MLC), Myosins I heavy chain, myosin II heavy chain, Myosin VI, Caldesmon, Desmin, Op18/stathmin, Merlin, Filamin A, LIM kinase (LIMK), p47^{phox}, BAD, caspase 3, estrogen and/or progesterone receptors, RhoGEF, GEF-H1, NET1, Gαz, phosphoglycerate mutase-B, RhoGDI, prolactin, p41^{Arc}, and/or Aurora-A (Bokoch et al., 2003, Annu. Rev. Biochem., 72:743; and Hofmann et al., 2004, J. Cell Sci., 117:4343; both of which are incorporated herein by reference). The following factors bind to PAK in cells and may fall downstream of PAK: CIB; sphingolipids; G-protein β and/or γ subunits; PIX/COOL; GIT/PKL; Nef; Paxillin; NESH; SH3-containing proteins (*e.g*. Nck and/or Grb2); kinases (*e.g*. Akt, PDK1, PI 3-kinase/p85, Cdk5, Cdc2, Src kinases, Abl, and/or protein kinase A (PKA)); and/or phosphatases (*e.g*. phosphatase PP2A, POPX1, and/or POPX2). Any of these factors may be targeted by PAK modulators in accordance with the present invention.

The present invention encompasses the recognition that PAK modulators may indirectly modulate ERK activity. For example, considering that PAK functions upstream of ERK, aberrant activation of PAK (Figure 9) could cause in the constitutive activation of ERK pathway kinases. Down-regulation of negative regulators of the ERK pathway, such as mitogen-activated protein (MAP) kinase phosphatases (MKPs) and Sprouty proteins, would also result in the constitutive activation of ERK (see, *e.g*., Kohno and Pouyssegur, 2006, Annals Med., 38:200; incorporated by reference herein).

As previously shown, *FMR1* KO mice exhibit enhanced basal ERK phosphorylation (and presumably activity) (Hou et al., 2006, Neuron, 51:41; incorporated herein by reference) while ERK is phosphorylated and activated by PAK at least in non-neuronal cells (Eblen et al. 2002, Mol Cell Biol, 22:6023; incorporated herein by reference). ERK is involved in regulation of spine morphology, synaptic plasticity and behaviors (Selcher et al., 2001, Learn Mem, 8:11; and Kelleher et al., 2004, Cell, 116:467; both of which are incorporated herein by reference), therefore, it is possible that PAK inhibition returns the levels of phospho-ERK in *FMR1* KO mice to wild-type levels, thereby reversing phenotypes in *FMR1* KO mice.

As shown in Figure 9, the ERK signaling cascade, starting from Ras and going downstream, comprises Ras, Raf, MEK, and ERK. PAK has been shown to phosphorylate and activate both MEK-1 (Frost et al., 1997, EMBO J., 16:6426; incorporated herein by reference) and Raf-1 (King et al., 1998, Nature, 396: 80; and Chaudhary et al., 2000, Curr. Biol., 10:551; both of which are incorporated herein by reference).

Thus, the present invention encompasses the recognition that inhibiting any member of the ERK signaling pathway (*e.g*. ERK, MEK, Ras, Raf) can be useful for treatment of FXS and/or other neurodevelopmental disorders. The present invention provides methods for treating FXS and/or other neurodevelopmental disorders comprising administering a therapeutically effective amount of an ERK pathway inhibitor (or pharmaceutical composition comprising an ERK pathway inhibitor) to a patient susceptible to, suffering from, and/or exhibiting one or more symptoms of FXS and/or other neurodevelopmental disorder. The general description of PAK modulators can be applied to ERK pathway inhibitors as well. To give but one example, ERK pathway inhibitors may be proteins, nucleic acids, small molecules, glycoproteins, proteoglycans, lipids, and/or carbohydrates, as described herein.

The signaling activity of Ras is dependent upon its association with the inner face of the plasma membrane, and this association is dependent upon a post-translational modification that places a farnesyl group on a cysteine residue near the C-terminus of Ras. This modification is catalyzed by famesyltransferase (FTase). Accordingly, FTase inhibitors have been developed as anti-Ras compounds. In some embodiments, ERK pathway modulators may be FTase inhibitors. Exemplary FTase inhibitors include, but are not limited to, FTI-276, FTI-2148, L-739,750, and BZA-2B (Sebti and Der, 2006, Nat. Rev. Cancer, 3:945; Zhu et al., 2003, Curr, Opin. Investig. Drugs, 4:1428; both of which are incorporated herein by reference); AZD-3409 (AstraZeneca; Lavelle, 1998, Exp. Opin. Invest. Drugs, 7:1015; Williams, 1998, Curr. Opin. Ther. Pat., 8:553; Singh and Lingham, 2002, Curr. Opin. Drug Discov. Develop., 5:225; Wilson et al., 2004, Eur. J. Cancer. Suppl., 2:Abstract 354; Smethurst et al., 2004, Eur. J. Cancer Suppl., 2:Abstract 376; Kelly et al., 2005, Proc. Amer. Assoc. Cancer Res., 46:Abstract 5962; all of which are incorporated herein by reference); Tipifamib (R-I15777, Zarnestra™; End et al., 2001, Cancer Res., 61:131; Cunningham et al., 2002, Proc. Amer. Soc. Clin. Oncol., 21:Abstract. 502; Lancet et al., 2004, Blood, 104:Abstract 874; and Adjei et al., 2001, Proc. Amer. Soc. Clin. Oncol., 20:Abstract 320; all of which are incorporated herein by reference); and/or lonafarnib (Schering-Plough; Sch-66336, Sarasar™; Yang et al., 2005, Proc. Amer. Soc. Clin. Oncol., 24:Abstract 5565; Long et al., 2005, Proc. Amer. Assoc. Cancer Res., 46:Abstract 5968; and Oh et al., 2005, Proc. Amer. Assoc. Cancer Res., 46:Abstract 5967; all of which are incorporated herein by reference).

In some embodiments, ERK pathway modulators may be antisense inhibitors of Raf (see, *e.g.,* Smith et al., 2006, Curr. Topics Med. Chem., 6:1071; and Sridhar et al., 2005, Mol. Cancer Ther., 4:677; and references therein, both of which are incorporated herein by reference). For example, the Raf antisense ISIS-5132 (ISIS Pharmaceuticals/Novartis) was designed as a 20-mer phosphorothioate oligonucleotide to inhibit translation of the Raf-1 RNA message into protein (Lan et al., 1998, Oncogene, 16:1899; Koller et al., 2000, Tr. Pharm. Sci., 21:142; Monia et al., 1996, Nature Med. 2:668; and Monia et al., 1996, Proc. Natl. Acad. Sci., USA 93:15481; all of which are incorporated herein by reference). To overcome degradation and improve intracellular delivery of ISIS-5132, a liposomal formulation (i.e. LErafAON) is under investigation.

In some embodiments, ERK pathway modulators in accordance with the present invention include Raf kinase destabilizers (see, *e.g*., Sridhar et al., 2005, Mol. Cancer Ther., 4:677 and references therein; incorporated herein by reference). In some embodiments, oxindoles include, but are not limited to, geldanamycin and MCP1.

In some embodiments, ERK pathway modulators in accordance with the present invention include small molecule inhibitors of MEK (see, *e.g*., Kohno and Pouyssegur, 2006, Annals Med., 38:200; incorporated herein by reference). In some embodiments, small molecule inhibitors of MEK include, but are not limited to, PD98059, U0126, PD184352 (CI-1040), PD0325901, and/or ARRY-14886.

In some embodiments, ERK pathway modulators may be ureas (see, e.g., Smith et al., 2006, Curr. Topics Med. Chem., 6:1071 and references therein; incorporated herein by reference). Ureas may be derivitized in any way that makes them suitable as ERK pathway modulators in accordance with the present invention. In some embodiments, ureas may be, for example, biaryl ureas, diphenyl ureas, heteroaryl aryl ureas, quinolinyl ureas, isoquinolinyl ureas, pyridinyl ureas, *etc.* Exemplary ureas that may be utilized as ERK pathway modulators include, but are not limited to, 3-thienyl urea 7, isoxazole, pyrazole, and/or BAY 43-9006. BAY 43-9006 (also known as sorafenib), a novel biaryl urea, inhibits Raf-1 kinase activity *in vittro.* The crystal structure of the Raf/BAY 43-9006 complex revealed that the inhibitor binds in the adenosine triphosphate (ATP) pocket and interacts with residues of the kinase activation loop of Raf proteins. This interaction prevents the activation loop and the catalytic residues from adopting a conformation that is competent to bind and phosphorylate substrates (Wan et al., 2004, Cell, 116:855; incorporated herein by reference). In some embodiments, ureas that can be utilized as ERK pathway modulators may be urea derivatives (*e.g*. benzylic ureas, pyridopyrimidinones, heteroaryl-substituted diaryl ureas, annelated ureas, *etc*.).

In some embodiments, ureas in accordance with the present invention include urea bioisosteres (see, *e.g*., Smith et al., 2006, Curr. Topics Med. Chem., 6:1071 and references therein; incorporated herein by reference). In some embodiments, urea bioisosteres include, but are not limited to, compounds in which methylene and/or carboxyl moieties have been inserted into the urea functionality; glycinamides in which a methylene moiety has been introduced into the urea; oxamides having a second carbonyl group added to the urea; malonamides having both a carbonyl and a methylene moiety inserted into the urea functionality; diacyl hydrazines; 2-amino-benzimidazole derivatives; and/or pyrrolecarboxamides.

In some embodiments, ERK pathway modulators in accordance with the present invention include bis-aryl imidazoles and related structures (see, *e.g*., Smith et al., 2006, Curr. Topics Med. Chem., 6:1071 and references therein; incorporated herein by reference). In some embodiments, bis-aryl imidazoles include, but are not limited to, SB-203580, L-779,450, SB-590885, pyridyl-naphthyl-imidazoles, and/or derivatives thereof.

In some embodiments, ERK pathway modulators in accordance with the present invention include benzamides (see, *e.g*., Smith et al., 2006, Curr Topics Med. Chem., 6:1071 and references therein; incorporated herein by reference). In some embodiments, benzamides include, but are not limited to, ZM-336372, imatinib, AMN-107, and/or derivatives thereof.

In some embodiments, ERK pathway modulators in accordance with the present invention include oxindoles (see, *e.g*., Smith et al., 2006, Curr. Topics Med. Chem., 6:1071 and references therein; incorporated herein by reference). In some embodiments, oxindoles include, but are not limited to, GW-5074.

In some embodiments, ERK pathway modulators in accordance with the present invention include PTK-787, thienopyrimidines, styrene (see, *e.g*., Smith et al., 2006, Curr. Topics Med Chem., 6:1071 and references therein; incorporated herein by reference).

Alternatively or additionally, PAK is activated by PI3K signaling via PDK, and PI3K signaling is involved in protein synthesis (Hou and Klann, 2004, J. Neurosci., 24:6352; incorporated herein by reference). Thus, the present invention encompasses the recognition that, a PAK modulator may be an inhibitor of the PI3K/PDK pathway.

*Biological Activities of PAK Modulators*

In some embodiments, substances that are known to modulate serine/threonine kinase activity may accordingly modulate PAK kinase activity, including but not limited to Staurosporin, PD098059, Genistein, tyrphostin B42, HA1077, K252a, H-7: (1(5-isoquinoline-sulfonyl)-2-methylpiperazine), CEP-1347, *etc*., including analogs, derivatives, and/or mimetics thereof, that retain the ability to modulate PAK activity.

list Table 3 Kumar and Table 2 Eswaran

In some embodiments, PAK modulators such as the ones described in Eswaren et al. (2007, Structure, 15:201; incorporated herein by reference) and Kumar et al. (2006, Nat. Rev. Cancer, 6:459; incorporated herein by reference) are used to treat FXS and/or other neurodevelopmental disorders. See, for example, Table 1 (adapted from Eswaren *et al*.) and Table 2 (adapted from Kumar *et al*.):

**Table 1. Exemplary PAK Modulators**

| | | **Tm Shift (°C)** | | | **% Activity at 10 µM** | | |
|---|---|---|---|---|---|---|---|
| **Compound Name** | **Chemical Structure** | **PAK4** | **PAR5** | **PAK6** | **PAK4** | **PAK5** | **PAK6** |
| Cdk1 Inhibitor | 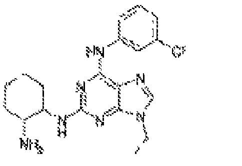 | 7.0 ± 15 | 71 ± 03 | 7.0 ± 03 | 56 | 12 | 23 |
| Cdk1/2 Inhibitor III | 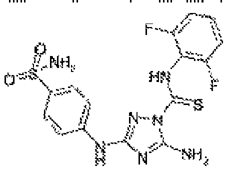 | 6.5 ± 0.2 | 5.6 ± 0.3 | 5.6 ± 0.8 | 62 | 7.0 | 18 |
| Purvalanol A | 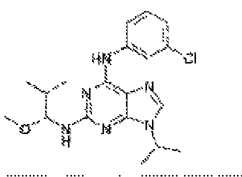 | 50 ± 0.3 | 45 ± 0.2 | 5.4 ± 0.5 | 20 | 24 | 48 |
| K252a | 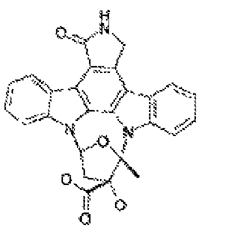 | 4.5 ± 03 | 5.9 ± 0.3 | 8.6 ± 1.0 | 16 | 22 | 16 |
| Staurosporine | 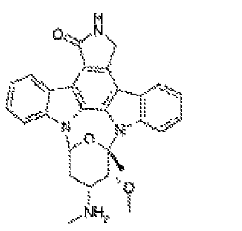 | 131 ± 1.5 | 12.5 ± 0.3 | 16.6 ± 0.5 | 0 | 0 | 0 |
| SU11652 | 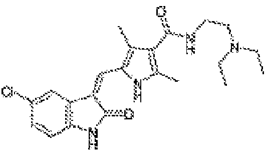 | 6.4 ± 1.2 | 5.3 ± 0.2 | 5.3 ± 0.3 | 34 | 43 | 75 |

**Table 2. Exemplary PAK1 Inhibitors**

| **Inhibitor** | **Mode of Action** | **References*** |
|---|---|---|
| hPIP | Binds to regulatory domain and blocks kinase activity | Xia *et al.,* 2001, *Proc. Natl Acad. Sci., USA,* 98:6174 |
| Merlin | Binds to PBD and inhibits recruitment to focal adhesions | Kissil *et al.,* 2003, *Mol. Cell,* 12:841 |
| Nischarin | Binds to kinase domain and inhibits kinase activity | Alahari *et al.,* 2004, *EMBO J.*, 23:2777 |
| P35/CDK5 | Phosphorylates Pak and inhibits kinase activity | Nikolic *et al.,* 1998, *Nature,* 395:194; and Rashid *et al.,* 2001, *J. Biol. Chem.,* 276:49043 |
| CDC2 | Phosphorylates xPAK2 and inhibits kinase activity | Cau *et al*., 2000, *J. Biol. Chem*., 275:2367 |
| p 110C | Binds to part of kinase domain and inhibits kinase activity | Chen *et al.*, 2003, *J. Biol. Chem*., 278:20029 |
| POPX1, POPX2 | Dephosphorylation of T422 in kinase activation loop | Koh *et al.,* 2002, *Curr. Biol.,* 12:317 |
| CRIPak | Binds to regulatory domain and blocks kinase activity | Talukder *et al.,* 2006, *Oncogene, 25:13* 11 |
| PAKI aa 89 -143 peptide | Competitive Inhibitor | Zhao *et al*., 1998, *M*o*l. Cell. Biol.,* 18:2153 |
| CEP-1347 | Small molecule - ATP antagonist | Nheu *et al.*, 2002, *Cancer J.,* 8:328 |
| GL-2003 | ERK inhibitor that blocks Pak activation | Hirokawa *et al.,* 2006, *Cancer Lett.* |

| | | |
|---|---|---|
| CDC2, cell division cycle 2; CDK5, cyclin-dependent kinase 5; CRIPak, cysteine-rich inhibitor of PAK1; ERK, extracellular signal regulated kinase; hPIP, human PAK/PLC interacting protein 1; PBD, p21-binding domain * The contents of all of which are incorporated herein by reference | | |

In some embodiments, PAK modulators may affect the ability of PAK to interact with its natural binding partners, including but not limited to FMRP. In certain embodiments, such binding blocks the interaction between PAK and its natural binding partners (for example, the interaction between PAK and FMRP). In some embodiments, such binding promotes the interaction between PAK and its natural binding partners. However, a PAK modulator need not necessarily bind directly to a catalytic and/or binding site, and may bind, for example, to an adjacent site, such as an adjacent site in the PAK polypeptide. A PAK modulator may even bind to another substance (for example, a protein, lipid, carbohydrate, *etc*. which is complexed with the enzyme), so long as its binding modulates PAK activity.

The present invention encompasses the discovery that the integrity of the FMRP KH domains facilitates FMRP's interaction with PAK. In specific embodiments, PAK modulators may affect the integrity of one or more KH domains of FMRP and modulate its ability to bind to PAK.

In some embodiments, a PAK modulator binds to a natural binding partner of PAK and inhibits and/or promotes the interaction of PAK with its natural binding partner. In another aspect, a PAK modulator binds to PAK and inhibits and/or promotes the interaction of a natural binding partner of PAK with PAK. Some modulators that regulate PAK function in this manner have been described in the art. For example, U.S. Patent Application 2004/0208880 (incorporated herein by reference) describes compounds that inhibit PAK1 function by modulating its interaction with dynein light chain 1/protein inhibitors of nitric oxide synthase (DLC1/PIN). U.S. Patent Application 2006/0172360 (incorporated herein by reference) describes compounds that inhibit PAK4 by modulating its interaction with MKK7. U.S. Patent Application 2004/0091907 (incorporated herein by reference) describes compounds that inhibit PAK4 by modulating its interaction with GEF/H1 (used to treat cancer). U.S. Patent Application 2002/0106690 (incorporated herein by reference) describes inhibitors that function by altering the interaction between PAK and the beta subunit of G-protein coupled receptors. U.S. Patent Application 2006/0088897 (incorporated herein by reference) describes substances that modulate PAK by altering the interaction between PAK and SH3 domain-containing proteins. U.S. Patents 6,013,500 and 6,667,168 (both of which are incorporated herein by reference) describe agents that bind to the GTP-binding domain of PAK4 and prevent PAK4 from binding to GTP-binding proteins and agents that bind the PAK4 cdc42-binding domain and prevent PAK4 from interacting with cdc42.

In some embodiments, a PAK modulator may bind to and/or compete for one or more sites on a relevant molecule, for example, a catalytic site and/or a binding site of PAK. In some embodiments, the PAK modulator interferes with and/or inhibits the binding of FMRP to PAK. In certain embodiments, the PAK modulator competes for an FMRP-binding region of PAK. In some embodiments, the PAK modulator competes for a PAK-binding region of FMRP.

Where modulators of an enzyme such as PAK are concerned, a modulator may include a substrate of PAK kinase and/or characteristic portion thereof which is capable of binding to PAK. Alternatively or additionally, whole or portions of a substrate isolated from a biological source (*e.g.* purified from tissues and/or cells) and/or by chemical synthesis may be used to compete with the substrate for binding sites on the enzyme. Alternatively or additionally, an antibody capable of binding to PAK may be used. A PAK modulator may include a peptide or other small molecule which is capable of modulating the binding interaction.

In some embodiments, PAK modulators are substances which bind to and/or block the kinase domain of PAK and/or the p21-binding domain of PAK, and/or the autophosphorylation sites of PAK. In some embodiments, PAK modulators are short peptides comprising sequences of PAK that dominant-negative activity (Kiosses *et al.,* 2002, *Circ. Res.,* 90:697; incorporated herein by reference). In some embodiments, such peptides may not block PAK kinase activity *per se,* but may displace PAK from sites of action within the cell, which may indirectly modulate PAK activity.

In certain embodiments, PAK modulators may function to alter the ability of PAK to phosphorylate its substrates. For example, U.S. Patent 6,383,734 (incorporated herein by reference) describes agents that inhibit PAK function by inhibiting the ability of PAK to phosphorylate Raf. U.S. Patents 6,013,500 and 6,667,168 (both of which are incorporated herein by reference) describe agents that block the ATP-binding domain of PAK4 in order to inhibit the kinase function of PAK4.

In certain embodiments, PAK modulators may function by altering the activity and/or expression of PAK activators. For example, U.S. Patent 6,046,224 (incorporated herein by reference) describes agents that inhibit PAK function by blocking 12(S)HETE receptors. 12(S)HETE stimulates PAK activity, so blocking 12(S)HETE function indirectly inhibits PAK function.

In certain embodiments, inventive PAK modulators may comprise phosphatases that inhibit PAK function by removing phosphate groups from targets that are phosphorylated by PAK kinase. Alternatively or additionally, inventive PAK modulators may comprise kinases that indirectly activate PAK function by phosphorylating targets that are phosphorylated by PAK kinase.

In some embodiments, PAK modulators may comprise the autoinhibitory domain of PAK, which may block and/or reduce PAK activity. In specific embodiments, the candidate substance is an autoinhibitory region of PAK protein, such as a peptide comprising at least 25, 50, 75, 100, 125, or 150 contiguous amino acids of PAK. In certain embodiments, the protein includes at least 25, 50, 75, 100, 125, 150, 200, or 300 of the N-terminal amino acids of PAK protein. In some embodiments, PAK modulators may comprise a dominant-negative PAK protein (*e.g.*, a mutant and/or a characteristic portion of PAK protein).

In some embodiments, PAK modulators function to modulate the expression, stability, and/or cellular levels of PAK. For example, U.S. Patent Applications 2004/0102326 (PAK1), 2002/0142325 (PAK2), 2004/0009935 (PAK2), and 2005/0191672 (PAK4) (all of which are incorporated herein by reference) describe nucleotide inhibitors of PAK expression that target PAK mRNA for degradation.

Alternatively or additionally, inventive PAK modulators affect PAK levels by increasing and/or decreasing transcription and/or translation of PAK, PAK substrates, and/or natural binding partners of PAK. In some embodiments, PAK modulators may affect RNA and/or protein half-life, for example, by directly affecting mRNA and/or protein stability. In certain embodiments, inventive PAK modulators cause the mRNA and/or protein to be more and/or less accessible and/or susceptible to nucleases, proteases, and/or the proteasome.

In some embodiments, inventive PAK modulators affect the processing of mRNAs encoding PAK, PAK substrates, and/or natural binding partners of PAK. For example, PAK modulators may function at the level of pre-mRNA splicing, 5' end formation (*e.g.* capping), 3' end processing (*e.g*. cleavage and/or polyadenylation), nuclear export, and/or association with the translational machinery and/or ribosomes in the cytoplasm.

In some embodiments, inventive PAK modulators affect translational control and/or post-translational modification of PAK, PAK substrates, and/or natural binding partners of PAK. For example, PAK modulators may function at the level of translation initiation, elongation, termination, and/or recycling. In some embodiments, PAK modulators may function at the step of protein folding into secondary, tertiary, and/or quaternary structures. Alternatively or additionally, PAK modulators may function at the level of intracellular transport (*e.g*. ER to Golgi transport, intra-Golgi transport, Golgi to plasma membrane transport, and/or secretion from the cell). In some embodiments, PAK modulators may function at the level of post-translational modification (*e.g.* cleavage of signal sequences and/or the addition of entities such as methyl groups, phosphates, glycan moieties, *etc.*).

In various embodiments, a PAK modulator is a purified and/or unpurified synthetic organic molecule and/or naturally occurring organic molecule.

In some embodiments, inventive PAK modulators may cause the level of PAK mRNA and/or protein, an activity of PAK protein, the half-life of PAK mRNA and/or protein, the binding of PAK mRNA and/or protein to its natural binding partners, and/or the level and/or activity of a substance that phosphorylates a PAK kinase to decrease by at least about 5%, at least about 10%, at least about 20%, at least about 30%, at least about 40%, at least about 50%, at least about 60%, at least about 80%, at least about 90%, at least about 95%, or substantially 100%.

PAK modulators may be used alone and/or in conjunction with other substances which affect PAK activity. Additional examples of PAK modulators will be apparent to the skilled person.

### Identification and/or characterization of PAK modulators

The present invention provides methods of identifying PAK modulators. In some embodiments, inventive methods screen for novel PAK modulators by identifying substances that improve and/or treat the symptoms of FXS and/or other neurodevelopmental disorders. In some embodiments, inventive methods identify novel PAK modulators by identifying substances that affect PAK's ability to interact with its natural binding partners (*e.g.* FMRP). In some embodiments, inventive methods identify novel PAK modulators by identifying substances that modulate PAK kinase activity. In some embodiments, inventive methods identify PAK modulators by identifying substances that modulate PAK expression and/or levels.

In some embodiments, inventive methods identify substances that are known to have a particular function, but were not previously known to function as PAK modulators. In some embodiments, inventive methods identify substances that have been identified and/or synthesized, but have not been attributed any particular function. In some embodiments, inventive methods identify novel substances that have never been identified and/or synthesized before.

As used herein, the term "test substance" refers to (1) a PAK protein, a nucleic acid encoding PAK, and/or homolog, portion, variant, mutant, and/or derivative thereof; (2) a natural binding partner of PAK, a nucleic acid encoding a natural binding partner of PAK, and/or a homolog, portion, variant, mutant, and/or derivative thereof; (3) an FMRP protein, a nucleic acid encoding FMRP, and/or a homolog, portion, variant, mutant, and/or derivative thereof; and/or (4) a substrate of PAK kinase, a nucleic acid encoding a substrate of PAK, and/or a homolog, portion, variant, mutant, and/or derivative thereof. In some embodiments, a test substance is a protein and/or characteristic portion thereof comprising a FMRP-binding portion of PAK. In some embodiments, a test substance is a protein and/or characterstic portion thereof comprising a PAK-binding portion of FMRP.

The efficacy of a candidate substance may be assessed by generating dose response curves from data obtained using various concentrations of the candidate substance. Moreover, a control assay may be performed to provide a baseline for comparison. In the control assay, the assay is performed in the absence of a candidate substance.

In some embodiments, inventive PAK modulators inhibit and/or activate PAK activity by at least about 10%, at least about 20%, at least about 30%, at least about 40%, at least about 50%, at least about 60%, at least about 70%, at least about 80%, at least about 90%, or greater than about 90% as compared with the activity observed under otherwise identical conditions lacking a candidate substance.

It will, of course, be understood that all screening methods of the present invention are useful in themselves notwithstanding the fact that effective PAK modulators may not be found. The invention provides methods for screening for candidate PAK modulators, not solely methods of finding them.

### Screening

In some embodiments, screening for PAK modulators is employed. In some embodiments, high throughput screening for PAK modulators is employed. In some embodiments, such screening identifies substances that bind to PAK. Typically, large numbers of candidate substances are immobilized on a solid substrate. Immobilized candidate substances are contacted with PAK and washed. Bound PAK is then detected by methods well known in the art.

Using high throughput assays, it is possible to screen up to several thousand candidate substances in a single day. In some embodiments, each well of a microtiter plate can be used to run a separate assay against a selected candidate substance, or, if concentration and/or incubation time effects are to be observed, every 5 -10 wells can test a single candidate substance. Thus, a single standard microtiter plate can assay up to 96 candidate substances. If 1536 well plates are used, then a single plate can assay up to 1536 candidate substances. It is possible to assay many plates per day; assay screens for up to about 6,000, about 20,000, about 50,000, or more than about 100,000 different candidate substances are possible using high throughput systems in accordance with the present invention.

For solid state reactions, a candidate substance may be bound to the solid state component, directly or indirectly, via covalent and/or non covalent linkage *e.g.*, via a tag. A tag may comprise any of a variety of components. In general, a substance which binds the tag (a tag binder) is fixed to a solid support, and the tagged candidate substance is attached to the solid support by interaction of the tag and/or the tag binder.

A number of tags and/or tag binders may be used, based upon known molecular interactions well described in the literature. For example, where a tag has a natural binder, for example, biotin, protein A, and/or protein G, it may be used in conjunction with appropriate tag binders (avidin, streptavidin, neutravidin, the Fc region of an immunoglobulin, *etc.).* Antibodies to molecules with natural binders such as biotin and/or appropriate tag binders are widely available (Sigma Immunochemicals, St. Louis, MO).

Similarly, any haptenic and/or antigenic compound may be used in combination with an appropriate antibody to form a tag/tag binder pair. Thousands of specific antibodies are commercially available and many additional antibodies are described in the literature. For example, in one common configuration, the tag is a first antibody and the tag binder is a second antibody which recognizes the first antibody. In addition to antibody-antigen interactions, receptor-ligand interactions are appropriate as tag and/or tag-binder pairs, including but not limited to transferrin, c-kit, viral receptor ligands, cytokine receptors, chemokine receptors, interleukin receptors, immunoglobulin receptors and/or antibodies, the cadherin family, the integrin family, the selectin family, *etc.* (see, *e.g.*, Pigott *et al., The Adhesion Molecule Facts Book I,* 1993). Similarly, toxins and/or venoms; viral epitopes; hormones *(e.g.* opiates, steroids, *etc.*); intracellular receptors *(e.g.* which mediate the effects of various small ligands, including steroids, thyroid hormone, retinoids, vitamin D, and/or peptides); drugs; lectins; carbohydrates; nucleic acids (linear and/or cyclic polymer configurations); proteins; phospholipids; and/or antibodies may interact with various cell receptors.

Synthetic polymers, such as polyurethanes, polyesters, polycarbonates, polyureas, polyamides, polyethyleneimines, polyarylene sulfides, polysiloxanes, polyimides, and/or polyacetates may form appropriate tags and/or tag binders. Many other tag/tag binder pairs are useful in assay systems described herein, as would be apparent to one skilled in the art.

Common linkers such as peptides, polyethers, and the like may serve as tags and may include polypeptide sequences, such as poly-Gly sequences of between about 5 and 200 amino acids. Such flexible linkers are known to persons of skill in the art. For example, poly(ethelyne glycol) linkers are available from Shearwater Polymers, Inc. (Huntsville, AL). These linkers optionally have amide linkages, sulfhydryl linkages, and/or heterofunctional linkages.

Tag binders are fixed to solid substrates using any of a variety of methods currently available. Solid substrates are commonly derivatized and/or functionalized by exposing all and/or a portion of the substrate to a chemical reagent which fixes a chemical group to the surface which is reactive with a portion of the tag binder. For example, groups which are suitable for attachment to a longer chain portion include amines, hydroxyl, thiol, and/or carboxyl groups. Aminoalkylsilanes and/or hydroxyalkylsilanes may be used to functionalize a variety of surfaces, such as glass surfaces. The construction of such solid phase biopolymer arrays is well described in the literature (see, *e.g.*, Merrifield, 1963, *J. Am. Chem. Soc.,* 85:2149, describing solid phase synthesis of, *e.g.,* peptides; Geysen *et al.,* 1987, *J. Immun. Meth.,* 102:259, describing synthesis of solid phase components on pins; Frank *et al.,* 1988, *Tetrahedron,* 44:6031, describing synthesis of various peptide sequences on cellulose disks; Fodor *et al.,* 1991, *Science,* 251:767; Sheldon *et al.,* 1993, *Clinical Chemistry,* 39(4):718; and Kozal *et al.,* 1996, *Nature Medicine,* 2:753; all describing arrays of biopolymers fixed to solid substrates; all of which are incorporated herein by reference). Non-chemical approaches for fixing tag binders to substrates include other common methods, such as heat, cross-linking by ultraviolet radiation, and the like.

### In Vitro Assays

*In vitro* assays can often be run quickly and/or in large numbers, thereby increasing the amount of information obtainable in a short period of time. A variety of vessels may be used to run the assays, including test tubes, plates, dishes, microtiter plates, and/or other surfaces such as dipsticks and/or beads. Some *in vitro* and *in cyto* assays have been described, for example, in PCT Publication WO 06/029337 (incorporated herein by reference).

The present invention provides *in vitro* methods for screening for PAK modulators. For example, in some embodiments, methods may comprise steps of: (1) providing a test substance (*e.g.* PAK protein, PAK gene, and/or characteristic portion thereof); (2) providing at least one candidate substance; and (3) measuring and/or detecting the influence of the candidate substance(s) on the test substance.

In some embodiments, a test substance (*e.g.* PAK protein, PAK gene, and/or characteristic portion thereof) is provided and brought directly and/or indirectly into contact with a candidate substance (*e.g*. in the form of a library). Then, the influence of the candidate substance on the test substance is detected and/or measured. Thereafter, suitable PAK modulators may be isolated and/or analyzed. For the screening of libraries, the use of high-throughput assays are contemplated and described herein.

In some embodiments, *in vitro* assays comprise binding assays. Binding of a candidate substance to a test substance (*e.g.* PAK protein, PAK gene, and/or characteristic portion thereof) may, in and of itself, be inhibitory, due to steric, allosteric, and/or charge-charge interactions. The test substance may be free in solution, fixed to a support, and/or expressed in and/or on the surface of a cell. The test substance and/or the candidate substance may be labeled, thereby permitting detection of binding. The test substance is frequently the labeled species, decreasing the chance that the labeling will interfere with and/or enhance binding. Competitive binding formats may be performed in which one of the substances is labeled, and one may measure the amount of free label versus bound label to determine the effect on binding.

In some embodiments, binding assays involve, for example, exposing a test substance to a candidate substance and detecting binding between the test substance and the candidate substance. A binding assay may be conducted *in vitro* (*e.g.* in a test tube, comprising substantially only the components mentioned; in cell-free extracts; and/or in substantially purified components). Alternatively or additionally, binding assays may be conducted *in cyto* and/or *in vivo* (*e.g.* within a cell, tissue, organ, and/or organism; described in further detail below).

In certain embodiments, at least one candidate substance is contacted with a test substance (*e.g.* PAK protein, PAK gene, and/or characteristic portion thereof) and an effect detected. In some embodiments, for example, a candidate substance is contacted with PAK protein, and binding to PAK protein is tested. In some embodiments, an assay may involve contacting a candidate substance with a characteristic portion of PAK protein, including but not limited to a FMRP-binding portion of PAK. Binding of the candidate substance to the PAK peptide is detected. It will be appreciated that fragments, portions, homologs, variants, and/or derivatives of PAK may be employed, provided that they comprise FMRP binding activity.

In some embodiments, assays may involve providing a test substance (*e.g.* immobilized on a solid support), and a non-immobilized candidate substance. The extent to which the test substance and candidate substance bind to one another is determined. Alternatively, the candidate substance may be immobilized and the test substance non-immobilized. Such assays may be used to identify candidate substances capable of binding to PAK and/or fragments, portions, homologs, variants, and/or derivatives thereof.

In some embodiments, an antibody that recognizes the test substance (*e.g.* an α-PAK antibody) is immobilized to a solid support (*e.g.* Protein-A beads). The antibody is contacted with the test substance, which binds to the immobilized antibody. The resulting complex is then brought into contact with the candidate substance (purified protein, cellular extract, combinatorial library, *etc.).* If the candidate substance interacts with the test substance, the candidate substance will become indirectly immobilized to the solid support. Presence of the candidate substance on the solid support can be assayed by any standard technique known in the art (including, but not limited to, western blotting). This type of assay is known in the art as an "immunoprecipitation" assay.

In some embodiments, a test substance (*e.g.* PAK protein, PAK gene, and/or characteristic portion thereof) is immobilized on a solid support (*e.g.* agarose beads). In specific embodiments, the test substance is expressed as a GST-fusion protein in bacteria, yeast, insect cells, and/or higher eukaryotic cell line and/or purified from crude cell extracts using glutathione-agarose beads. As a control, binding of the candidate substance, which is not a GST-fusion protein, to the immobilized PAK protein is determined in the absence of PAK protein. The binding of the candidate substance to the immobilized PAK protein is then determined. This type of assay is known in the art as a "GST pulldown" assay. Alternatively or additionally, the candidate substance may be immobilized and the test substance non-immobilized.

It is possible to perform this type of assay using different affinity purification systems for immobilizing one of the components, for example Ni-NTA agarose- and/or histidine-tagged components.

Binding of a test substance to the candidate substance may be determined by a variety of methods well-known in the art. For example, the non-immobilized component may be labeled (with for example, a radioactive label, an epitope tag, and/or an enzyme-antibody conjugate). Alternatively or additionally, binding may be determined by immunological detection techniques. For example, the reaction mixture may be subjected to Western blotting and the blot probed with an antibody that detects the non-immobilized component. Alternatively or additionally, enzyme linked immunosorbent assay (ELISA) may be utilized to assay for binding.

In some embodiments, screening methods of the present invention comprise: (1) obtaining a candidate substance; (2) contacting the candidate substance with PAK (and/or characteristic portion thereof) and FMRP; and (3) detecting inhibition and/or activation of binding between PAK and FMRP in the presence and/or absence of the candidate substance.

In some embodiments, screening methods of the present invention comprise: (1) obtaining a candidate substance; and (2) contacting the candidate substance with a preformed PAK (and/or characteristic portion thereof)-FMRP complex; and (3) determining whether the candidate substance affects the PAK (and/or characteristic portion thereof)-FMRP complex.

In some embodiments, screening methods of the present invention comprise (1) obtaining a candidate substance; (2) contacting the candidate substance with PAK (and/or characteristic portion thereof) and a natural binding partner; and (3) detecting whether the candidate substance can compete with the binding interaction between PAK (and/or characteristic portion thereof) and the natural binding partner.

In some embodiments, a candidate substance is determined to be a PAK inhibitor if administering the candidate substance to PAK and the natural binding partner results in decreased binding between PAK and the natural binding partner. In some embodiments, a candidate substance is determined to be a PAK inhibitor if administering the candidate substance to PAK and the natural binding partner results in an at least 2-fold decrease in binding between PAK and the natural binding partner. In some embodiments, a candidate substance is determined to be a PAK inhibitor if administering the candidate substance to PAK and the natural binding partner results in an at least 3-fold, at least 4-fold, at least 5-fold, at least 10-fold, at least 20-fold, at least 50-fold, at least 100-fold, at least 200-fold, at least 500-fold, at least 1000-fold, at least 10,000-fold, or greater than 10,000-fold decrease in binding between PAK and the natural binding partner. In some embodiments, a candidate substance is determined to be a PAK inhibitor if administering the candidate substance to PAK and the natural binding partner results in an at least 25%, 50%, 75%, 100%, 200%, 500%, 1000%, or greater than 1000% decrease in binding between PAK and the natural binding partner.

In some embodiments, a candidate substance is determined to be a PAK activator if administering the candidate substance to PAK and the natural binding partner results in decreased binding between PAK and the natural binding partner. In some embodiments, a candidate substance is determined to be a PAK activator if administering the candidate substance to PAK and the natural binding partner results in an at least 2-fold decrease in binding between PAK and the natural binding partner. In some embodiments, a candidate substance is determined to be a PAK activator if administering the candidate substance to PAK and the natural binding partner results in an at least 3-fold, at least 4-fold, at least 5-fold, at least 10-fold, at least 20-fold, at least 50-fold, at least 100-fold, at least 200-fold, at least 500-fold, at least 1000-fold, at least 10,000-fold, or greater than 10,000-fold decrease in binding between PAK and the natural binding partner. In some embodiments, a candidate substance is determined to be a PAK activator if administering the candidate substance to PAK and the natural binding partner results in an at least 25%, 50%, 75%, 100%, 200%, 500%, 1000%, or greater than 1000% decrease in binding between PAK and the natural binding partner.

In some embodiments, a candidate substance is determined to be a PAK inhibitor if administering the candidate substance to PAK and the natural binding partner results in increased binding between PAK and the natural binding partner. In some embodiments, a candidate substance is determined to be a PAK inhibitor if administering the candidate substance to PAK and the natural binding partner results in an at least 2-fold increase in binding between PAK and the natural binding partner. In some embodiments, a candidate substance is determined to be a PAK inhibitor if administering the candidate substance to PAK and the natural binding partner results in an at least 3-fold, at least 4-fold, at least 5-fold, at least 10-fold, at least 20-fold, at least 50-fold, at least 100-fold, at least 200-fold, at least 500-fold, at least 1000-fold, at least 10,000-fold, or greater than 10,000-fold increase in binding between PAK and the natural binding partner. In some embodiments, a candidate substance is determined to be a PAK inhibitor if administering the candidate substance to PAK and the natural binding partner results in an at least 25%, 50%, 75%, 100%, 200%, 500%, 1000%, or greater than 1000% increase in binding between PAK and the natural binding partner.

In some embodiments, a candidate substance is determined to be a PAK activator if administering the candidate substance to PAK and the natural binding partner results in increased binding between PAK and the natural binding partner. In some embodiments, a candidate substance is determined to be a PAK activator if administering the candidate substance to PAK and the natural binding partner results in an at least 2-fold increase in binding between PAK and the natural binding partner. In some embodiments, a candidate substance is determined to be a PAK activator if administering the candidate substance to PAK and the natural binding partner results in an at least 3-fold, at least 4-fold, at least 5-fold, at least 10-fold, at least 20-fold, at least 50-fold, at least 100-fold, at least 200-fold, at least 500-fold, at least 1000-fold, at least 10,000-fold, or greater than 10,000-fold increase in binding between PAK and the natural binding partner. In some embodiments, a candidate substance is determined to be a PAK activator if administering the candidate substance to PAK and the natural binding partner results in an at least 25%, 50%, 75%, 100%, 200%, 500%, 1000%, or greater than 1000% increase in binding between PAK and the natural binding partner.

The activity of PAK modulators of the present invention may be determined by, for example, assaying for kinase activity of PAK. In such assays PAK and/or a characteristic portion thereof produced by recombinant means as described above is contacted with a substrate in the presence of a suitable phosphate donor (*e.g.* ATP) containing radiolabeled phosphate, and PAK-dependent incorporation of radiolabel into the substrate is measured. By "substrate," one means any substance containing a suitable hydroxyl moiety that acts as an acceptor for the γ-phosphate group transferred from a donor molecule such as ATP in a reaction catalyzed by PAK. A substrate may be an endogenous substrate of PAK, *i.e.* a naturally-occurring substance that is phosphorylated in unmodified cells by naturally-occurring PAK and/or any other substance that is not normally phosphorylated by PAK in a physiological situation, by that may be phosphorylated by PAK in the reaction conditions employed. A substrate may be a protein or peptide, and the phosphorylation reaction may occur on a substrate serine and/or threonine residue. It is well-known to those skilled in the art that non-natural substrates can act as suitable substrates in kinase assays such as that described above.

It is well known to those skilled in the art that detection of kinase-dependent substrate phosphorylation can be effected by a number of means other than measurement of radiolabeled phosphate incorporation into the substrate. For example, incorporation of phosphate groups can affect physicochemical properties of the substrate, such as electrophoretic mobility, light absorbance, fluorescence and/or phosphorescence, chromatographic properties, *etc.* Such alterations of substrate physicochemical properties can be readily measured by one skilled in the art and used as an indicator of kinase activity.

Alternatively or additionally, it is well known that monoclonal or polyclonal antibodies can be generated which selectively recognize phosphorylated forms of the substrate, and thus the degree of binding of such antibodies to substrate subsequent to the kinase reaction may be used as an indirect method of determining kinase activity. Furthermore, it is known that many kinases, including PAK kinases, possess the capacity to phosphorylated residues on the same kinase molecule. Such phosphorylation reactions are termed autophosphorylation, and therefore measurement of incorporation of phosphate into PAK itself catalyzed by the same may be used to monitor PAK activity. Kinase assays such as those described above may be performed using purified, partially recombinant PAK, and/or PAK which is purified from cells that naturally express the protein using purification procedures such as those described above.

ELISA-based assays may be used to screen for novel PAK substrates. One such assay employs random biotinylated peptides that can be phosphorylated by a kinase, such as PAK. PAK-specific antibodies are immobilized in the wells of a microtiter dish, and samples comprising PAK protein may be diluted into a reaction buffer and subsequently added to plate wells. Reactions are initiated, for example, by the addition of the biotinylated peptide substrates to the PAK sample. Reactions are stopped by removing the mixtures, and then the plates are washed. After washing, streptavidin-horseradish peroxidase (HRP) is added. Thereafter, unbound streptavidin-HRP is removed, the peroxidase color reaction is initiated by addition of the peroxidase substrate, and the optical density is measured in a suitable densitometer.

In some embodiments, a candidate substance suspected of modulating the binding between FMRP and PAK is a candidate substance suspected of modulating the phosphorylation of FMRP by PAK. For example, the candidate substance may be a substance suspected of promoting the phosphorylation of FMRP by PAK. In some embodiments, a candidate substance suspected of modulating the phosphorylation of FMRP by PAK pertains to a candidate substance suspected of inhibiting the phosphorylation of FMRP by PAK.

Alternatively or additionally, PAK activity may be measured by assaying its kinase activity, its ability to interact with its natural binding partners, and/or by the detection of events that lead to and/or are consequent of PAK activity in intact cells, in cell lysates, and/or in systems in which signaling events are reconstituted *in vitro.* For example, it is known that PAK proteins bind to and are activated by GTP-binding proteins such as Rac and/or Cdc42. Thus detection of interaction of PAK with naturally-occurring activators such as Rac/Cdc42, and/or substrates may be used as an indicator of PAK activity.

In some embodiments, a candidate substance is determined to be a PAK inhibitor if administering the candidate substance to PAK and a substrate results in decreased ability of PAK to phosphorylate the substrate. In some embodiments, a candidate substance is determined to be a PAK inhibitor if administering the candidate substance to PAK and the substrate results in an at least 2-fold decrease in the ability of PAK to phosphorylate the substrate. In some embodiments, a candidate substance is determined to be a PAK inhibitor if administering the candidate substance to PAK and the substrate results in an at least 3-fold, at least 4-fold, at least 5-fold, at least 10-fold, at least 20-fold, at least 50-fold, at least 100-fold, at least 200-fold, at least 500-fold, at least 1000-fold, at least 10,000-fold, or greater than 10,000-fold decrease in the ability of PAK to phosphorylate the substrate.

In some embodiments, a candidate substance is determined to be a PAK activator if administering the candidate substance to PAK and a substrate results in increased ability of PAK to phosphorylate the substrate. In some embodiments, a candidate substance is determined to be a PAK activator if administering the candidate substance to PAK and the substrate results in an at least 2-fold increase in the ability of PAK to phosphorylate the substrate. In some embodiments, a candidate substance is determined to be a PAK activator if administering the candidate substance to PAK and the substrate results in an at least 3-fold, at least 4-fold, at least 5-fold, at least 10-fold, at least 20-fold, at least 50-fold, at least 100-fold, at least 200-fold, at least 500-fold, at least 1000-fold, at least 10,000-fold, or greater than 10,000-fold increase in the ability of PAK to phosphorylate the substrate.

### In Cyto Assays

In some embodiments, the present invention provides methods for screening for PAK modulators wherein a candidate substance is contacted with a cell. The cell can then be assayed for various parameters associated with PAK activity. For example, parameters associated with PAK activity include, but are not limited to, PAK's ability to interact with its natural binding partners (*e.g.* FMRP) and/or PAK's ability to phosphorylate its substrates.

In certain embodiments, cells may be directly assayed for binding between FMRP and PAK. Immunohistochemical techniques, confocal techniques, and/or other techniques to assess binding are well known to those of skill in the art. In some embodiments, a cell is assayed for phosphorylation of FMRP by PAK. Various cell lines may be utilized for such screening assays, including cells specifically engineered for this purpose. Examples of cells used in the screening assays include neuronal cells and/or dendritic cells. The cell may be a stimulated cell, such as a cell stimulated with a growth factor. One of skill in the art would understand that the invention disclosed herein contemplates a wide variety of *in cyto* assays for measuring parameters that correlate with the activity of PAK.

Depending on the assay, cell and/or tissue culture may be required. A cell may be examined using any of a number of different physiologic assays, as discussed above for binding between FMRP and PAK. Alternatively or additionally, molecular analysis may be performed, including, but not limited to, western blotting to monitor protein expression and/or test for protein-protein interactions; northern blotting, differential display of RNA, and/or microarray analysis to monitor RNA expression; kinase assays to monitor phosphorylation; mass spectrometry to monitor other chemical modifications; *etc.*

The present invention provides methods for identifying substances that bind to PAK and, therefore, may modulate PAK activity. One *in cyto* method of identifying substances that bind to PAK is the two-hybrid system assay (Fields *et al.,* 1994, *Trends in Genetics,* 10:286; and Colas *et al.,* 1998, *TIBTECH,* 16:355; both of which are incorporated herein by reference). In this assay, yeast cells express a first fusion protein comprising a test substance in accordance with the present invention (*e.g.* PAK protein, PAK gene, and/or a characteristic portion thereof) and a DNA-binding domain of a transcription factor such as Gal4 and/or LexA. The cells additionally contain a reporter gene whose promoter contains binding sites for the corresponding DNA-binding domain. By transforming the cells with a vector that expresses a second fusion protein comprising a candidate substance fused to an activation domain (*e.g.* from Gal4 and/or herpes simplex virus VP16) expression of the reporter gene may be increased if the candidate substance interacts with the test substance. Consequently this assay may be used for screening for substances that modulate an interaction between PAK and any number of candidate substances. In this way, it is possible rapidly to identify novel PAK modulators.

The present invention provides assays involving solid phase-bound PAK proteins and detecting their interactions with one or more candidate substances. Thus, a test substance (*e.g.* PAK protein and/or a characteristic portion thereof) may contain a detectable marker, such as a radioactive, fluorescent, and/or luminescent label. Furthermore, candidate substances can be coupled to substances which permit indirect detection *(e.g.* by means of employing an enzyme which uses a chromogenic substrate and/or by means of binding a detectable antibody). Changes in the conformation of PAK as the result of an interaction with a candidate substance may be detected, for example, by the change in the emission of the detectable marker. Alternatively or additionally, the solid phase-bound protein complexes may be analyzed by means of mass spectrometry.

The present invention provides assays involving monitoring activities of downstream effectors of PAK. For example, the invention provides assays involving monitoring the activity of ERK pathway members and/or PI3K pathway members. Kinase activity can be assayed using any method known in the art. To give but one example, kinase activity can be assayed by using phospho-specific antibodies, as the phosphorylation state of members of these pathways indicates whether the protein is active. For example, a Western blot can be conducted for phosphoERK1/2, and quantification of changes in pathway activity can be obtained by normalization to total ERK1/2 levels. Alternatively or additionally, the activity of downstream members of the ERK signaling pathway (*e.g.* translation initiation factors S6, eIF4E, 4EBP1, *etc.)* can also be determined with Western blots probed with phospho-specific antibodies against these proteins (Kelleher *et al.,* 2004, *Cell,* 116:467; incorporated herein by reference). In some embodiments, PI3K pathways activity can be monitored through detection of phosphorylated PI3K or downstream signaling proteins Akt and PDK1.

In some embodiments, screening assays may assay PAK activity by monitoring the downstream cellular effects of PAK activity. Such effects include, but are not limited to, the formation of peripheral actin microspikes and/or associated loss of stress fibers (Zhao et al., 1998, Mol. Cell Biol., 18:2153; incorporated herein by reference) and/or other cellular responses, such as growth, growth arrest, differentiation, and/or apoptosis. In some embodiments, yeast cells are used for this type of screening assay. For example, in a PAK yeast functional assay, when cultured on glucose containing medium, PAK yeast cells grow like normal yeast cells. Upon exposure to galactose, however, intracellular expression of PAK is induced, causing yeast cells to die. Substances that inhibit PAK activity are identified by their ability to prevent yeast cells from dying.

In some embodiments, PAK levels are determined by measuring levels of protein and/or mRNA. Levels of PAK protein and/or characteristic portions thereof are measured using immunoassays such as western blotting and/or ELISA using antibodies that selectively bind to PAK. For measurement of mRNA, amplification (*e.g.*, using polymerase chain reaction [PCR], ligase chain reaction [LCR], *etc.)* and/or hybridization assays (*e.g.*, northern hybridization, RNAse protection, dot blotting, *etc.)* may be used. In some embodiments, levels of protein and/or mRNA can be detected using directly- and/or indirectly-labeled detection agents, *e.g.*, fluorescently and/or radioactively labeled nucleic acids, radioactively and/or enzymatically labeled antibodies, *etc.* as described herein.

Alternatively or additionally, PAK expression may be measured using a reporter gene system. Such a system may be devised using a PAK protein promoter operably-linked to a reporter gene such as chloramphenicol acetyltransferase, firefly luciferase, bacterial luciferase, O-galactosidase, alkaline phosphatase, *etc.* Furthermore, PAK may be used as an indirect reporter via attachment to a second reporter such as red and/or green fluorescent protein (see, *e.g.,* Mistili et al., 1997, Nature Biotech., 15:961; incorporated herein by reference). The reporter construct is typically transfected into a cell. After treatment with a candidate substance, the amount of reporter gene transcription, translation, and/or activity is measured according to standard techniques known to those of skill in the art.

In some embodiments, the present invention provides methods to determine whether PAK can induce phosphorylation of FMRP *in vivo.* In such methods, cells are transfected with vectors expressing constitutively active-PAK and/or FMRP. Cell extracts are prepared at specified time points after transfection, and the degree of phosphorylation of FMRP is analyzed by western blot analysis.

### In vivo Assays

*In vivo* assays involve the use of various animal models, including transgenic animals that have been engineered to have specific defects and/or carry markers that can be used to measure the ability of a candidate substance to reach and/or affect different cells within the organism. Due to their size, ease of handling, and/or information on their physiology and/or genetic makeup, mice are often used, especially for transgenics. However, other animals are suitable as well, including rats, rabbits, hamsters, guinea pigs, gerbils, woodchucks, cats, dogs, sheep, goats, pigs, cows, horses and/or monkeys (including chimps, gibbons and/or baboons). Assays for PAK modulators may be conducted using an animal model derived from any of these species and/or other useful species not listed herein.

In such assays, one or more candidate substances are administered to an animal, and the ability of the candidate substance(s) to alter one or more characteristics, as compared to a control animal not treated with the candidate substance(s), is determined. The characteristics may be any of those discussed herein with regard to the symptoms associated with FXS (*e.g.*, behavioral symptoms, abnormal synaptic function, improperly-formed dendritic spines, *etc.).*

The present invention provides methods of screening for candidate substances that may treat, alleviate, ameliorate, relieve, delay onset of, inhibit progression of, reduce severity of, and/or reduce incidence of one or more symptoms or features of FXS and/or other neurodevelopmental disorder. In some embodiments, a candidate substance comprises a PAK modulator. Treatment of these animals with candidate substances may involve the administration of the substance, in an appropriate form, to the animal. Administration is discussed in further detail below, in the section entitled *"Administration."*

Accordingly, in some embodiments, the invention provides screening systems, including methods and/or compositions, for determining whether a candidate substance is useful for treating, alleviating, ameliorating, relieving, delaying onset of, inhibiting progression of, reducing severity of, and/or reducing incidence of one or more symptoms or features of a FXS in a mammal. In some embodiments, a PAK modulator is identified that can be used to treat, alleviate, ameliorate, relieve, delay onset of, inhibit progression of, reduce severity of, and/or reduce incidence of one or more symptoms or features of FXS and/or other neurodevelopmental disorder.

In some embodiments, screening systems of the present invention may involve the use of the *FMR1* knockout *(FMR1* KO) mouse. *FMR1* KO mice and FXS patients show similar behavioral phenotypes and/or similar abnormalities in synaptic morphology in the brain. Their brains have more dendritic spines and/or a higher proportion of longer and/or thinner spines compared to normal individuals. Furthermore, they display abnormal synaptic function, such as enhanced long-term depression (LTD) mediated by metabotropic glutamate receptor in the hippocampus and/or impaired long-term potentiation (LTP) in the cortex. Therefore, the *FMR1* KO mouse serves as an accurate model of FXS, and the *FMR1* KO mouse exhibits several quantifiable phenotypes that are useful in the screening methods of the present invention. Such phenotypes include behavior, synaptic morphology, and/or synaptic function.

In specific embodiments, the present invention provides methods for identifying PAK modulators that may be utilized for treatment of FXS and/or other neurodevelopmental disorders comprising steps of (1) providing an *FMR* KO mouse exhibiting symptoms of FXS and/or other neurodevelopmental disorders, (2) administering a candidate substance to the mouse, and (3) measuring the effect(s) of the candidate substance on the symptoms of FXS and/or other neurodevelopmental disorder.

In some embodiments, screening methods of the present invention can measure behavioral symptoms. Such behavioral symptoms can include hyperactivity, stereotypy, perseverative behavior, anxiety, hypo-anxiety, seizure, impaired social behavior, and/or cognitive delay. In some embodiments, behavioral symptoms can be measured using an open field test. In an open field test, a subject is allowed to run freely in an open arena (*e.g.* VersaMax activity monitor chamber from Accuscan Instruments) and certain behaviors are analyzed. These behaviors include, but are not limited to: (1) hyperactivity, determined by measuring the distance and/or length of time traveled by the subject; (2) stereotypy, determined by measuring the number of repetitive behaviors exhibited by the subject; (3) hypo-anxiety, determined by measuring the amount of time the subject remains in the center field relative to the time spent in the corners of the field; and/or (4) combinations of these.

In some embodiments, behavioral symptoms can be measured using a trace fear conditioning task. For example, a subject is placed in a training chamber (Chamber A), where a tone is sounded, followed by a blank time (also called trace), and then shock. The sequence is repeated several times to let the subject learn the association between tone and shock across the time gap. To examine whether the subject remembers this association, at various time point after conditioning, the subject is placed into a new chamber (Chamber B) with a different shape and smell from Chamber A and its response to the tone is monitored. If the subject learns and remembers that tone is associated with shock, it will become immobile (called "freezing"). Previous studies have shown that attention-distracting stimuli and/or lesion of prefrontal cortex and/or hippocampus can interfere with trace fear conditioning. Thus, the subject's attention and/or associative memory may be measured by comparing the degree of freezing both pre- and post-conditioning, often relative to a control subject.
In some embodiments, relevant behavior tests can include eight-arm maze test and/or sensitization test to amphetamine-induced stereotypy for stereotypy and perseverative behavior. In some embodiments, relevant behavior tests can include elevated plus maze, light-dark transition, and/or novelty suppressed feeding test for anxiety, audiogenic seizure, social interaction, and/or social learning for social behaviors. In some embodiments, relevant behavior tests can include Morris water maze (including the reversal version) and/or fear conditioning for learning and memory.

In some embodiments, behavioral symptoms can be measured using an audiogenic seizure (AGS) assay. Fragile X humans and mice are susceptible to seizures at early ages. Fragile X mice show a robust phenotype in an AGS assay. While no 19 - 21 day old (p19-21) wildtype mice typically have seizures in this task, the majority of fragile X mice do have seizures. AGS can be performed essentially as described (Yan et al., 2005, Neuropharmacol., 49:1053; incorporated herein by reference). Briefly, mice are habituated to a behavioral chamber and then exposed to a high intensity siren of frequency peak 1800 Hz - 6300 Hz at an average sound pressure level above 120 dB at approximately 10 cm for 5 minutes. Behaviors of the mice can be monitored after administration of sound. Fragile X mice typically (1) run wildly, (2) have seizures, and/or (3) die. Wildtype mice typically do not exhibit these responses. An AGS phenotype is scored based on the animal's endpoint.

In some embodiments, behavioral symptoms can be measured using any of several social interaction tests. In some embodiments, home cage behavior and social interaction are assessed with a variety of tests (Kwon et al., 2006, Neuron, 50:377; Spencer et al., 2005, Genes Brain Behav., 4:420; and Lijam et al., 1997, Cell, 90:895; all of which are incorporated herein by reference). For example, mice can be observed in their home cage by videorecording and scored for various nonsocial behaviors and/or social behaviors (*e.g.* grooming, mounting, tail pulling, and sniffing).

In some embodiments, direct social interaction is assessed by exposing mice to a novel conspecific mouse and observing approaching and sniffing behaviors. Percent of time spent interacting can be recorded. This is typically repeated about 3 days later with the same mice. Control mice usually exhibit a decrease in social interaction the second time, indicating recognition of the familiar mouse and/or normal social learning. *FMR* KO mice typically do not exhibit a decrease in social interaction the second time, indicating impaired social learning, memory, and/or behavior. This test can be conducted in a novel or familiar environment, as significant differences between *FMR* KO and wild-type mice have been observed in social interaction assays depending on the degree of familiarity with the environment. Additional social behaviors can be monitored in this assay including active behaviors (*e.g.* aggressive attacks, lateral threats, and/or chasing) and passive behaviors (*e.g.* receiving sniffing from other mouse and/or not showing signs of submissive and/or defensive behavior).

Prior to an indirect social interaction test mice can be housed individually for 4 days. Indirect social interaction tasks typically take place in a cage divided in half by a clear perforated partition. The task is run in two different modes: one involves a familiar environment, by pre-exposure to the testing chamber, while the other involves a novel environment. In some embodiments, test mice are exposed to novel or familiar mice. Typically, *FMR* KO mice behave similarly to wild-type mice in a novel environment, but behave significantly differently in a familiar cage. Time spent at the partition can be recorded in 2 to 5 minute intervals for a 20 minute test. *FMR* KO mice tend to spend significantly less time interacting (i.e. at the partition) in the first few minutes and take longer to first approach the partition than wild-type mice. In contrast, *FMR* KO mice usually spend more time at the partition during the last time intervals than controls.

In some experiments, the indirect social interaction test is conducted in a chamber divided into three rooms. The central room - which is connected to two rooms independent from each other, one on the left and one on the right - is empty. The left room contains an empty cage (*i*.*e.* a novel object and/or inanimate target), while the right room contains a similar cage enclosing a novel mouse (i.e. a social target). This task involves a choice between spending time with a social target or an inanimate target, and therefore is called a social preference test. Percent of total time interacting with each obj ect can be recorded. The present invention encompasses the recognition that *FMR* KO mice may spend a significantly different amount of time (*e.g.* more or less) interacting with the social target than control mice.

In some embodiments, a social dominance tube test is conducted in a tube approximately 30 cm long and 3 cm - 4 cm in diameter. Mice are placed at opposite ends of the tube and released simultaneously. A mouse is pronounced the "winner" when his opponent backs out completely. *FMR1* KO mice win significantly fewer matches against unfamiliar wild-type mice than expected by chance. When a *FMR1* KO mouse competes against a wild-type non-cagemate, the wild-type mouse is the winner in approximately 73% of matches (Kwon et al., 2006, Neuron, 50:377; Spencer et al., 2005, Genes Brain Behav., 4:420; and Lijam et al., 1997, Cell, 90:895; all of which are incorporated herein by reference). The present invention encompasses the recognition that modulation of PAK activity via administration of a PAK modulator may ameliorate this phenotype.

Since *FMR1* KO mice have been shown to display some abnormal social behaviors, the present invention encompasses the recognition that home cage social behavior, including nest building and sleeping behavior, may be altered in *FMR1* KO mice. Nesting patterns are evaluated by placing a cotton nestle into a cage of approximately two, three, or four mice of the same genotype (*e.g.* wild-type, *FMR1* KO, *etc*.) that receive identical drug treatments. After about 30 minutes to 1 hour, the nest can be removed and the height measured. Wild-type mice build nests with depths that average 20 mm to 50 mm. Mice which display abnormal social behavior, such as *FMR1* KO mice, frequently build shallower nests *(e.g.* < 20 mm).

In some embodiments, sleeping positions of mice in their home cages can be recorded two to four times a day over five consecutive days. Wild-type mice sleep huddled in the well-formed, fluffy nests they build. Observations will determine whether *FMR1* KO mice sleep in scattered, random patterns, do not build full nests, or sleep on top of intact nestle material. The present invention encompasses the recognition that, if *FMR1* KO mice display abnormal phenotypes, amelioration of those phenotypes may occur following modulation of PAK activity.

In some embodiments, behavioral symptoms such as hippocampus-dependent spatial learning are assessed using the classical Morris Water Maze test. *FMR1* KO mice, just like the wild-type controls, learn to find the visible or hidden platform with decreasing latency scores over the course of the standard training protocol. However, some groups observe an abnormal phenotype in a reversal trial, a test in which the platform is transferred to the quadrant opposite the initial training quadrant. In particular, *FMR1* KO mice display increased escape latency and path length, suggesting that they have low response flexibility or high memory interference (see, *e.g.* D'Hooge et al., 1997, Neuroscience, 76:367; incorporated herein by reference).

In some embodiments, hippocampal synaptic plasticity is assessed using a standard protocol for mGluR-LTD (see, *e.g.* Example 5 for a more detailed protocol). Hippocampal slices can be prepared from *FMR* KO mice and control littermates per standard procedures. All experiments are generally performed blind to genotype. Schaffer collaterals are stimulated and extracellular field potentials measured in *str*. *radiatum* of CA1. mGluR-dependent LTD is elicited both electrophysiologically (by pairs of stimuli delivered at 1 Hz for 15 minutes to 20 minutes; "PP-LFS") and pharmacologically (by bath application of 50 µM to 100 µM 3,4-dihydroxyphenylglycine (DHPG) for 5 minutes). The initial slope of the field potential is recorded as an indicator of synaptic strength. Hippocampal slices from *FMR1* KO mice show enhanced mGluR-LTD relative to control mice (see, *e.g.*, Huber et al., 2002, Proc. Natl. Acad. Sci., USA, 99:7746; and Nosyreva and Huber, 2006, J. Neurophysiol., 95:3291; both of which are incorporated herein by reference).

Cortical long-term potentiation (LTP) is reduced in slices from *FMR1* KO mice (see Figure 14). Coronol brain slices containing temporal cortex are prepared from two- to three-month-old male littermates, and left to recover for at least 1 hour before recording in oxygenated (95% O₂ and 5% CO₂) warm (30 °C) artificial cerebrospinal fluid containing 124 mM NaCl, 5 mM KCl, 1.25 mM NaH₂PO₄, 1 mM MgCl₂, 2 mM CaCl₂, 26 mM NaHCO₃, 10 mM dextrose. Field potentials (FPs) in layer II/III evoked by layer IV stimulation are measured as previously described and responses are quantified as the amplitude of FP in cortex. LTP is induced by TBS, which consisted of eight brief bursts (each with 4 pulses at 100 Hz) of stimuli delivered every 200 msec. Genetic inhibition of PAK rescues the reduced cortical LTP in the *FMR1* KO mice, and the present invention encompasses the recognition that pharmacological inhibition may have the same effect (Hayashi et al., 2007, Proc. Natl. Acad. Sci., USA, 104:11489; incorporated herein by reference).

In some embodiments, screening methods of the present invention involve measurement of synaptic morphology. In some embodiments, the number of dendritic spines is counted. In some embodiments, spine number is examined in Golgi-stained layer II/III pyramidal neurons of the temporal cortex. For example, serial brain sections can be obtained following the Golgi-Cox technique. Layer II/III pyramidal neurons in the temporal cortex are visualized by microscopy (*e.g.* under Olympus upright BX61 with motorized XY stage using Neurolucida/ stereology software [Microbrightfield]). On each primary apical dendritic branch, regularly-sized segments (*e.g.* ten consecutive 10-µm-long dendritic segments) are analyzed to quantify spine density (*e.g.* the number of spines per 10 µm long dendritic segment). In some embodiments, length and/or width of dendritic spines is measured. In some embodiments, spine length is measured from Golgi-stained neurons.

In some embodiments, dendritic spine head size can be examined by electron microscopic analysis of the length of the postsynaptic density (PSD). In some embodiments, the dendritic spine heads can be examined by conducting electron microscopic analyses of the proportion of larger, perforated synapses. For example, for electron microscopy, subjects are anaesthetized and perfused. Blocks of temporal cortex are embedded, from which sections (*e.g.* 1 µm thick) are cut and stained with toluidine blue *(e.g.* diluted to 1%) to guide the further trimming to isolate layer II/III of temporal cortex. Ultrathin sections (*e.g.* 90 nm) are then cut and stained with uracyl acetate and lead citrate. Randomly selected neuropil areas are photographed *(e.g.* at a 10,000X magnification with a JEOL 1200EX electron microscope) and image negatives are scanned and analyzed.

In some embodiments, neurons can be labeled by fluorescent proteins (directly or via indirect staining) in an animal and/or in culture established from an animal. Fluorescent microscopy can be used to visualize neurons and measure the number, length, and/or size of spines. In some embodiments, the size of presynaptic terminals is measured. In some embodiments, the size of presynaptic terminals is examined by conducting electron microscopic analysis of the number of synaptic vesicles and/or docked vesicles. In some embodiments, presynaptic terminals are labeled by fluorescent proteins and fluorescent microscopy is used to visualize the presynaptic terminals and measure their size.

In certain embodiments, screening methods of the present invention involve measuring synaptic function. In some embodiments, long-term depression (LTD) mediated by metabotropic glutamate receptor (mGluR) can be measured in the hippocampus. For example, hippocampal slices are prepared and allowed to recover before recording in oxygenated artificial cerebrospinal fluid (ACSF). Field potentials (FPs) in stratum radiatum of area CA1 are evoked by a current pulse to Schaffer collateral axons. Stable baseline responses are regularly collected by a stimulation intensity (10 µA - 30 µA) yielding 50% - 60% of the maximal response. mGluR-LTD can be induced by application of mGluR agonist 3,5-dihydroxyphenylglycine (DHPG; *e.g.* at 100 µM) and/or by using paired-pulse low-frequency stimulation consisting of 900 pairs of stimuli delivered at 1 Hz in the presence of the N-methyl-D-aspartate receptor (NMDAR) antagonist D-(-)-2-amino-5-phosphono-pentanoic acid (D-APV; *e.g.* at 50 µM). DHPG is a chiral compound, and mGluR-LTD can be induced by application of RS-DHPG and S-DHPG, but typically not by application ofR-DHPG.

In some embodiments, long-term potentiation (LTP) can be measured in the cortex. For example, coronal brain slices containing cortex are prepared and left to recover before recording in oxygenated ACSF. FPs in layer II/III evoked by layer IV stimulation are measured and responses are quantified as the amplitude of FP. Cortical LTP can be induced by theta-burst stimulation (TBS), which consists of brief bursts of stimuli delivered at regular intervals (*e.g.* eight brief bursts, each with 4 pulses at 100 Hz, of stimuli delivered every 200 msec).

In some embodiments, synaptic currents mediated by α-amino-3-hydroxy-5-methylisoxazole-4- propionic acid receptors (AMPARs) and/or NMDARs can be measured in the cortex. For example, for measurement of AMPAR-mediated miniature excitatory postsynaptic current (mEPSC), tetrodotoxin, APV, and bicuculline are added in a bath of ACSF. Continuous traces *(e.g.* 30 msec - 60 msec) are collected at regular intervals (*e.g.* every 8 seconds) and filtered (*e.g.* at 2 KHz). Cells with series resistance above a certain threshhold level (*e.g.* ≥13 mΩ) are discarded. The measurement of NMDAR-EPSC/ AMPAR-EPSC ratio is typically done in ACSF containing Mg²⁺, bicuculline, and glycine. NMDAR-dependent and AMPAR-dependent responses are discriminated based on their distinct kinetics and voltage dependence. Thus, NMDAR-mediated response is valued as the currents recorded at +40 mV and measured 100 msec after the response onset. The AMPAR-mediated response is taken from the peak amplitude response recorded at -80 mV.

In some embodiments, the present invention provides methods for identifying PAK modulators that can be utilized to treat FXS and/or other neurodevelopmental disorders comprising steps of: (1) providing an *FMR* KO mouse exhibiting symptoms of FXS and/or other neurodevelopmental disorders, (2) administering a candidate substance to the mouse, and (3) measuring the effect(s) of the candidate substance on PAK mRNA and/or protein.

In some embodiments, screening methods of the present invention involve measuring the interaction between PAK and its natural binding partners. The candidate substance is determined to treat, alleviate, ameliorate, relieve, delay onset of, inhibit progression of, reduce severity of, and/or reduce incidence of one or more symptoms or features of FXS and/or other neurodevelopmental disorder if the candidate substance modulates the interaction between PAK and its natural binding partners. In certain embodiments, the candidate substance is determined to treat, alleviate, ameliorate, relieve, delay onset of, inhibit progression of, reduce severity of, and/or reduce incidence of one or more symptoms or features of FXS and/or other neurodevelopmental disorder if the candidate substance modulates the interaction between PAK and FMRP. The interaction between PAK and its natural binding partners may be measured using standard methods, which are described herein and in Sambrook et al. (Molecular Cloning: A Laboratory Manual, 3rd ed., Cold Spring Harbor Laboratory Press, Cold Spring Harbor, N.Y., 2001; incorporated herein by reference).

In some embodiments, screening methods of the present invention involve measuring PAK kinase activity in a cell, tissue, and/or mammal in the presence and/or absence of the candidate substance. A candidate substance is determined to treat, alleviate, ameliorate, relieve, delay onset of, inhibit progression of, reduce severity of, and/or reduce incidence of one or more symptoms or features of FXS and/or other neurodevelopmental disorder if the candidate substance modulates PAK kinase activity. PAK kinase activity may be measured using standard methods, which are described herein and in Sambrook et al. (Molecular Cloning: A Laboratory Manual, 3rd ed., Cold Spring Harbor Laboratory Press, Cold Spring Harbor, N.Y., 2001; incorporated herein by reference).

In some embodiments, the invention provides screening methods for determining whether a candidate substance is useful for treating, alleviating, ameliorating, relieving, delaying onset of, inhibiting progression of, reducing severity of, and/or reducing incidence of one or more symptoms or features of a FXS and/or other neurodevelopmental disorder in a mammal. This method involves measuring the phosphorylation level of FMRP in a cell, tissue, and/or mammal in the presence and/or absence of the candidate substance. The candidate substance is determined to treat, alleviate, ameliorate, relieve, delay onset of, inhibit progression of, reduce severity of, and/or reduce incidence of one or more symptoms or features of FXS and/or other neurodevelopmental disorder if the candidate substance decreases the phosphorylation level of FMRP.

In some embodiments, screening methods of the present invention involve measuring PAK mRNA and/or protein levels in a cell, tissue, and/or mammal in the presence and/or absence of the candidate substance. The candidate substance is determined to treat, alleviate, ameliorate, relieve, delay onset of, inhibit progression of, reduce severity of, and/or reduce incidence of one or more symptoms or features of FXS and/or other neurodevelopmental disorder if the substance modulates PAK mRNA and/or protein levels. PAK mRNA and/or protein levels may be measured using standard methods, which are described herein and in Sambrook et al. (Molecular Cloning: A Laboratory Manual, 3rd ed., Cold Spring Harbor Laboratory Press, Cold Spring Harbor, N.Y., 2001; incorporated herein by reference).

In some embodiments, a strain of *C*. *elegans* is utilized which is characterized by a phenotype of sterility and/or embryonic lethality and/or a defective gonad migration and which harbors an impaired and/or missing PAK function. The strain of *C*. *elegans* utilized by the present invention could be obtainable and/or could be obtained by one or more of the methods for generating a *C*. *elegans* having said phenotypes. Such a model may be used, amongst other things, for characterizing the signaling pathways linked to PAK. Such a model may be used for identifying substances that interfere and/or interact with one or more proteins that are part of a signaling pathway linked to PAK. Such a model may be used for identification of a PAK modulator using any of the *in vivo* assays described herein.

In some embodiments, other animal models for FXS and/or other neurodevelopmental disorders can be utilized. For example, a *D*. *melanogaster* FXS model could be utilized in accordance with the present invention. A *Drosophila* FXS model was established in 2001 (Zhang et al., 2001, Cell, 107:591; incorporated herein by reference) to provide evolutionary perspective on conserved FMRP functions and to allow genetic studies of molecular and cellular functions in the context of a relatively simple brain Zhang et al. , 2001, Cell, 107:591; Dockendorff et al., 2002, Neuron, 34:973; Morales et al., 2002, Neuron, 34:961; Lee et al., 2003, Development, 130:5543; Michel et al., 2004, J. Neurosci., 24:5798; and Pan et al., 2004, Curr. Biol., 14:1863; all of which arc incorporated herein by reference). In *Drosophila,* loss of dFMRP causes significant changes in neuronal structural morphogenesis and circuit formation (Michel et al., 2004, J. Neurosci., 24:5798; and Pan et al., 2004, Curr. Biol., 14:1863; both of which are incorporated herein by reference) and synaptic differentiation and neurotransmission properties (Zhang et al., 2001, Cell, 107:591; and Pan et al., 2004, Curr. Biol., 14:1863; both of which are incorporate herein by reference) and alterations in behavioral output (Zhang et al., 2001, Cell, 107:591; Dockendorff et al., 2002, Neuron, 34:973; Morales et al, 2002, Neuron, 34:961; all of which are incorporated herein by reference) comparable to the human disease. To establish molecular bases for these cellular and behavioral phenotypes, studies were carried out using this model to identify proteins that arc misregulated in the absence of dFMRP. Several such proteins were identified using this model system (Zhang et al., 2005, Mol. Cell. Proteomics, 4:278; incorporated herein by reference). Such a model may be used, amongst other things, for characterizing the signaling pathways linked to PAK. Such a model may be used for identifying substances that interfere and/or interact with one or more proteins that are part of a signaling pathway linked to PAK. Such a model may be used for identification of a PAK modulator using any of the *in vivo* assays described herein.

The FXS model systems described above are merely exemplary model systems that can be used in accordance the present invention. One of ordinary skill will recognize that the present invention is not limited to these particular in vivo models, but that any in vivo model in which expression of FMRP is disrupted may serve as a model system for FXS and/or other developmental disorders. In some embodiments, any in vivo model in which the organism displays one or more symptoms of FXS may serve as a model system for FXS and/or other neurodevelopmental disorders.

Determining the effectiveness of a candidate substance *in vivo* may involve a variety of different criteria. Measuring toxicity and/or dose response may be performed in subjects in a more meaningful fashion than in *in nitro* and/or *in cyto* assays.

### Methods of Use

The present invention provides methods of treating FXS and/or other neurodevelopmental disorders (*e.g*. POF, FXTAS, various forms of mental retardation, and/or autism spectrum disorders (ASD)). In some embodiments, the present invention provides methods of treating POF, FXTAS, various forms of mental retardation, and/or autism spectrum disorders (ASD). In certain embodiments, such methods involve modulating PAK activity. In some embodiments, PAK activity is modulated in any manner described herein. In some embodiments, PAK activity is inhibited. PAK activity may be inhibited by disrupting its ability to interact with FMRP and/or to phosphorylate FMRP. In some embodiments, PAK activity is activated.

In some embodiments, the invention provides pharmaceutical compositions comprising at least one PAK modulator and at least one pharmaceutically acceptable excipient. In other aspects, a pharmaceutical composition comprising a candidate substance is administered to a cell, such as one in a patient suffering from and/or susceptible to FXS S and/or other neurodevelopmental disorders.

In some embodiments, PAK modulators of the present invention are used in the treatment of abnormal synaptic formation. In certain embodiments, abnormal synaptic formation is caused by FXS and/or other neurodevelopmental disorders. In specific embodiments, inventive PAK modulators are used in the treatment of improperly-formed dendritic spines. In some embodiments, dendritic spines are characterized as "improperly-formed" when an individual possesses abnormally high numbers of dendritic spines. In some embodiments, dendritic spines are characterized as "improperly-formed" when an individual possesses spines that are abnormally long and/or thin. In some embodiments, improperly-formed dendritic spines are caused by FXS and/or other neurodevelopmental disorders.

In some embodiments, PAK modulators of the present invention are used in the treatment of abnormal synaptic function. In certain embodiments, abnormal synaptic function is caused by FXS and/or other neurodevelopmental disorders. In specific embodiments, inventive PAK modulators are used in the treatment of enhanced long-term depression (LTD) mediated by metabotropic glutamate receptor in the hippocampus and/or impaired long-term potentiation (LTP) in the cortex. In some embodiments, defective enhanced long-term depression (LTD) mediated by metabotropic glutamate receptor in the hippocampus and/or impaired long-term potentiation (LTP) in the cortex are caused by FXS and/or other neurodevelopmental disorders.

In some embodiments, PAK modulators of the present invention are used in the treatment of one or more of the following symptoms: hyperactivity, stereotypy, anxiety, seizure, impaired social behavior, and/or cognitive delay. In certain embodiments, these symptoms are caused by FXS and/or other neurodevelopmental disorders.

In some embodiments, the present invention provides a method of treating FXS and/or other neurodevelopmental disorders comprising steps of (1) providing a patient exhibiting symptoms of FXS and/or other neurodevelopmental disorders, and (2) administering a therapeutic amount of one or more PAK modulators to the patient. In some embodiments, the present invention provides a method of treating FXS and/or other neurodevelopmental disorders comprising steps of (1) providing a patient suffering from FXS and/or other neurodevelopmental disorders, and (2) administering a therapeutic amount of one or more PAK modulators to the patient. In some embodiments, the present invention provides a method of treating FXS and/or other neurodevelopmental disorders comprising steps of (1) providing a patient susceptible to FXS and/or other neurodevelopmental disorders, and (2) administering a therapeutic amount of one or more PAK modulators to the patient.

In some embodiments, the present invention provides methods of treating FXS and/or other neurodevelopmental disorders comprising steps of (1) providing a patient exhibiting symptoms of, suffering from, and/or susceptible to FXS and/or other neurodevelopmental disorders, and (2) administering a substance that modulates the ability of PAK to interact with its natural binding partners.

In some embodiments, the present invention provides methods of treating FXS and/or other neurodevelopmental disorders comprising steps of (1) providing a patient exhibiting symptoms of, suffering from, and/or susceptible to FXS and/or other neurodevelopmental disorders, and (2) administering a substance that inhibits and/or disrupts the binding of PAK and FMRP.

In certain embodiments, the present invention provides methods of treating FXS and/or other neurodevelopmental disorders comprising steps of (1) providing a patient exhibiting symptoms of, suffering from, and/or susceptible to FXS and/or other neurodevelopmental disorders, and (2) administering a substance that modulates the kinase activity of PAK.

In some embodiments, the present invention provides methods of treating FXS and/or other neurodevelopmental disorders comprising steps of (1) providing a patient exhibiting symptoms of, suffering from, and/or susceptible to FXS and/or other neurodevelopmental disorders, and (2) administering a substance that modulates ability of PAK to phosphorylate FMRP.

In some embodiments, the present invention provides methods of treating FXS and/or other neurodevelopmental disorders comprising steps of (1) providing a patient exhibiting symptoms of FXS and/or other neurodevelopmental disorders, and (2) administering a substance that modulates the levels of PAK RNA and/or protein.

In some embodiments, inventive PAK modulators may be used to treat diseases, conditions, and/or disorders other than FXS. For example, some *FMR1* mutations do not cause FXS, but instead cause other disorders. "Alleles" of the *FMR1* gene are defined by the number of CGG repeats and/or by the methylation status of the *FMR1* locus. *FMR1* normally has between 7-52 CGG repeats (most often 30 repeats) located in the 5' untranslated region of exon 1. These sequences are normally unmethylated, but methylation can occur at each C of CG dinucleotide repeats, which silences expression of *FMR1.*

A "full mutation" is characterized by > 200 CGG repeats that are methylated. All males and 50% of females possessing the full mutation will develop FXS. In contrast, a "premutation" is characterized by 55 - 200 CGG repeats that are not methylated. These individuals are not likely to develop FXS, but females exhibit increased twinning and/or an increased occurrence of premature ovarian failure (POF).

Furthermore, the premutation may lead to fragile X-associated tremor ataxia (FXTAS), especially in males. FXTAS is characterized by intention tremor, ataxia, cognitive decline, and/or brain atrophy and is thought to be related to RNA levels.

The premutation may lead to cognitive effects. For example, a premutation carrier's IQ may be within the average range, but is likely to be on lower end of the average range. There is evidence of socio-emotional effects, including increased shyness and/or social anxiety.

Thus, improper expression and/or activity of the *FMR1* gene may lead to diseases, disorders, and/or conditions other than FXS. In some embodiments, inventive PAK modulators may be used in the treatment of any disease, condition, and/or disorder caused by improper expression and/or function of the *FMR1* gene.

### Pharmaceutical compositions

The present invention provides PAK modulators used in the treatment of FXS and/or other neurodevelopmental disorders. In some embodiments, the present invention provides pharmaceutical compositions comprising PAK modulators and at least one phamaceutically acceptable excipient. The present invention provides pharmaceutical compositions comprising a therapeutically effective amount of inventive PAK modulators) appropriately formulated for administration to a subject suffering from and/or susceptible to FXS and/or other neurodevelopmental disorders. Such pharmaceutical compositions may optionally comprise one or more additional therapeutically-active substances.

In accordance with some embodiments, methods of treating FXS and/or other neurodevelopmental disorders are provided. In some embodiments, inventive methods comprise administering a pharmaceutical composition comprising at least one PAK modulator of the present invention to a subject in need thereof. Inventive PAK modulators may be administered with other medications used to treat the symptoms of FXS. In some embodiments, the compositions are administered to humans.

The invention encompasses the preparation and/or use of pharmaceutical compositions comprising a PAK modulator for treatment of FXS and/or other neurodevelopmental disorders as an active ingredient. Such a pharmaceutical composition may consist of the active ingredient alone, in a form suitable for administration to a subject, or the pharmaceutical composition may comprise the active ingredient and one or more pharmaceutically acceptable excipients, one or more additional ingredients, and/or a combination of these. The active ingredient may be present in the pharmaceutical composition in the form of a physiologically acceptable ester and/or salt, such as in combination with a physiologically acceptable cation and/or anion, as is well known in the art.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation. Patients to which administration of the pharmaceutical compositions of the invention is contemplated include, but are not limited to, humans and/or other primates; mammals, including commercially relevant mammals such as cattle, pigs, horses, sheep, cats, and/or dogs; and/or birds, including commercially relevant birds such as chickens, ducks, geese, and/or turkeys.

Pharmaceutical compositions as described herein may be prepared by any method known or hereafter developed in the art of pharmaceutics. In general, such preparatory methods include the step of bringing the active ingredient into association with one or more excipients and/or one or more other accessory ingredients, and then, if necessary and/or desirable, shaping and/or packaging the product into a desired single- or multi-dose unit.

A pharmaceutical composition of the invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

The relative amounts of the active ingredient, the pharmaceutically acceptable excipient(s), and/or any additional ingredients in a pharmaceutical composition of the invention will vary, depending upon the identity, size, and/or condition of the subject treated and further depending upon the route by which the composition is to be administered. By way of example, the composition may comprise between 0.1% and 100% (w/w) active ingredient.

Pharmaceutical formulations of the present invention may additionally comprise a pharmaceutically acceptable excipient, which, as used herein, includes any and all solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro, (Lippincott, Williams & Wilkins, Baltimore, MD, 2006) discloses various excipients used in formulating pharmaceutical compositions and known techniques for the preparation thereof Except insofar as any conventional excipient is incompatible with a substance or its derivatives, such as by producing any undesirable biological effect or otherwise interacting in a deleterious manner with any other component(s) of the pharmaceutical composition, its use is contemplated to be within the scope of this invention.

In some embodiments, the pharmaceutically acceptable excipient is at least 95%, 96%, 97%, 98%, 99%, or 100% pure. In some embodiments, the excipient is approved for use in humans and for veterinary use. In some embodiments, the excipient is approved by United States Food and Drug Administration. In some embodiments, the excipient is pharmaceutical grade. In some embodiments, the excipient meets the standards of the United States Pharmacopoeia (USP), the European Pharmacopoeia (EP), the British Pharmacopoeia, and/or the International Pharmacopoeia.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in the inventive formulations. Excipients such as cocoa butter and suppository waxes, coloring agents, coating agents, sweetening, flavoring, and perfuming agents can be present in the composition, according to the judgment of the formulator.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, *etc.,* and combinations thereof

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linlced poly(vinyl-pynolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (VEEGUM), sodium lauryl sulfate, quaternary ammonium compounds, *etc*., and combinations thereof.

Exemplary surface active agents and/or emulsifiers include, but are not limited to, natural emulsifiers (*e.g*. acacia, agar, alginic acid, sodium alginate, tragacanth, chondrux, cholesterol, xanthan, pectin, gelatin, egg yolk, casein, wool fat, cholesterol, wax, and lecithin), colloidal clays (*e.g*. bentonite [aluminum silicate] and VEEGUM [magnesium aluminum silicate]), long chain amino acid derivatives, high molecular weight alcohols (*e.g*. stearyl alcohol, cetyl alcohol, oleyl alcohol, triacetin monostearate, ethylene glycol distearate, glyceryl monostearate, and propylene glycol monostearate, polyvinyl alcohol), carbomers (e.g. carboxy polymethylene, polyacrylic acid, acrylic acid polymer, and carboxyvinyl polymer), carrageenan, cellulosic derivatives (*e.g*. carboxymethylcellulose sodium, powdered cellulose, hydroxymethyl cellulose, hydroxypropyl cellulose, hydroxypropyl methylcellulose, methylcellulose), sorbitan fatty acid esters (*e.g*. polyoxyethylene sorbitan monolaurate [TWEEN®20], polyoxyethylene sorbitan [TWEEN®60], polyoxyethylene sorbitan monooleate [TWEEN®80], sorbitan monopalmitate [SPAN®40], sorbitan monostearate [SPAN^{®}60], sorbitan tristearate [SPAN^{®}65], glyceryl monooleate, sorbitan monooleate [SPAN^{®}80]), polyoxyethylene esters (*e.g*. polyoxyethylene monostearate [MYRJ^{®}45], polyoxyethylene hydrogenated castor oil, polyethoxylated castor oil, polyoxymethylene stearate, and SOLUTOL), sucrose fatty acid esters, polyethylene glycol fatty acid esters (*e.g*. CREMOPHOR), polyoxyethylene ethers, (*e.g*. polyoxyethylene lauryl ether [BRIJ^{®}30]), poly(vinyl-pyrrolidone), diethylene glycol monolaurate, triethanolamine oleate, sodium oleate, potassium oleate, ethyl oleate, oleic acid, ethyl laurate, sodium lauryl sulfate, PLURONIC^{®}F 68, POLOXAMER 188, cetrimonium bromide, cetylpyridinium chloride, benzalkonium chloride, docusate sodium, *etc*. and/or combinations thereof.

Exemplary binding agents include, but are not limited to, starch (*e.g*. cornstarch and starch paste); gelatin; sugars (*e.g*. sucrose, glucose, dextrose, dextrin, molasses, lactose, lactitol, mannitol,); natural and synthetic gums (*e.g*. acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, poly(vinylpyrrolidone), magnesium aluminum silicate (VEEGUM), and larch arabogalactan); alginates; polyethylene oxide; polyethylene glycol; inorganic calcium salts; silicic acid; polymethacrylates; waxes; water; alcohol; *etc.;* and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfte, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal. Exemplary antifungal preservatives include, but are not limited to, butyl paraben, methyl paraben, ethyl paraben, propyl paraben, benzoic acid, hydroxybenzoic acid, potassium benzoate, potassium sorbate, sodium benzoate, sodium propionate, and sorbic acid. Exemplary alcohol preservatives include, but are not limited to, ethanol, polyethylene glycol, phenol, phenolic compounds, bisphenol, chlorobutanol, hydroxybenzoate, and phenylethyl alcohol. Exemplary acidic preservatives include, but arc not limited to, vitamin A, vitamin C, vitamin E, beta-carotene, citric acid, acetic acid, dehydroacetic acid, ascorbic acid, sorbic acid, and phytic acid. Other preservatives include, but are not limited to, tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, GLYDANT PLUS^{®}, PHENONIP^{®}, methylparaben, GERMALL^{®}115, GERMABEN^{®}II, NEOLONE^{™}, KATHON, and EUXYL. In certain embodiments, the preservative is an anti-oxidant. In other embodiments, the preservative is chelating agent.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, *etc.,* and combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, *etc*., and combinations thereof.

Exemplary oils include, but are not limited to, almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavaladin, lavender, lemon, litsea cubeba, macademia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and combinations thereof.

Liquid dosage forms for oral and parenteral administration include, but are not limited to, pharmaceutically acceptable emulsions, microemulsions, solutions, suspensions, syrups and elixirs. In addition to the active ingredient(s), liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, oral compositions can include adjuvants such as wetting agents, emulsifying and suspending agents, sweetening, flavoring, and perfuming agents. In certain embodiments for parenteral administration, an active ingredient can be mixed with solubilizing agents such as CREMOPHOR, alcohols, oils, modified oils, glycols, polysorbates, cyclodextrins, polymers, and combinations thereof.

Injectable preparations, for example, sterile injectable aqueous or oleaginous suspensions may be formulated according to the known art using suitable dispersing or wetting agents and suspending agents. A sterile injectable preparation may be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or di-glycerides. In addition, fatty acids such as oleic acid are used in the preparation of injectables.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Compositions for rectal or vaginal administration are typically suppositories which can be prepared by mixing the active ingredients of this invention with suitable nonirritating excipients such as cocoa butter, polyethylene glycol, or a suppository wax which are solid at ambient temperature but liquid at body temperature and therefore melt in the rectum or vaginal cavity and release the active ingredient.

Solid dosage forms for oral administration include capsules, tablets, pills, powders, and granules. In such solid dosage forms, the active ingredient is mixed with at least one inert, pharmaceutically acceptable excipient such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidinone, sucrose, and acacia, (c) humectants such as glycerol, (d) disintegrating agents such as agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and/or (i) lubricants such as talc, calcium stearate, magnesium stearate, solid polyethylene glycols, sodium lauryl sulfate, and mixtures thereof. In the case of capsules, tablets and pills, the dosage form may comprise buffering agents.

Solid compositions of a similar type may be employed as fillers in soft and hard-filled gelatin capsules using such excipients as lactose or milk sugar as well as high molecular weight polyethylene glycols and the like. Solid dosage forms of tablets, dragees, capsules, pills, and granules can be prepared with coatings and shells such as enteric coatings and other coatings well known in the pharmaceutical formulating art. They may optionally comprise opacifying agents and can be of a composition that they release the active ingredient(s) only, or preferentially, in a certain part of the intestinal tract, optionally, in a delayed manner. Examples of embedding compositions which can be used include polymeric substances and waxes. Solid compositions of a similar type may be employed as fillers in soft and hard-filled gelatin capsules using such excipients as lactose or milk sugar as well as high molecular weight polethylene glycols and the like.

Active ingredients can be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active ingredient may be admixed with at least one inert diluent such as sucrose, lactose or starch. Such dosage forms may comprise, as is normal practice, additional substances other than inert diluents, e.g., tableting lubricants and other tableting aids such a magnesium stearate and microcrystalline cellulose. In the case of capsules, tablets and pills, dosage forms may comprise buffering agents. They may optionally comprise opacifying agents and can be of a composition that they release the active ingredient(s) only, or preferentially, in a certain part of the intestinal tract, optionally, in a delayed manner. Examples of embedding compositions which can be used include polymeric substances and waxes.

Dosage forms for topical and/or transdermal administration of a composition of this invention may include ointments, pastes, creams, lotions, gels, powders, solutions, sprays, inhalants and/or patches. Generally, an active ingredient is admixed under sterile conditions with a pharmaceutically acceptable excipient and/or any needed preservatives and/or buffers as may be required. Additionally, the present invention contemplates the use of transdermal patches, which often have the added advantage of providing controlled delivery of an active ingredient to the body. Such dosage forms may be prepared, for example, by dissolving and/or dispensing the active ingredient in the proper medium. Alternatively or additionally, the rate may be controlled by either providing a rate controlling membrane and/or by dispersing the active ingredient in a polymer matrix and/or gel.

Suitable devices for use in delivering intradermal pharmaceutical compositions described herein include short needle devices such as those described in U.S. Patents 4,886,499; 5,190,521; 5,328,483; 5,527,288; 4,270,537; 5,015,235; 5,141,496; and 5,417,662. Intradermal compositions may be administered by devices which limit the effective penetration length of a needle into the skin, such as those described in PCT publication WO 99/34850 and functional equivalents thereof. Jet injection devices which deliver liquid vaccines to the dermis via a liquid jet injector and/or via a needle which pierces the stratum corneum and produces a jet which reaches the dermis are suitable. Jet injection devices are described, for example, in U.S. Patents 5,480,381; 5,599,302; 5,334,144; 5,993,412; 5,649,912; 5,569,189; 5,704,911; 5,383,851; 5,893,397; 5,466,220; 5,339,163; 5,312,335; 5,503,627; 5,064,413; 5,520,639; 4,596,556; 4,790,824; 4,941,880; 4,940,460; and PCT publications WO 97/37705 and WO 97/13537. Ballistic powder/particle delivery devices which use compressed gas to accelerate vaccine in powder form through the outer layers of the skin to the dermis are suitable. Alternatively or additionally, conventional syringes may be used in the classical mantoux method of intradermal administration.

Formulations suitable for topical administration include, but are not limited to, liquid and/or semi liquid preparations such as liniments, lotions, oil in water and/or water in oil emulsions such as creams, ointments and/or pastes, and/or solutions and/or suspensions. Topically-administrable formulations may, for example, comprise from about 1.0% to about 10% (w/w) active ingredient, although the concentration of the active ingredient may be as high as the solubility limit of the active ingredient in the solvent. Formulations for topical administration may further comprise one or more of the excipients and/or additional ingredients described herein.

A pharmaceutical composition of the invention may be prepared, packaged, and/or sold in a formulation suitable for pulmonary administration via the buccal cavity. Such a formulation may comprise dry particles which comprise the active ingredient and which have a diameter in the range from about 0.5 µm to about 7 µm or from about 1 µm to about 6 µm. Such compositions are conveniently in the form of dry powders for administration using a device comprising a dry powder reservoir to which a stream of propellant may be directed to disperse the powder and/or using a self propelling solvent/powder dispensing container such as a device comprising the active ingredient dissolved and/or suspended in a low-boiling propellant in a sealed container. Such powders comprise particles wherein at least 98% of the particles by weight have a diameter greater than 0.5 µm and at least 95% of the particles by number have a diameter less than 7 µm. Alternatively, at least 95% of the particles by weight have a diameter greater than 1 µm and at least 90% of the particles by number have a diameter less than 6 µm. Dry powder compositions may include a solid fine powder diluent such as sugar and are conveniently provided in a unit dose form.

Low boiling propellants generally include liquid propellants having a boiling point of below 65 °F at atmospheric pressure. Generally the propellant may constitute 50% to 99.9% (w/w) of the composition, and the active ingredient may constitute 0.1% to 20% (w/w) of the composition. The propellant may further comprise additional ingredients such as a liquid non-ionic and/or solid anionic surfactant and/or a solid diluent (which may have a particle size of the same order as particles comprising the active ingredient).

Pharmaceutical compositions of the invention formulated for pulmonary delivery may provide the active ingredient in the form of droplets of a solution and/or suspension. Such formulations may be prepared, packaged, and/or sold as aqueous and/or dilute alcoholic solutions and/or suspensions, optionally sterile, comprising the active ingredient, and may conveniently be administered using any nebulization and/or atomization device. Such formulations may further comprise one or more additional ingredients including, but not limited to, a flavoring agent such as saccharin sodium, a volatile oil, a buffering agent, a surface active agent, and/or a preservative such as methylhydroxybenzoate. Droplets provided by this route of administration may have an average diameter in the range from about 0.1 µm to about 200 µm.

Formulations described herein as being useful for pulmonary delivery are useful for intranasal delivery of a pharmaceutical composition of the invention. Another formulation suitable for intranasal administration is a coarse powder comprising the active ingredient and having an average particle from about 0.2 µm to 500 µm. Such a formulation is administered in the manner in which snuff is taken, *i.e*. by rapid inhalation through the nasal passage from a container of the powder held close to the nares.

Formulations suitable for nasal administration may, for example, comprise from about as little as 0.1% (w/w) and as much as 100% (w/w) of the active ingredient, and may comprise one or more of the excipients and/or additional ingredients described herein. A pharmaceutical composition of the invention may be prepared, packaged, and/or sold in a formulation suitable for buccal administration. Such formulations may, for example, be in the form of tablets and/or lozenges made using conventional methods, and may contain, for example, 0.1% to 20% (w/w) active ingredient, the balance comprising an orally dissolvable and/or degradable composition and, optionally, one or more of the excipients and/or additional ingredients described herein. Alternately, formulations suitable for buccal administration may comprise a powder and/or an aerosolized and/or atomized solution and/or suspension comprising the active ingredient. Such powdered, aerosolized, and/or aerosolized formulations, when dispersed, may have an average particle and/or droplet size in the range from about 0.1 µm to about 200 µm, and may further comprise one or more of the excipients and/or additional ingredients described herein.

A pharmaceutical composition of the invention may be prepared, packaged, and/or sold in a formulation suitable for ophthalmic administration. Such formulations may, for example, be in the form of eye drops including, for example, a 0.1%/1.0% (w/w) solution and/or suspension of the active ingredient in an aqueous or oily liquid excipient. Such drops may further comprise buffering agents, salts, and/or one or more other of the excipients and/or additional ingredients described herein. Other opthalmically-administrable formulations which are useful include those which comprise the active ingredient in microcrystalline form and/or in a liposomal preparation. Ear drops and/or eye drops are contemplated as being within the scope of this invention.

General considerations in the formulation and/or manufacture of pharmaceutical agents may be found, for example, in Remington: The Science and Practice of Pharmacy 21st ed., Lippincott Williams & Wilkins, 2005 (incorporated herein by reference).

### Administration

Inventive PAK modulators are useful in the treatment of FXS and/or other neurodevelopmental disorders. Thus, pharmaceutical compositions containing one or more inventive PAK modulators may be administered to one or more individuals suffering from, susceptible to, and/or exhibiting symptoms of FXS. The present invention therefore encompasses methods of modulating PAK activity, as well as methods of treating FXS and/or other neurodevelopmental disorders in subjects.

In some embodiments, a therapeutically effective amount of a pharmaceutical composition comprising a PAK modulator is delivered to a subject and/or organism prior to, simultaneously with, and/or after diagnosis with FXS and/or other neurodevelopmental disorder. In some embodiments, a therapeutic amount of a pharmaceutical composition is delivered to a patient and/or organism prior to, simultaneously with, and/or after onset of symptoms of FXS and/or other neurodevelopmental disorder. In some embodiments, the amount of pharmaceutical composition is sufficient to treat, alleviate, ameliorate, relieve, delay onset of, inhibit progression of, reduce severity of, and/or reduce incidence of one or more symptoms or features of FXS and/or other neurodevelopmental disorder.

Pharmaceutical compositions, according to the method of the present invention, may be administered using any amount and any route of administration effective for treatment. The exact amount required will vary from subject to subject, depending on the species, age, and general condition of the subject, the seventy of the infection, the particular composition, its mode of administration, its mode of activity, and the like. Compositions of the invention are typically formulated in dosage unit form for ease of administration and uniformity of dosage. It will be understood, however, that the total daily usage of the compositions of the present invention will be decided by the attending physician within the scope of sound medical judgment. The specific therapeutically effective dose level for any particular subject or organism will depend upon a variety of factors including the disorder being treated and the severity of the disorder; the activity of the specific active ingredient employed; the specific composition employed; the age, body weight, general health, sex and diet of the subject; the time of administration, route of administration, and rate of excretion of the specific active ingredient employed; the duration of the treatment; drugs used in combination or coincidental with the specific active ingredient employed; and like factors well known in the medical arts.

Pharmaceutical compositions of the present invention may be administered by any route. In some embodiments, pharmaceutical compositions of the present invention are administered by a variety of routes, including oral, intravenous, intramuscular, intra-arterial, intramedullary, intrathecal, parenteral, subcutaneous, intraventricular, transdermal, interdermal, rectal, intravaginal, intraperitoneal, topical (*e.g*. by powders, ointments, creams, gels, and/or drops), transdennal, mucosal, nasal, buccal, enteral, sublingual; by intratracheal instillation, bronchial instillation, and/or inhalation; and/or as an oral spray, nasal spray, and/or aerosol. In some embodiments, inventive compositions are administered intravenously. In some embodiments, inventive compositions are administered orally. In some embodiments, inventive compositions are administered using a continuous IV drip. In some embodiments, inventive compositions are administered by retro-orbital injection.

In some embodiments, inventive compositions can be delivered using a pump. In some embodiments, a pump to be used in accordance with the invention is an external pump. In some embodiments, a pump to be used in accordance with the invention is a pump that is implanted within the body of a subject. In some embodiments, a pump to be used in accordance with the invention is a mechanical pump and/or an osmotic pump.

In general the most appropriate route of administration will depend upon a variety of factors including the nature of the composition (*e.g*., its stability in the environment of the gastrointestinal tract), the condition of the subject (*e.g*., whether the subject is able to tolerate oral administration), *etc*.

In certain embodiments, a composition may be administered in amounts ranging from about 0.001 mg/kg to about 100 mg/kg, from about 0.01 mg/kg to about 50 mg/kg, from about 0.1 mg/kg to about 40 mg/kg, from about 0.5 mg/kg to about 30 mg/kg, from about 0.01 mg/kg to about 10 mg/kg, from about 0.1 mg/kg to about 10 mg/kg, or from about 1 mg/kg to about 25 mg/kg, of subject body weight per day, one or more times a day, to obtain the desired therapeutic effect. The desired dosage may be delivered three times a day, two times a day, once a day, every other day, every third day, every week, every two weeks, every three weeks, or every four weeks. In certain embodiments, the desired dosage may be delivered using multiple administrations (*e.g*., two, three, four, five, six, seven, eight, nine, ten, eleven, twelve, thirteen, fourteen, or more administrations).

It will be appreciated that therapeutic agents and pharmaceutical compositions of the present invention can be employed in combination therapies. In some embodiments, the present invention encompasses "therapeutic cocktails" comprising inventive compositions. The particular combination of therapies (therapeutics or procedures) to employ in a combination regimen will take into account compatibility of the desired therapeutics and/or procedures and the desired therapeutic effect to be achieved. It will be appreciated that the therapies employed may achieve a desired effect for the same purpose. For example, a PAK modulator may be administered with another agent that is used to treat symptoms of FXS and/or other neurodevelopmental disorder. In some embodiments, the therapies employed may achieve different effects (*e.g*., control of any adverse side effects).

Pharmaceutical compositions of the present invention may be administered either alone or in combination with one or more other therapeutic agents. By "in combination with," it is not intended to imply that the agents must be administered at the same time and/or formulated for delivery together, although these methods of delivery are within the scope of the invention. Compositions can be administered concurrently with, prior to, or subsequent to, one or more other desired therapeutics or medical procedures. In general, each agent will be administered at a dose and/or on a time schedule determined for that agent. Additionally, the invention encompasses the delivery of the inventive pharmaceutical compositions in combination with agents that may improve their bioavailability, reduce and/or modify their metabolism, inhibit their excretion, and/or modify their distribution within the body.

The particular combination of therapies (therapeutics and/or procedures) to employ in a combination regimen will take into account compatibility of the desired therapeutics and/or procedures and/or the desired therapeutic effect to be achieved. It will be appreciated that the therapies employed may achieve a desired effect for the same disorder (for example, an inventive agent may be administered concurrently with another therapeutic agent used to treat the same disorder), and/or they may achieve different effects (e.g., control of any adverse side effects). In some embodiments, compositions of the invention are administered with a second therapeutic agent that is approved by the U.S. Food and Drug Administration.

In will further be appreciated that therapeutically active agents utilized in combination may be administered together in a single composition or administered separately in different compositions.

In general, it is expected that agents utilized in combination with be utilized at levels that do not exceed the levels at which they are utilized individually. In some embodiments, the levels utilized in combination will be lower than those utilized individually.

The pharmaceutical compositions of the present invention may be administered alone and/or in combination with other agents that are used to treat the symptoms of FXS. In some embodiments, such agents may treat seizures and/or mood instability, including but not limited to as Carbamazepine (TEGRETOL^{®}), Valproic Acid, Divalproex (DEPAKOTE^{®}), Lithium Carbonate, Gabapentin (NEURONTIN^{®}), Lamotrigine (LAMICTAL^{®}), Topiramate (TOPAMAX^{®}), Tiagabine (GABITRIL^{®}), Vigabatrin (SABRIL^{®}), Phenobarbital, Primidone (MYSOUNE^{®}), and/or Phenytoin (DILANTIN^{®}).

In some embodiments, such agents may be central nervous system stimulants, including but not limited to Methylphenidate (RITALIN^{®}), Dextro amphetamine (DEXEDRINE^{®}; ADDERALL^{®}), Ditropan (CONCERTA^{®}), L-acetylcarnitine, Venlafaxine (EFFEXO^{®}), Nefazodone (SERZONE^{®}), Amantadine (SYMMETREL^{®}), Buproprion (WELLBUTRIN^{®}), Desipramine, Imipramine, and/or Buspirone (BUSPARI^{®}).

In some embodiments, such agents may be antihypertensive drugs, including but not limited to Clonidine (CATAPRES^{®}) and/or Guanfacine (TENEX^{®}).

In certain embodiments, such agents may include folic acid.

In some embodiments, such agents may be selected serotonin reuptake inhibitors, including but not limited to Fluoxetine (PROZAC^{®}), Sertraline (ZOLOFT^{®}), and/or Citalopram (CELEXA^{®}).

In some embodiments, such agents may be antipsychotics, including but not limited to Risperidone (RISPERIDAL^{®}), Olanzepine (ZYPREXA^{®}), and/or Quetiapine (SEROQUEL^{®}).

In certain embodiments, such agents may be used to treat sleep disturbances, including but not limited to Desyrel (TRAZODONE^{®}) and/or Melatonin.

In will further be appreciated that therapeutically active agents utilized in combination may be administered together in a single composition or administered separately in different compositions.

In some embodiments, inventive PAK modulators may be administered in combination with one or more other PAK modulators.

One of ordinary skill in the art will understand that the examples presented above are not meant to be limiting. The principles presented in the examples above can be generally applied to any combination therapies for treatment of FXS and/or other neurodevelopmental disorders.

### Kits

The invention provides a variety of kits comprising one or more of PAK modulators of the invention. For example, the invention provides kits comprising at least one PAK modulator and instructions for use. A kit may comprise multiple different PAK modulators. A kit may comprise any of a number of additional components or reagents in any combination. All of the various combinations are not set forth explicitly but each combination is included in the scope of the invention.

According to certain embodiments of the invention, a kit may include, for example, (i) a PAK modulator; (ii) instructions for administering the PAK modulator to a subject suffering from, susceptible to, and/or exhibiting symptoms of FXS and/or other neurodevelopmental disorder.

According to certain embodiments of the invention, a kit for identifying PAK modulators may include, for example, (i) an *FMR* KO mouse; (ii) at least one candidate substance (*e.g*. small molecule library, collection of peptides, *etc*.); (iii) a positive control (*e.g*. known PAK modulator); (iv) a negative control (*e.g*. a substance known not to be a PAK modulator); (v) pharmaceutically acceptable excipients and equipment necessary to prepare candidate substances and controls for administration to the mouse; and (vii) instructions for administering the candidate substance to the mouse in order to determine whether it treats, alleviates, ameliorates, relieves, delays onset of, inhibits progression of, reduces severity of, and/or reduces incidence of one or more symptoms or features of FXS and/or other neurodevelopmental disorder in the mouse.

Kits typically include instructions for use of inventive PAK modulators. Instructions may, for example, comprise protocols and/or describe conditions for identification of PAK modulators, administration of PAK modulators to a subject in need thereof, *etc.* Kits generally include one or more vessels or containers so that some or all of the individual components and reagents may be separately housed. Kits may also include a means for enclosing individual containers in relatively close confinement for commercial sale, *e.g.,* a plastic box, in which instructions, packaging materials such as styrofoam, *etc*., may be enclosed. An identifier, *e.g*., a bar code, radio frequency identification (ID) tag, *etc*., may be present in or on the kit or in or one or more of the vessels or containers included in the kit. An identifier can be used, *e.g*., to uniquely identify the kit for purposes of quality control, inventory control, tracking, movement between workstations, *etc.*

### Exemplification

The representative Examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following Examples contain important additional information, exemplification and guidance that can be adapted to the practice of this invention in its various embodiments and the equivalents thereof. It will be appreciated, however, that these examples do not limit the invention. Variations of the invention, now known and/or further developed, are considered to fall within the scope of the present invention as described herein and as hereinafter claimed.

Previous studies have consistently associated mental retardation with abnormalities in the number and size of synapses. In FXS patients and in *FMR1* KO mice, cortical neurons display more postsynaptic dendritic spines and a higher proportion of longer and thinner spines compared to normal individuals. The *FMR1* KO mice exhibit impaired cortical LTP compared to wild-type mice. Strikingly, the abnormalities in cortical synaptic morphology and plasticity of *FMR1* KO mice are opposite to those we observed in transgenic (*dnPAK* TG) mice in which activity of p21-activated kinase (PAK) is inhibited by its dominant negative form (dnPAK), specifically in the postnatal forebrain. In the *dnPAK* TG mice, we found that cortical neurons display fewer dendritic spines and a lower proportion of longer and thinner spines compared to wild type mice (Hayashi et al., 2004, Neuron, 42:773; incorporated herein by reference). These transgenic mice exhibit enhanced cortical LTP, in contrast to the impaired cortical LTP in *FMR1* KO mice. One hypothesis that may explain these results is that signaling pathways mediated by PAK and FMR1 may antagonize each other to regulate synaptic morphology and function, and this hypothesis was tested in the following examples.

### Example 1: PAK inhibition rescues spine morphological abnormalities in FMR1 KO mice.

### Materials and Methods

### Golgi Analysis

Following the Golgi-Cox technique (Ramon-Moliner, 1970, Contemporary Research Methods in Neuroanatomy, Springer, Berlin, Heidelberg, New York), 120 µm thick serial sections were obtained from brains of two-month-old male littermates. Slides containing these sections were coded before quantitative analysis, and the code was broken only after the analysis was completed. Layer II/III pyramidal neurons in the temporal cortex were visualized under Olympus upright BX61 with motorized XY stage using Neurolucida/ stereology software (Microbrightfield). On each primary apical dendritic branch, ten consecutive 10 µm-long dendritic segments were analyzed to quantify spine density. To ensure sampling consistency among Golgi analysis and electrophysiology experiments, analyses in the temporal cortex were all carried out in slices or sections corresponding to Figure 62 - 67 of the mouse brain atlas (Franklin et al., The mouse brain in stereotaxic coordinates, Academic, San Diego, CA, 1997).

### Electron Microscopy

Two-month-old male littermates were anaesthetized and perfused according to standard procedures. Blocks of temporal cortex were then embedded, from which 1 µm thick sections were cut and stained with 1% toluidine blue to guide the further trimming to isolate layer II/III of temporal cortex. 90 nm ultrathin sections were then cut and stained with uracyl acetate and lead citrate. Randomly selected neuropil areas were photographed at a 10,000X magnification with a JEOL 1200EX electron microscopy. Image negatives were scanned at 1200 dpi and analyzed by OpenLab Program (Improvision). Excitatory synapses bearing spines were defined by the presence of a clear post-synaptic density (PSD) facing at least three presynaptic vesicles. Micrographs covering 500 µm² - 1000 µm² neuropil regions from each mouse were analyzed and used for quantitation. PSD length and percentage of perforated synapses were quantified from the same population of synapses. The measurements were all performed by an experimenter blind to the genotype.

### Results

Since spine abnormality is a pathological hallmark in FXS patients and *FMR1* KO mice at the cellular level, dendritic spine morphology was examined by measuring spine density in apical dendrites of Golgi-stained layer II/III pyramidal neurons of the temporal cortex of dMT mice as well as their littermates, *dnPAK* TG, *FMR1* KO, and wild-type mice. The number of spines per 10 µm of dendritic segments that run proximal to distal to the neuronal soma was quantified. In proximal dendritic segments, spine density was lower in *dnPAK* TG mice compared to wild-type mice while it was higher in *FMR1* KO mice compared to wild-type mice (Figures 10 and 11). In contrast, spine density in dMT mice was comparable to that in wild-type controls in all dendritic segments except segments 7 and 8 (Figure 11). When averaged over all segments, mean spine density in dMT mice was significantly lower than that in *FMR1* KO mice and significantly higher than that in *dnPAK* TG mice (Figure 12). These results indicate that PAK inhibition partially restores the abnormality of spine density in *FMR1* KO mice.

In addition to in increased spine density, cortical neurons from FXS patients and *FMR1* KO mice exhibit increased spine length (Hinton et al., 1991, Am. J. Med. Genet., 41:289; Comery et al., 1997, Proc. Natl. Acad. Sci., USA, 94:5401; Irwin et al., 2001, Am. J. Med. Genet., 98:161; and McKinney et al., 2005, Am. J. Med. Genet. B. Neuropsychiatr. Genet., 136:98; all of which are incorporated herein by reference). To investigate whether dnPAK can also restore this abnormality, spine length (the radial distance from tip of spine head to dendritic shaft) of Golgi-stained pyramidal neurons in the four genotypes was measured. In cumulative frequency plots, *FMR1* KO neurons exhibited a significant shift in the overall spine distribution towards spines of longer length compared to wild-type neurons, while *dnPAK* TG neurons exhibited the opposite shift to shorter spines (Figure 13). In contrast, spine length distribution of dMT neurons overlapped well with that of wild-type neurons (Figure 13), indicating that PAK inhibition is sufficient to restore the cortical spine length abnormality in *FMR1* KO mice.

### Example 2: PAK Inhibition Rescues Reduced Cortical LTP in FMR1 KO Mice.

### Materials and Methods

### Electrophysiology

From three-month-old male littermates, coronal brain slices containing temporal cortex were prepared and left to recover for at least 1 hour before recording in oxygenated (95% O₂ and 5% CO₂) warm (30 °C) artificial cerebrospinal fluid containing 124 mM NaCl, 5 mM KCl, 1.25 mM NaH₂PO₄, 1 mM MgCl₂, 2 mM CaCl₂, 26 mM NaHCO₃, and 10 mM dextrose. Field potentials (FPs) in layer II/III evoked by layer IV stimulation were measured as previously described (Hayashi et al., 2004, Neuron 42:773) and responses were quantified as the amplitude of FP in cortex. LTD was induced by TBS, which consisted of eight brief bursts (each with 4 pulses at 100 Hz) of stimuli delivered every 200 msec.\

### Results

Cortical long-term potentiation (LTP) has been shown to be reduced in *FMR1* KO mice while it is enhanced in *dnPAK* TG mice (Li et al., 2002, Mol. Cell. Neurosci., 19:138; Zhao et al., 2005, J. Neurosci., 25:7385; and Hayashi et al., 2004, Neuron, 42:773; all of which are incorporated herein by reference). To assess the effect of PAK inhibition on the cortical synaptic transmission and plasticity in *FMR1* KO mice, extracellular field recordings in temporal cortex layer II/III synapses were carried out while stimulating layer IV. Basal synaptic transmission, as measured by field potential responses to a range of stimulus intensities, did not differ between the four genotypes (Figure 14A). However, as expected, administration of theta-burst stimulation (TBS) at 100 Hz produced LTP of a lower magnitude in *FMR1* KO mice than in wild-type mice and LTP of a higher magnitude in *dnPAK* TG than in wild-type mice (Figure 14B). In contrast, the magnitude of LTP was indistinguishable between dMT mice and wild-type controls at various times following the application of the stimulus (Figure 14B). This demonstrates that PAK inhibition rescues LTP defects in *FMR1* KO mice.

### Example 3: PAK inhibition rescues multiple behavioral defects in FMR1 KO mice.

### Materials and Methods

### Open Field Test

Two-month-old male littermates were subjected to the open field test according to standard procedures. Each mouse ran for 10 minutes in a VersaMax activity monitor chamber (Accuscan Instruments). Open field activity was detected by photobeam breaks and analyzed by the VersaMax software. Stereotypy is recorded when the mouse breaks the same beam (or set of beams) repeatedly. Stereotypy count is the number of beam breaks that occur during this period of sterotypic activity.

### Trace Fear Conditioning Task

Three-month-old male littermates were subjected to trace fear conditioning as previously described (Zhao et al., 2005, J. Neurosci., 25:7385). On day 1, mice were placed in the training chamber (Chamber A, Coulboum Instruments) for 60 seconds before the onset of a 15-second white noise tone (conditioned stimulus or CS). 30 seconds later, mice received a 1-second shock (0.7 mA intensity; unconditioned stimulus or US). Thus, one trial is composed of tone (CS), 30 seconds blank time (also called "trace"), and then shock (US). Seven trials with an intertrial interval (ITI) of 210 seconds were performed to let the mice learn the association between tone and shock across a time gap. To examine whether mice remember this association, on day 2, mice were placed into a new chamber (Chamber B) with a different shape and smell from those in Chamber A. After 60 seconds, a 15-second tone was repeated seven times with an ITI of 210 seconds. Video images were digitized and the percentage of freezing time during each ITI was analyzed by Image FZ program (O'Hara & Co). Freezing was defmed as the absence of all but respiratory movement for a 1-second period.

### Results

### PAK inhibition rescues multiple behavioral defects in FMR1 KO mice

To test if the partial rescue of spine morphology and the complete rescue of cortical LTP by PAK inhibition could ameliorate behavioral deficits present in *FMR1* KO mice, the mice of various genotypes were subjected to a series of behavioral tasks. In an open field test where mice are placed in a box and allowed to run freely for ten minutes, *FMR1* KO mice exhibited three abnormal behaviors compared to wild-type mice (Peier *et al*., 2000, *Hum. Mol. Genet.,* 9:1145). (1) Hyperactivity: they traveled a longer distance and moved for a longer period of time (Figure 15); (2) Stereotypy: they exhibited a higher number of repetitive behaviors (Figure 15); and (3) Hypo-anxiety; they stayed in the center field for a longer period of time and in the corners of the field for a shorter period of time (Figure 15). In all three behaviors, dMT mice exhibited performance comparable to wild-type controls (Figure 15). This indicated that PAK inhibition in *FMR1* KO mice restores locomotion, repetitive behavior, and anxiety to wild-type levels.

To further examine whether PAK inhibition can rescue abnormal cortex-dependent behaviors, trace fear conditioning was performed, which is a test that depends on the integrity of the prefrontal cortex and is sensitive to attention-distracting stimuli (McEchron et al., 1998, Hippocampus, 8:638; and Han et al., 2003, Proc. Natl. Acad. Sci., USA, 100:13087; both of which are incorporated herein by reference). It was previously shown that *FMR1* KO mice are impaired in this form of conditioning, which may relate to the attention deficits in FXS patients (Zhao et al., 2005, J. Neurosci., 25:7385; incorporated herein by reference). In this task, a conditioning trial was composed of a tone (as the conditioned stimulus or CS), then a 30-second time gap (also called "trace") and finally an electric shock (as the unconditioned stimulus or US). Seven trials were given to allow the mice to learn the association between the tone and the shock across the 30-second time gap. Mice that learn and remember this association will become immobile (or "freeze") in response to the tone, even when they are placed into a new chamber with a different shape and smell compared to the training chamber. During training, the four genotypes exhibited comparable amounts of freezing in all conditioning trials (Figure 16), suggesting normal memory acquisition. However, when placed in a new chamber 24 hours after training, both *FMR1* KO mice and *dnPAK* TG mice exhibited a significant reduction in tone-induced freezing compared to wild-type controls (Figure 16), indicating an impaired trace fear memory in these two genotypes. dMT mice also showed freezing deficits during the first several tone sessions (sessions 1 to 4) compared to wild-type controls (Figure 16), although the deficits during these sessions were, on average, less pronounced compared to *dnPAK* TG or *FMR1* KO mice (Figure 17). However, with additional tone sessions (sessions 5 to 7), freezing by dMT caught up to that of wild-type while its difference from *FMR1* KO mice almost reached statistical significance (*p* = 0.07, Figure 17). Thus, dMT mice are slow in expressing the memory and/or require a repetition of the recall cue (tone), but they can eventually (after 5 tone sessions) recall the memory at the level that is not significantly different from the wild-type level.

### Example 4: PAK1 and FMRP physically interact

### Materials and Methods

### Animal Handling, Experimental Design, and Data Analysis

All strains of mice are of the C57B6 background. *FMR1* KO mice were obtained from Dr. Steven Warren. *dnPAK* TG mice were generated previously (Hayashi et al., 2004, Neuron, 42:773). Mouse maintenance and all experimental procedures were performed in compliance with National Institute of Health guidelines. All experiments were conducted in a blind fashion. Unless specified otherwise, data were analyzed with Statview software (SAS) using one-way ANOVA test followed by Fisher's protected least significance difference (PLSD) *post hoc* test. Values are presented as mean ± SEM.

### Immunoprecipitation and Western Blotting

Mouse brains were homogenized in ice-cold homogenization buffer (0.32 M sucrose; 10 mM Tris-HCl, pH 7.4; 5 mM EDTA; Complete Protease Inhibitor Cocktail Tablets (Roche)) and centrifuged at 1,000 x g for 10 minutes at 4 °C. The supernatant was collected and centrifuged at 21,000 x g for 15 minutes at 4 °C. The pellet was resuspended in TE buffer (10 mM Tris-HCl pH 7.4; 5 mM EDTA) and one-ninth volume of cold DOC buffer (500 mM Tris-HCl, pH 9.0; 10% sodium deoxycholate) was added. The mixture was incubated in a 37 °C water bath for 30 minutes while shaking and mixed with one-ninth volume of Buffer T (1% Triton X-100; 1% sodium deoxycholate; 500 mM Tris-HCl, pH 9.0). The membrane extract was dialyzed against binding/dialysis buffer (50 mM Tris-HCl, pH 7.4; 0.1% Triton X-100) at 4 °C overnight.

For immunoprecipitation, the dialyzed membrane extract was pre-cleared with protein A-sepharose beads, then incubated with α-PAK1 (N-20 from Santa Cruz Biotech) or control rabbit serum (Sigma) in binding/dialysis buffer for 3 hours, and then incubated with protein A-sepharose beads overnight at 4 °C. To test binding specificity, α-PAK1 was also incubated with its corresponding blocking peptide (Santa Cruz Biotech) prior to incubation with the membrane extract. The beads were washed three times with binding/dialysis buffer.

Proteins that bound to the beads were separated by SDS-PAGE, transferred to a nitrocellulose membrane, and subjected to western blot analysis. For PAK1 western blots, the membrane was blocked in 10% milk, then incubated with α-PAK1 antibody diluted at 1:1000, then incubated with α-rabbit horse radish peroxidase (HRP, Sigma) diluted at 1:1000, and then developed with enhanced chemiluminescence (ECL) Renaissance kit (New England Nuclear). For FMRP western blots, the membrane was processed with the Blast blotting amplification system (Perkin Elmer) with α-FMRP antibody (Chemicon) diluted at 1:1000, biotinylated α-mouse diluted at 1:1000, and streptavidin-HRP diluted at 1:1000.

### GST Pull-Downs

GEX6p-1 plasmid encoding GST was purchased from Pharmacia. GST-PAK1 plasmid was obtained from Dr. Joe Kissil (Kissil et al., 2003, Mol. Cell, 12:841; incorporated herein by reference). Plasmids encoding FMRP and its mutants were obtained from Dr. Edouard Khandjian (Mazroui et al., 2003, Hum. Mol. Genet., 12:3087; incorporated herein by reference). GST and GST-PAK1 proteins were expressed in BL21 *E. coli,* purified on glutathione sepharose 4B (GS4B) beads (Pharmacia), and dialyzed with PBS overnight. FMRP and its mutants were in vitro-translated with the TNT coupled reticulocyte lysate systems kit (Promega) and labeled with Transcend tRNA (Promega). GST or GST-PAK1 was incubated with FMRP or its mutants in binding buffer (50 mM Tris-HCl, pH 7.5; 120 mM NaCl; 10 mM MgCl₂, 5% glycerol; 1% Triton X-100) for 3 hours. GS4B beads were added and incubated for 1 hour. The beads were washed three times with the binding buffer. Proteins that bound to the beads were separated by SDS-PAGE, transferred to a nitrocellulose membrane, and subjected to western blot analysis. To detect in vitro-translated FMRP or its mutants, the membrane was blocked, incubated with streptavidin-HRP, washed, and developed with ECL Renaissance kit.

### Results

The morphological, electrophysiological and behavioral data presented in Examples 1 - 3 demonstrate that PAK inhibition rescues (at least partially) multiple abnormalities in *FMR1* KO mice. To begin to understand the underlying mechanism, it was determined whether PAK1 and FMRP physically interact via immunoprecipitation followed by Western blot analysis. Since PAK1 and FMRP are both localized in synapses (Weiler et al., 1997, Proc. Natl. Acad. Sci., USA, 94:5395; and Hayashi et al., 2004, Neuron, 42:773; both of which are incorporated herein by reference), synapse-enriched membrane extract was prepared from mouse brain and subjected the extract to immunoprecipitation with a PAK1 antibody (α-PAK1). Proteins that may co-precipitate through their direct or indirect interaction with PAK1 were separated by SDS-PAGE and subjected to Western blot analysis with an FMRP antibody. FMRP immuno-reactivity was observed in PAK1 immunoprecipitates but not in control serum immunoprecipitates (Figure 18). This interaction is specific because it did not occur when PAK1 antibody was pre-incubated with a blocking peptide, which competes with PAK1 for binding to the PAK1 antibody, prior to immunoprecipitation (Figure 18). This result shows that the endogenous PAK1 and FMRP interact, directly or indirectly, in the brain.

To examine whether PAK1 directly interacts with FMRP, a glutathione S-transferase (GST)-pull down assay was performed in which *in vitro*-translated FMRP was incubated with either GST or GST-tagged PAK1 (GST-PAK1). GST-PAK1, but not GST alone, bound to FMRP (Figures 19 and 20), suggesting a direct interaction between PAK1 and FMRP. FMRP contains a primary phosphorylation site at Ser 499 and three RNA-binding domains (KH1, KH2 and RGG) that are conserved among species (Figure 21; O'Donnell and Warren, 2002, Annu. Rev. Neurosci., 25:315; and Ceman et al., 2003, Hum. Mo/. Genet., 12:3295; both of which are incorporated herein by reference). To map the PAK1-binding region on FMRP, a series of deletion or point mutants of FMRP were used in the GST-pull down assay. An FMRP mutant without the RGG box (ΔRGG) or phosphorylation domain containing Ser 499 (ΔS499) was still able to bind to PAK1, whereas an FMRP mutant without KH domains (ΔKH) or with a point mutation in the KH2 domain previously found in a human with severe FXS (I304N; Feng et al., 1997, Mol. Cell, 1:109) was unable to bind to PAK1 (Figures 20 and 21). These results show that PAK1 directly binds to FMRP and this interaction requires the integrity of the KH domains of FMRP.

### Example 5: LTD Mediated by mGluR in the Hippocampus

Long-term depression (LTD) mediated by metabotropic glutamate receptor (mGluR) can be measured in the hippocampus. Hippocampal slices are prepared in ice-cold dissection buffer (212 mM sucrose, 2.6 mM KCl, 1.25 mM NaH₂PO₄, 26 mM NaHCO₃, 5 mM MgCl₂, 0.5 mM CaCl₂, and 10 mM dextrose) and allowed to recover for 1 hours - 5 hours before recording in oxygenated (95% O₂ and 5% CO₂) 30 °C artificial cerebrospinal fluid (ACSF; 124 mM NaCl, 5 mM KCl, 1.25 mM NaH₂PO₄, 1 mM MgCl₂, 2 mM CaCl₂, 26 mM NaHCO₃, and 10 mM dextrose). Field potentials (FPs) in stratum radiatum of area CA1 are evoked by a 200 µsec current pulse to Schaffer collateral axons. Stable baseline responses are collected every 30 seconds by a stimulation intensity (10 µA- 30 µA) yielding 50% - 60% of the maximal response. mGluR-LTD is induced by a 5 minute application of mGluR. agonist 3,5-dihydroxyphenylglycine (DHPG; at 100 µM) and/or by using paired-pulse low-frequency stimulation consisting of 900 pairs of stimuli delivered at 1 Hz in the presence of the N-methyl-D-aspartate receptor (NMDAR) antagonist D-(-)-2-amino-5-phosphono-pentanoic acid (D-APV; at 50 µM). DHPG is a chiral compound, and mGluR-LTD can be induced by application of RS-DHPG and S-DHPG, but typically not by application of R-DHPG. The initial slope of the field potential is recorded as an indicator of synaptic strength.

### Example 6: Synaptic Currents Mediated by AMPARs or NMDARs in the Cortex

Synaptic currents mediated by AMPA receptors (AMPARs) and/or NMDARs can be measured in the cortex. For measurement of AMPAR-mediated miniature excitatory postsynaptic current (mEPSC), 1 µM tetrodotoxin, 100 µM APV, and 10 µM bicuculline are added in a bath of ACSF. The cells are held at -80 mV, and recordings are done at 30 °C. Continuous 30 msec- 60 msec traces are collected at 8 second interval and filtered at 2 KHz. Cells with series resistance ≥13 mΩ are discarded. The measurement of NMDAR-EPSC/ AMPAR-EPSC ratio is done in ACSF containing 2 mM Mg²⁺, 10 µM bicuculline, and 50 µM glycine. NMDAR-dependent and AMPAR-dependent responses are discriminated based on their distinct kinetics and voltage dependence. Thus, NMDAR-mediated response is valued as the currents recorded at +40 mV and measured 100 msec after the response onset. The AMPAR-mediated response is taken from the peak amplitude response recorded at -80 mV.

### Example 7: Spine Morphology in dnPAK TG, FMR1 KO Mice

Spine morphology in *dnPAK* TG, *FMR1* KO mice ("dMT mice") is largely normal compared to wild type mice, indicating that signaling pathways mediated by PAK and FMRP antagonize each other to regulate spine morphogenesis. FMRP is known to bind to mRNA encoding Rac1, the upstream activator of PAK, and to antagonize the effect of Rac1 on dendritic development in *Drosophila.* If in the mouse, FMRP binds to and subsequently represses the translation of *Rac1* mRNA, *FMR1* removal would result in increased levels of Rac1 and enhanced Rac1/PAK-mediated signaling. Thus, in dMT mice, Rac1/PAK-mediated signaling would be reversed to wild type level by simultaneous *FMR1* removal and PAK inhibition, leading to normal actin dynamics and spine morphogenesis.

### Example 8: Analysis of FMRP in Translational Repression of Rac1, PAK1, PAK2, and/or PAK3 mRNA

In addition to an indirect signaling interaction between PAK and FMRP, possibly via FMRP's capability to bind to and repress the translation of *Rac1* mRNA, FMRP may directly bind to and repress the translation of mRNAs that encode PAKs, such as PAK1, PAK2, and/or PAK3. In this case, *FMR* removal would result in increased levels of total PAK, including active PAK. Thus, in dMT mice, the levels of active PAK would be reversed to wild type level.

### Example 9: Assaying Whether PAK Competes with RNA for Binding to FMRP KH Domains

PAK may bind to and negatively regulate FMRP's activity. Example 4 shows that the KH RNA binding domains of FMRP mediate the binding of FMRP to PAK1. Thus, PAK may compete with RNAs for binding to the KH domains of FMAP, thus relieving FMRP's activity to repress translation of these RNAs. To determine whether PAK may compete with RNAs for binding to the KH domains of FMRP, binding assays (such as immunoprecipitation and GST pulldown, described herein) may be performed in the presence of a substance that may compete with PAK for binding to the KH domains of FMRP.

In such assays, FMRP may be free in solution, fixed to a support, and/or expressed in and/or on the surface of a cell. The candidate RNAs and/or PAK may be labeled, thereby permitting detection of binding. Competitive binding formats may be performed in which one of the substances is labeled, and one may measure the amount of free label versus bound label to determine the effect of the competitor on binding. For example, PAK may be labeled and the candidate RNAs may be unlabeled in order to detect the ability of the candidate RNAs to compete with PAK for binding to FMRP

### Example 10: Assaying Whether PAK Phosphorylates FMRP

PAK, a serine/threonine kinase, may phosphorylate FMRP. In the brain, FMRP is phosphorylated on Ser 499 and its phosphorylation status affects its association with RNA and polyribosomes. Additional putative kinase substrates in the mouse brain include Thr 454, Ser 496, Thr 501, Ser 503, Thr 517 (Ceman et al., 2003, Hum. Mol. Genet., 12:3295; and Mazroui et al., 2003, Hum. Mol. Genet., 12:3087; both of which are incorporated herein by reference). Kinase assays may be performed in order to determine whether PAK phosphorylates FMRP. In such ass ays PAK and/or a characteristic portion thereof is contacted with FMRP and/or a characteristic portion thereof in the presence of a suitable phosphate donor, such as ATP, containing radiolabeled phosphate, and PAK-dependent incorporation of radiolabel into FMRP is measured.

PAK-dependent FMRP phosphorylation can be measured by monitoring physicochemical properties of FMRP that may occur as a result of incorporation of phosphate groups. Such properties may include electrophoretic mobility, light absorbance, fluorescence and/or phosphorescence, chromatographic properties, *etc.* Such alterations of substrate physicochemical properties can be readily measured by one skilled in the art and used as an indicator of kinase activity.

Alternatively or additionally, monoclonal or polyclonal antibodies may be generated which selectively recognize phosphorylated forms of FMRP, and thus the degree of binding of such antibodies to FMRP subsequent to the kinase reaction may be used as an indirect method of determining the ability of PAK to phosphorylate FMRP. Kinase assays may be performed using purified, partially recombinant PAK and/or FMRP, and/or PAK and/or FMRP which is purified from cells that naturally express the protein using standard purification procedures, such as those described herein.

### Example 11: PAKModulators and Autism

FXS patients constitute ~5% population of patients with autism, a complex multigenic disorder characterized by impaired communication, impaired social interactions and repetitive interests and behavior. Due to the difficulty in identifying susceptible chromosomal loci and genes, little is known on the causes and pathogenesis of autism. The association between FXS and autism suggests that these two disorders may share common genetic factors and underlying patho-physiological mechanisms. PAK modulators, therefore, may represent a novel treatment for certain types of autism. In support of this idea, the *PAK3* gene locates in chromosomal locus Xq22, a region linked to autism. It could, therefore, be determined whether *PAK3* and/or other *PAK* genes are mutated in autistic patients. To directly test the link between PAK modulation and autism, *dnPAK* TG mice could be crossed to available autism mouse models (Lijam et al., 1997, Cell, 90:895; Moretti et al., 2005, Hum. Mol. Genet., 14:205; and Kwon et al., 2006, Neuron, 50:377; all of which are incorporated herein by reference) to examine whether social behavior is reversed to wild type level in the double mutants.

### Example 12: Treatment of FMR KO Mice with Small Molecule PAK Inhibitors

### Preparation of Stock Solutions

Emodin (also known as 1,3,8-Tri-hydroxy-6-methyl-anthra-quinone; 6-Methyl-1,3,8-tri-hydroxy-anthra-quinone; Emodol; Frangula-emodin) is obtained from Sigma-Aldrich (# E7881). Emodin is an orange powder that is soluble in DMSO, ethanol, or I N dilute aqueous ammonia. A stock solution of 1 mg/ml - 50 mg/ml is made and stored at -20 °C.

OSU-03012 (also known as 2-amino-N-{4-[5-(2-phenanthrenyl)-3-(trifluoromethyl)-1H-pyrazol-1-yl]-phenyl} acetamide) is obtained from Cayman Chemicals (#10008005; Figures 6 and 7). OSU-03012 is a white solid that is soluble in organic solvents such as DMSO, ethanol, and DMF. A stock solution of 30 mg/ml - 100 mg/ml is made and stored at -20°C.

### Administration of Small Molecule Therapeutics to FMR KO Mice

Drug effects are optimized by determining the ideal delivery method, dose, volume, and dosage schedule. The level of drug exposure and duration is compared in regions of the brain as well as in other organs and the blood following various drug administration methods.

Small molecule therapeutics, including, but not limited to, Emodin and/or OSU-03012, can be administered to *FMR* KO mice by injection, and injected mice can be evaluated for therapeutic efficacy. Studies are frequently performed using male mice, but heterozygous and/or homozygous female mice can also be used in accordance with the invention. Studies are frequently performed using adult mice, but prenatal, infant, and/or juvenile mice can also be utilized in accordance with the present invention.

Behavioral phenotypes vary slightly with mouse strain. In some embodiments, a C57BL6 genetic background can be used for small molecule PAK inhibitor studies. In some embodiments, a FVB/NJ (FVB) genetic background can be used for small molecule PAK inhibitor studies. In some embodiments, F1 hybrids produced by crossing a male FVB mouse to a female C57BL6 heterozygous *FMR1* KO can be used for small molecule PAK inhibitor studies. In some embodiments, F1 hybrids produced by crossing a male C57BL6 mouse to a female FVB heterozygous mouse can be used for small molecule PAK inhibitor studies.

In general, small molecule PAK modulators are frequently soluble in organic solvents, but not in aqueous solutions. A variety of different solvents are tested to achieve maximal therapeutic effects and minimal adverse side effects. For example, small molecule therapeutics (*e.g*. Emodin and/or OSU-03012) are formulated using water, saline (*e.g*. phosphate-buffered saline), 2-hydroxypropyl-beta-cyclodextrin, Tween, methyl cellulose, polyethylene glycol, Cremophor EL (Sigma), oils (*e.g*. vegetable oil), and/or Maalox. The small molecule agent may be formulated as a homogenous solution or colloidal dispersion.

A variety of amounts of Emodin and/or OSU-03012 are administered to *FMR* KO mice. For example, dosages of about 5 µl, about 10 µl, about 20 µl, about 50 µl, about 100 µl, about 500 µl, about 1000 µl, about 2000 µl, and/or about 3000 µl of Emodin and/or OSU-03012 can be administered per dose. Typically, dosages of about 50 µl to about 1.0 ml are utilized.

Emodin and/or OSU-03012 can be administered at concentrations of about 0.5 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 20 mg/kg, about 50 mg/kg, about 100 mg/kg, about 250 mg/kg, or about 500 mg/kg per dose (mg/kg corresponds to # mg Emodin and/or OSU-03012 per kg of animal subject).

A variety of dosage schedules for emodin and/or OSU-03012 are utilized. Emodin and/or OSU-03012 are administered one time only and/or are administered multiple times (e.g. at regular intervals). For example, Emodin and/or OSU-03012 can be administered one time per month, two times per month, one time per week, two times per week, three times per week, every other day, one time per day, two times per day, three times per day, and/or four times per day.

Emodin and/or OSU-03012 can be administered by oral, intravenous, intramuscular, intra-arterial, intramedullary, intrathecal, parenteral, subcutaneous, intraventricular, transdermal, interdermal, rectal, intravaginal, intraperitoneal, topical (*e.g*. by powders, ointments, creams, gels, and/or drops), transdermal, mucosal, nasal, buccal, enteral, and/or sublingual administration; by intratracheal instillation, bronchial instillation, and/or inhalation; and/or as an oral spray, nasal spray, and/or aerosol. In some embodiments, Emodin and/or OSU-03012 can be administered by a continuous IV drip. In some embodiments, Emodin and/or OSU-03012 can be administered by retro-orbital injection.

Time lapse between last dose and biochemical or behavioral analyses ranges between 15 minutes and 2 days. For example, time lapse between last dose and biochemical or behavioral analyses can be approximately 15 minutes, approximately 30 minutes, approximately 1 hour, approximately 2 hours, approximately 3 hours, approximately 4 hours, approximately 5 hours, approximately 6 hours, approximately 9 hours, approximately 12 hours, approximately 15 hours, approximately 18 hours, approximately 24 hours, approximately 30 hours, approximately 36 hours, approximately 42 hours, approximately 48 hours, approximately 60 hours, or approximately 72 hours.

### Exemplary Dosage Schedule #1

Adult male *FMR1* KO mice and littermates are administered either placebo or PAK inhibitor (1 mg/kg, 10 mg/kg, 30 mg/kg, 100 mg/kg, or 300 mg/kg) in a total volume of 200 µl via oral gavage. Mice are monitored for signs of toxicity or decline in health. At various time points (1 hour, 4 hours, 12 hours, or 24 hours) following the single drug administration, animals are sacrificed. At least two mice are used for each of the 48 conditions. In some experiments, between two and ten mice are used. In some experiments, more than 10 mice are used. In some experimental runs, only a subset of these conditions is performed. At the time of sacrifice, blood, urine, and organs are collected. Brains are dissected into subregions, including the hippocampus, cortex and cerebellum. Analysis of drug concentration and efficacy in various fluids and tissues is used to inform future studies by suggesting optimal drug dosing concentrations and schedules.

### Exemplary Dosage Schedule #2

Adult male *FMR1* KO mice and littermates are treated with PAK inhibitor (e*.g.* a small molecule such as Emodin and/or OSU-03012) for 3 days, 1 week, 2 weeks, or 4 weeks prior to behavior analysis. Mice of each genotype will be randomly divided into three dosing groups with at least two mice in each group: vehicle control and drug treated (10 mg/kg or 100 mg/kg; or another dose found to be optimal in the previously described pilot experiment). In some experiments, about five mice are used. In some experiments, about ten mice are used. In some experiments, more than 10 mice are used. All drugs are administered once per day in a total volume of 200 µl by oral gavage. In some experimental runs, only a subset of these conditions is performed. Behavior experiments - including open field, trace fear conditioning, and/or social interaction experiments - are conducted 12 -18 hours after the last dosing (or at another time point found to be optimal in the previous dosing experiment). Mice are sacrificed immediately following final behavior assay and subregions of the brain, as well as other organs and blood, are collected and stored.

### Exemplary Dosage Schedule #3

Male *FMR1* KO pups and littermates to be used for audiogenic seizure (AGS) experiments are treated with PAK inhibitor (*e.g*. a small molecule such as Emodin and/or OSU-03012) for 1 day, 3 days, 5 days, or 7 days immediately prior to post natal day 20 (p20), at which time behavioral testing occurs. Therefore, PAK inhibitor treatments begin when the pups are p13, p15, p17, or p19. Subcutaneous injections of placebo or PAK inhibitor (1 mg/kg, 3 mg/kg, or 10 mg/kg) in a total volume of 50 µl/g body weight are given twice per day. For each of the 16 conditions (combinations of days of administration and drug dose), 6 - 8 *FMR1* KO pups and 2 control littermates are used. In some experimental runs, only a subset of these conditions is performed. Behavior experiments are conducted 4 - 8 hours after final injection.

One of ordinary skill in the art will readily recognize that these are exemplary, dosage schedules and are not meant to be limiting. A skilled artisan will recognize how the conditions of these exemplary dosage schedules may be modified in order to optimize dosage. Such optimizations are contained within the scope of the present invention.

### Post-Treatment Biochemical Analyses

After treatment, mice are subjected to a number of biochemical analyses in order to evaluate the efficacy of small molecule treatment. Typically, biological samples to be used in biochemical analyses are obtained post-mortem. However, biochemical analyses can be performed using biological samples obtained from living mice.

After the final administration of the compound, mice are subjected to a number of biochemical analyses in order to evaluate the bioavailability and efficacy of small molecule treatment. Mice are sacrificed at 1 hour, 2 hours, 6 hours, 12 hours, 24 hours, or 48 hours after final drug administration either by cervical dislocation or overdose of anesthesia (e.g. Avertin or Isoflurane), and blood is collected in heparinized tubes immediately thereafter by cardiac puncture. Blood plasma is separated by centrifugation (*e.g*. 20,000 x g), frozen, and stored at -80°C until analysis. Cerebrospinal fluid (CSF) is collected from the ventricles, briefly centrifuged to pellet cell debris, frozen, and stored at - 80 °C until analysis. To determine route and rate of elimination of the compound, urine is collected from the bladder, briefly centrifuged to pellet cell debris, and the supernatant frozen and stored at 80 °C until analysis. Using dissection, tissues including liver, kidney, spleen, lymph node, heart, lung, muscle, skin, spinal cord and brain, which can be subdivided into multiple areas including but not limited to cerebral cortex, hippocampus, and cerebellum are collected. Emodin, OSU-03012, and their metabolites are extracted from tissues using methanol.

For determination of Emodin and metabolite concentrations, samples (e.g. blood plasma, CSF, urine, and methanol extracts from tissues) are subjected to High-Performance Liquid Chromatography (HPLC) using a C18 column as the solid phase and either 88%methanol: 12% 0.1%phosphoric acid (water) or 40:60 0.1M phosphoric acid:methanol as the mobile phase. Emodin is detected by UV absorbance at 220 nm. Samples are spiked with a known amount of emodin prior to HPLC as an internal standard. The amount of emodin in the sample is determined by comparison to the known amount of internal standard, or with samples obtained from untreated mice spiked with known amount of emodin. The concentration of emodin is thus determined on a per mL basis (blood plasma / urine) or per mg basis (sample), based upon the amount of sample used for the procedure.

For determination of OSU-03012 and metabolite concentrations, samples (*e.g.* blood plasma, CSF, urine, and methanol extracts from tissues) are subjected to High-Performance Liquid Chromatography (HPLC) using a C18 column as the solid phase and a linear gradient from acetonitrile/0.025 M ammonium acetate, pH 4.5 (20:80) to acetonitrile/0.025 M ammonium acetate, pH 4.5 (60:40) as the mobile phase. OSU-03012 and metabolites are detected by UV absorbance at 240 nm or by fluorescence with excitation at 240 nm and emission at 380 nm. Samples are spiked with a known amount of OSU-03012 prior to HPLC as an internal standard. The amount of OSU-03012 in the sample is determined by comparison to the known amount of internal standard, or with samples obtained from untreated mice spiked with known amounts of OSU-03012. The concentration of OSU-03012 is thus determined on a per mL basis (blood plasma / urine) or per mg basis (sample), based upon the amount of sample used for the procedure.

Pharmacokinetic analysis includes plotting mean plasma, CSF, and/or urine concentrations of drug as a function of time, and calculating area under the curve (AUC) for different times post-injection. Half-life of PAK inhibitors is determined by fitting the concentration-time curve after the peak levels have been reached to a single exponential. Peak levels of small molecule PAK inhibitor in collected fluids are determined for each drug, dose, and time point combination.

Similarly, blood and urine can be collected and analyzed at set intervals throughout the dosage regimen. For example, the peri-orbital sinus can be used as a source of venous blood, and approximately 0.25 ml of blood can be safely collected in a capillary tube at weekly intervals under anesthesia. Similarly, tail vein venipuncture can be used to collect small blood volumes (typically a few drops) from restrained mice.

Post-mortem, various tissues are collected and analyzed for drug compounds and associated metabolites (as described for blood, CSF, and urine samples) and efficacy of PAK inhibition. Tissues collected include liver, kidney, spleen, lymph node, heart, lung, muscle, skin, spinal cord and brain, which can be subdivided into multiple areas including but not limited to cerebral cortex, hippocampus, and cerebellum. Efficacy of drug treatment in the brain is determined by homogenization of brain tissue, followed by gel electrophoresis and Western blotting to monitor activities of PAK and its downstream effectors through the use of phospho-specific antibodies.

These Western blots are performed as previously described (Hayashi et al., 2004, Neuron, 42:773; and Kelleher et al., 2004, Cell, 116:467; both of which are incorporated herein by reference). Briefly, mouse cerebral cortex, hippocampus, and cerebellum are dissected and homogenized in cold (*e.g.* 4 °C) RIPA buffer (50 mM Tris-HCl pH 7.4, 150 mM NaCl, 1% NP-40, 0.5% sodium deoxycholate, 0 mM or 1mM SDS) with protease (*e.g*. some or all of Roche Complete®, Mini Complete®, Calbiochem Protease Inhibitor Cocktails I, II and III®), and phosphatase (*e.g*. some or all of Calbiochem Phosphatase Inhibitor Cocktails I, II and III®) inhibitors. Homogenates are centrifuged to remove debris and protein concentrations are determined (*e.g*. Biorad Bradford assay, Biorad Rc-Dc assay, or Molecular Probes NanoOrange protocols as per manufacturer's instructions). In some cases, further centrifugation through a sucrose gradient and treatment with detergents is conducted to fractionate the extracts, thereby allowing enrichment of membrane fractions, synaptosome fractions, and post-synaptic density fractions. These fractions are subjected to Western blot analysis using antibodies (*e.g*. obtainable from Cell Signaling) that recognize phospho-specific forms of PAK and downstream signaling proteins, including p-PAK1 Thr 423 antibody (recognizes phosphorylated forms of PAK1, PAK2, and PAK3), dually phosphorylated p-ERK1/2, p-S6 (S235/S236), p-eIF4E (S209), p-4E-BP1 (S65), and p-Akt (S473). Alternatively or additionally, these fractions are subjected to Western blot analysis using a p-vimentin antibody (S56; Medical & Biological Laboratories). Blots are stripped and reprobed with anti-sera against total PAK1 (Santa Cruz), PAK3 (Upstate), ERK1/2 (Cell Signaling), S6 (Cell Signaling), eIF4E (Cell Signaling), 4E-BP1 (Cell Signaling), and Akt (Cell Signaling). Results are quantified with software (e.g. NIH Image J or Li-Cor Odyssey).

Alternatively or additionally, the extent to which small molecule inhibitors like Emodin and OSU-03012 inhibit endogenous PAK catalytic activity in the mouse brain is determined using an *in vitro* kinase assay as described in Hayashi et al., 2004, Neuron, 42:773; and Zenke et al., 1999, J. Biol. Chem., 274:32565; both of which are incorporated herein by reference). Briefly, catalytic activity of PAK can be stimulated by GTP-γS-Rac in total forebrain homogenates and measured by the amount of phosphorylated myelin basic protein (MBP).

For Western blots and *in vitro* PAK kinase assays, level of PAK activity (phospho-protein levels for all mentioned proteins above relative to no-drug condition or *in vitro* PAK catalytic activity level relative to no-drug condition) is monitored as a function of dose and dosage length.

Measurements of drug toxicity are conducted by monitoring weight loss, death, motor activity (*e.g.* open-field test), and/or coordination (*e.g*. rotorod) for all mentioned drug doses and throughout the dosage schedules.

In some embodiments, the optimum concentration of PAK to be used for behavior and electrophysiology experiments is approximately the smaller of the lowest amount of inhibitor required to achieve maximum inhibition of PAK activity (*e.g*. from Western Blot and/or kinase assay studies) or the lowest amount inhibitor that causes adverse toxic effect (*e.g*. weight loss, death, motor activity difference, coordination problems, *etc.).*

Typically, one group of mice is sacrificed and utilized in biochemical experiments (*e.g.* to determine the level of PAK kinase activity inhibition in the brain for various dosing schedules, times after final administration, *etc.)* A separate group of mice are used for behavior analyses. A mouse can be used for a single behavioral test or a battery of tests. If a single mouse is used for multiple behavioral tests, the tests are typically performed in the following order: open field, social-interaction, trace fear conditioning, and audiogenic seizure analysis.

### Post-Treatment Morphological Analyses

After treatment, mice are subjected to a number of histological and/or morphological analyses in order to evaluate the efficacy of small molecule treatment. A few exemplary histological and/or morphological analyses are described below and are explained in further detail in Example 1.

Since dendritic spine abnormalities are a hallmark of loss of FMRP in both humans and mice, Golgi analysis is performed on *FMR* KO mice following PAK inhibitor administration using the Golgi-Cox technique. Dendritic spine density in areas of the cortex, such as pyramidal neurons of layer II/III of temporal cortex, is measured on each primary or secondary apical or basal dendritic branch in 10 µm long dendritic segments. Spine number per 10 µm segment and mean spine density averaged over segments are quantified and compared (as in Figures 11 and 12). *FMR* KO mice show increase spine density with respect to their wild-type littermates. This phenotype is rescued or partially rescued by PAK inhibition in the *FMR* KO mice.

The Golgi preparation is used to quantify and compare dendritic spine length. Increased spine length, and perhaps concurrent decreased synapse size, of Golgi stained pyramidal neurons is observed in *FMR* KO mice and may be rescued by inhibition of PAK activity (as in Figure 13). Spine length is measured as the radial distance from the tip of the spine head to the dendritic shaft (see, *e.g*. Hayashi et al., 2007, Proc. Natl. Acad. Sci., USA, 104:11489; and Ramon-Moliner, 1970, Contemporary Research Methods in Neuroanatomy, Springer, Berlin; both of which are incorporated herein by reference).

Dendritic spine abnormalities are also observed in green fluorescent protein (GFP) labeled neurons in the cortex, such as those in layer V neurons of the barrel cortex or cortical neurons in culture. Neurons are labeled with GFP through the use of either a viral vector injected into the cortex or a transgenic mouse that expresses this fluorescent protein in some cortical neurons. Fluorescent neurons are imaged in fixed brain sections or primary cultures using a two-photon laser scanning microscope. Spine length and density are quantified as described above. *FMR* KO neurons display increased spine length and increased spine density, as phenotype which may be rescued by inhibition of PAK kinase activity (see, *e.g*., Nimchinsky et al., 2001, J. Neurosci., 21:5139; and Livet et al., 2007, Nature, 450:56; both of which are incorporated herein by reference).

### Post-Treatment Analysis of LTP and LTD

In some embodiments, hippocampal synaptic plasticity is assessed using a standard protocol for mGluR-LTD (see, *e.g*. Example 5 for a more detailed protocol). Hippocampal slices are prepared from *FMR* KO mice and control littermates per standard procedures. All experiments are performed blind to genotype. Schaffer collaterals are stimulated and extracellular field potentials measured in *str. radiatum* of CA1. mGluR-dependent LTD is elicited both electrophysiologically (by pairs of stimuli delivered at 1 Hz for 15 minutes to 20 minutes; "PP-LFS") and pharmacologically (by bath application of 50 µM to 100 µM 3,4-dihydroxyphenylglycine (DHPG) for 5 minutes). The initial slope of the field potential is recorded as an indicator of synaptic strength. Hippocampal slices from *FMR1* KO mice show enhanced mGluR-LTD relative to control mice (see, *e.g*., Huber et al., 2002, Proc. Natl. Acad. Sci., USA, 99:7746; and Nosyreva and Huber, 2006, J. Neurophysiol., 95:3291; both of which are incorporated herein by reference).

Cortical long-term potentiation (LTP) is reduced in slices from *FMR1* KO mice (see Figure 14). Coronol brain slices containing temporal cortex are prepared from two- to three-month-old male littermates, and left to recover for at least 1 hour before recording in oxygenated (95% O₂ and 5% CO₂) warm (30 °C) artificial cerebrospinal fluid containing 124 mM NaCl, 5 mM KCl, 1.25 mM NaH₂PO₄, 1 mM MgCl₂, 2 mM CaCl₂, 26 mM NaHCO₃, 10 mM dextrose. Field potentials (FPs) in layer II/III evoked by layer IV stimulation are measured as previously described and responses are quantified as the amplitude of FP in cortex. LTP is induced by TBS, which consisted of eight brief bursts (each with 4 pulses at 100 Hz) of stimuli delivered every 200 msec. Genetic inhibition of PAK rescues the reduced cortical LTP in the *FMR1* KO mice, and the present invention encompasses the recognition that pharmacological inhibition may have the same effect (Hayashi et al., 2007, Proc. Natl. Acad. Sci., USA, 104:11489; incorporated herein by reference).

### Post-Treatment Behavioral Analyses

Fragile X mice and littermates treated with PAK inhibitors or placebo are subjected to various behavioral tasks to determine whether pharmacological inhibition of PAK can ameliorate symptoms of Fragile X Syndrome in the mouse model. The experimenter is blind to genotype and prior drug treatment.

### Open Field Test

As described in the specification, the open field test records parameters such as horizontal and vertical activity, time spent in the center, and stereotopy. Mice are allowed to run freely in an open arena (*e.g*. VersaMax activity monitor chamber from Accuscan Instruments). Mice are allowed to run about 5 minutes, about 10 minutes, about 15 minutes, about 20 minutes, about 30 minutes, or about 1 hour. After mice are allowed to run, behaviors are analyzed, including (1) hyperactivity, determined by measuring the distance and/or length of time traveled by the subject; (2) stereotypy, determined by measuring the number of repetitive behaviors exhibited by the subject; (3) hypo-anxiety, determined by measuring the amount of time the subject remains in the center field relative to the time spent in the corners of the field; and (4) combinations of these.

In certain experiments, open field activity can be detected by photobeam breaks and analyzed by VersaMax software. Activities measured are the amount of time the mouse spends in the center of the field, the number of times the mouse exhibits repetitive behaviors ("stereotypy"), and the total distance traveled by the mouse.

### Audiogenic Seizure (AGS) Assay

Fragile X humans and mice are susceptible to seizures at early ages. Fragile X mice show a robust phenotype in an audiogenic seizure (AGS) assay. While no 19 - 21 day old (p19-21) wildtype mice typically have seizures in this task, the majority of fragile X mice do have seizures.

AGS is performed essentially as described (Yan *et al.,* 2005, *Neuropharmacol.,* 49:1053; incorporated herein by reference). Briefly, mice are habituated to a behavioral chamber and then exposed to a high intensity siren of frequency peak 1800 Hz - 6300 Hz at an average sound pressure level above 120 dB at approximately 10 cm for 5 minutes. Behaviors of the mice are monitored after administration of sound. Fragile X mice typically (1) run wildly, (2) have seizures, and/or (3) die. Wildtype mice typically do not exhibit these responses. An AGS phenotype is scored based on the animal's endpoint.

### Trace Fear Conditioning

Mice are subjected to the trace fear conditioning task according to standard procedures. On day 1 ("conditioning"), mice are placed into a training chamber for ~60 seconds before the onset of a ~15-second white noise tone. Another ~30 seconds later, mice received a ~1-second shock (~0.7 mA intensity). Thus, one trial is composed of tone, 30 seconds blank time (also called "trace"), and then shock. Five to ten trials with an intertrial interval (ITI) of 210 seconds are performed. To examine whether mice remember this association, on day 2 ("tone test"), mice are placed into a new chamber with a different shape and smell from the first chamber. After ~60 seconds, a ~15-second tone is repeated for five to ten times with an ITI of 210 seconds. Video images are digitized and the percentage of freezing time during each ITI is analyzed (*e.g*. by Image FZ program). Freezing is defined as the absence of all but respiratory movement for a 1-second period. See, *e.g.* Zhao et al. (2005, J. Neurosci., 25:7385; incorporated herein by reference).

### Social Interaction Tests

Home cage behavior and social interaction are assessed with a variety of tests (Kwon et al., 2006, Neuron, 50:377; Spencer et al., 2005, Genes Brain Behav., 4:420; and Lijam et al., 1997, Cell, 90:895; all of which are incorporated herein by reference). For example, mice can be observed in their home cage by videorecording and scored for various nonsocial behaviors and/or social behaviors (*e.g*. grooming, mounting, tail pulling, and sniffing).

Direct social interaction is assessed by exposing mice to a novel conspecific mouse and observing approaching and sniffing behaviors. Percent of time spent interacting is recorded. This is repeated about 3 days later with the same mice. Control mice exhibit a decrease in social interaction the second time, indicating recognition of the familiar mouse and/or normal social learning. *FMR* KO mice do not exhibit a decrease in social interaction the second time, indicating impaired social learning, memory, and/or behavior. This test can be conducted in a novel or familiar environment, as significant differences between *FMR* KO and wild-type mice have been observed in social interaction assays depending on the degree of familiarity with the environment. Additional social behaviors can be monitored in this assay including active behaviors (*e.g*. aggressive attacks, lateral threats, and/or chasing) and passive behaviors (*e.g*. receiving sniffing from other mouse and/or not showing signs of submissive and/or defensive behavior).

Prior to an indirect social interaction test mice are housed individually for 4 days. Indirect social interaction tasks typically take place in a cage divided in half by a clear perforated partition. The task is run in two different modes: one involves a familiar environment, by pre-exposure to the testing chamber, while the other involves a novel environment. Test mice are exposed to novel or familiar mice. Typically, *FMR* KO mice behave similarly to wild-type mice in a novel environment, but behave significantly differently in a familiar cage. Time spent at the partition is recorded in 2 to 5 minute intervals for a 20 minute test. *FMR* KO mice spend significantly less time interacting (i.e. at the partition) in the first few minutes and take longer to first approach the partition than wild-type mice. In contrast, *FMR* KO mice spend more time at the partition during the last time intervals than controls.

In some experiments, the indirect social interaction test is conducted in a chamber divided into three rooms. The central room - which is connected to two rooms independent from each other, one on the left and one on the right - is empty. The left room contains an empty cage (*i.e*. a novel object and/or inanimate target), while the right room contains a similar cage enclosing a novel mouse (i.e. a social target). This task involves a choice between spending time with a social target or an inanimate target, and therefore is called a social preference test. Percent of total time interacting with each object is recorded. *FMR* KO mice may spend a significantly different amount of time (*e.g*. more or less) interacting with the social target than control mice.

Social dominance tube test is conducted in a tube approximately 30 cm long and 3 cm - 4 cm in diameter. Mice are placed at opposite ends of the tube and released simultaneously. A mouse is pronounced the "winner" when his opponent backs out completely. *FMR1* KO mice win significantly fewer matches against unfamiliar wild-type mice than expected by chance. When a *FMR1* KO mouse competes against a wild-type non-cagemate, the wild-type mouse is the winner in approximately 73% of matches (Kwon et al., 2006, Neuron, 50:377; Spencer et al., 2005, Genes Brain Behav., 4:420; and Lijam et al., 1997, Cell, 90:895; all of which are incorporated herein by reference). Inhibition of PAK via administration of a small molecule inhibitor may ameliorate this phenotype.

Since *FMR1* KO mice have been shown to display some abnormal social behaviors, home cage social behavior, including nest building and sleeping behavior, may be altered in *FMR1* KO mice. Nesting patterns are evaluated by placing a cotton nestle into a cage of approximately two, three, or four mice of the same genotype (*e.g*. wild-type, *FMR1* KO, *etc.)* that receive identical drug treatments. After about 30 minutes to 1 hour, the nest can be removed and the height measured. Wild-type mice build nests with depths that average 20 mm to 50 mm. Mice which display abnormal social behavior, such as *FMR1* KO mice, frequently build shallower nests (*e.g*. < 20 mm).

In another assay, sleeping positions of mice in their home cages can be recorded two to four times a day over five consecutive days. Wild-type mice sleep huddled in the well-formed, fluffy nests they build. Observations will determine whether *FMR1* KO mice sleep in scattered, random patterns, do not build full nests, or sleep on top of intact nestle material. If *FMR1* KO mice display abnormal phenotypes, amelioration of those phenotypes may occur following PAK inhibition using small molecule inhibitors such as Emodin and/or OSU-03012.

### Eight-Arm Maze Test

FXS patients have cognitive deficits (*e.g*. short-term memory impairments) and/or perseverative language and movements. FXS patients often become anxious with any breach of their normal daily routine. The 8-arm radial maze is a task used to see if a similar phenotype is also present in the mouse model of the disease. The maze consists of a central arena surrounded by eight doors which guard the entrances to eight maze arms. Prominent distal cues surround the maze. A food pellet is hidden at the distal end of the arms and serves as a reward. In a version of the task specifically designed to test working memory, all 8 arms are baited with a food pellet. In each trial, the mouse must visit each of the 8 arms only once to consume the food reward. A second visit to an arm is considered a working memory error. Mice with poor short-term memory accrue more errors than wild-type mice. In the reference memory version of the task, only 4 arms are baited. In this case, the same 4 arms are baited each day during the training period (which typically lasts 10 - 16 days with 1 - 3 trials per day). An entry into an unbaited arm is considered a reference memory error. Just as in the previous version of the task, a second entry into any arm is considered a working memory error. Once the mice have learned the task, a reversal phase begins in which the previously unbaited arms now have the 4 food pellets and the previously baited arms contain no food reward. Mice with deficits in memory flexibility are unable to learn the new task and continue to visit the arms baited in the training phase. Errors are recorded and compared among *FMR* KO mice that receive PAK inhibitor, placebo, or no treatment.

### Morris Water Maze Test

Hippocampus-dependent spatial learning is assessed using the classical Morris Water Maze test. *FMR1* KO mice, just like the wild-type controls, learn to find the visible or hidden platform with decreasing latency scores over the course of the standard training protocol. However, some groups observe an abnormal phenotype in a reversal trial, a test in which the platform is transferred to the quadrant opposite the initial training quadrant. In particular, *FMR1* KO mice display increased escape latency and path length, suggesting that they have low response flexibility or high memory interference (see, *e.g*. D'Hooge et al., 1997, Neuroscience, 76:367; incorporated herein by reference).

### Equivalents and Scope

The foregoing has been a description of certain non-limiting preferred embodiments of the invention. Those skilled in the art will recognize, or be able to ascertain using no more than routine experimentation, many equivalents to the specific embodiments of the invention described herein. Those of ordinary skill in the art will appreciate that various changes and modifications to this description may be made without departing from the spirit or scope of the present invention, as defined in the following claims.

In the claims articles such as "a,", "an" and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process. Furthermore, it is to be understood that the invention encompasses all variations, combinations, and permutations in which one or more limitations, elements, clauses, descriptive terms, etc., from one or more of the claims or from relevant portions of the description is introduced into another claim. For example, any claim that is dependent on another claim can be modified to include one or more limitations found in any other claim that is dependent on the same base claim. Furthermore, where the claims recite a composition, it is to be understood that methods of using the composition for any of the purposes disclosed herein are included, and methods of making the composition according to any of the methods of making disclosed herein or other methods known in the art are included, unless otherwise indicated or unless it would be evident to one of ordinary skill in the art that a contradiction or inconsistency would arise. For example, it is to be understood that any of the compositions of the invention can be used for inhibiting the formation, progression, and/or recurrence of adhesions at any of the locations, and/or due to any of the causes discussed herein or known in the art. It is also to be understood that any of the compositions made according to the methods for preparing compositions disclosed herein can be used for inhibiting the formation, progression, and/or recurrence of adhesions at any of the locations, and/or due to any of the causes discussed herein or known in the art. In addition, the invention encompasses compositions made according to any of the methods for preparing compositions disclosed herein.

Where elements are presented as lists, *e.g*., in Markush group format, it is to be understood that each subgroup of the elements is also disclosed, and any element(s) can be removed from the group. It is also noted that the term "comprising" is intended to be open and permits the inclusion of additional elements or steps. It should be understood that, in general, where the invention, or aspects of the invention, is/are referred to as comprising particular elements, features, steps, etc., certain embodiments of the invention or aspects of the invention consist, or consist essentially of, such elements, features, steps, etc. For purposes of simplicity those embodiments have not been specifically set forth *in haec verba* herein. Thus for each embodiment of the invention that comprises one or more elements, features, steps, etc., the invention also provides embodiments that consist or consist essentially of those elements, features, steps, etc.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

In addition, it is to be understood that any particular embodiment of the present invention may be explicitly excluded from any one or more of the claims. Any embodiment, element, feature, application, or aspect of the compositions and/or methods of the invention (*e.g*., any PAK modulator, any biological activity of PAK modulators, any method of identifying PAK modulators, any method of treatment of FXS and/or other neurodevelopmental disorder, any neurodevelopmental disorder, *etc*.), can be excluded from any one or more claims. For purposes of brevity, all of the embodiments in which one or more elements, features, purposes, or aspects is excluded are not set forth explicitly herein.
Aspects and features of the present disclosure are set out in the following numbered clauses which contain the subject matter of the clauses of the parent application as filed.
1. A method comprising steps of: providing a subject susceptible to, suffering from, or exhibiting at least one symptom associated with fragile X syndrome (FXS); and administering an amount of at least one modulator of p21 -activated kinase (PAK) to the subject effective to treat, alleviate, ameliorate, relieve, delay onset of, inhibit progression of, reduce severity of, or reduce incidence of the at least one symptom associated with FXS.
2. A method, comprising steps of: providing a subject susceptible to, suffering from, or exhibiting at least one symptom associated with fragile X syndrome (FXS); and administering an amount of a pharmaceutical composition comprising at least one modulator of p21 -activated kinase (PAK) to the subject effective to treat, alleviate, ameliorate, relieve, delay onset of, inhibit progression of, reduce severity of, or reduce incidence of the at least one symptom associated with FXS.
3. A method, comprising steps of: providing a subject susceptible to, suffering from, or exhibiting at least one symptom associated with mental retardation or autism spectrum disorders; and administering an amount of at least one modulator of p21 - activated kinase (PAK) to the subject effective to treat, alleviate, ameliorate, relieve, delay onset of, inhibit progression of, reduce severity of, or reduce incidence of the at least one symptom associated with mental retardation or autism spectrum disorders.
4. A method, comprising steps of: providing a subject susceptible to, suffering from, or exhibiting at least one symptom associated with mental retardation or autism spectrum disorders; and administering an amount of a pharmaceutical composition comprising at least one modulator of p21 -activated kinase (PAK) to the subject effective to treat, alleviate, ameliorate, relieve, delay onset of, inhibit progression of, reduce severity of, or reduce incidence of the at least one symptom associated with mental retardation or autism spectrum disorders.
5. The method of any on of clauses 1 - 4, wherein the modulator of PAK is an inhibitor of PAK.
6. The method of clause 5, wherein the inhibitor of PAK functions by modulating the interaction of PAK with at least one natural binding partner of PAK.
7. The method of clause 6, wherein the natural binding partner of PAK is FMRP.
8. The method of clause 6, wherein the natural binding partner of PAK is selected from the group consisting of Myosin light chain kinase, regulatory Myosin light chain, Myosin I heavy chain, Myosin II heavy chain, Myosin VI, Caldesmon, Desmin, Op18/stathmin, Merlin, Filamin A, LIM kinase, Ras, Raf, Mek, p47<phox>, BAD, caspase 3, estrogen receptor, progesterone receptor; RhoGEF, GEF-H1, NET1, G[alpha]z, phosphoglycerate mutase-B, RhoGDI, prolactin, p41<Arc>, Aurora-A, Rac/Cdc42, CIB, sphingolipids, G-protein [beta] subunit, G-protein [gamma] subunit, PIX/COOL, GIT/PKL, Paxillin, Nef, NESH, SH3 -containing proteins, Nek, Grb2, kinases, Akt, PDK1, PB- kinase/p85, Cdk5, Cdc2, Src kinases, AbI, protein kinase A, phosphatases phosphatase PP2A, POPX1, and POPX2.
9. The method of clause 5, wherein wherein the step of administering comprises administering an amount effective to inhibit PAK's kinase activity.
10. The method of clause 9, wherein the inhibitor of PAK reduces PAK' s ability to phosphorylate a PAK substrate.
11. The method of clause 10, wherein the PAK substrate is selected from the group consisting of Myosin light chain kinase, regulatory Myosin light chain, Myosin I heavy chain, Myosin II heavy chain, Myosin VI, Caldesmon, Desmin, Op18/stathmin, Merlin, Filamin A, LIM kinase, Ras, Raf, Mek, p47<phox>, BAD, caspase 3, estrogen receptor, progesterone receptor; RhoGEF, GEF-H1, NETI, G[alpha]z, phosphoglycerate mutase-B, RhoGDI, prolactin, p41<Arc>, and Aurora-A.
12. The method of clause 9, wherein the inhibitor of PAK sterically blocks PAK's kinase domain.
13. The method of clause 9, wherein the inhibitor of PAK sterically blocks PAK's p21- binding domain.
14. The method of clause 9, wherein the inhibitor of PAK sterically blocks at least one autophosphorylation site of PAK.
15. The method of clause 5, wherein the step of administering comprises administering an amount effective to reduce expression of PAK.
16. The method of clause 5, wherein the step of administering comprises administering an amount effective to reduce cellular levels of PAK.
17. The method of clause 5, wherein the step of administering comprises administering an amount effective to reduce expression of at least one PAK natural binding partner.
18. The method of clause 5, wherein the step of administering comprises administering an amount effective to increase expression of at least one PAK natural binding partner.
19. The method of clause 5, wherein the inhibitor of PAK functions by reducing cellular levels of at least one PAK natural binding partner.
20. The method of clause 5, wherein the step of administering comprises administering an amount effective to increase cellular levels of at least one PAK natural binding partner.
21. The method of clause 5, wherein the step of administering comprises administering an amount effective to reduce expression of an activator of PAK transcription.
22. The method of clause 5, wherein the step of administering comprises administering an amount effective to increase expression of an inhibitor of PAK transcription.
23. The method of clause 5, wherein the step of administering comprises administering an amount effective to reduce expression of an activator of PAK translation.
24. The method of clause 5, wherein the step of administering comprises administering an amount effective to increase expression of inhibitor of PAK translation.
25. The method of clause 5, wherein the step of administering comprises administering an amount effective to reduce stability of PAK mRNA or protein.
26. The method of clause 5, wherein the step of administering comprises administering an amount effective to increase susceptibility of PAK mRNA or protein to nuclease, protease, or proteasome.
27. The method of clause 5, wherein the step of administering comprises administering an amount effective to inhibit mRNA processing of PAK mRNA.
28. The method of clause 5, wherein the step of administering comprises administering an amount effective to repress translation of PAK mRNA.
29. The method of any one of clauses 1-5, wherein the modulator of PAK is a small molecule.
30. The method of clause 29, wherein the small molecule is Emodin.
31. The method of clause 29, wherein the small molecule is a derivative of Emodin.
32. The method of clause 29, wherein the small molecule is OSU-03012.
33. The method of clause 29, wherein the small molecule is a derivative of OSU-03012.
34. The method of clause 29, wherein the small molecule is staurosporin.
35. The method of clause 29, wherein the small molecule is a derivative of staurosporin.
36. The method of clause 29, wherein the small molecule is PD098059.
37. The method of clause 29, wherein the small molecule is a derivative of PD098059.
38. The method of clause 29, wherein the small molecule is genisteintyrphostin B42.
39. The method of clause 29, wherein the small molecule is a derivative of genisteintyrphostin B42.
40. The method of clause 29, wherein the small molecule is HA 1077.
41. The method of clause 29, wherein the small molecule is a derivative of HA1077.
42. The method of clause 29, wherein the small molecule is K252a.
43. The method of clause 29, wherein the small molecule is a derivative of K252a.
44. The method of clause 29, wherein the small molecule is 1(5-isoquinoline-sulfonyl)-2- methylpiperazine (H-7).
45. The method of clause 29, wherein the small molecule is a derivative of 1(5-isoquinoline-sulfonyl)-2-methylpiperazine (H-7).
46. The method of clause 29, wherein the small molecule is CEP- 1347.
47. The method of clause 29, wherein the small molecule is a derivative of CEP-1347.
48. The method of clause 29, wherein the small molecule is selected from the group consisting of hPIP, Merlin, Nischarin, P35/CDK5, CDC2, p 11OC, POPX1, POPX2, CRIPak, PAK1 amino acids 89 - 143, GL-2003, SU1 1652, Cdk1 Inhibitor, Cdk1/2 Inhibitor III, and Purvalanol A.
49. The method of clause 29, wherein the small molecule is a derivative of a small molecule selected from the group consisting of hPIP, Merlin, Nischarin, P35/CDK5, CDC2, p HOC, POPX1, POPX2, CRIPak, PAK1 amino acids 89 - 143, GL-2003, SU1 1652, Cdk1 Inhibitor, Cdk1/2 Inhibitor III, and Purvalanol A.
50. The method of any one of clauses 1-5, wherein the modulator of PAK is a protein.
51. The method of clause 50, wherein the protein is an antibody.
52. The method of clause 50, wherein the protein is a peptide.
53. The method of clause 50, wherein the protein comprises more than one polypeptide.
54. The method of any one of clauses 1 - 5 wherein the modulator of PAK is a nucleic acid.
55. The method of clause 54, wherein the nucleic acid is an RNA interference (RNAi)- inducing entity.
56. The method of clause 55, wherein the RNAi-inducing entity is a short interfering RNA.
57. The method of clause 55, wherein the RNAi-inducing entity is a short hairpin RNA.
58. The method of clause 54, wherein the nucleic acid is an antisense oligonucleotide.
59. The method of clause 54, wherein the nucleic acid is a ribozyme.
60. The method of clause 54, wherein the nucleic acid is a triple helix inducing agent.
61. The method of any one of clauses 1-5, wherein the modulator of PAK comprises a lipid.
62. The method of any one of clauses 1-5, wherein the modulator of PAK comprises a carbohydrate.
63. The method of clause 62, wherein the modulator of PAK is a proteoglycan or glycoprotein.
64. The method of any one of clauses 1 - 5, wherein the modulator of PAK is administered in combination with an additional therapeutic agent.
65. The method of clause 64, wherein the additional therapeutic agent is an anti-seizure agent.
66. The method of clause 64, wherein the additional therapeutic agent is a mood stabilizer.
67. The method of clause 64, wherein the additional therapeutic agent is a central nervous system stimulant.
68. The method of clause 64, wherein the additional therapeutic agent is an antihypertensive agent.
69. The method of clause 64, wherein the additional therapeutic agent is folic acid.
70. The method of clause 64, wherein the additional therapeutic agent is a selected serotonin reuptake inhibitor.
71. The method of clause 64, wherein the additional therapeutic agent is an antipsychotic agent.
72. The method of clause 64, wherein the additional therapeutic agent is an agent used to treat sleep disturbances.
73. A method comprising steps of: providing an FMR knockout mouse exhibiting at least one symptom of fragile
   X syndrome (FXS); administering at least one candidate substance to the mouse; and measuring the effect of the at least one candidate substance on the at least one symptom of FXS.
74. The method of clause 73, wherein the at least one symptom of FXS is stereotypy.
75. The method of clause 73, wherein the at least one symptom of FXS is hyperactivity
76. The method of clause 73, wherein the at least one symptom of FXS is anxiety.
77. The method of clause 73, wherein the at least one symptom of FXS is seizure.
78. The method of clause 73, wherein the at least one symptom of FXS is impaired social behavior.
79. The method of clause 73, wherein the at least one symptom of FXS is cognitive delay.
80. The method of clause 73, wherein the step of measuring the effect of the candidate substance on the at least one symptom of FXS comprises assaying synaptic morphology.
81. The method of clause 80, wherein assaying synaptic morphology comprises measuring dendritic spine density.
82. The method of clause 81, wherein a candidate substance is determined to be a PAK inhibitor if administering the candidate substance to the subject results in decreased dendritic spine density.
83. The method of clause 80, wherein assaying synaptic morphology comprises measuring the length or width of dendritic spines.
84. The method of clause 83, wherein a candidate substance is determined to be a PAK inhibitor if administering the candidate substance to the subject results in shorter dendritic spines.
85. The method of clause 83, wherein a candidate substance is determined to be a PAK inhibitor if administering the candidate substance to the subject results in thicker dendritic spines.
86. The method of clause 80, wherein assaying synaptic morphology comprises measuring the size of dendritic spine heads.
87. The method of clause 73, wherein the step of measuring the effect of the candidate substance on the at least one symptom of FXS comprises assaying synaptic function.
88. The method of clause 87, wherein assaying synaptic function comprises measuring long-term depression in the hippocampus.
89. The method of clause 88, wherein a candidate substance is determined to be a PAK inhibitor if administering the candidate substance to the subject results in decreased long-term depression in the hippocampus.
90. The method of clause 87, wherein assaying synaptic function comprises measuring long-term potentiation in the cortex.
91. The method of clause 90, wherein a candidate substance is determined to be a PAK inhibitor if administering the candidate substance to the subject results in increased long-term potentiation in the cortex.
92. The method of clause 87, wherein assaying synaptic function comprises measuring synaptic currents in the cortex.
93. The method of clause 73, wherein the step of measuring the effect of the candidate substance on the at least one symptom of FXS comprises assaying behavioral symptoms.
94. The method of clause 93, wherein assaying behavioral symptoms comprises performing an open-field test.
95. The method of clause 93, wherein assaying behavioral symptoms comprises performing a trace-fear conditioning task.
96. The method of clause 93, wherein assaying behavioral symptoms comprises performing an eight-arm maze task.
97. The method of clause 93, wherein assaying behavioral symptoms comprises performing a social interaction test.
98. The method of clause 93, wherein assaying behavioral symptoms comprises performing an audiogenic seizure assay.
99. A method comprising steps of: providing an FMR knockout mouse exhibiting at least one symptom of mental retardation or autism spectrum disorders; administering at least one candidate substance to the mouse; and measuring the effect of the at least one candidate substance on the at least one symptom of mental retardation or autism spectrum disorders.
100. A method comprising steps of: providing p21 -activated kinase (PAK); providing a natural binding partner of PAK; administering at least one candidate substance to PAK and the natural binding partner; and measuring the effect of the at least one candidate substance on the binding interaction between PAK and the natural binding partner.
101. The method of clause 100, wherein the step of measuring the effect of the at least one candidate substance on the binding interaction between PAK and the natural binding partner comprises: performing immunoprecipitations in the presence and in the absence of the at least one candidate substance; and comparing the results of the immunoprecipitation in the presence of the at least one candidate substance to the results of the immunoprecipitation in the absence of the at least one candidate substance.
102. The method of clause 100, wherein the step of measuring the effect of the at least one candidate substance on the binding interaction between PAK and the natural binding partner comprises: performing GST pull-down assays in the presence and in the absence of the at least one candidate substance; and comparing the results of the GST pull-down assay in the presence of the at least one candidate substance to the results of the GST pull-down assay in the absence of the at least one candidate substance.
103. A method comprising steps of: providing p21 -activated kinase (PAK); providing a natural binding partner of PAK; administering at least one candidate substance to PAK and the natural binding partner; and measuring the ability of the at least one candidate substance to compete with the binding interaction between PAK and the natural binding partner.
104. The method of clause 100 or 103, wherein a candidate substance is determined to be a PAK inhibitor if administering the candidate substance to PAK and the natural binding partner results in decreased binding between PAK and the natural binding partner.
105. The method of clause 104, wherein a candidate substance is determined to be a PAK inhibitor if administering the candidate substance to PAK and the natural binding partner results in an at least 2-fold decrease in binding between PAK and the natural binding partner.
106. The method of clause 104, wherein a candidate substance is determined to be a PAK inhibitor if administering the candidate substance to PAK and the natural binding partner results in an at least 5 -fold decrease in binding between PAK and the natural binding partner.
107. The method of clause 104, wherein a candidate substance is determined to be a PAK inhibitor if administering the candidate substance to PAK and the natural binding partner results in an at least 10-fold decrease in binding between PAK and the natural binding partner.
108. The method of clause 104, wherein a candidate substance is determined to be a PAK inhibitor if administering the candidate substance to PAK and the natural binding partner results in an at least 100-fold decrease in binding between PAK and the natural binding partner.
109. A method comprising steps of: providing a p21 -activated kinase (PAK); providing a phosphorylation substrate of PAK; administering at least one candidate substance to PAK and the phosphorylation substrate of PAK; and measuring the effect of the at least one candidate substance on ability of PAK to phosphorylate the substrate.
110. The method of clause 109, wherein the step of measuring the effect of the at least one candidate substance on ability of PAK to phosphorylate the substrate comprises: performing kinase assays in the presence and in the absence of the at least one candidate substance; and comparing the results of the kinase assay in the presence of the at least one candidate substance to the results of the kinase assay in the absence of the at least one candidate substance.
111. The method of clause 109, wherein a candidate substance is determined to be a PAK inhibitor if administering the candidate substance to PAK and the natural binding partner results in decreased ability of PAK to phosphorylate the substrate.
112. The method of clause 111, wherein a candidate substance is determined to be a PAK inhibitor if administering the candidate substance to PAK and the natural binding partner results in an at least 2-fold decrease in the ability of PAK to phosphorylate the substrate.
113. The method of clause 111, wherein a candidate substance is determined to be a PAK inhibitor if administering the candidate substance to PAK and the natural binding partner results in an at least 5-fold decrease in the ability of PAK to phosphorylate the substrate.
114. The method of clause 111, wherein a candidate substance is determined to be a PAK inhibitor if administering the candidate substance to PAK and the natural binding partner results in an at least 10-fold decrease in the ability of PAK to phosphorylate the substrate.
115. The method of clause 111, wherein a candidate substance is determined to be a PAK inhibitor if administering the candidate substance to PAK and the natural binding partner results in an at least 100-fold decrease in the ability of PAK to phosphorylate the substrate.
116. The method of any one of clauses 73, 99, 100, 103, or 109, wherein the at least one candidate substance comprises exactly one candidate substance.
117. The method of any one of clauses 73, 99, 100, 103, or 109, wherein the at least one candidate substance comprises exactly two candidate substances.
118. The method of any one of clauses 73, 99, 100, 103, or 109, wherein the at least one candidate substance comprises more than two candidate substances.
119. The method of any one of clauses 73, 99, 100, 103, or 109, wherein the at least one candidate substance comprises a library of candidate substances.
120. The method of any one of clauses 100, 103, or 109, wherein PAK is PAK1.
121. The method of any one of clauses 100, 103, or 109, wherein PAK is PAK2.
122. The method of any one of clauses 100, 103, or 109, wherein PAK is PAK3.
123. The method of any one of clauses 100, 103, or 109, wherein PAK is PAK4.
124. The method of any one of clauses 100, 103, or 109, wherein PAK is PAK5.
125. The method of any one of clauses 100, 103, or 109, wherein PAK is PAK6.
126. The method of any one of clauses 100, 103, or 109, wherein PAK is PAK7.
127. A modulator of p21 -activated kinase (PAK) identified by any one of the methods of clauses 73, 99, 100, 103, or 109.
128. A pharmaceutical composition comprising: the modulator of clause 127; and at least one pharmaceutically acceptable excipient.
129. A pharmaceutical composition comprising: a modulator of p21 -activated kinase (PAK); and at least one pharmaceutically acceptable excipient.
130. A kit comprising: the pharmaceutical composition of clause 129; and instructions for administering the pharmaceutical composition to a subject suffering from, susceptible to, or exhibiting symptoms of FXS.
131. A kit comprising: an FMR KO mouse; a least one candidate substance; a positive control, wherein the positive control comprises a known modulator of p21 -activated kinase (PAK); a negative control, wherein the negative control comprises a substance known to not be a PAK modulator; instructions for administering the candidate substance to the mouse in order to determine whether the candidate substance treats, alleviates, ameliorates, relieves, delays onset of, inhibits progression of, reduces severity of, or reduces incidence of one or more symptoms or features of FXS.

## Claims

1. At least one inhibitor of p21-activated kinase (PAK) for use in treating a neurodevelopmental disorder.

2. The inhibitor for the use of claim 1, wherein the inhibitor of PAK inhibits the interaction of PAK1 with FMRP.

3. The inhibitor for the use of claim 1, wherein the inhibitor of PAK is Cdk1 inhibitor, Cdk1/2 inhibitor III or GL-2003.

4. The inhibitor for the use of claim 2, wherein the inhibitor of PAK is Emodin, OSU-03012, staurosporin, PD098059, genistein, tyrphostin B42, HA 1077, K252a, 1(5-isoquinoline-sulfonyl)-2-methylpiperazine (H-7), CEP-1347, hPIP, Merlin, Nischarin, P35/CDK5, CDC2, p110C, POPX1, POPX2, CRIPak, PAK1 amnio acids 89-143, GL-2003, SU11652, or Purvalanol A.

5. The inhibitor for the use of claim 1, wherein the inhibitor of PAK is a short interfering RNA (siRNA) or a short hairpin RNA (shRNA).

6. The inhibitor for the use of claim 1, wherein the inhibitor of PAK is an antibody.

7. The inhibitor for the use of claim 1, wherein the inhibitor of PAK is administered in combination with an additional therapeutic agent, for example an anti-seizure agent, a mood stabilizer, a central nervous system stimulant, an antihypertensive agent, folic acid, a selected serotonin reuptake inhibitor, or an antipsychotic agent.

8. Use of at least one inhibitor of p21-activated kinase (PAK) in the manufacture of a medicament for treating a neurodevelopmental disorder.

9. At least one inhibitor of p21-activated kinase (PAK) for use in treating a disease, disorder and/or condition caused by and/or associated with one or more of the following:
(1) a mutation in FMR1 (the nucleotide sequence encoding FMRP);
(2) defective FMR1 expression;
(3) increased and/or decreased expression of FMRP;
(4) defective FMRP function;
(5) increased and/or decreased expression of FMRP's natural binding partners;
(6) an increased and/or decreased ability of FMRP to bind to its natural binding partners;
(7) decreased or absent arginine methylation of FMRP;
(8) the increased methylation of *FMRl* CpG repeats in the 5' UTR of exon 1;
(9) the mislocalization or misexpression of FMRP within the cell or within the organism;
(10) the modulation of function of *FMRl* transcription factors SpI and/or NRF1;
(11) an increased and/or decreased ability of FMRP to associate with polysomes;
(12) the loss of function of *FXRl* and/or FXR2, which are homologous to FMR1 and may compensate for loss of *FMRl* function;
(13) the decreased ability of FMRP to recognize RNA secondary structures that FMRP normally recognizes;
(14) an increased and/or decreased ability of FMRP to interact with miRNAs and/or members of the miRNA pathway;
(15) an increased and/or decreased ability of FMRP to interact with its known target RNAs, such as RNAs encoding Racl, microtubule-associated protein IB, activity-regulated cytoskeleton-associated protein, and/or alpha-calcium/calmodulin-dependent protein kinase II;
(16) an increased and/or decreased ability of phosphatase PP2A to act on FMRP;
(17) the increased and/or decreased activity of mGluR5, which is known to decrease activity of phosphatase PP2A
(18) exaggerated signaling in mGluR pathways;
(19) disruption of a KH domain of FMRP, which decreases the ability of FMRP to interact with PAK; and/or
(20) an 1304N mutation in FMRP, which decreases the ability of FMRP to interact with PAK.


SEQUENCE LISTING

<110>  Massachusetts Institute of Technology

<120>  PAK MODULATORS

<130>  0492612-0568

<140>  PCT/US2007/084325
<141>  2007-11-09

<160>  18

<170>  PatentIn version 3.5

<210>  1
<211>  681
<212>  PRT
<213>  Artificial Sequence

<220>
<223>  FMR and FXR amino acid sequences

<400>  1
   Met Glu Asp Leu Leu Val Glu Val Arg Leu Asp Asn Gly Ala Tyr Tyr
   1               5                   10                  15
   Lys Gly Gln Val Thr Ala Val Ala Asp Asp Gly Ile Phe Val Asp Val
               20                  25                  30
   Asp Gly Val Pro Glu Ser Met Lys Tyr Pro Phe Val Asn Val Arg Leu
           35                  40                  45
   Pro Pro Glu Glu Thr Val Glu Val Ala Ala Pro Ile Phe Glu Glu Gly
       50                  55                  60
   Met Glu Val Glu Val Phe Thr Arg Thr Asn Asp Arg Glu Thr Cys Gly
   65                  70                  75                  80
   Trp Trp Val Gly Ile Ile Lys Met Arg Lys Ala Glu Ile Tyr Ala Val
                   85                  90                  95
   Ala Tyr Ile Gly Phe Glu Thr Ser Tyr Thr Glu Ile Cys Glu Leu Gly
               100                 105                 110
   Arg Leu Arg Ala Lys Asn Ser Asn Pro Pro Ile Thr Ala Lys Thr Phe
           115                 120                 125
   Tyr Gln Phe Thr Leu Pro Val Pro Glu Glu Leu Arg Glu Glu Ala Gln
       130                 135                 140
   Lys Asp Gly Ile His Lys Glu Phe Gln Arg Thr Ile Asp Ala Gly Val
   145                 150                 155                 160
   Cys Asn Tyr Ser Arg Asp Leu Asp Ala Leu Ile Val Ile Ser Lys Phe
                   165                 170                 175
   Glu His Thr Gln Lys Arg Ala Ser Met Leu Lys Asp Met His Phe Arg
               180                 185                 190
   Asn Leu Ser Gln Lys Val Met Leu Leu Lys Arg Thr Glu Glu Ala Ala
           195                 200                 205
   Arg Gln Leu Glu Thr Thr Lys Leu Met Ser Arg Gly Asn Tyr Val Glu
       210                 215                 220
   Glu Phe Arg Val Arg Asp Asp Leu Met Gly Leu Ala Ile Gly Ser His
   225                 230                 235                 240
   Gly Ser Asn Ile Gln Ala Ala Arg Thr Val Asp Gly Val Thr Asn Ile
                   245                 250                 255
   Glu Leu Glu Glu Lys Ser Cys Thr Phe Lys Ile Ser Gly Glu Thr Glu
               260                 265                 270
   Glu Ser Val Gln Arg Ala Arg Ala Met Leu Glu Tyr Ala Glu Glu Phe
           275                 280                 285
   Phe Gln Val Pro Arg Glu Leu Val Gly Lys Val Ile Gly Lys Asn Gly
       290                 295                 300
   Arg Ile Ile Gln Glu Ile Val Asp Lys Ser Gly Val Phe Arg Ile Lys
   305                 310                 315                 320
   Ile Ala Gly Asp Asp Glu Gln Asp Gln Asn Ile Pro Arg Glu Leu Ala
                   325                 330                 335
   His Val Pro Leu Val Phe Ile Gly Thr Val Glu Ser Ile Ala Asn Ala
               340                 345                 350
   Lys Val Leu Leu Leu Tyr His Leu Ser His Leu Lys Glu Val Glu Gln
           355                 360                 365
   Leu Arg Gln Glu Lys Met Glu Ile Asp Gln Gln Leu Arg Ala Ile Gln
       370                 375                 380
   Glu Ser Ser Met Gly Ser Thr Gln Ser Phe Pro Val Thr Arg Arg Ser
   385                 390                 395                 400
   Glu Arg Gly Tyr Ser Ser Asp Ile Glu Ser Val Arg Ser Met Arg Gly
                   405                 410                 415
   Gly Gly Gly Gly Gln Arg Gly Arg Val Arg Gly Arg Gly Gly Gly Gly
               420                 425                 430
   Pro Gly Gly Gly Asn Gly Leu Asn Gln Arg Tyr His Asn Asn Arg Arg
           435                 440                 445
   Asp Glu Asp Asp Tyr Asn Ser Arg Gly Asp His Gln Arg Asp Gln Gln
       450                 455                 460
   Arg Gly Tyr Asn Asp Arg Gly Gly Gly Asp Asn Thr Gly Ser Tyr Arg
   465                 470                 475                 480
   Gly Gly Gly Gly Gly Ala Gly Gly Pro Gly Asn Asn Arg Arg Gly Gly
                   485                 490                 495
   Ile Asn Arg Arg Pro Pro Arg Asn Asp Gln Gln Asn Gly Arg Asp Tyr
               500                 505                 510
   Gln His His Asn His Thr Thr Glu Glu Val Arg Glu Thr Arg Glu Met
           515                 520                 525
   Ser Ser Val Glu Arg Ala Asp Ser Asn Ser Ser Tyr Glu Gly Ser Ser
       530                 535                 540
   Arg Arg Arg Arg Arg Gln Lys Asn Asn Asn Gly Pro Ser Asn Thr Asn
   545                 550                 555                 560
   Gly Ala Val Ala Asn Asn Asn Asn Lys Pro Gln Ser Ala Gln Gln Pro
                   565                 570                 575
   Gln Gln Gln Gln Pro Pro Ala Pro Gly Asn Lys Ala Ala Leu Asn Ala
               580                 585                 590
   Ser Asp Ala Ser Lys Gln Asn Ser Gly Asn Ala Asn Ala Ala Gly Gly
           595                 600                 605
   Ala Ser Lys Pro Lys Asp Ala Ser Arg Asn Gly Asp Lys Gln Gln Ala
       610                 615                 620
   Gly Thr Gln Gln Gln Gln Pro Ser Gln Val Gln Gln Gln Gln Ala Ala
   625                 630                 635                 640
   Gln Gln Gln Gln Pro Lys Pro Arg Arg Asn Lys Asn Arg Ser Asn Asn
                   645                 650                 655
   His Thr Asp Gln Pro Ser Gln Gln Gln Gln Leu Ala Glu Asn Val Lys
               660                 665                 670
   Lys Glu Gly Leu Val Asn Gly Thr Ser
           675                 680

<210>  2
<211>  632
<212>  PRT
<213>  Artificial Sequence

<220>
<223>  FMR and FXR amino acid sequences

<400>  2

Met Glu Glu Leu Val Val Glu Val Arg Gly Ser Asn Phe Ala Phe Tyr
1               5                   10                  15

Lys Ala Phe Val Lys Asp Val His Glu Asp Ser Ile Thr Val Ala Phe
            20                  25                  30

Glu Asn Asn Trp Gln Pro Asp Arg Gln Ile Pro Phe His Asp Val Arg
        35                  40                  45

Phe Pro Pro Pro Val Gly Tyr Asn Lys Gly Ile Asn Glu Ser Asp Glu
    50                  55                  60

Val Glu Val Tyr Ser Arg Ala Asn Glu Lys Glu Pro Cys Cys Trp Trp
65                  70                  75                  80

Leu Ala Lys Val Arg Met Ile Lys Gly Glu Phe Tyr Val Ile Glu Tyr
                85                  90                  95

Ala Ala Cys Asp Ala Thr Tyr Asn Glu Ile Val Thr Ile Glu Arg Leu
            100                 105                 110

Arg Ser Val Asn Pro Asn Lys Pro Ala Thr Lys Asp Thr Glu Phe His
        115                 120                 125

Lys Ile Lys Leu Asp Val Pro Glu Asp Leu Arg Gln Met Cys Ala Lys

130                 135                 140

Glu Ala Ala His Lys Asp Phe Lys Lys Ala Val Gly Ala Phe Ser Val
145                 150                 155                 160

Thr Tyr Asp Pro Glu Asn Tyr Gln Leu Val Ile Leu Ser Ile Asn Glu
                165                 170                 175

Val Thr Ser Lys Arg Ala His Met Leu Ile Asp Met His Phe Arg Ser
            180                 185                 190

Leu Arg Thr Lys Leu Ser Leu Ile Met Arg Asn Glu Glu Ala Ser Lys
        195                 200                 205

Gln Leu Glu Ser Ser Arg Gln Leu Ala Ser Arg Phe His Glu Gln Phe
    210                 215                 220

Ile Val Arg Glu Asp Leu Met Gly Leu Ala Ile Gly Thr His Gly Ala
225                 230                 235                 240

Asn Ile Gln Gln Ala Arg Lys Val Pro Gly Val Thr Ala Ile Leu Asp
                245                 250                 255

Glu Asp Thr Cys Thr Phe His Ile Tyr Gly Glu Asp Gln Asp Ala Val
            260                 265                 270

Lys Lys Ala Arg Ser Phe Leu Glu Phe Ala Glu Asp Tyr Ile Gln Val
        275                 280                 285

Pro Arg Asn Leu Val Gly Lys Val Ile Gly Lys Asn Gly Leu Lys Ile
    290                 295                 300

Gln Glu Ile Val Asp Lys Ser Gly Val Val Arg Val Arg Ile Glu Ala
305                 310                 315                 320

Glu Asn Glu Lys Asn Val Pro Gln Glu Glu Glu Ile Met Pro Pro Asn
                325                 330                 335

Ser Leu Pro Ser Asn Asn Ser Arg Val Gly Pro Asn Ala Pro Glu Glu
            340                 345                 350

Lys Lys His Leu Asp Ile Lys Glu Asn Ser Thr His Phe Ser Gln Pro
        355                 360                 365

Asn Ser Thr Lys Val Gln Arg Val Leu Val Ala Ser Ser Val Val Ala

    370                 375                 380

Gly Glu Ser Gln Lys Pro Glu Leu Lys Ala Trp Gln Gly Met Val Pro
385                 390                 395                 400

Phe Val Phe Val Gly Thr Lys Asp Ser Ile Ala Asn Ala Thr Val Leu
                405                 410                 415

Leu Asp Tyr His Leu Asn Tyr Leu Lys Glu Val Asp Gln Leu Arg Leu
            420                 425                 430

Glu Arg Leu Gln Ile Asp Glu Gln Leu Arg Gln Ile Gly Ala Ser Ser
        435                 440                 445

Arg Pro Pro Pro Asn Arg Thr Asp Lys Glu Lys Ser Tyr Val Thr Asp
    450                 455                 460

Asp Gly Gln Gly Met Gly Arg Gly Ser Arg Pro Tyr Arg Asn Arg Gly
465                 470                 475                 480

His Gly Arg Arg Gly Pro Gly Tyr Thr Ser Gly Thr Asn Ser Glu Ala
                485                 490                 495

Ser Asn Ala Ser Glu Thr Glu Ser Asp His Arg Asp Glu Leu Ser Asp
            500                 505                 510

Trp Ser Leu Ala Pro Thr Glu Glu Glu Arg Glu Ser Phe Leu Arg Arg
        515                 520                 525

Gly Asp Gly Arg Arg Arg Gly Gly Gly Gly Arg Gly Gln Gly Gly Arg
    530                 535                 540

Gly Arg Gly Gly Gly Phe Lys Gly Asn Asp Asp His Ser Arg Thr Asp
545                 550                 555                 560

Asn Arg Pro Arg Asn Pro Arg Glu Ala Lys Gly Arg Thr Thr Asp Gly
                565                 570                 575

Ser Leu Gln Ile Arg Val Asp Cys Asn Asn Glu Arg Ser Val His Thr
            580                 585                 590

Lys Thr Leu Gln Asn Thr Ser Ser Glu Gly Ser Arg Leu Arg Thr Gly
        595                 600                 605

Lys Asp Arg Asn Gln Lys Lys Glu Lys Pro Asp Ser Val Asp Gly Gln

610                 615                 620

Gln Pro Leu Val Asn Gly Val Pro
625                 630

<210>  3
<211>  612
<212>  PRT
<213>  Artificial Sequence

<220>
<223>  FMR and FXR amino acid sequences

<400>  3

Met Ala Asp Val Thr Val Glu Val Arg Gly Ser Asn Gly Ala Phe Tyr
1               5                   10                  15

Lys Gly Phe Ile Lys Asp Val His Glu Asp Ser Leu Thr Val Val Phe
            20                  25                  30

Glu Asn Asn Trp Gln Pro Glu Arg Gln Val Pro Phe Asn Glu Val Arg
        35                  40                  45

Leu Pro Pro Pro Pro Asp Ile Lys Lys Glu Ile Ser Glu Gly Asp Glu
    50                  55                  60

Val Glu Val Tyr Ser Arg Ala Asn Asp Gln Glu Pro Cys Gly Trp Trp
65                  70                  75                  80

Leu Ala Lys Val Arg Met Met Lys Gly Glu Phe Tyr Val Ile Glu Tyr
                85                  90                  95

Ala Ala Cys Asp Ala Thr Tyr Asn Glu Ile Val Thr Phe Glu Arg Leu
            100                 105                 110

Arg Pro Val Asn Gln Asn Lys Thr Val Lys Lys Asn Thr Phe Phe Lys
        115                 120                 125

Cys Thr Val Asp Val Pro Glu Asp Leu Arg Glu Ala Cys Ala Asn Glu
    130                 135                 140

Asn Ala His Lys Asp Phe Lys Lys Ala Val Gly Ala Cys Arg Ile Phe
145                 150                 155                 160

Tyr His Pro Glu Thr Thr Gln Leu Met Ile Leu Ser Ala Ser Glu Ala
                165                 170                 175

Thr Val Lys Arg Val Asn Ile Leu Ser Asp Met His Leu Arg Ser Ile
        180                 185                 190

Arg Thr Lys Leu Met Leu Met Ser Arg Asn Glu Glu Ala Thr Lys His
    195                 200                 205

Leu Glu Cys Thr Lys Gln Leu Ala Ala Ala Phe His Glu Glu Phe Val
    210                 215                 220

Val Arg Glu Asp Leu Met Gly Leu Ala Ile Gly Thr His Gly Ser Asn
225                 230                 235                 240

Ile Gln Gln Ala Arg Lys Val Pro Gly Val Thr Ala Ile Glu Leu Asp
                245                 250                 255

Glu Asp Thr Gly Thr Phe Arg Ile Tyr Gly Glu Ser Ala Asp Ala Val
        260                 265                 270

Lys Lys Ala Arg Gly Phe Leu Glu Phe Val Glu Asp Phe Ile Gln Val
    275                 280                 285

Pro Arg Glu Leu Val Gly Lys Val Ile Gly Lys Asn Gly Arg Val Ile
    290                 295                 300

Gln Glu Ile Val Asp Lys Ser Gly Val Val Arg Ile Arg Ile Glu Gly
305                 310                 315                 320

Asp Asn Glu Asn Lys Leu Pro Arg Glu Asp Gly Met Val Pro Phe Val
                325                 330                 335

Phe Val Gly Thr Lys Glu Ser Ile Gly Asn Val Gln Val Leu Leu Glu
        340                 345                 350

Tyr His Ile Ala Tyr Leu Lys Glu Val Glu Gln Leu Arg Met Glu Arg
    355                 360                 365

Leu Gln Ile Asp Glu Gln Leu Arg Gln Ile Gly Ser Arg Ser Tyr Ser
    370                 375                 380

Gly Arg Gly Arg Gly Arg Arg Gly Pro Asn Tyr Thr Ser Gly Tyr Gly
385                 390                 395                 400

Thr Asn Ser Glu Leu Ser Asn Pro Ser Glu Thr Glu Ser Glu Arg Lys
                405                 410                 415

Asp Glu Leu Ser Asp Trp Ser Leu Ala Gly Glu Asp Asn Arg Asp Ser
            420                 425                 430

Arg His Gln Arg Asp Ser Arg Arg Arg Pro Gly Gly Arg Gly Arg Ser
        435                 440                 445

Val Ser Gly Gly Arg Gly Arg Gly Gly Pro Arg Gly Arg Lys Ser Ser
    450                 455                 460

Ile Ser Ser Val Leu Lys Asp Pro Asp Ser Asn Pro Tyr Ser Leu Leu
465                 470                 475                 480

Asp Asn Thr Glu Ser Asp Gln Thr Ala Asp Thr Asp Ala Ser Glu Ser
                485                 490                 495

His His Ser Thr Asn Arg Arg Arg Arg Ser Arg Arg Arg Arg Thr Asp
            500                 505                 510

Glu Asp Ala Val Leu Met Asp Gly Met Thr Glu Ser Asp Thr Ala Ser
        515                 520                 525

Val Asn Glu Asn Gly Leu Val Thr Val Ala Asp Tyr Ile Ser Arg Ala
    530                 535                 540

Glu Ser Gln Ser Arg Gln Arg Asn Leu Pro Arg Glu Thr Leu Ala Lys
545                 550                 555                 560

Asn Lys Lys Glu Met Ala Lys Asp Val Ile Glu Leu His Gly Pro Ser
                565                 570                 575

Glu Lys Ala Ile Asn Gly Pro Thr Ser Ala Ser Gly Asp Asp Ile Ser
            580                 585                 590

Lys Leu Gln Arg Thr Pro Gly Glu Glu Lys Ile Asn Thr Leu Lys Glu
        595                 600                 605

Glu Asn Thr Gln
    610

<210>  4
<211>  664
<212>  PRT
<213>  Artificial Sequence

<220>
<223>  FMR and FXR amino acid sequence

<400>  4
   Met Gly Gly Leu Ala Ser Gly Gly Asp Val Glu Pro Gly Leu Pro Val
   1               5                   10                  15
   Glu Val Arg Gly Ser Asn Gly Ala Phe Tyr Lys Gly Phe Val Lys Asp
               20                  25                  30
   Val His Glu Asp Ser Val Thr Ile Phe Phe Glu Asn Asn Trp Gln Ser
           35                  40                  45
   Glu Arg Gln Ile Pro Phe Gly Asp Val Arg Leu Pro Pro Pro Ala Asp
       50                  55                  60
   Tyr Asn Lys Glu Ile Thr Glu Gly Asp Glu Val Glu Val Tyr Ser Arg
   65                  70                  75                  80
   Ala Asn Glu Gln Glu Pro Cys Gly Trp Trp Leu Ala Arg Val Arg Met
                   85                  90                  95
   Met Lys Gly Asp Phe Tyr Val Ile Glu Tyr Ala Ala Cys Asp Ala Thr
               100                 105                 110
   Tyr Asn Glu Ile Val Thr Leu Glu Arg Leu Arg Pro Val Asn Pro Asn
           115                 120                 125
   Pro Leu Ala Thr Lys Gly Ser Phe Phe Lys Val Thr Met Ala Val Pro
       130                 135                 140
   Glu Asp Leu Arg Glu Ala Cys Ser Asn Glu Asn Val His Lys Glu Phe
   145                 150                 155                 160
   Lys Lys Ala Leu Gly Ala Asn Cys Ile Phe Leu Asn Ile Thr Asn Ser
                   165                 170                 175
   Glu Leu Phe Ile Leu Ser Thr Thr Glu Ala Pro Val Lys Arg Ala Ser
               180                 185                 190
   Leu Leu Gly Asp Met His Phe Arg Ser Leu Arg Thr Lys Leu Leu Leu
           195                 200                 205
   Met Ser Arg Asn Glu Glu Ala Thr Lys His Leu Glu Thr Ser Lys Gln
       210                 215                 220
   Leu Ala Ala Ala Phe Gln Glu Glu Phe Thr Val Arg Glu Asp Leu Met
   225                 230                 235                 240
   Gly Leu Ala Ile Gly Thr His Gly Ala Asn Ile Gln Gln Ala Arg Lys
                   245                 250                 255
   Val Pro Gly Val Thr Ala Ile Glu Leu Gly Glu Glu Thr Cys Thr Phe
               260                 265                 270
   Arg Ile Tyr Gly Glu Thr Pro Glu Ala Cys Arg Gln Ala Arg Ser Tyr
           275                 280                 285
   Leu Glu Phe Ser Glu Asp Ser Val Gln Val Pro Arg Glu Leu Val Gly
       290                 295                 300
   Lys Val Ile Gly Lys Asn Gly Arg Val Ile Gln Glu Ile Val Asp Lys
   305                 310                 315                 320
   Ser Gly Val Val Arg Val Arg Val Glu Gly Asp Asn Asp Lys Lys Asn
                   325                 330                 335
   Pro Arg Glu Glu Gly Met Val Pro Phe Ile Phe Val Gly Thr Arg Glu
               340                 345                 350
   Asn Ile Ser Asn Ala Gln Ala Leu Leu Leu Tyr His Leu Ser Tyr Leu
           355                 360                 365
   Gln Glu Val Glu Gln Leu Arg Leu Glu Arg Leu Gln Ile Asp Glu Gln
       370                 375                 380
   Leu Arg Gln Ile Gln Leu Gly Phe Arg Pro Pro Gly Ser Gly Arg Gly
   385                 390                 395                 400
   Ser Gly Gly Ser Asp Lys Ala Gly Tyr Ser Thr Asp Glu Ser Ser Ser
                   405                 410                 415
   Ser Ser Leu His Ala Thr Arg Thr Tyr Gly Gly Ser Tyr Gly Gly Arg
               420                 425                 430
   Gly Arg Gly Arg Arg Thr Gly Gly Pro Ala Tyr Gly Pro Ser Ser Asp
           435                 440                 445
   Val Ser Thr Ala Ser Glu Thr Glu Ser Glu Lys Arg Glu Leu Pro Asn
       450                 455                 460
   Arg Ala Gly Pro Gly Asp Arg Asp Pro Pro Thr Arg Gly Glu Glu Ser
   465                 470                 475                 480
   Arg Arg Arg Pro Thr Gly Gly Arg Gly Arg Gly Pro Pro Pro Ala Pro
                   485                 490                 495
   Arg Pro Thr Ser Arg Tyr Asn Ser Ser Ser Ile Ser Ser Val Leu Lys
               500                 505                 510
   Asp Pro Asp Ser Asn Pro Tyr Ser Leu Leu Asp Thr Ser Glu Pro Glu
           515                 520                 525
   Pro Pro Val Asp Ser Glu Pro Gly Glu Pro Pro Pro Ala Ser Ala Arg
       530                 535                 540
   Arg Arg Arg Ser Arg Arg Arg Arg Thr Asp Glu Asp Arg Thr Val Met
   545                 550                 555                 560
   Asp Gly Gly Leu Glu Ser Asp Gly Pro Asn Met Thr Glu Asn Gly Leu
                   565                 570                 575
   Glu Asp Glu Ser Arg Pro Gln Arg Arg Asn Arg Ser Arg Arg Arg Arg
               580                 585                 590
   Asn Arg Gly Asn Arg Thr Asp Gly Ser Ile Ser Gly Asp Arg Gln Pro
           595                 600                 605
   Val Thr Val Ala Asp Tyr Ile Ser Arg Ala Glu Ser Gln Ser Arg Gln
       610                 615                 620
   Ser Ala Pro Leu Glu Arg Thr Lys Pro Ser Leu Asp Ser Leu Ser Gly
   625                 630                 635                 640
   Gln Lys Gly Asp Ser Val Ser Lys Leu Pro Lys Gly Pro Ser Glu Asn
                   645                 650                 655
   Gly Glu Leu Ser Ala Pro Leu Glu
               660

<210>  5
<211>  614
<212>  PRT
<213>  Artificial Sequence

<220>
<223>  Amino acid sequence for mouse FMR1

<400>  5

Met Glu Glu Leu Val Val Glu Val Arg Gly Ser Asn Gly Ala Phe Tyr
1               5                   10                  15

Lys Ala Phe Val Lys Asp Val His Glu Asp Ser Ile Thr Val Ala Phe
            20                  25                  30

Glu Asn Asn Trp Gln Pro Glu Arg Gln Ile Pro Phe His Asp Val Arg
        35                  40                  45

Phe Pro Pro Pro Val Gly Tyr Asn Lys Asp Ile Asn Glu Ser Asp Glu
    50                  55                  60

Val Glu Val Tyr Ser Arg Ala Asn Glu Lys Glu Pro Cys Cys Trp Trp
65                  70                  75                  80

Leu Ala Lys Val Arg Met Ile Lys Gly Glu Phe Tyr Val Ile Glu Tyr
                85                  90                  95

Ala Ala Cys Asp Ala Thr Tyr Asn Glu Ile Val Thr Ile Glu Arg Leu
            100                 105                 110

Arg Ser Val Asn Pro Asn Lys Pro Ala Thr Lys Asp Thr Phe His Lys
        115                 120                 125

Ile Lys Leu Glu Val Pro Glu Asp Leu Arg Gln Met Cys Ala Lys Glu
    130                 135                 140

Ser Ala His Lys Asp Phe Lys Lys Ala Val Gly Ala Phe Ser Val Thr
145                 150                 155                 160

Tyr Asp Pro Glu Asn Tyr Gln Leu Val Ile Leu Ser Ile Asn Glu Val
                165                 170                 175

Thr Ser Lys Arg Ala His Met Leu Ile Asp Met His Phe Arg Ser Leu
            180                 185                 190

Arg Thr Lys Leu Ser Leu Ile Leu Arg Asn Glu Glu Ala Ser Lys Gln
        195                 200                 205

Leu Glu Ser Ser Arg Gln Leu Ala Ser Arg Phe His Glu Gln Phe Ile
    210                 215                 220

Val Arg Glu Asp Leu Met Gly Leu Ala Ile Gly Thr His Gly Ala Asn
225                 230                 235                 240

Ile Gln Gln Ala Arg Lys Val Pro Gly Val Thr Ala Ile Asp Leu Asp
                245                 250                 255

Glu Asp Thr Cys Thr Phe His Ile Tyr Gly Glu Asp Gln Asp Ala Val
            260                 265                 270

Lys Lys Ala Arg Ser Phe Leu Glu Phe Ala Glu Asp Val Ile Gln Val
        275                 280                 285

Pro Arg Asn Leu Val Gly Lys Val Ile Gly Lys Asn Gly Lys Leu Ile
    290                 295                 300

Gln Glu Ile Val Asp Lys Ser Gly Val Val Arg Val Arg Ile Glu Ala
305                 310                 315                 320

Glu Asn Glu Lys Ser Val Pro Gln Glu Glu Glu Ile Met Pro Pro Ser
                325                 330                 335

Ser Leu Pro Ser Asn Asn Ser Arg Val Gly Pro Asn Ser Ser Glu Glu
            340                 345                 350

Lys Lys His Leu Asp Thr Lys Glu Asn Thr His Phe Ser Gln Pro Asn
        355                 360                 365

Ser Thr Lys Val Gln Arg Val Leu Val Val Ser Ser Ile Val Ala Gly
    370                 375                 380

Gly Pro Gln Lys Pro Glu Pro Lys Ala Trp Gln Gly Met Val Pro Phe
385                 390                 395                 400

Val Phe Val Gly Thr Lys Asp Ser Ile Ala Asn Ala Thr Val Leu Leu
                405                 410                 415

Asp Tyr His Leu Asn Tyr Leu Lys Glu Val Asp Gln Leu Arg Leu Glu
            420                 425                 430

Arg Leu Gln Ile Asp Glu Gln Leu Arg Gln Ile Gly Ala Ser Ser Arg
        435                 440                 445

Pro Pro Pro Asn Arg Thr Asp Lys Glu Lys Gly Tyr Val Thr Asp Asp
    450                 455                 460

Gly Gln Gly Met Gly Arg Gly Ser Arg Pro Tyr Arg Asn Arg Gly His
465                 470                 475                 480

Gly Arg Arg Gly Pro Gly Tyr Thr Ser Gly Thr Asn Ser Glu Ala Ser
                485                 490                 495

Asn Ala Ser Glu Thr Glu Ser Asp His Arg Asp Glu Leu Ser Asp Trp
            500                 505                 510

Ser Leu Ala Pro Thr Glu Glu Glu Arg Glu Ser Phe Leu Arg Arg Gly
        515                 520                 525

Asp Gly Arg Arg Arg Gly Gly Gly Gly Arg Gly Gln Gly Gly Arg Gly
    530                 535                 540

Arg Gly Gly Gly Phe Lys Gly Asn Asp Asp His Ser Arg Thr Asp Asn
545                 550                 555                 560

Arg Pro Arg Asn Pro Arg Glu Ala Lys Gly Arg Thr Ala Asp Gly Ser
                565                 570                 575

Leu Gln Ser Ala Ser Ser Glu Gly Ser Arg Leu Arg Thr Gly Lys Asp
            580                 585                 590

Arg Asn Gln Lys Lys Glu Lys Pro Asp Ser Val Asp Gly Leu Gln Pro
        595                 600                 605

Leu Val Asn Gly Val Pro
    610


<210>  6
<211>  545
<212>  PRT
<213>  Artificial Sequence

<220>
<223>  Human PAK1, PAK2 and PAK3 amino acid sequences

<400>  6

Met Ser Asn Asn Gly Leu Asp Ile Gln Asp Lys Pro Pro Ala Pro Pro
1               5                   10                  15

Met Arg Asn Thr Ser Thr Met Ile Gly Val Gly Ser Lys Asp Ala Gly
            20                  25                  30

Thr Leu Asn His Gly Ser Lys Pro Leu Pro Pro Asn Pro Arg Arg Lys
        35                  40                  45

Lys Lys Lys Asp Arg Phe Tyr Arg Ser Ile Leu Pro Gly Asp Lys Thr

50                  55                  60

Asn Lys Lys Lys His Lys Glu Arg Pro Arg Ile Ser Leu Pro Ser Asp
65                  70                  75                  80

Phe Glu His Thr Ile His Val Gly Phe Asp Ala Val Thr Gly Glu Phe
                85                  90                  95

Thr Gly Met Pro Glu Gln Met Ala Arg Leu Leu Gln Thr Ser Asn Ile
            100                 105                 110

Thr Lys Ser Glu Gln Lys Lys Asn Pro Gln Ala Val Leu Asp Val Leu
        115                 120                 125

Glu Phe Tyr Asn Ser Lys Lys Thr Ser Asn Ser Gln Lys Tyr Met Ser
    130                 135                 140

Phe Thr Asp Lys Ser Ala Glu Asp Tyr Asn Ser Ser Asn Ala Leu Asn
145                 150                 155                 160

Val Lys Ala Val Ser Glu Thr Pro Ala Val Pro Pro Val Ser Arg Asp
                165                 170                 175

Glu Asp Asp Asp Asp Asp Asp Ala Thr Pro Pro Pro Val Ile Ala Pro
            180                 185                 190

Arg Pro Glu His Thr Lys Ser Val Tyr Thr Arg Ser Val Ile Arg Pro
        195                 200                 205

Leu Pro Val Thr Pro Thr Arg Asp Val Ala Thr Ser Pro Ile Ser Pro
    210                 215                 220

Thr Glu Asn Asn Thr Thr Pro Pro Asp Ala Leu Thr Arg Asn Thr Glu
225                 230                 235                 240

Lys Gln Lys Lys Lys Pro Lys Met Ser Asp Glu Glu Ile Leu Arg Lys
                245                 250                 255

Leu Arg Ser Ile Val Ser Val Gly Asp Pro Lys Lys Lys Tyr Thr Arg
            260                 265                 270

Phe Glu Lys Asp Gly Gln Gly Ala Ser Gly Thr Val Tyr Thr Ala Met
        275                 280                 285

Asp Val Ala Thr Gly Gln Glu Val Ala Ile Lys Gln Met Asn Leu Gln

290                 295                 300

Gln Gln Pro Lys Lys Glu Leu Ile Ile Asn Arg Ile Leu Val Met Arg
305                 310                 315                 320

Glu Asn Lys Asn Pro Asn Ile Val Asn Tyr Leu Asp Ser Tyr Leu Val
                325                 330                 335

Gly Asp Glu Leu Met Val Val Met Glu Tyr Leu Ala Gly Gly Ser Leu
            340                 345                 350

Thr Asp Val Val Thr Glu Thr Cys Met Asp Glu Gly Gln Ile Ala Ala
        355                 360                 365

Val Cys Arg Glu Cys Leu Gln Ala Leu Glu Phe Leu His Ser Asn Gln
    370                 375                 380

Val Ile His Arg Asp Ile Lys Ser Asp Asn Ile Leu Leu Gly Met Asp
385                 390                 395                 400

Gly Ser Val Lys Leu Thr Asp Phe Gly Phe Cys Ala Gln Ile Thr Pro
                405                 410                 415

Glu Gln Ser Lys Arg Ser Thr Met Val Gly Thr Pro Tyr Met Met Ala
            420                 425                 430

Pro Glu Val Val Thr Arg Lys Ala Tyr Gly Pro Lys Val Asp Ile Met
        435                 440                 445

Ser Leu Gly Ile Met Ala Ile Glu Met Ile Glu Gly Glu Pro Pro Tyr
    450                 455                 460

Leu Asn Glu Asn Pro Leu Arg Ala Leu Tyr Leu Ile Ala Thr Asn Gly
465                 470                 475                 480

Thr Pro Glu Leu Gln Asn Pro Glu Lys Leu Ser Ala Ile Phe Arg Asp
                485                 490                 495

Phe Leu Asn Arg Cys Leu Asp Met Asp Val Glu Lys Arg Gly Ser Ala
            500                 505                 510

Lys Glu Leu Leu Gln His Gln Phe Leu Lys Ile Ala Lys Pro Leu Ser
        515                 520                 525

Ser Leu Thr Pro Leu Ile Ala Ala Ala Lys Glu Ala Thr Lys Asn Asn

530                 535                 540

His
545

<210>  7
<211>  525
<212>  PRT
<213>  Artificial Sequence

<220>
<223>  Human PAK1, PAK2 and PAK3 amino acid sequences

<400>  7

Met Ser Asp Asn Gly Glu Leu Arg Asp Lys Pro Pro Ala Pro Pro Val
1               5                   10                  15

Arg Met Ser Ser Thr Ile Phe Ser Thr Gly Gly Lys Asp Pro Leu Ser
            20                  25                  30

Ala Asn His Ser Leu Lys Pro Leu Pro Ser Val Pro Arg Arg Lys Lys
        35                  40                  45

Pro Arg His Lys Ile Ile Ser Ile Phe Ser Gly Thr Glu Lys Gly Ser
    50                  55                  60

Lys Lys Lys Glu Lys Glu Arg Pro Arg Ile Ser Pro Pro Ser Asp Phe
65                  70                  75                  80

Glu His Thr Ile His Val Gly Phe Asp Ala Val Thr Gly Glu Phe Thr
                85                  90                  95

Gly Met Pro Glu Gln Met Ala Arg Leu Leu Gln Thr Ser Asn Ile Thr
            100                 105                 110

Lys Leu Glu Gln Lys Lys Asn Pro Gln Ala Val Leu Asp Val Leu Lys
        115                 120                 125

Phe Tyr Asp Ser Asn Thr Val Lys Gln Lys Tyr Leu Ser Phe Thr Pro
    130                 135                 140

Pro Glu Lys Asp Gly Leu Pro Ser Gly Thr Pro Ala Leu Asn Ala Lys
145                 150                 155                 160

Gly Thr Glu Ala Pro Ala Val Val Thr Phe Glu Glu Asp Asp Asp Glu
                165                 170                 175

Glu Thr Ala Pro Pro Val Ile Ala Pro Arg Pro Asp His Thr Lys Ser
            180                 185                 190

Ile Tyr Thr Arg Ser Val Ile Asp Pro Val Pro Ala Pro Val Gly Asp
        195                 200                 205

Ser His Val Asp Cys Ala Ala Lys Ser Leu Asp Lys Gln Lys Lys Lys
    210                 215                 220

Pro Lys Met Thr Asp Glu Glu Ile Met Arg Lys Leu Arg Thr Ile Val
225                 230                 235                 240

Ser Ile Gly Asn Pro Lys Lys Lys Tyr Thr Arg Tyr Glu Lys Asp Gly
                245                 250                 255

Gln Gly Ala Ser Gly Thr Val Phe Thr Ala Thr Asp Val Ala Leu Gly
            260                 265                 270

Gln Glu Val Ala Ile Lys Gln Ile Asn Leu Gln Lys Gln Pro Lys Lys
        275                 280                 285

Glu Leu Ile Ile Asn Arg Ile Leu Val Met Lys Glu Leu Lys Asn Pro
    290                 295                 300

Asn Ile Val Asn Phe Leu Asp Ser Tyr Leu Val Gly Asp Glu Leu Phe
305                 310                 315                 320

Val Val Met Glu Tyr Leu Ala Gly Gly Ser Leu Thr Asp Val Val Thr
                325                 330                 335

Glu Thr Ala Cys Met Asp Glu Ala Gln Ile Ala Ala Val Cys Arg Glu
            340                 345                 350

Cys Leu Gln Ala Leu Glu Phe Leu His Ala Asn Gln Val Ile His Arg
        355                 360                 365

Asp Ile Lys Ser Asp Asn Val Leu Leu Gly Met Glu Gly Ser Val Lys
    370                 375                 380

Leu Thr Asp Phe Gly Phe Cys Ala Gln Ile Thr Pro Glu Gln Ser Lys
385                 390                 395                 400

Arg Ser Thr Met Val Gly Thr Pro Tyr Met Met Ala Pro Glu Val Val
                405                 410                 415

Thr Arg Lys Ala Tyr Gly Pro Lys Val Asp Ile Met Ser Leu Gly Ile
            420                 425                 430

Met Ala Ile Glu Met Val Glu Gly Glu Pro Pro Tyr Leu Asn Glu Asn
        435                 440                 445

Pro Leu Arg Ala Leu Tyr Leu Ile Ala Thr Asn Gly Thr Pro Glu Leu
    450                 455                 460

Gln Asn Pro Glu Lys Leu Ser Pro Ile Phe Arg Asp Phe Leu Asn Arg
465                 470                 475                 480

Cys Leu Glu Met Asp Val Glu Lys Arg Gly Ser Ala Lys Glu Leu Leu
                485                 490                 495

Gln His Pro Phe Leu Lys Leu Ala Lys Pro Leu Ser Ser Leu Thr Pro
            500                 505                 510

Leu Ile Met Ala Ala Lys Glu Ala Met Lys Ser Asn Arg
        515                 520                 525

<210>  8
<211>  544
<212>  PRT
<213>  Artificial Sequence

<220>
<223>  Human PAK1, PAK2 and PAK3 amino acid sequences

<400>  8

Met Ser Asp Gly Leu Asp Asn Glu Glu Lys Pro Pro Ala Pro Pro Leu
1               5                   10                  15

Arg Met Asn Ser Asn Asn Arg Asp Ser Ser Ala Leu Asn His Ser Ser
            20                  25                  30

Lys Pro Leu Pro Met Ala Pro Ser Glu Lys Asn Lys Lys Ala Arg Leu
        35                  40                  45

Arg Ser Ile Phe Pro Gly Gly Gly Asp Lys Thr Asn Lys Lys Lys Glu
    50                  55                  60

Lys Glu Arg Pro Arg Ile Ser Leu Pro Ser Asp Phe Glu His Thr Ile
65                  70                  75                  80

His Val Gly Phe Asp Ala Val Thr Gly Glu Phe Thr Gly Ile Pro Glu

85                  90                  95

Gln Met Ala Arg Leu Leu Gln Thr Ser Asn Ile Thr Lys Leu Glu Gln
        100                 105                 110

Lys Lys Asn Pro Gln Ala Val Leu Asp Val Leu Lys Phe Tyr Asp Ser
        115                 120                 125

Lys Glu Thr Val Asn Asn Gln Lys Tyr Met Ser Phe Thr Ser Gly Asp
    130                 135                 140

Lys Ser Ala His Gly Tyr Ile Ala Ala His Pro Ser Ser Thr Lys Thr
145                 150                 155                 160

Ala Ser Glu Pro Pro Leu Ala Pro Pro Val Ser Arg Arg Arg Asp Arg
                165                 170                 175

Arg Arg Arg Arg Arg Arg Asp Arg Asn Glu Pro Pro Pro Val Ile Ala
        180                 185                 190

Pro Arg Pro Glu His Thr Lys Ser Ile Tyr Thr Arg Ser Val Val Glu
        195                 200                 205

Ser Ile Ala Ser Pro Ala Val Pro Asn Lys Glu Val Thr Pro Pro Ser
    210                 215                 220

Ala Glu Asn Ala Asn Ser Ser Thr Leu Tyr Arg Asn Thr Asp Arg Gln
225                 230                 235                 240

Arg Lys Lys Ser Lys Asn Thr Asp Glu Glu Ile Leu Arg Lys Leu Arg
                245                 250                 255

Ser Ile Val Ser Val Gly Asp Pro Lys Lys Lys Tyr Thr Arg Glu Arg
            260                 265                 270

Lys Asp Gly Gln Gly Ala Ser Gly Thr Val Tyr Thr Ala Leu Asp Ile
        275                 280                 285

Ala Thr Gly Gln Glu Val Ala Ile Lys Gln Met Asn Leu Gln Gln Gln
    290                 295                 300

Pro Lys Lys Glu Leu Ile Ile Asn Arg Ile Leu Val Met Arg Glu Asn
305                 310                 315                 320

Lys Asn Pro Asn Ile Val Asn Tyr Leu Asp Ser Tyr Leu Val Gly Asp

325                 330                 335

Glu Leu Met Val Val Met Glu Tyr Leu Ala Gly Gly Ser Leu Thr Asp
        340                 345                 350

Val Val Thr Glu Thr Cys Met Asp Glu Gly Gln Ile Ala Ala Val Cys
        355                 360                 365

Arg Glu Cys Leu Gln Ala Leu Asp Phe Leu His Ser Asn Gln Val Ile
    370                 375                 380

His Arg Asp Ile Lys Ser Asp Asn Ile Leu Leu Gly Met Asp Gly Ser
385                 390                 395                 400

Val Lys Leu Thr Asp Phe Gly Phe Cys Ala Gln Ile Thr Pro Glu Gln
                405                 410                 415

Ser Lys Arg Ser Thr Met Val Gly Thr Pro Tyr Met Met Ala Pro Glu
        420                 425                 430

Val Val Thr Arg Lys Ala Tyr Gly Pro Lys Val Asp Ile Met Ser Leu
        435                 440                 445

Gly Ile Met Ala Ile Glu Met Val Glu Gly Glu Pro Pro Tyr Leu Asn
    450                 455                 460

Glu Asn Pro Leu Arg Ala Leu Tyr Leu Ile Ala Thr Asn Gly Thr Pro
465                 470                 475                 480

Glu Leu Gln Asn Pro Glu Arg Leu Ser Ala Val Pro Arg Asp Phe Leu
                485                 490                 495

Asn Arg Cys Leu Glu Met Asp Val Glu Arg Arg Gly Ser Ala Lys Glu
        500                 505                 510

Leu Leu Gln His Phe Pro Leu Lys Leu Ala Lys Pro Leu Ser Ser Leu
        515                 520                 525

Thr Pro Leu Ile Ile Ala Ala Lys Glu Ala Ile Lys Asn Ser Ser Arg
    530                 535                 540

<210>  9
<211>  591
<212>  PRT
<213>  Artificial Sequence

<220>
<223>  Human PAK4, PAK5, PAK6, and PAK7 amino acid sequences

<400>  9

Met Phe Gly Lys Arg Lys Lys Arg Val Glu Ile Ser Ala Pro Ser Asn
1               5                   10                  15

Phe Glu His Arg Val His Thr Gly Phe Asp Gln His Glu Gln Lys Phe
            20                  25                  30

Thr Gly Leu Pro Arg Gln Trp Gln Ser Leu Ile Glu Glu Ser Ala Arg
        35                  40                  45

Arg Pro Lys Pro Leu Val Asp Pro Ala Cys Ile Thr Ser Ile Gln Pro
    50                  55                  60

Gly Ala Pro Lys Thr Ile Val Arg Gly Ser Lys Gly Ala Lys Asp Gly
65                  70                  75                  80

Ala Leu Thr Leu Leu Leu Asp Glu Phe Glu Asn Met Ser Val Thr Arg
                85                  90                  95

Ser Asn Ser Leu Arg Arg Asp Ser Pro Pro Pro Pro Ala Arg Ala Arg
            100                 105                 110

Gln Glu Asn Gly Met Pro Glu Glu Pro Ala Thr Thr Ala Arg Gly Gly
        115                 120                 125

Pro Gly Lys Ala Gly Ser Arg Gly Arg Phe Ala Gly His Ser Glu Ala
    130                 135                 140

Gly Gly Gly Ser Gly Asp Arg Arg Arg Ala Gly Pro Glu Lys Arg Pro
145                 150                 155                 160

Lys Ser Ser Arg Glu Gly Ser Gly Gly Pro Gln Glu Ser Ser Arg Asp
                165                 170                 175

Lys Arg Pro Leu Ser Gly Pro Asp Val Gly Thr Pro Gln Pro Ala Gly
            180                 185                 190

Leu Ala Ser Gly Ala Lys Leu Ala Ala Gly Arg Pro Phe Asn Thr Tyr
        195                 200                 205

Pro Arg Ala Asp Thr Asp His Pro Ser Arg Gly Ala Gln Gly Glu Pro
    210                 215                 220

His Asp Val Ala Pro Asn Gly Pro Ser Ala Gly Gly Leu Ala Ile Pro
225                 230                 235                 240

Gln Ser Ser Ser Ser Ser Ser Arg Pro Pro Thr Arg Ala Arg Gly Ala
                245                 250                 255

Pro Ser Pro Gly Val Leu Gly Pro His Ala Ser Glu Pro Gln Leu Ala
            260                 265                 270

Pro Pro Ala Cys Thr Pro Ala Ala Pro Ala Val Pro Gly Pro Pro Gly
        275                 280                 285

Pro Arg Ser Pro Gln Arg Glu Pro Gln Arg Val Ser His Glu Gln Phe
    290                 295                 300

Arg Ala Ala Leu Gln Leu Val Val Asp Pro Gly Asp Pro Arg Ser Tyr
305                 310                 315                 320

Leu Asp Asn Phe Ile Lys Ile Gly Glu Gly Ser Thr Gly Ile Val Cys
                325                 330                 335

Ile Ala Thr Val Arg Ser Ser Gly Lys Leu Val Ala Val Lys Lys Met
            340                 345                 350

Asp Leu Arg Lys Gln Gln Arg Arg Glu Leu Leu Phe Asn Glu Val Val
        355                 360                 365

Ile Met Arg Asp Tyr Gln His Glu Asn Val Val Glu Met Tyr Asn Ser
    370                 375                 380

Tyr Leu Val Gly Asp Glu Leu Trp Val Val Met Glu Phe Leu Glu Gly
385                 390                 395                 400

Gly Ala Leu Thr Asp Ile Val Thr His Thr Arg Met Asn Glu Glu Gln
                405                 410                 415

Ile Ala Ala Val Cys Leu Ala Val Leu Gln Ala Leu Ser Val Leu His
            420                 425                 430

Ala Gln Gly Val Ile His Arg Asp Ile Lys Ser Asp Ser Ile Leu Leu
        435                 440                 445

Thr His Asp Gly Arg Val Lys Leu Ser Asp Phe Gly Phe Cys Ala Gln
    450                 455                 460

Val Ser Lys Glu Val Pro Arg Arg Lys Ser Leu Val Gly Thr Pro Tyr
465                 470                 475                 480

Trp Met Ala Pro Glu Leu Ile Ser Arg Leu Pro Tyr Gly Pro Glu Val
                485                 490                 495

Asp Ile Trp Ser Leu Gly Ile Met Val Ile Glu Trp Val Asp Gly Glu
            500                 505                 510

Pro Pro Tyr Phe Asn Glu Pro Pro Leu Lys Ala Met Lys Met Ile Arg
        515                 520                 525

Asp Asn Leu Pro Pro Arg Leu Lys Asn Leu His Lys Val Ser Pro Ser
    530                 535                 540

Leu Lys Gly Phe Leu Asp Arg Leu Leu Val Arg Asp Pro Ala Gln Arg
545                 550                 555                 560

Ala Thr Ala Ala Glu Leu Leu Lys His Pro Phe Leu Ala Lys Ala Gly
                565                 570                 575

Pro Pro Ala Ser Ile Val Pro Leu Met Arg Gln Asn Arg Thr Arg
            580                 585                 590

<210>  10
<211>  718
<212>  PRT
<213>  Artificial Sequence

<220>
<223>  Human PAK4, PAK5, PAK6, and PAK7 amino acid sequences

<400>  10

Met Phe Gly Lys Lys Lys Lys Lys Ile Glu Ile Ser Gly Pro Ser Asn
1               5                   10                  15

Phe Glu His Arg Val His Thr Gly Phe Asp Pro Gln Glu Gln Lys Phe
            20                  25                  30

Thr Gly Leu Pro Gln Gln Trp His Ser Leu Leu Ala Asp Thr Ala Asn
        35                  40                  45

Arg Pro Lys Pro Met Val Asp Pro Ser Cys Ile Thr Pro Ile Gln Leu
    50                  55                  60

Ala Pro Met Lys Thr Ile Val Arg Gly Asn Lys Pro Cys Lys Glu Thr

65                  70                  75                  80

Ser Ile Asn Gly Leu Leu Glu Asp Phe Asp Asn Ile Ser Val Thr Arg
                85                  90                  95

Ser Asn Ser Leu Arg Lys Glu Ser Pro Pro Thr Pro Asp Gln Gly Ala
            100                 105                 110

Ser Ser His Gly Pro Gly His Ala Glu Glu Asn Gly Phe Ile Thr Phe
        115                 120                 125

Ser Gln Tyr Ser Ser Glu Ser Asp Thr Thr Ala Asp Tyr Thr Thr Glu
    130                 135                 140

Lys Tyr Arg Glu Lys Ser Leu Tyr Gly Asp Asp Leu Asp Pro Tyr Tyr
145                 150                 155                 160

Arg Gly Ser His Ala Ala Lys Gln Asn Gly His Val Met Lys Met Lys
                165                 170                 175

His Gly Glu Ala Tyr Tyr Ser Glu Val Lys Pro Leu Lys Ser Asp Phe
            180                 185                 190

Ala Arg Phe Ser Ala Asp Tyr His Ser His Leu Asp Ser Leu Ser Lys
        195                 200                 205

Pro Ser Glu Tyr Ser Asp Leu Lys Trp Glu Tyr Gln Arg Ala Ser Ser
    210                 215                 220

Ser Ser Pro Leu Asp Tyr Ser Phe Gln Phe Thr Pro Ser Arg Thr Ala
225                 230                 235                 240

Gly Thr Ser Gly Cys Ser Lys Glu Ser Leu Ala Tyr Ser Glu Ser Glu
                245                 250                 255

Trp Gly Pro Ser Leu Asp Asp Tyr Asp Arg Arg Pro Lys Ser Ser Tyr
            260                 265                 270

Leu Asn Gln Thr Ser Pro Gln Pro Thr Met Arg Gln Arg Ser Arg Ser
        275                 280                 285

Gly Ser Gly Leu Gln Glu Pro Met Met Pro Phe Gly Ala Ser Ala Phe
    290                 295                 300

Lys Thr His Pro Gln Gly His Ser Tyr Asn Ser Tyr Thr Tyr Pro Arg

305                 310                 315                 320

Leu Ser Glu Pro Thr Met Cys Ile Pro Lys Val Asp Tyr Asp Arg Ala
                325                 330                 335

Gln Met Val Leu Ser Pro Pro Leu Ser Gly Ser Asp Thr Tyr Pro Arg
            340                 345                 350

Gly Pro Ala Lys Leu Pro Gln Ser Gln Ser Lys Ser Gly Tyr Ser Ser
        355                 360                 365

Ser Ser His Gln Tyr Pro Ser Gly Tyr His Lys Ala Thr Leu Tyr His
    370                 375                 380

His Pro Ser Leu Gln Ser Ser Ser Gln Tyr Ile Ser Thr Ala Ser Tyr
385                 390                 395                 400

Leu Ser Ser Leu Ser Leu Ser Ser Ser Thr Tyr Pro Pro Pro Ser Trp
                405                 410                 415

Gly Ser Ser Ser Asp Gln Gln Pro Ser Arg Val Ser His Glu Gln Phe
            420                 425                 430

Arg Ala Ala Leu Gln Leu Val Val Ser Pro Gly Asp Pro Arg Glu Tyr
        435                 440                 445

Leu Ala Asn Phe Ile Lys Ile Gly Glu Gly Ser Thr Gly Ile Val Cys
    450                 455                 460

Ile Ala Thr Glu Lys His Thr Gly Lys Gln Val Ala Val Lys Lys Met
465                 470                 475                 480

Asp Leu Arg Gln Gln Arg Arg Glu Leu Leu Phe Asn Glu Val Val Ile
                485                 490                 495

Met Arg Asp Tyr His His Asp Asn Val Val Asp Met Tyr Ser Ser Tyr
            500                 505                 510

Leu Val Gly Asp Glu Leu Trp Val Val Met Glu Phe Leu Glu Gly Gly
        515                 520                 525

Ala Leu Thr Asp Ile Val Thr His Thr Arg Met Asn Glu Glu Gln Ile
    530                 535                 540

Ala Thr Val Cys Leu Ser Val Leu Arg Ala Leu Ser Tyr Leu His Asn

545                 550                 555                 560

Gln Gly Val Ile His Arg Asp Ile Lys Ser Asp Ser Ile Leu Leu Thr
                565                 570                 575

Ser Asp Gly Arg Ile Lys Leu Ser Asp Phe Gly Phe Cys Ala Gln Val
            580                 585                 590

Ser Lys Glu Val Pro Lys Arg Lys Ser Leu Val Gly Thr Pro Tyr Trp
        595                 600                 605

Met Ala Pro Glu Val Ile Ser Arg Leu Pro Tyr Gly Thr Glu Val Asp
    610                 615                 620

Ile Trp Ser Leu Gly Ile Met Val Ile Glu Met Ile Asp Gly Glu Pro
625                 630                 635                 640

Pro Tyr Phe Asn Glu Pro Pro Leu Gln Ala Met Arg Arg Ile Arg Asp
                645                 650                 655

Ser Leu Pro Pro Arg Val Lys Asp Leu His Lys Val Ser Ser Val Leu
            660                 665                 670

Arg Gly Phe Leu Asp Leu Met Leu Val Arg Glu Pro Ser Gln Arg Ala
        675                 680                 685

Thr Ala Gln Glu Leu Leu Gly His Pro Phe Leu Lys Leu Ala Gly Pro
    690                 695                 700

Pro Ser Cys Ile Val Pro Leu Met Arg Gln Tyr Arg His His
705                 710                 715

<210>  11
<211>  681
<212>  PRT
<213>  Artificial Sequence

<220>
<223>  Human PAK4, PAK5, PAK6 and PAK7 amino acid sequences

<400>  11
   Met Phe Arg Lys Lys Lys Lys Lys Arg Pro Glu Ile Ser Ala Pro Gln
   1               5                   10                  15
   Asn Phe Gln His Arg Val His Thr Ser Phe Asp Pro Lys Glu Gly Lys
               20                  25                  30
   Phe Val Gly Leu Pro Pro Gln Trp Gln Asn Ile Leu Asp Thr Leu Arg
   35 40 45
   Arg Pro Lys Pro Val Val Asp Pro Ser Arg Ile Thr Arg Val Gln Leu
   50 55 60
   Gln Pro Met Lys Thr Val Val Arg Gly Ser Ala Met Pro Val Asp Gly
   65 70 75 80
   Tyr Ile Ser Gly Leu Leu Asn Asp Ile Gln Lys Leu Ser Val Ile Ser
   85 90 95
   Ser Asn Thr Leu Arg Gly Arg Ser Pro Thr Ser Arg Arg Arg Ala Gln
   100 105 110
   Ser Leu Gly Leu Leu Gly Asp Glu His Trp Ala Thr Asp Pro Asp Met
   115 120 125
   Tyr Leu Gln Ser Pro Gln Ser Glu Arg Thr Asp Pro His Gly Leu Tyr
   130 135 140
   Leu Ser Cys Asn Gly Gly Thr Pro Ala Gly His Lys Gln Met Pro Gln
   145 150 155 160
   Pro Glu Pro Gln Ser Pro Arg Val Leu Pro Asn Gly Leu Ala Ala Lys
   165 170 175
   Gln Ser Leu Gly Pro Ala Glu Phe Gln Gly Ala Ser Gln Arg Cys Leu
   180 185 190
   Gln Leu Gly Ala Cys Leu Gln Ser Ser Pro Pro Gly Ala Ser Pro Pro
   195 200 205
   Thr Gly Thr Asn Arg His Gly Met Lys Ala Ala Lys His Gly Ser Glu
   210 215 220
   Glu Ala Arg Pro Gln Ser Cys Leu Val Gly Ser Ala Thr Gly Arg Pro
   225 230 235 240
   Gly Gly Glu Gly Ser Pro Ser Pro Lys Thr Arg Glu Ser Ser Leu Lys
   245 250 255
   Arg Arg Leu Phe Arg Ser Met Phe Leu Ser Thr Ala Ala Thr Ala Pro
   260 265 270
   Pro Ser Ser Ser Lys Pro Gly Pro Pro Pro Gln Ser Lys Pro Asn Ser
           275                 280                 285
   Ser Phe Arg Pro Pro Gln Lys Asp Asn Pro Pro Ser Leu Val Ala Lys
       290                 295                 300
   Ala Gln Ser Leu Pro Ser Asp Gln Pro Val Gly Thr Phe Ser Pro Leu
   305                 310                 315                 320
   Thr Thr Ser Asp Thr Ser Ser Pro Gln Lys Ser Leu Arg Thr Ala Pro
                   325                 330                 335
   Ala Thr Gly Gln Leu Pro Gly Arg Ser Ser Pro Ala Gly Ser Pro Arg
               340                 345                 350
   Thr Trp His Ala Gln Ile Ser Thr Ser Asn Leu Tyr Leu Pro Gln Asp
           355                 360                 365
   Pro Thr Val Ala Lys Gly Ala Leu Ala Gly Glu Asp Thr Gly Val Val
       370                 375                 380
   Thr His Glu Gln Phe Lys Ala Ala Leu Arg Met Val Val Asp Gln Gly
   385                 390                 395                 400
   Asp Pro Arg Leu Leu Leu Asp Ser Tyr Val Lys Ile Gly Glu Gly Ser
                   405                 410                 415
   Thr Gly Ile Val Cys Leu Ala Arg Glu Lys His Ser Gly Arg Gln Val
               420                 425                 430
   Ala Val Lys Met Met Asp Leu Arg Lys Gln Gln Arg Arg Glu Leu Leu
           435                 440                 445
   Phe Asn Glu Val Val Ile Met Arg Asp Tyr Gln His Phe Asn Val Val
       450                 455                 460
   Glu Met Tyr Lys Ser Tyr Leu Val Gly Glu Glu Leu Trp Val Leu Met
   465                 470                 475                 480
   Glu Phe Leu Gln Gly Gly Ala Leu Thr Asp Ile Val Ser Gln Val Arg
                   485                 490                 495
   Leu Asn Glu Glu Gln Ile Ala Thr Val Cys Glu Ala Val Leu Gln Ala
               500                 505                 510
   Leu Ala Tyr Leu His Ala Gln Gly Val Ile His Arg Asp Ile Lys Ser
           515                 520                 525
   Asp Ser Ile Leu Leu Thr Leu Asp Gly Arg Val Lys Leu Ser Asp Phe
       530                 535                 540
   Gly Phe Cys Ala Gln Ile Ser Lys Asp Val Pro Lys Arg Lys Ser Leu
   545                 550                 555                 560
   Val Gly Thr Pro Tyr Trp Trp Met Ala Pro Glu Val Ile Ser Arg Ser
                   565                 570                 575
   Leu Tyr Ala Thr Glu Val Asp Ile Trp Ser Leu Gly Ile Met Val Ile
               580                 585                 590
   Glu Met Val Asp Gly Glu Pro Pro Tyr Phe Ser Asp Ser Pro Val Gln
           595                 600                 605
   Ala Met Lys Arg Leu Arg Asp Ser Pro Pro Pro Lys Leu Lys Asn Ser
       610                 615                 620
   His Lys Val Ser Pro Val Leu Arg Asp Phe Leu Glu Arg Met Leu Val
   625                 630                 635                 640
   Arg Asp Pro Gln Glu Arg Ala Thr Ala Gln Glu Leu Leu Asp His Pro
                   645                 650                 655
   Phe Leu Leu Gln Thr Gly Leu Pro Glu Cys Leu Val Pro Leu Ile Gly
               660                 665                 670
   Leu Tyr Arg Lys Gln Thr Ser Thr Cys
           675                 680

<210>  12
<211>  681
<212>  PRT
<213>  Artificial Sequence

<220>
<223>  Human PAK4, PAK5, PAK6 and PAK7 amino acid sequences

<400>  12

Met Phe Arg Lys Lys Lys Lys Lys Arg Pro Glu Ile Ser Ala Pro Gln
1               5                   10                  15

Asn Phe Gln His Arg Val His Thr Ser Phe Asp Pro Lys Glu Gly Lys

20                  25                  30

Phe Val Gly Leu Pro Pro Gln Trp Gln Asn Ile Leu Asp Thr Leu Arg
        35                  40                  45

Arg Pro Lys Pro Val Val Asp Pro Ser Arg Ile Thr Arg Val Gln Leu
    50                  55                  60

Gln Pro Met Lys Thr Val Val Arg Gly Ser Ala Met Pro Val Asp Gly
65                  70                  75                  80

Tyr Ile Ser Gly Leu Leu Asn Asp Ile Gln Lys Leu Ser Val Ile Ser
                85                  90                  95

Ser Asn Thr Leu Arg Gly Arg Ser Pro Thr Ser Arg Arg Arg Ala Gln
            100                 105                 110

Ser Leu Gly Leu Leu Gly Asp Glu His Trp Ala Thr Asp Pro Asp Met
        115                 120                 125

Tyr Leu Gln Ser Pro Gln Ser Glu Arg Thr Asp Pro His Gly Leu Tyr
    130                 135                 140

Leu Ser Cys Asn Gly Gly Thr Pro Ala Gly His Lys Gln Met Pro Trp
145                 150                 155                 160

Pro Glu Pro Gln Ser Pro Arg Val Leu Pro Asn Gly Leu Ala Ala Lys
                165                 170                 175

Ala Gln Ser Leu Gly Pro Ala Glu Phe Gln Gly Ala Ser Gln Arg Cys
            180                 185                 190

Leu Gln Leu Gly Ala Cys Leu Gln Ser Ser Pro Pro Gly Ala Ser Pro
        195                 200                 205

Pro Thr Gly Thr Asn Arg His Gly Met Lys Ala Ala Lys His Gly Ser
    210                 215                 220

Glu Glu Ala Arg Pro Gln Ser Cys Leu Val Gly Ser Ala Thr Gly Arg
225                 230                 235                 240

Pro Gly Gly Glu Gly Ser Pro Ser Pro Lys Thr Arg Glu Ser Ser Leu
                245                 250                 255

Lys Arg Arg Leu Phe Arg Ser Met Phe Leu Ser Thr Ala Ala Thr Ala

260                 265                 270

Pro Pro Ser Ser Ser Lys Pro Gly Pro Pro Pro Gln Ser Lys Pro Asn
        275                 280                 285

Ser Ser Phe Arg Pro Pro Gln Lys Asp Asn Pro Pro Ser Leu Val Ala
    290                 295                 300

Lys Ala Gln Ser Leu Pro Ser Asp Gln Pro Val Gly Thr Phe Ser Pro
305                 310                 315                 320

Leu Thr Thr Ser Asp Thr Ser Ser Pro Gln Lys Ser Leu Arg Thr Ala
                325                 330                 335

Pro Ala Thr Gly Gln Leu Pro Gly Arg Ser Ser Pro Ala Gly Ser Pro
            340                 345                 350

Arg Thr Trp His Ala Gln Ile Ser Thr Ser Asn Leu Tyr Leu Pro Gln
        355                 360                 365

Asp Pro Thr Val Ala Lys Gly Ala Leu Ala Gly Glu Asp Thr Gly Val
    370                 375                 380

Val Thr His Glu Gln Phe Lys Ala Ala Leu Arg Met Val Val Asp Gln
385                 390                 395                 400

Gly Asp Pro Arg Leu Leu Leu Asp Ser Tyr Val Lys Ile Gly Glu Gly
                405                 410                 415

Ser Thr Gly Ile Val Cys Leu Ala Arg Glu Lys His Ser Gly Arg Gln
            420                 425                 430

Val Ala Val Lys Met Met Asp Leu Arg Lys Gln Gln Arg Arg Glu Leu
        435                 440                 445

Leu Phe Asn Glu Val Val Ile Met Arg Asp Tyr Gln His Phe Asn Val
    450                 455                 460

Val Glu Met Tyr Lys Ser Tyr Leu Val Gly Glu Glu Leu Trp Val Leu
465                 470                 475                 480

Met Glu Phe Leu Gln Gly Gly Ala Leu Thr Asp Ile Val Ser Gln Val
                485                 490                 495

Arg Leu Asn Glu Glu Gln Ile Ala Thr Val Cys Glu Ala Val Leu Gln

500                 505                 510

Ala Leu Ala Tyr Leu His Ala Gln Gly Val Ile His Arg Asp Ile Lys
        515                 520                 525

Asp Ser Asp Ile Leu Leu Thr Leu Asp Gly Arg Val Lys Leu Ser Asp
    530                 535                 540

Phe Gly Phe Cys Ala Gln Ile Ser Lys Asp Val Pro Lys Arg Lys Ser
545                 550                 555                 560

Leu Val Gly Thr Pro Tyr Trp Met Ala Pro Glu Val Ile Ser Arg Ser
                565                 570                 575

Leu Tyr Ala Thr Glu Val Asp Ile Trp Ser Leu Gly Ile Met Val Ile
            580                 585                 590

Glu Met Val Asp Gly Glu Pro Pro Tyr Phe Ser Asp Ser Pro Val Gln
        595                 600                 605

Ala Met Lys Arg Leu Arg Asp Ser Pro Pro Pro Lys Leu Lys Asn Ser
    610                 615                 620

His Lys Val Ser Pro Val Leu Arg Asp Phe Leu Glu Arg Met Leu Val
625                 630                 635                 640

Arg Asp Pro Gln Glu Arg Ala Thr Ala Gln Glu Leu Leu Asp His Pro
                645                 650                 655

Phe Leu Leu Gln Thr Gly Leu Pro Glu Cys Leu Val Pro Leu Ile Gln
            660                 665                 670

Leu Tyr Arg Lys Gln Thr Ser Thr Cys
        675                 680

<210>  13
<211>  719
<212>  PRT
<213>  Artificial Sequence

<220>
<223>  Human PAK4, PAK5, and PAK6 amino acid sequences

<400>  13

Met Phe Gly Lys Lys Lys Lys Lys Ile Glu Ile Ser Gly Pro Ser Asn
1               5                   10                  15

Phe Glu His Arg Val His Thr Gly Phe Asp Pro Gln Glu Gln Lys Phe
            20                  25                  30

Thr Gly Leu Pro Gln Gln Trp His Ser Leu Leu Ala Asp Thr Ala Asn
        35                  40                  45

Arg Pro Lys Pro Met Val Asp Pro Ser Cys Ile Thr Pro Ile Gln Leu
    50                  55                  60

Ala Pro Met Lys Thr Ile Val Arg Gly Asn Lys Pro Cys Lys Glu Thr
65                  70                  75                  80

Ser Ile Asn Gly Leu Leu Glu Asp Phe Asp Asn Ile Ser Val Thr Arg
                85                  90                  95

Ser Asn Ser Leu Arg Lys Glu Ser Pro Pro Thr Pro Asp Gln Gly Ala
            100                 105                 110

Ser Ser His Gly Pro Gly His Ala Glu Glu Asn Gly Phe Ile Thr Phe
        115                 120                 125

Ser Gln Tyr Ser Ser Glu Ser Asp Thr Thr Ala Asp Tyr Thr Thr Glu
    130                 135                 140

Lys Tyr Arg Glu Lys Ser Leu Tyr Gly Asp Asp Leu Asp Pro Tyr Tyr
145                 150                 155                 160

Arg Gly Ser His Ala Ala Lys Gln Asn Gly His Val Met Lys Met Lys
                165                 170                 175

His Gly Glu Ala Tyr Tyr Ser Glu Val Lys Pro Leu Lys Ser Asp Phe
            180                 185                 190

Ala Arg Phe Ser Ala Asp Tyr His Ser His Leu Asp Ser Leu Ser Lys
        195                 200                 205

Pro Ser Glu Tyr Ser Asp Leu Lys Trp Glu Tyr Gln Arg Ala Ser Ser
    210                 215                 220

Ser Ser Pro Leu Asp Tyr Ser Phe Gln Phe Thr Pro Ser Arg Thr Ala
225                 230                 235                 240

Gly Thr Ser Gly Cys Ser Lys Glu Ser Leu Ala Tyr Ser Glu Ser Glu
                245                 250                 255

Trp Gly Pro Ser Leu Asp Asp Tyr Asp Arg Arg Pro Lys Ser Ser Tyr
            260                 265                 270

Leu Asn Gln Thr Ser Pro Gln Pro Thr Met Arg Gln Arg Ser Arg Ser
        275                 280                 285

Gly Ser Gly Leu Gln Glu Pro Met Met Pro Phe Gly Ala Ser Ala Phe
    290                 295                 300

Lys Thr His Pro Gln Gly His Ser Tyr Asn Ser Tyr Thr Tyr Pro Arg
305                 310                 315                 320

Leu Ser Glu Pro Thr Met Cys Ile Pro Lys Val Asp Tyr Asp Arg Ala
                325                 330                 335

Gln Met Val Leu Ser Pro Pro Leu Ser Gly Ser Asp Thr Tyr Pro Arg
            340                 345                 350

Gly Pro Ala Lys Leu Pro Gln Ser Gln Ser Lys Ser Gly Tyr Ser Ser
        355                 360                 365

Ser Ser His Gln Tyr Pro Ser Gly Tyr His Lys Ala Thr Leu Tyr His
    370                 375                 380

His Pro Ser Leu Gln Ser Ser Ser Gln Tyr Ile Ser Thr Ala Ser Tyr
385                 390                 395                 400

Leu Ser Ser Leu Ser Leu Ser Ser Ser Thr Tyr Pro Pro Pro Ser Trp
                405                 410                 415

Gly Ser Ser Ser Asp Gln Gln Pro Ser Arg Val Ser His Glu Gln Phe
            420                 425                 430

Arg Ala Ala Leu Gln Leu Val Val Ser Pro Gly Asp Pro Arg Glu Tyr
        435                 440                 445

Leu Ala Asn Phe Ile Lys Ile Gly Glu Gly Ser Thr Gly Ile Val Cys
    450                 455                 460

Ile Ala Thr Glu Lys His Thr Gly Lys Gln Val Ala Val Lys Lys Met
465                 470                 475                 480

Asp Leu Arg Lys Gln Gln Arg Arg Glu Leu Leu Phe Asn Glu Val Val
                485                 490                 495

Ile Met Arg Asp Tyr His His Asp Asn Val Val Asp Met Tyr Ser Ser
            500                 505                 510

Tyr Leu Val Gly Asp Glu Leu Trp Val Val Met Glu Phe Leu Glu Gly
        515                 520                 525

Gly Ala Leu Thr Asp Ile Val Thr His Thr Arg Met Asn Glu Glu Gln
    530                 535                 540

Ile Ala Thr Val Cys Leu Ser Val Leu Arg Ala Leu Ser Tyr Leu His
545                 550                 555                 560

Asn Gln Gly Val Ile His Arg Asp Ile Lys Ser Asp Ser Ile Leu Leu
                565                 570                 575

Thr Ser Asp Gly Arg Ile Lys Leu Ser Asp Glu Gly Phe Cys Ala Gln
            580                 585                 590

Val Ser Lys Glu Val Pro Lys Arg Lys Ser Leu Val Gly Thr Pro Tyr
        595                 600                 605

Trp Met Ala Pro Glu Val Ile Ser Arg Leu Pro Tyr Gly Thr Glu Val
    610                 615                 620

Asp Ile Trp Ser Leu Gly Ile Met Val Ile Glu Met Ile Asp Gly Glu
625                 630                 635                 640

Pro Pro Tyr Phe Asn Glu Pro Pro Leu Gln Ala Met Arg Arg Ile Arg
                645                 650                 655

Asp Ser Leu Pro Pro Arg Val Lys Asp Leu His Lys Val Ser Ser Val
            660                 665                 670

Leu Arg Gly Phe Leu Asp Leu Met Leu Val Arg Glu Pro Ser Gln Arg
        675                 680                 685

Ala Thr Ala Gln Glu Leu Leu Gly His Pro Phe Leu Lys Leu Ala Gly
    690                 695                 700

Pro Pro Ser Cys Ile Val Pro Leu Met Arg Gln Tyr Arg His His
705                 710                 715

<210>  14
<211>  591
<212>  PRT

<213>  Artificial Sequence

<220>
<223>  Human PAK4, PAK5 and PAK6 amino acid sequences

<400>  14

Met Phe Gly Lys Arg Lys Lys Arg Val Glu Ile Ser Ala Pro Ser Asn
1               5                   10                  15

Phe Glu His Arg Val His Thr Gly Phe Asp Gln His Glu Gln Lys Phe
            20                  25                  30

Thr Gly Leu Pro Arg Gln Trp Gln Ser Leu Ile Glu Glu Ser Ala Arg
        35                  40                  45

Arg Pro Lys Pro Leu Val Asp Pro Ala Cys Ile Thr Ser Ile Gln Pro
    50                  55                  60

Gly Ala Pro Lys Thr Ile Val Arg Gly Ser Lys Gly Ala Lys Asp Gly
65                  70                  75                  80

Ala Leu Thr Leu Leu Leu Asp Glu Phe Glu Asn Met Ser Val Thr Arg
                85                  90                  95

Ser Asn Ser Leu Arg Arg Asp Ser Pro Pro Pro Pro Ala Arg Ala Arg
            100                 105                 110

Gln Glu Asn Gly Met Pro Glu Glu Pro Ala Thr Thr Ala Arg Gly Gly
        115                 120                 125

Pro Gly Lys Ala Gly Ser Arg Gly Arg Phe Ala Gly His Ser Glu Ala
    130                 135                 140

Gly Gly Gly Ser Gly Asp Arg Arg Arg Ala Gly Pro Glu Lys Arg Pro
145                 150                 155                 160

Lys Ser Ser Arg Glu Gly Ser Gly Gly Pro Gln Glu Ser Ser Arg Asp
                165                 170                 175

Lys Arg Pro Leu Ser Gly Pro Asp Val Gly Thr Pro Gln Pro Ala Gly
            180                 185                 190

Leu Ala Ser Gly Ala Lys Leu Ala Ala Gly Arg Pro Phe Asn Thr Tyr
        195                 200                 205

Pro Arg Ala Asp Thr Asp His Pro Ser Arg Gly Ala Gln Gly Glu Pro

210                 215                 220

His Asp Val Ala Pro Asn Gly Pro Ser Ala Gly Gly Leu Ala Ile Pro
225                 230                 235                 240

Gln Ser Ser Ser Ser Ser Ser Arg Pro Pro Thr Arg Ala Arg Gly Ala
                245                 250                 255

Pro Ser Pro Gly Val Leu Gly Pro His Ala Ser Glu Pro Gln Leu Ala
            260                 265                 270

Pro Pro Ala Cys Thr Pro Ala Ala Pro Ala Val Pro Gly Pro Pro Gly
        275                 280                 285

Pro Arg Ser Pro Gln Arg Glu Pro Gln Arg Val Ser His Glu Gln Phe
    290                 295                 300

Arg Ala Ala Leu Gln Leu Val Val Asp Pro Gly Asp Pro Arg Ser Tyr
305                 310                 315                 320

Leu Asp Asn Phe Ile Lys Ile Gly Glu Gly Ser Thr Gly Ile Val Cys
                325                 330                 335

Ile Ala Thr Val Arg Ser Ser Gly Lys Leu Val Ala Val Lys Lys Met
            340                 345                 350

Asp Leu Arg Lys Gln Gln Arg Arg Glu Leu Leu Phe Asn Glu Val Val
        355                 360                 365

Ile Met Arg Asp Tyr Gln His Glu Asn Val Val Glu Met Tyr Asn Ser
    370                 375                 380

Tyr Leu Val Gly Asp Glu Leu Trp Val Val Met Glu Phe Leu Glu Gly
385                 390                 395                 400

Gly Ala Leu Thr Asp Ile Val Thr His Thr Arg Met Asn Glu Glu Gln
                405                 410                 415

Ile Ala Ala Val Cys Leu Ala Val Leu Gln Ala Leu Ser Val Leu His
            420                 425                 430

Ala Gln Gly Val Ile His Arg Asp Ile Lys Ser Asp Ser Ile Leu Leu
        435                 440                 445

Thr His Asp Gly Arg Val Lys Leu Ser Asp Phe Gly Phe Cys Ala Gln

450                 455                 460

Val Ser Lys Glu Val Pro Arg Arg Lys Ser Leu Val Gly Thr Pro Tyr
465                 470                 475                 480

Trp Met Ala Pro Glu Leu Ile Ser Arg Leu Pro Tyr Gly Pro Glu Val
                485                 490                 495

Asp Ile Trp Ser Leu Gly Ile Met Val Ile Glu Met Val Asp Gly Glu
            500                 505                 510

Pro Pro Tyr Phe Asn Glu Pro Pro Leu Lys Ala Met Lys Met Ile Arg
        515                 520                 525

Asp Asn Leu Pro Pro Arg Leu Lys Asn Leu His Lys Val Ser Pro Ser
    530                 535                 540

Leu Lys Gly Phe Leu Asp Arg Leu Leu Val Arg Asp Pro Ala Gln Arg
545                 550                 555                 560

Ala Thr Ala Ala Glu Leu Leu Lys His Pro Phe Leu Ala Lys Ala Gly
                565                 570                 575

Pro Pro Ala Ser Ile Val Pro Leu Met Arg Gln Asn Arg Thr Arg
            580                 585                 590

<210>  15
<211>  681
<212>  PRT
<213>  Artificial Sequence

<220>
<223>  Human PAK4, PAK5, and PAK6 amino acid sequences

<400>  15

Met Phe Arg Lys Lys Lys Lys Lys Arg Pro Glu Ile Ser Ala Pro Gln
1               5                   10                  15

Asn Phe Gln His Arg Val His Thr Ser Phe Asp Pro Lys Glu Gly Lys
            20                  25                  30

Phe Val Gly Leu Pro Pro Gln Trp Gln Asn Ile Leu Asp Thr Leu Arg
        35                  40                  45

Arg Pro Lys Pro Val Val Asp Pro Ser Arg Ile Thr Arg Val Gln Leu
    50                  55                  60

Gln Pro Met Lys Thr Val Val Arg Gly Ser Ala Met Pro Val Asp Gly
65                  70                  75                  80

Tyr Ile Ser Gly Leu Leu Asn Asp Ile Gln Lys Leu Ser Val Ile Ser
                85                  90                  95

Ser Asn Thr Leu Arg Gly Arg Ser Pro Thr Ser Arg Arg Arg Ala Gln
            100                 105                 110

Ser Leu Gly Leu Leu Gly Asp Glu His Trp Ala Thr Asp Pro Asp Met
        115                 120                 125

Tyr Leu Gln Ser Pro Gln Ser Glu Arg Thr Asp Pro His Gly Leu Tyr
    130                 135                 140

Leu Ser Cys Asn Gly Gly Thr Pro Ala Gly His Lys Gln Met Pro Trp
145                 150                 155                 160

Pro Glu Pro Gln Ser Pro Arg Val Leu Pro Asn Gly Leu Ala Ala Lys
                165                 170                 175

Ala Gln Ser Leu Gly Pro Ala Glu Phe Gln Gly Ala Ser Gln Arg Cys
            180                 185                 190

Leu Gln Leu Gly Ala Cys Leu Gln Ser Ser Pro Pro Gly Ala Ser Pro
        195                 200                 205

Pro Thr Gly Thr Asn Arg His Gly Met Lys Ala Ala Lys His Gly Ser
    210                 215                 220

Glu Glu Ala Arg Pro Gln Ser Cys Leu Val Gly Ser Ala Thr Gly Arg
225                 230                 235                 240

Pro Gly Gly Glu Gly Ser Pro Ser Pro Lys Thr Arg Glu Ser Ser Leu
                245                 250                 255

Lys Arg Arg Leu Phe Arg Ser Met Phe Leu Ser Thr Ala Ala Thr Ala
            260                 265                 270

Pro Pro Ser Ser Ser Lys Pro Gly Pro Pro Pro Gln Ser Lys Pro Asn
        275                 280                 285

Ser Ser Phe Arg Pro Pro Gln Lys Asp Asn Pro Pro Ser Leu Val Ala
    290                 295                 300

Lys Ala Gln Ser Leu Pro Ser Asp Gln Pro Val Gly Thr Phe Ser Pro
305                 310                 315                 320

Leu Thr Thr Ser Asp Thr Ser Ser Pro Gln Lys Ser Leu Arg Thr Ala
                325                 330                 335

Pro Ala Thr Gly Gln Leu Pro Gly Arg Ser Ser Pro Ala Gly Ser Pro
            340                 345                 350

Arg Thr Trp His Ala Gln Ile Ser Thr Ser Asn Leu Tyr Leu Pro Gln
        355                 360                 365

Asp Pro Thr Val Ala Lys Gly Ala Leu Ala Gly Glu Asp Thr Gly Val
    370                 375                 380

Val Thr His Glu Gln Phe Lys Ala Ala Leu Arg Met Val Val Asp Gln
385                 390                 395                 400

Gly Asp Pro Arg Leu Leu Leu Asp Ser Tyr Val Lys Ile Gly Glu Gly
                405                 410                 415

Ser Thr Gly Ile Val Cys Leu Ala Arg Glu Lys His Ser Gly Arg Gln
            420                 425                 430

Val Ala Val Lys Met Met Asp Leu Arg Lys Gln Gln Arg Arg Glu Leu
        435                 440                 445

Leu Phe Asn Glu Val Val Ile Met Arg Asp Tyr Gln His Phe Asn Val
    450                 455                 460

Val Glu Met Tyr Lys Ser Tyr Leu Val Gly Glu Glu Leu Trp Val Leu
465                 470                 475                 480

Met Glu Phe Leu Gln Gly Gly Ala Leu Thr Asp Ile Val Ser Gln Val
                485                 490                 495

Arg Leu Asn Glu Glu Gln Ile Ala Thr Val Cys Glu Ala Val Leu Gln
            500                 505                 510

Ala Leu Ala Tyr Leu His Ala Gln Gly Val Ile His Arg Asp Ile Lys
        515                 520                 525

Ser Asp Ser Ile Leu Leu Thr Leu Asp Gly Arg Val Lys Leu Ser Asp
    530                 535                 540

Phe Gly Phe Cys Ala Gln Ile Ser Lys Asp Val Pro Lys Arg Lys Ser
545                 550                 555                 560

Leu Val Gly Thr Pro Tyr Trp Met Ala Pro Glu Val Ile Ser Arg Ser
                565                 570                 575

Leu Tyr Ala Thr Glu Val Asp Ile Trp Ser Leu Gly Ile Met Val Ile
            580                 585                 590

Glu Met Val Asp Gly Glu Pro Pro Tyr Phe Ser Asp Ser Pro Val Gln
        595                 600                 605

Ala Met Lys Arg Leu Arg Asp Ser Pro Pro Pro Lys Leu Lys Asn Ser
    610                 615                 620

His Lys Val Ser Pro Val Leu Arg Asp Phe Leu Glu Arg Met Leu Val
625                 630                 635                 640

Arg Asp Pro Gln Glu Arg Ala Thr Ala Gln Glu Leu Leu Asp His Pro
                645                 650                 655

Phe Leu Leu Gln Thr Gly Leu Pro Glu Cys Leu Val Pro Leu Ile Gln
            660                 665                 670

Leu Tyr Arg Lys Gln Thr Ser Thr Cys
        675                 680

<210>  16
<211>  632
<212>  PRT
<213>  Artificial Sequence

<220>
<223>  Caenorhabditis elegans, Drosophila melanogaster, and rat PAK1
       amino acid sequences

<400>  16

Met Lys Ala Phe Ser Ser Tyr Asp Glu Lys Pro Pro Ala Pro Pro Ile
1               5                   10                  15

Arg Phe Ser Ser Ser Ala Thr Arg Glu Asn Gln Val Val Gly Leu Lys
            20                  25                  30

Pro Leu Pro Lys Glu Pro Glu Cys Glu Pro Ala Lys Ala Thr Lys Lys
        35                  40                  45

Lys Lys Thr Met Pro Asn Pro Phe Met Lys Lys Asn Lys Asp Lys Lys
    50                  55                  60

Glu Ala Ser Glu Lys Pro Val Ile Ser Arg Pro Ser Asn Phe Glu His
65                  70                  75                  80

Thr Ile His Val Gly Tyr Asp Pro Lys Thr Gly Glu Phe Thr Gly Met
                85                  90                  95

Pro Glu Ala Trp Ala Arg Leu Cys Glu Pro Ala Lys Leu Thr Asp Ser
            100                 105                 110

Gln Ile Ser Lys Gln Glu Gln Gln Gln Asn Pro Gln Ala Val Leu Asp
        115                 120                 125

Ala Leu Lys Tyr Tyr Thr Gln Gly Glu Ser Ser Gly Gln Lys Trp Leu
    130                 135                 140

Gln Tyr Asp Met Met Phe Ile Asp Asp Ala Pro Ser Arg Thr Pro Ser
145                 150                 155                 160

Tyr Gly Leu Lys Pro Gln Pro Cys Glu Pro Ala Lys Tyr Ser Thr Ser
                165                 170                 175

Ser Leu Pro Tyr His Gly Asn Lys Ile Gln Asp Pro Arg Lys Met Asn
            180                 185                 190

Pro Met Thr Thr Ser Thr Ser Ser Ala Gly Tyr Asn Ser Lys Gln Cys
        195                 200                 205

Glu Pro Ala Lys Gly Val Pro Pro Thr Thr Phe Ser Val Asn Glu Asn
    210                 215                 220

Arg Ser Ser Met Pro Pro Ser Tyr Ala Pro Pro Pro Val Pro His Gly
225                 230                 235                 240

Glu Thr Pro Ala Asp Ile Val Pro Pro Ala Ile Pro Asp Arg Pro Ala
                245                 250                 255

Arg Cys Glu Pro Ala Lys Thr Leu Ser Ile Tyr Thr Lys Pro Lys Glu
            260                 265                 270

Glu Cys Glu Pro Ala Lys Glu Glu Lys Ile Pro Asp Leu Ser Lys Gly
        275                 280                 285

Ala Gln Gly Val Gln Ala Arg Gly Gln Lys Ala Lys Lys Met Thr
    290                 295                 300

Asp Ala Glu Cys Glu Pro Ala Lys Val Leu Thr Lys Leu Arg Thr Ile
305 310 315 320

Val Ser Ile Gly Asn Pro Asp Arg Lys Tyr Arg Lys Val Asp Lys Ile
325 330 335

Gly Ser Gly Ala Ser Gly Ser Val Tyr Thr Ala Ile Glu Ile Ser Thr
                340                 345                 350

Glu Ala Glu Val Ala Ile Lys Gln Met Asn Leu Lys Asp Gln Pro Lys
            355                 360                 365

Lys Glu Leu Ile Cys Glu Pro Ala Lys Ile Asn Glu Ile Leu Val Met
        370                 375                 380

Arg Glu Asn Lys His Ala Asn Ile Val Asn Tyr Leu Asp Ser Tyr Leu
385 390 395 400

Val Cys Asp Glu Leu Trp Val Val Met Glu Tyr Leu Ala Gly Gly Ser
405 410 415

Leu Thr Asp Val Val Thr Glu Cys Gln Met Glu Asp Gly Ile Ile Ala
                420                 425                 430

Ala Val Cys Arg Glu Cys Glu Pro Ala Lys Val Leu Gln Ala Leu Glu
            435                 440                 445

Phe Leu His Ser Arg His Val Ile His Arg Asp Ile Lys Ser Asp Asn
        450                 455                 460

Ile Leu Leu Gly Met Asp Gly Ser Val Lys Leu Thr Asp Phe Gly Phe
465 470 475 480

Cys Ala Gln Leu Ser Pro Glu Gln Arg Lys Arg Thr Thr Met Val Gly
485 490 495

Thr Pro Tyr Trp Met Ala Cys Glu Pro Ala Lys Pro Glu Val Val Thr
                500                 505                 510

Arg Lys Gln Tyr Gly Pro Lys Val Asp Val Trp Ser Leu Gly Ile Met
            515                 520                 525

Ala Ile Glu Met Val Glu Gly Glu Pro Pro Tyr Leu Asn Glu Asn Pro
    530                 535                 540

Leu Arg Ala Ile Tyr Leu Ile Ala Thr Asn Gly Lys Pro Asp Phe Pro
545                 550                 555                 560

Gly Arg Asp Ser Met Thr Leu Cys Glu Pro Ala Lys Leu Phe Lys Asp
                565                 570                 575

Phe Val Asp Ser Ala Leu Glu Val Gln Val Glu Asn Arg Trp Ser Ala
            580                 585                 590

Ser Gln Leu Leu Thr His Pro Phe Leu Arg Cys Ala Lys Pro Leu Ala
        595                 600                 605

Ser Leu Tyr Tyr Leu Ile Val Ala Ala Lys Lys Ser Ile Ala Glu Ala
    610                 615                 620

Ser Asn Ser Cys Glu Pro Ala Lys
625                 630

<210>  17
<211>  752
<212>  PRT
<213>  Artificial Sequence

<220>
<223>  Caenorhabditis elegans, Drosophila melanogaster, and rat PAK1
       amino acid sequences

<400>  17

Met Ser Ser Glu Glu Asp Lys Pro Pro Ala Pro Pro Val Arg Leu Thr
1               5                   10                  15

Ser Asn Arg Gly Gly Asn Glu Arg Ser Gly Gly Gly Val Gly Val Gly
            20                  25                  30

Gly Gly Gly Leu Gly Gly Gly Gly Met Gly Asp Val Pro Pro Asp Met
        35                  40                  45

Arg Pro Leu Pro Lys Glu Pro Asp Asp Pro Ala Lys Asp Ser Asp Arg
    50                  55                  60

Lys Lys Lys Thr Leu Lys Ser Lys Ile Lys Gly Ser Lys Pro Ser His
65                  70                  75                  80

Thr Asp Ser Lys Pro Asn Ile Ser Tyr Pro Thr Asn Phe Glu His Thr

85                  90                  95

Val His Val Gly Phe Asp Ala Val Thr Gly Glu Phe Thr Gly Met Pro
            100                 105                 110

Glu Ala Trp Ala Arg Leu Asp Pro Ala Lys Leu Met Asn Ser Asn Ile
        115                 120                 125

Ser Lys Gln Glu Gln Lys Lys Asn Pro Gln Ala Val Leu Asp Val Leu
    130                 135                 140

Lys Trp Phe Asp Asn Thr Thr Lys Gln Arg Pro Ser Ser Lys Tyr Met
145                 150                 155                 160

Thr Asn Ala Ile Thr Thr His Ser Gly Ser Ser Leu Ser Arg Val Ser
                165                 170                 175

Ser Ser Ser Pro Ser Asp Pro Ala Lys Ser Thr Pro Thr Asp Ser Glu
            180                 185                 190

Leu His Gly Ser Asn Ser Gly Gly Asn Leu Ile Gly Val Gln Leu Gly
        195                 200                 205

Ser Met Thr Leu Gly Pro Asn Ala Asn Asn Val Ala Val Ala Gly Ile
    210                 215                 220

Leu Gly Asn His Tyr Gln Gln Gln Gln Gln His Leu Leu Gln Gln Gln
225                 230                 235                 240

Gln Pro Leu Leu Asp Pro Ala Lys His Gln Asn His Asn Gln His His
                245                 250                 255

Met Gly Ile Ser Gln Ser His Ser Tyr Asn Phe Val Gly His Thr Val
            260                 265                 270

Ser Ser Ser Thr Ser Gln His Ser Ser Ala Asn Glu Asp Asp Met Leu
        275                 280                 285

Gly Pro Gln His Pro Gln Gln Gln Pro Pro Pro Pro Pro Val Ala Ser
    290                 295                 300

Arg Pro Glu Arg Asp Pro Ala Lys Thr Lys Ser Ile Tyr Thr Arg Pro
305                 310                 315                 320

Ile Glu Asp Leu Gln Pro Ala Ile Ile Pro Met Pro Val Ala Pro Ala

325                 330                 335

Thr Thr Pro Ala Thr Pro Leu Gln Asn His Arg Thr Pro Gly Gly Ile
            340                 345                 350

Ser Ala Pro Ala Thr Ser Pro Met Met His Asn Asn Ala Thr Thr Thr
        355                 360                 365

Leu Asp Lys Asn Asp Pro Ala Lys Lys Asn Asn Ala Asn Leu Tyr Thr
    370                 375                 380

Pro Glu Pro Thr Val Ala Gln Val Ser Ala Gly Gly Pro Ser Ser Gln
385                 390                 395                 400

Val Ala Gly Asn Gln Ile Ala Val Pro Gln Ala Ala Val Ala Pro Ala
                405                 410                 415

Ala Thr Pro Asn Thr Arg Ala Ala Asn Ala Lys Lys Lys Lys Met Ser
            420                 425                 430

Asp Glu Glu Asp Pro Ala Lys Ile Leu Glu Lys Leu Arg Thr Ile Val
        435                 440                 445

Ser Ile Gly Asn Pro Asn Arg Lys Tyr Thr Lys Met Glu Lys Ile Gly
    450                 455                 460

Gln Gly Ala Ser Gly Thr Val Tyr Thr Ala Ile Glu Ser Ser Thr Gly
465                 470                 475                 480

Met Glu Val Ala Ile Lys Gln Met Asn Leu Ser Gln Gln Pro Lys Lys
                485                 490                 495

Glu Leu Ile Asp Pro Ala Lys Ile Asn Glu Ile Leu Val Met Arg Glu
            500                 505                 510

Asn Lys His Pro Asn Val Val Asn Tyr Leu Asp Ser Tyr Leu Val Ser
        515                 520                 525

Glu Glu Leu Trp Val Val Met Glu Tyr Leu Pro Gly Gly Ser Leu Thr
    530                 535                 540

Asp Val Val Thr Glu Thr Cys Met Asp Glu Gly Gln Ile Ala Ala Val
545                 550                 555                 560

Cys Arg Glu Asp Pro Ala Lys Val Leu Gln Ala Leu Glu Phe Leu His

565                 570                 575

Ala Asn Gln Val Ile His Arg Asp Ile Lys Ser Asp Asn Ile Leu Leu
            580                 585                 590

Gly Leu Asp Gly Ser Val Lys Leu Thr Asp Phe Gly Phe Cys Ala Gln
        595                 600                 605

Ile Ser Pro Glu Gln Ser Lys Arg Thr Thr Met Val Gly Thr Pro Tyr
    610                 615                 620

Trp Met Ala Asp Pro Ala Lys Pro Glu Val Val Thr Arg Lys Gln Tyr
625                 630                 635                 640

Gly Pro Lys Val Asp Leu Gln Trp Ser Leu Gly Ile Met Ala Ile Glu
                645                 650                 655

Met Val Glu Gly Glu Pro Pro Tyr Leu Asn Glu Asn Pro Leu Lys Ala
            660                 665                 670

Leu Tyr Leu Ile Ala Thr Asn Gly Lys Pro Glu Ile Lys Glu Lys Asp
        675                 680                 685

Lys Leu Ser Ser Asp Pro Ala Lys Ala Phe Gln Asp Phe Leu Asp Gln
    690                 695                 700

Cys Leu Glu Val Glu Val Asp Arg Arg Ala Ser Ala Leu Asp Leu Leu
705                 710                 715                 720

Lys His Pro Phe Leu Lys Leu Ala Arg Pro Leu Ala Ser Leu Thr Pro
                725                 730                 735

Leu Ile Met Ala Ala Lys Glu Ala Thr Lys Gly Asn Asp Pro Ala Lys
            740                 745                 750

<210>  18
<211>  574
<212>  PRT
<213>  Artificial Sequence

<220>
<223>  Caenorhabditis elegans, Drosophila melanogaster, and rat PAK1
       amino acid sequences

<400>  18

Met Ser Asn Asn Gly Leu Asp Val Gln Asp Lys Pro Pro Ala Pro Pro
1               5                   10                  15

Met Arg Asn Thr Ser Thr Met Ile Gly Ala Gly Ser Lys Asp Pro Gly
                20                  25                  30

Thr Leu Asn His Gly Ser Lys Pro Leu Pro Pro Asn Pro Glu Pro Ala
        35                  40                  45

Lys Glu Lys Lys Lys Lys Asp Arg Phe Tyr Arg Ser Ile Leu Ala Gly
    50                  55                  60

Asp Lys Thr Asn Lys Lys Lys Glu Lys Glu Arg His Glu Ile Ser Leu
65                  70                  75                  80

Pro Ser Asp Phe Glu His Thr Ile His Val Gly Phe Asp Ala Val Thr
                85                  90                  95

Gly Glu Phe Thr Gly Met Pro Glu Gln Trp Gln Ala Arg Leu Pro Ala
            100                 105                 110

Lys Leu Gln Thr Ser Asn Ile Thr Lys Ser Glu Gln Lys Lys Asn Pro
        115                 120                 125

Gln Ala Val Leu Asp Val Leu Glu Phe Tyr Asn Ser Lys Lys Thr Ser
    130                 135                 140

Asn Ser Gln Lys Tyr Met Ser Phe Thr Asp Lys Ser Ala Pro Ala Lys
145                 150                 155                 160

Glu Asp Tyr Pro Ala Lys Asn Ser Ser Asn Thr Leu Asn Val Lys Thr
                165                 170                 175

Val Ser Glu Thr Pro Ala Val Pro Pro Val Ser Glu Asp Glu Asp Asp
            180                 185                 190

Asp Asp Asp Ala Thr Pro Pro Pro Val Ile Ala Pro Arg Pro Glu His
        195                 200                 205

Pro Ala Lys Thr Lys Ser Val Tyr Thr Arg Ser Val Ile Glu Pro Leu
    210                 215                 220

Pro Val Thr Pro Thr Arg Asp Val Ala Thr Ser Pro Ile Ser Pro Thr
225                 230                 235                 240

Glu Asn Asn Thr Thr Pro Pro Asp Ala Leu Thr Arg Asn Thr Glu Lys
                245                 250                 255

Gln Lys Lys Lys Pro Lys Met Ser Asp Glu Glu Pro Ala Lys Ile Leu
            260                 265                 270

Glu Lys Leu Arg Asn Ile Val Ser Val Gly Asp Pro Lys Lys Lys Tyr
        275                 280                 285

Thr Arg Phe Glu Lys Ile Gly Gln Gly Ala Ser Gly Thr Val Tyr Thr
    290                 295                 300

Ala Met Asp Val Ala Thr Gly Gln Glu Val Ala Ile Lys Gln Met Asn
305                 310                 315                 320

Leu Gln Gln Gln Pro Lys Lys Glu Leu Ile Pro Ala Lys Ile Asn Glu
                325                 330                 335

Ile Leu Val Met Arg Glu Asn Lys Asn Pro Asn Ile Val Asn Tyr Leu
            340                 345                 350

Asp Ser Tyr Leu Val Gly Asp Glu Leu Trp Val Val Met Glu Tyr Leu
        355                 360                 365

Ala Gly Gly Ser Leu Thr Asp Val Val Thr Glu Thr Cys Met Asp Glu
    370                 375                 380

Gly Gln Ile Ala Ala Val Cys Arg Glu Pro Ala Lys Cys Leu Gln Ala
385                 390                 395                 400

Leu Glu Phe Leu His Ser Asn Gln Val Ile His Arg Asp Ile Lys Ser
                405                 410                 415

Asp Asn Ile Leu Leu Gly Met Asp Gly Ser Val Lys Leu Thr Asp Phe
            420                 425                 430

Gly Cys Ala Gln Ile Thr Pro Glu Gln Ser Lys Arg Ser Thr Met Val
        435                 440                 445

Gly Thr Pro Tyr Trp Met Ala Pro Ala Lys Pro Glu Val Val Thr Arg
    450                 455                 460

Lys Ala Tyr Gly Pro Lys Val Asp Ile Trp Ser Leu Gly Ile Met Ala
465                 470                 475                 480

Ile Glu Met Ile Glu Gly Glu Pro Pro Tyr Leu Asn Glu Asn Pro Leu
                485                 490                 495

Arg Ala Leu Tyr Leu Ile Ala Thr Asn Gly Thr Pro Glu Leu Gln Asn
            500                 505                 510

Pro Glu Lys Leu Ser Ala Pro Ala Lys Ile Phe Arg Asp Phe Leu Asn
        515                 520                 525

Arg Cys Leu Glu Met Asp Val Glu Lys Arg Gly Ser Ala Lys Glu Leu
    530                 535                 540

Leu Gln His Gln Phe Leu Lys Ile Ala Lys Pro Leu Ser Ser Leu Thr
545                 550                 555                 560

Pro Leu Ile Ala Ala Ala Lys Glu Ala Thr Lys Asn Asn His
                565                 570